(19) Europäisches Patentamt
European Patent Office
Office européen des brevets

(11) EP 4 273 129 A1

(12) EUROPEAN PATENT APPLICATION
published in accordance with Art. 153(4) EPC

(43) Date of publication:
**08.11.2023 Bulletin 2023/45**

(21) Application number: **21914503.4**

(22) Date of filing: **29.12.2021**

(51) International Patent Classification (IPC):
***C07D 213/75*** *(2006.01)* ***C07D 213/84*** *(2006.01)*
***C07D 401/06*** *(2006.01)* ***C07D 405/06*** *(2006.01)*
***C07D 417/06*** *(2006.01)* ***C07D 239/04*** *(2006.01)*
***A61K 31/44*** *(2006.01)* ***A61K 31/4402*** *(2006.01)*
***A61K 31/4427*** *(2006.01)* ***A61K 31/4439*** *(2006.01)*
***A61K 31/505*** *(2006.01)* ***A61K 31/506*** *(2006.01)*
***A61P 17/06*** *(2006.01)* ***A61P 25/00*** *(2006.01)*
***A61P 1/00*** *(2006.01)* ***A61P 29/00*** *(2006.01)*
***A61P 37/00*** *(2006.01)* ***A61P 11/00*** *(2006.01)*

(52) Cooperative Patent Classification (CPC):
**A61K 31/44; A61K 31/4402; A61K 31/4427; A61K 31/4439; A61K 31/505; A61K 31/506; A61P 1/00; A61P 11/00; A61P 17/06; A61P 25/00; A61P 29/00; A61P 37/00; C07D 213/75; C07D 213/84; C07D 239/04;** (Cont.)

(86) International application number:
**PCT/CN2021/142477**

(87) International publication number:
**WO 2022/143771 (07.07.2022 Gazette 2022/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.12.2020 CN 202011626107**
**19.07.2021 CN 202110814131**

(71) Applicant: **Shanghai Pharmaceuticals Holding Co., Ltd.**
**Shanghai 201203 (CN)**

(72) Inventors:
- **WANG, Qian**
  **Shanghai 201203 (CN)**
- **ZHANG, Bingbin**
  **Shanghai 201203 (CN)**
- **XIA, Guangxin**
  **Shanghai 201203 (CN)**
- **SHU, Sijie**
  **Shanghai 201203 (CN)**
- **HUO, Guoyong**
  **Shanghai 201203 (CN)**
- **XIANG, Zhijun**
  **Shanghai 201203 (CN)**
- **WU, Guosheng**
  **Shanghai 201203 (CN)**
- **LIANG, Tao**
  **Shanghai 201203 (CN)**
- **SHI, Chen**
  **Shanghai 201203 (CN)**
- **ZHAO, Yongxin**
  **Shanghai 201203 (CN)**
- **LI, Lingwen**
  **Shanghai 201203 (CN)**
- **KE, Ying**
  **Shanghai 201203 (CN)**

(74) Representative: **Petraz, Gilberto Luigi et al**
**GLP S.r.l.**
**Viale Europa Unita, 171**
**33100 Udine (IT)**

(54) **RORgammaT MODULATOR, AND PREPARATION METHOD THEREFOR AND APPLICATION THEREOF**

(57) Provided are an RORγt modulator, and a preparation method therefor and an application thereof. The RORγt modulator is selected from a compound represented by formula I, and a pharmaceutically acceptable salt, solvate,



EP 4 273 129 A1

solvate of pharmaceutically acceptable salt, metabolite or prodrug thereof. The compound has inhibitory activity on RORyt, and can effectively inhibit an RORyt protein receptor, thereby regulating the differentiation of Th17 cells, inhibiting generation of IL-17, and then treating RORyt-mediated relevant autoimmune diseases.

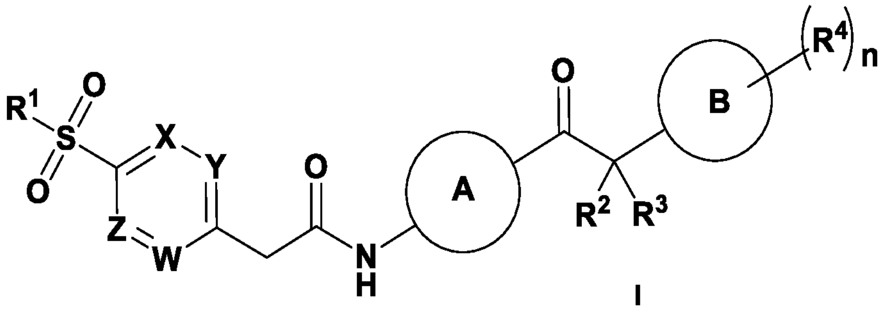

I

(52) Cooperative Patent Classification (CPC): (Cont.)
**C07D 401/06; C07D 405/06; C07D 417/06**

## Description

### TECHNICAL FIELD

The invention relates to the field of medicinal chemistry, in particular to a class of RORγt modulators, a preparation method therefor and a use thereof.

### BACKGROUND ART

Retinoic acid receptor-related orphan receptors (RORs) are members of the nuclear receptor superfamily of ligand-dependent transcription factors, and play important roles in a series of physiological and pathological processes such as reproductive development, circadian rhythm regulation, metabolic disorders, inflammation and immune system regulation. RORs mainly include three members, RORα, RORβ and RORγ. RORα is mainly distributed in liver, skeletal muscle, skin, lung, adipose tissue, kidney, thymus, brain and blood. RORβ is mainly distributed in the central nervous system, including brain, retina and pineal gland. RORγ is highly expressed in the thymus, and is also distributed in kidney, liver, heart, skeletal muscle, adipose tissue, testis, prostate and pancreas. According to the site of transcription and splicing, RORγ is divided into two subtypes, RORγ1 and RORγt (also known as RORγ2), the former is mainly expressed in thymus, testis, pancreas, heart, liver, skeletal muscle and kidney, while RORγt is only expressed in immune organs.

Th17 cells are a subtype of T helper cells, and characterized by the secretion of interleukin 17 (IL-17) cytokines. They were originally thought to mainly exert immune functions by recruiting neutrophils in resisting bacterial and fungal infections. Subsequent studies have found that Th17 cells play critical roles in many mouse models of autoimmune diseases, and in some human autoimmune diseases including psoriasis, multiple sclerosis (MS), rheumatoid arthritis (RA) and inflammatory bowel disease (IBD), increased IL-17 level can also be detected. The numbers of Th17 cells are found to be increased in tissue and peripheral blood samples from patients with autoimmune diseases. Therefore, Th17 cells or the cytokine IL-17 produced by them are closely related to the pathogenesis of autoimmune diseases and inflammation, and inhibiting the differentiation of Th17 cells can be used to treat associated diseases.

Studies have shown that RORγt is a key modulator of Th17 cell differentiation. Littman et al. first reported that RORγt is necessary for the differentiation of naive CD4+ T cells into Th17 cells. Mice lacking RORγt lack lymphoid organs such as lymph nodes and Peyer's patches, and the process of T cell development and maturation is also affected, and the numbers of various T cells are lower than those of normal mice. The regulation of RORγt activity by a small molecule compound can directly affect the differentiation of Th17 cells and inhibit RORγt, resulting in significantly reduced level of cytokine IL-17 secreted by Th17. Therefore, RORγt can be used as a new target for the treatment of autoimmune diseases, and it is of great significance to develop RORγt small molecule modulators and use them in the treatment of RORγt-mediated associated diseases such as autoimmune diseases and inflammatory diseases.

At present, patent applications WO2015083130, WO2016193459, WO2017010399, etc. have disclosed some RORγt small molecule modulators, but there are no related products on the market yet. It still needs to develop new RORγt small molecule modulators with better activity and higher safety in this field.

### SUMMARY OF THE INVENTION

The invention provides a heterocyclic compound different from the prior art, a preparation method therefor and a use thereof. This type of compound has inhibitory activity on RORγt, can effectively inhibit the RORγt protein receptor, thereby regulating the differentiation of Th17 cells, inhibiting the production of IL-17, and then treating associated autoimmune diseases mediated by RORγt, especially various diseases such as psoriasis, multiple sclerosis, atopic dermatitis, inflammatory bowel disease.

The present invention provides a compound represented by formula I, or a pharmaceutically acceptable salt thereof, a solvate thereof, a solvate of the pharmaceutically acceptable salt, a metabolite thereof or a prodrug thereof:

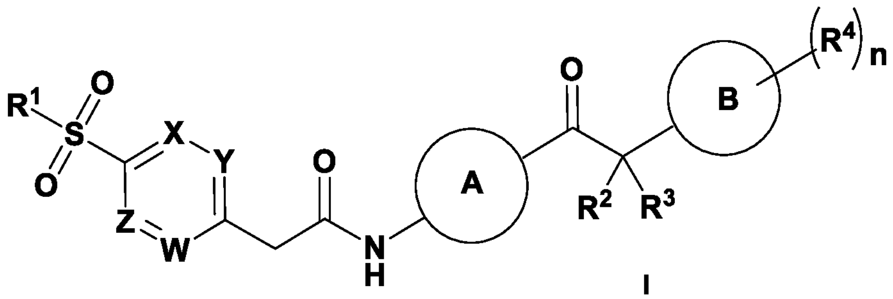

I

wherein, n is 0, 1, 2, 3, 4 or 5;

W, X, Y and Z are independently CH or N;

ring A and ring B are each independently $C_3$-$C_{14}$ cycloalkyl, 3- to 14-membered heterocycloalkyl, $C_3$-$C_{14}$ cycloalkenyl, 3- to 14-membered heterocycloalkenyl, $C_6$-$C_{12}$ aryl, or 5- to 10-membered heteroaryl, the 3- to 14-membered heterocycloalkyl, the 3- to 14-membered heterocycloalkenyl and the 5- to 10-membered heteroaryl independently contain one or more heteroatoms selected from the group conisisting of boron, silicon, oxygen, sulfur, selenium, nitrogen and phosphorus, and the number of heteroatoms is independently 1, 2, 3 or 4; particularly, ring A and ring B are each independently $C_3$-$C_{10}$ cycloalkyl, 3- to 10-membered heterocycloalkyl, $C_6$-$C_{10}$ aryl or 5- to 6-membered heteroaryl, the 5- to 6-membered heteroaryl and the 3- to 10-membered heterocycloalkyl each independently contain one or more heteroatoms selected from the group consisting of oxygen, sulfur and nitrogen, and the number of heteroatoms is independently 1, 2 or 3; more particularly, ring A and ring B are each independently cyclopropyl, cyclobutyl, cyclopentyl, cyclohexyl, oxetanyl, pyrrolidinyl, tetrahydrofuryl, tetrahydrothiophenyl, tetrahydropyranyl, tetrahydrothiopyranyl, piperidinyl, piperazinyl, morpholinyl, phenyl, naphthyl, pyrazolyl, imidazolyl, thiazolyl, oxazolyl, indolyl, furyl, thienyl, benzothienyl, benzofuryl, pyrazinyl, pyridazinyl, pyridyl, pyrimidyl or pyrrolyl;

wherein, ring A is optionally substituted by 1 to 3 substituents selected from the group consisting of halogen, $C_1$-$C_4$ alkyl, $C_1$-$C_4$ alkoxy, hydroxyl and cyano;

$R^1$ is independently -$OR^{1-1}$, -$NR^{1-2}R^{1-3}$, $C_1$-$C_7$ alkyl, $C_3$-$C_{14}$ cycloalkyl, 3- to 14-membered heterocycloalkyl, $R^{1-4}$-substituted $C_1$-$C_7$ alkyl, or $R^{1-4}$-substituted 3- to 14-membered heterocycloalkyl, said 3- to 14-membered heterocycloalkyl independently contains one or more heteroatoms selected from the group consisting of boron, silicon, oxygen, sulfur, selenium, nitrogen and phosphorus, and the number of heteroatoms is independently 1, 2, 3 or 4; particularly, $R^1$ is independently -$OR^{1-1}$, $C_1$-$C_4$ alkyl, -$NR^{1-2}R^{1-3}$, or $R^{1-4}$-substituted $C_1$-$C_4$ alkyl; more particularly, $R^1$ is independently methoxy, ethoxy, propoxy, methyl, ethyl, n-propyl, isopropyl, methylamino, dimethylamino, halogen-substituted methyl, halogen-substituted ethyl, halogen-substituted n-propyl, halogen-substituted isopropyl (such as trifluoromethyl, dichloroethyl, etc.);

$R^2$ and $R^3$ are each independently hydrogen, halogen, -$OR^{2-1}$, -CN, -$NR^{2-2}R^{2-3}$, $C_1$-$C_7$ alkyl, $C_3$-$C_{14}$ cycloalkyl, 3- to 14-membered heterocycloalkyl, $R^{2-4}$-substituted $C_1$-$C_7$ alkyl, $R^{2-4}$-substituted $C_3$-$C_{14}$ cycloalkyl, or $R^{2-4}$-substituted 3- to 14-membered heterocycloalkyl, or $R^2$ and $R^3$ together form oxo (=O), the 3- to 14-membered heterocycloalkyl group independently contains one or more heteroatoms selected from the group consisting of boron, silicon, oxygen, sulfur, selenium, nitrogen and phosphorus, and the number of heteroatoms is independently 1, 2, 3 or 4; particularly, $R^2$ and $R^3$ are each independently hydrogen, halogen, -CN, $C_1$-$C_4$ alkyl, $C_3$-$C_8$ cycloalkyl, or $R^{2-4}$-substituted $C_1$-$C_4$ alkyl; more particularly, $R^2$ and $R^3$ are each independently hydrogen, halogen, -CN, methyl, ethyl, n-propyl, isopropyl, cyclopropyl, cyclobutyl, halogen-substituted methyl, halogen-substituted ethyl (such as trifluoromethyl, dichloroethyl, etc.);

or, $R^2$ and $R^3$ together with the carbon atom attached thereto form a $C_3$-$C_{14}$ cycloalkyl, a 3- to 14-membered heterocycloalkyl, a $R^{3-1}$-substituted $C_3$-$C_{14}$ cycloalkyl, or a $R^{3-1}$-substituted 3- to 14-heterocycloalkyl, the 3- to 14-membered heterocycloalkyl independently contains one or more heteroatoms selected from the group consisting of boron, silicon, oxygen, sulfur, selenium, nitrogen and phosphorus, and the number of the heteroatoms is independently 1, 2, 3 or 4; particularly, $R^2$ and $R^3$ together with the carbon atom attached thereto form a $C_3$-$C_8$ cycloalkyl, a 3- to 8-membered heterocycloalkyl, a $R^{3-1}$-substituted $C_3$-$C_8$ cycloalkyl, or a $R^{3-1}$-substituted 3- to 8-membered heterocycloalkyl, the 3- to 8-membered heterocycloalkyl independently contains one or more heteroatoms selected from the group consisting of oxygen, sulfur and nitrogen, and the number of heteroatoms is independently 1, 2 or 3; more particularly, $R^2$ and $R^3$ together with the carbon atom attached thereto form cyclopropyl, halogen-substituted cyclopropyl, cyclobutyl, halogen-substituted cyclobutyl, cyclopentyl, cyclohexyl, oxetanyl, pyrrolidinyl, tetrahydrofuranyl, tetrahydrothienyl, tetrahydropyranyl, piperidinyl or morpholinyl;

Each $R^4$ is independently hydrogen, halogen, -$OR^{4-1}$, -CN, -$NR^{4-2}R^{4-3}$, -C(=O)$R^{4-4}$, -S(=O)$_2R^{4-5}$, $C_3$-$C_{14}$ cycloalkyl, 3- to 14-membered heterocycloalkyl, $C_1$-$C_7$ alkyl, $R^{4-6}$-substituted $C_1$-$C_7$ alkyl, $R^{4-6}$-substituted $C_3$-$C_{14}$ cycloalkyl, $R^{4-6}$-substituted 3- to 14-membered heterocycloalkyl, or oxo, the 3- to 14-membered heterocycloalkyl independently

contains one or more heteroatoms selected from the group consisting of boron, silicon, oxygen, sulfur, selenium, nitrogen and phosphorus, and the number of heteroatoms is independently 1, 2, 3 or 4; particularly, each $R^4$ is independently hydrogen, halogen, $-OR^{4-1}$, -CN, $C_3$-$C_8$ cycloalkyl, 3- to 8-membered heterocycloalkyl, $C_1$-$C_4$ alkyl, $R^{4-6}$-substituted $C_1$-$C_4$ alkyl, or oxo, the 3- to 8-membered heterocycloalkyl independently contains one or more heteroatoms selected from the group consisting of oxygen, sulfur and nitrogen, and the number of heteroatoms is independently 1, 2 or 3; more particularly, each $R^4$ is independently hydrogen, halogen, $C_1$-$C_4$ alkoxy, halogen- or deuterium-substituted $C_1$-$C_4$ alkoxy, -CN, $C_1$-$C_4$ alkyl, or halogen- or deuterium-substituted $C_1$-$C_4$ alkyl; more particularly, each $R^4$ is independently hydrogen, halogen, methoxy, ethoxy, halogen- or deuterium-substituted methoxy or ethoxy (such as $-OCF_3$), -CN, methyl, ethyl, or halogen- or deuterium-substituted methyl or ethyl (such as trifluoromethyl, dichloroethyl, $-CD_3$, etc.);

alternatively, any two adjacent $R^4$s together with the ring atoms attached thereto form a substituted or unsubstituted saturated or unsaturated non-aromatic $C_3$-$C_{10}$ cyclic hydrocarbyl, a substituted or unsubstituted saturated or unsaturated non-aromatic 4- to 6-membered heterocyclyl, a substituted or unsubstituted $C_6$-$C_{10}$ aryl, or a substituted or unsubstituted 4- to 6-membered heteroaryl, wherein, the 4- to 6-membered heterocyclyl and the 4- to 6-membered heteroaryl each independently contain one or more heteroatoms selected from the group consisting of oxygen, sulfur and nitrogen, and the number of heteroatoms is independently 1, 2 or 3; particularly, any two adjacent $R^4$s together with the ring atoms attached thereto form a substituted or unsubstituted, saturated or unsaturated non-aromatic $C_3$-$C_6$ cyclic hydrocarbyl, a substituted or unsubstituted saturated or unsaturated non-aromatic 5- to 6-membered heterocyclyl, a substituted or unsubstituted $C_6$-$C_{10}$ aryl, or a substituted or unsubstituted 5- to 6-membered heteroaryl, wherein, the 5- to 6-membered heterocyclyl and the 5- to 6-membered heteroaryl each independently contain one or more heteroatoms selected from the group consisting of oxygen, sulfur and nitrogen, and the number of heteroatoms is independently 1, 2 or 3; wherein, the substituent for the "substituted" includes 1, 2 or 3 groups selected from the group consisting of $C_1$-$C_6$ alkyl, $C_1$-$C_6$ alkoxy, halogen, -CN, amino, -OH, halogen-substituted $C_1$-$C_6$ alkyl, $C_1$-$C_6$ alkyl-monosubstituted or disubstituted amino, $C_3$-$C_6$ cycloalkyl, 3-6 membered heterocycloalkyl containing one or more heteroatoms independently selected from the group consisting of oxygen, sulfur and nitrogen, and oxo;

$R^{1-1}$, $R^{2-1}$ and $R^{4-1}$ are each independently hydrogen, deuterium, $C_1$-$C_7$ alkyl, halogen-substituted $C_1$-$C_7$ alkyl, $C_3$-$C_{14}$ cycloalkyl, or 3- to 14-membered heterocycloalkyl, the 3- to 14-membered heterocycloalkyl contains one or more heteroatoms selected from the group consisting of boron, silicon, oxygen, sulfur, selenium, nitrogen and phosphorus, and the number of heteroatoms is independently 1, 2, 3 or 4; particularly, $R^{1-1}$, $R^{2-1}$ and $R^{4-1}$ are each independently hydrogen, deuterium, $C_1$-$C_7$ alkyl, or halogen-substituted $C_1$-$C_7$ alkyl; more particularly, $R^{1-1}$, $R^{2-1}$ and $R^{4-1}$ are each independently hydrogen, deuterium, methyl, ethyl, n-propyl, isopropyl, halogen-substituted methyl, halogen-substituted ethyl, halogen-substituted n-propyl, halogen-substituted isopropyl (such as trifluoromethyl, dichloroethyl, etc.);

$R^{1-2}$, $R^{1-3}$, $R^{2-2}$, $R^{2-3}$, $R^{4-2}$, $R^{4-3}$ are independently hydrogen, $C_1$-$C_7$ alkyl, halogen-substituted $C_1$-$C_7$ alkyl, $C_3$-$C_{14}$ cycloalkyl or 3- to 14-membered heterocycloalkyl; the 3- to 14- membered heterocycloalkyl contains one or more heteroatoms selected from the group consisting of boron, silicon, oxygen, sulfur, selenium, nitrogen and phosphorus, and the number of heteroatoms may be 1, 2, 3 or 4;

alternatively, $R^{1-2}$ and $R^{1-3}$ together with the nitrogen atom attached thereto form a 3- to 14-membered heterocycloalkyl, or a $R^{1-2-1}$-substituted 3- to 14-membered heterocycloalkyl; the 3- to 14-membered heterocycloalkyl and the $R^{1-2-1}$-substituted 3-to 14-membered heterocycloalkyl independently contain one or more heteroatoms selected from the group consisting of boron, silicon, oxygen, sulfur, selenium, nitrogen and phosphorus, and the number of heteroatoms is independently 1, 2, 3 or 4; said $R^{1-2-1}$ is independently hydroxyl, halogen, oxo, -CN, $C_1$-$C_7$ alkyl or $C_1$-$C_7$ alkoxy;

alternatively, $R^{2-2}$ and $R^{2-3}$ together with the nitrogen atom attached thereto form a 3- to 14-membered heterocycloalkyl, or a $R^{2-2-1}$-substituted 3- to 14-membered heterocycloalkyl; the 3- to 14-membered heterocycloalkyl and the $R^{2-2-1}$-substituted 3-to 14-membered heterocycloalkyl independently contain one or more heteroatoms selected from the group consisting of boron, silicon, oxygen, sulfur, selenium, nitrogen and phosphorus, and the number of heteroatoms is independently 1, 2, 3 or 4; said $R^{2-2-1}$ is independently hydroxyl, halogen, oxo, -CN, $C_1$-$C_7$ alkyl or $C_1$-$C_7$ alkoxy;

alternatively, $R^{4-2}$ and $R^{4-3}$ together with the nitrogen atom attached thereto form a 3- to 14-membered heterocycloalkyl, or a $R^{4-2-1}$-substituted 3- to 14-membered heterocycloalkyl; the 3- to 14-membered heterocycloalkyl and the $R^{4-2-1}$-substituted 3-to 14-membered heterocycloalkyl independently contain one or more heteroatoms selected from the group consisting of boron, silicon, oxygen, sulfur, selenium, nitrogen and phosphorus, and the number of heteroatoms is independently 1, 2, 3 or 4; said $R^{4-2-1}$ is independently hydroxyl, halogen, oxo, -CN, $C_1$-$C_7$ alkyl or $C_1$-$C_7$ alkoxy;

$R^{1-4}$, $R^{2-4}$, $R^{3-1}$ and $R^{4-6}$ represent 1 to 3 substituents, and are each independently one or more selected from the group consisting of hydrogen, deuterium, -CN, halogen, $-OR^{1-4-1}$, $NR^{1-4-2}R^{1-4-3}$, $C_1$-$C_7$ alkyl, $C_3$-$C_{14}$ cycloalkyl and

3- to 14-membered heterocycloalkyl; the 3- to 14-membered heterocycloalkyl contains one or more heteroatoms selected from the group consisting of boron, silicon, oxygen, sulfur, selenium, nitrogen and phosphorus, and the number of heteroatoms is independently 1, 2, 3 or 4;

$R^{4-4}$ and $R^{4-5}$ are independently hydrogen, $-OR^{4-4-1}$, $NR^{4-4-2}R^{4-4-3}$, $C_1$-$C_7$ alkyl, $C_3$-$C_{14}$ cycloalkyl, or 3- to 14-membered heterocycloalkyl; the 3- to 14-membered heterocycloalkyl contains one or more heteroatoms selected from the group consisting of boron, silicon, oxygen, sulfur, selenium, nitrogen and phosphorus, and the number of heteroatoms is independently 1, 2, 3 or 4;

$R^{1-4-1}$ and $R^{4-4-1}$ are independently hydrogen, halogen, $C_1$-$C_7$ alkyl, $C_3$-$C_{14}$ cycloalkyl, or 3- to 14-membered heterocycloalkyl, the 3- to 14-membered heterocycloalkyl contains one or more heteroatoms selected from the group consisting of boron, silicon, oxygen, sulfur, selenium, nitrogen and phosphorus, and the number of heteroatoms is independently 1, 2, 3 or 4;

$R^{1-4-2}$, $R^{1-4-3}$, $R^{4-4-2}$ and $R^{4-4-3}$ are independently hydrogen, $C_1$-$C_7$ alkyl, $C_3$-$C_{14}$ cycloalkyl, or 3- to 14-membered heterocycloalkyl, the 3- to 14-membered heterocycloalkyl contains one or more heteroatoms selected from the group consisting of boron, silicon, oxygen, sulfur, selenium, nitrogen and phosphorus, and the number of heteroatoms is independently 1, 2, 3 or 4;

alternatively, $R^{1-4-2}$ and $R^{1-4-3}$ together with the nitrogen atom attached thereto form a 3- to 14-membered heterocycloalkyl, or a $R^{1-4-2-1}$-substituted 3- to 14-membered heterocycloalkyl; the 3- to 14-membered heterocycloalkyl and the $R^{1-4-2-1}$-substituted 3-to 14-membered heterocycloalkyl independently contain one or more heteroatoms selected from the group consisting of boron, silicon, oxygen, sulfur, selenium, nitrogen and phosphorus, and the number of heteroatoms is independently 1, 2, 3 or 4; the $R^{1-4-2-1}$ is independently hydroxyl, halogen, oxo, -CN, $C_1$-$C_7$ alkyl or $C_1$-$C_7$ alkoxy;

alternatively, $R^{4-4-2}$ and $R^{4-4-3}$ together with the nitrogen atom attached thereto form a 3- to 14-membered heterocycloalkyl, or a $R^{4-4-2-1}$-substituted 3- to 14-membered heterocycloalkyl; the 3- to 14-membered heterocycloalkyl and the $R^{4-4-2-1}$-substituted 3-to 14-membered heterocycloalkyl independently contain one or more heteroatoms selected from the group consisting of boron, silicon, oxygen, sulfur, selenium, nitrogen and phosphorus, and the number of heteroatoms is independently 1, 2, 3 or 4; the $R^{4-4-2-1}$ is independently hydroxyl, halogen, oxo, -CN, $C_1$-$C_7$ alkyl or $C_1$-$C_7$ alkoxy.

According to one embodiment of the present invention, in the compound represented by formula I,

n is 0, 1, 2, 3, 4 or 5;

W, X, Y and Z are independently CH or N;

ring A is 5- to 10-membered heteroaryl, preferably 5- to 6-membered heteroaryl, wherein the 5- to 10-membered heteroaryl or 5- to 6-membered heteroaryl independently contains one or more heteroatoms selected from the group conisisting of oxygen, sulfur, and nitrogen, and the number of heteroatoms is independently 1, 2, 3 or 4; wherein, ring A is optionally substituted by 1 to 3 substituents selected from the group consisting of halogen, $C_1$-$C_4$ alkyl, $C_1$-$C_4$ alkoxy, hydroxyl and cyano;

ring B is $C_3$-$C_{14}$ cycloalkyl, 3- to 14-membered heterocycloalkyl, $C_6$-$C_{12}$ aryl, or 5-to 10-membered heteroaryl, the 5- to 10-membered heteroaryl and 3- to 14-membered heterocycloalkyl each independently contain one or more heteroatoms selected from the group consisting of oxygen, sulfur and nitrogen, and the number of heteroatoms is independently 1, 2, 3 or 4;

$R^1$ is $-NR^{1-2}R^{1-3}$ or $C_1$-$C_7$ alkyl, $R^{1-2}$ and $R^{1-3}$ are each as defined above;

$R^2$ and $R^3$ are each independently hydrogen, halogen, $C_1$-$C_7$ alkyl, or $C_3$-$C_{14}$ cycloalkyl; or, $R^2$ and $R^3$ together with the carbon atom attached thereto form a $C_3$-$C_{14}$ cycloalkyl, a $R^{3-1}$-substituted $C_3$-$C_{14}$ cycloalkyl, or a 3- to 14-membered heterocycloalkyl, wherein $R^{3-1}$ is as defined above, the 3- to 14-membered heterocycloalkyl independently contains one or more heteroatoms selected from the group consisting of boron, silicon, oxygen, sulfur, selenium, nitrogen and phosphorus, and the number of heteroatoms is independently 1, 2, 3 or 4;

Each $R^4$ is independently hydrogen, halogen, $-OR^{4-1}$, -CN, $C_1$-$C_7$ alkyl, or $R^{4-6}$-substituted $C_1$-$C_7$ alkyl;

$R^{4-1}$s are each independently hydrogen, deuterium, $C_1$-$C_7$ alkyl, or halogen-substituted $C_1$-$C_7$ alkyl; $R^{4-6}$ is as defined above, preferably independently one or more selected from the group consisting of halogen and deuterium.

According to one embodiment of the present invention, in the compound represented by formula I,

n is 0, 1, 2, 3, 4 or 5;

W, X, Y and Z are independently CH or N;

ring A is 5- to 6-membered heteroaryl, the 5- to 6-membered heteroaryl contains one or more heteroatoms selected from the group consisting of oxygen, sulfur and nitrogen, and the number of heteroatoms is independently 1 or 2; wherein, ring A is optionally substituted by 1 to 3 substituents selected from the group consisting of halogen, $C_1$-$C_4$

alkyl, $C_1$-$C_4$ alkoxy, hydroxyl, and cyano;
ring B is $C_3$-$C_{10}$ cycloalkyl, 3- to 10-membered heterocycloalkyl, $C_6$-$C_{10}$ aryl or 5-to 6-membered heteroaryl, the 5- to 6-membered heteroaryl and 3- to 10-membered heterocycloalkyl each independently contain one or more heteroatoms selected from the group consisting of oxygen, sulfur and nitrogen, and the number of heteroatoms is independently 1 or 2;
$R^1$ is -$NR^{1-2}R^{1-3}$ or $C_1$-$C_7$ alkyl, wherein $R^{1-2}$ and $R^{1-3}$ are each as defined above;
$R^2$ and $R^3$ are each independently hydrogen, halogen, $C_1$-$C_7$ alkyl, or $C_3$-$C_{14}$ cycloalkyl; or, $R^2$ and $R^3$ together with the carbon atom attached thereto form a $C_3$-$C_8$ cycloalkyl, a $R^{3-1}$-substituted $C_3$-$C_8$ cycloalkyl, or a 3- to 8-membered heterocycloalkyl, wherein $R^{3-1}$ is as defined above, the 3- to 8-membered heterocycloalkyl independently contains one or more heteroatoms selected from the group consisting of oxygen, sulfur and nitrogen, and the number of heteroatoms is independently 1 or 2;
Each $R^4$ is independently hydrogen, halogen, -$OR^{4-1}$, -CN, $C_1$-$C_7$ alkyl, or halogen- or deuterium-substituted $C_1$-$C_7$ alkyl;
$R^{4-1}$s are each independently hydrogen, deuterium, $C_1$-$C_7$ alkyl, or halogen-substituted $C_1$-$C_7$ alkyl.

According to one embodiment of the present invention, in the compound represented by formula I,

n is 0, 1, 2, 3, 4 or 5;
W, X, Y and Z are independently CH or N;
ring A is pyridyl, pyrimidyl or thiazolyl;
ring B is $C_3$-$C_8$ cycloalkyl, 4- to 8-membered heterocycloalkyl, $C_6$-$C_{10}$ aryl or 5- to 6-membered heteroaryl, the 5- to 6-membered heteroaryl and 4- to 8-membered heterocycloalkyl each independently contain one or more heteroatoms selected from the group consisting of oxygen, sulfur and nitrogen, and the number of heteroatoms is independently 1 or 2;
$R^1$ is -$NR^{1-2}R^{1-3}$ or $C_1$-$C_7$ alkyl (including methyl, ethyl, n-propyl, isopropyl), wherein $R^{1-2}$ and $R^{1-3}$ are each as defined above;
$R^2$ and $R^3$ are each independently hydrogen, halogen, $C_1$-$C_7$ alkyl or $C_3$-$C_{14}$ cycloalkyl; or, $R^2$ and $R^3$ together with the carbon atom attached thereto form a $C_3$-$C_8$ cycloalkyl, a $R^{3-1}$-substituted $C_3$-$C_8$ cycloalkyl, or a 3- to 8-membered heterocycloalkyl, wherein $R^{3-1}$ is as defined above, the 3- to 8-membered heterocycloalkyl independently contains one or more heteroatoms selected from the group consisting of oxygen, sulfur and nitrogen, and the number of heteroatoms is independently 1 or 2;
Each $R^4$ is independently hydrogen, halogen, -$OR^{4-1}$, -CN, $C_1$-$C_7$ alkyl, or halogen- or deuterium-substituted $C_1$-$C_7$ alkyl;
$R^{4-1}$s are each independently hydrogen, deuterium, $C_1$-$C_7$ alkyl, or halogen-substituted $C_1$-$C_7$ alkyl.

According to one embodiment of the present invention, in the compound represented by formula I,

n is 0, 1, 2, 3, 4 or 5;
W, X, Y and Z are independently CH or N;
ring A is pyridyl, pyrimidyl or thiazolyl;
ring B is cyclopropyl, cyclobutyl, cyclopentyl, cyclohexyl, oxetanyl, pyrrolidinyl, tetrahydrofuryl, tetrahydrothiophenyl, tetrahydropyranyl, tetrahydrothiopyranyl, piperidyl, piperazinyl, morpholinyl, phenyl, pyrimidinyl, thiazolyl, pyrazolyl, imidazolyl, oxazolyl or pyridyl;
$R^1$ is -$NR^{1-2}R^{1-3}$ or $C_1$-$C_7$ alkyl (including methyl, ethyl, n-propyl, isopropyl), wherein $R^{1-2}$ and $R^{1-3}$ are each as defined above;
$R^2$ and $R^3$ are each independently hydrogen, halogen or $C_1$-$C_7$ alkyl; or, $R^2$ and $R^3$ together with the carbon atom attached thereto form a $C_3$-$C_6$ cycloalkyl (such as cyclopropyl, cyclobutyl, cyclopentyl, cyclohexyl), a halogen-substituted $C_3$-$C_6$ cycloalkyl (such as fluorine-substituted cyclopropyl, fluorine-substituted cyclobutyl, the number of fluorine atoms may be 1 to 3), or a 3- to 6-membered heterocycloalkyl (such as oxetanyl), the 3- to 6-membered heterocycloalkyl contains one or more heteroatoms selected from the group consisting of oxygen, sulfur and nitrogen, and the number of heteroatoms is independently 1 or 2;
Each $R^4$ is independently hydrogen, halogen, -$OR^{4-1}$, -CN, $C_1$-$C_7$ alkyl, or halogen- or deuterium-substituted $C_1$-$C_7$ alkyl;
$R^{4-1}$s are each independently hydrogen, deuterium, $C_1$-$C_7$ alkyl, or halogen-substituted $C_1$-$C_7$ alkyl.

According to one embodiment of the present invention, in the compound represented by formula I,
ring A is selected from the group consisting of

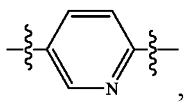, 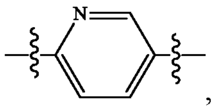,

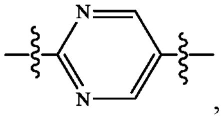, and 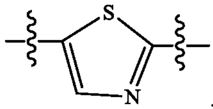,

wherein n, W, X, Y, Z, $R^1$, $R^2$, $R^3$, $R^4$ and ring B are each as defined above, and it is connected to the amido group at the left side and to the carbonyl group at the right side.

According to one embodiment of the present invention, in the compound represented by formula I,

N is 1, 2, 3 or 4 ;
W, X, Y and Z are independently CH or N;
ring A is selected from the group consisting of

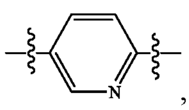, 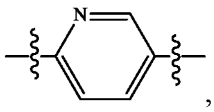,

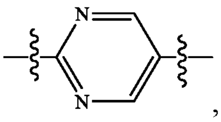, and 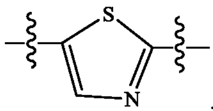,

and it is connected to the amido group at the left side and to the carbonyl group at the right side;
ring B is selected from the group consisting of

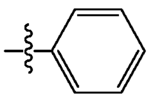, 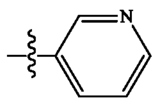,

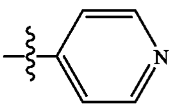, 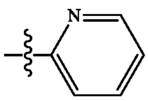, 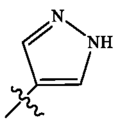, 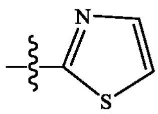, 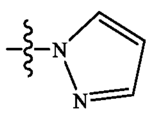, 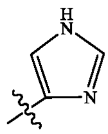,

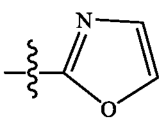, 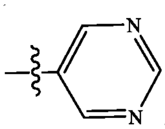, and 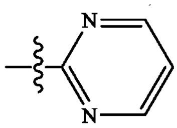;

$R^1$ is methyl, ethyl, n-propyl, methylamino, ethylamino or dimethylamino;
$R^2$ and $R^3$ are each independently hydrogen, fluorine, chlorine, methyl, ethyl, n-propyl or isopropyl; or, $R^2$ and $R^3$ together with the carbon atom attached thereto form

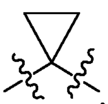, 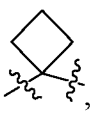, 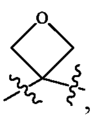, or 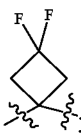;

Each $R^4$ is independently hydrogen, fluorine, chlorine, bromine, trifluoromethoxy, -CN, methyl, ethyl, trifluoromethyl, 2,2,2-trifluoroethyl, 1,1-difluoroethyl group, or trideuteromethyl.

According to one embodiment of the present invention, in the compound represented by formula I, W, X, Y and Z are all CH, or the compound represented by formula I is shown as the following formula I-A,

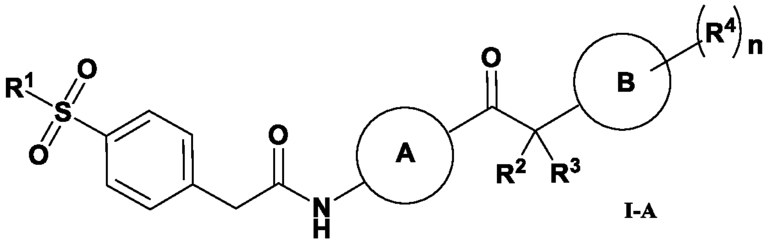

I-A

wherein n, $R^1$, $R^2$, $R^3$, $R^4$, ring A and ring B are each as defined above.

In one preferable embodiment, the compound represented by formula I is optionally any one of the following compounds:

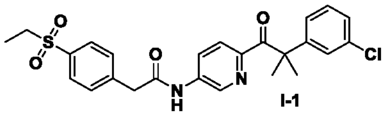
I-1 ,
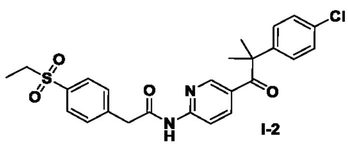
I-2 ,
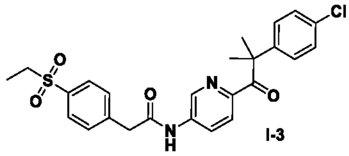
I-3 ,
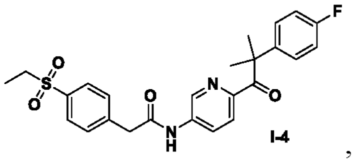
I-4 ,
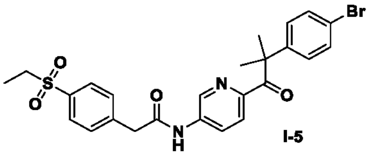
I-5 ,
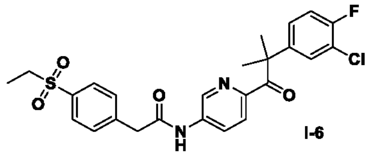
I-6 ,
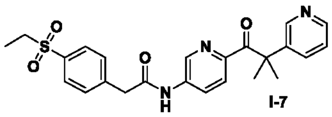
I-7 ,
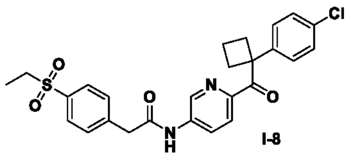
I-8 ,
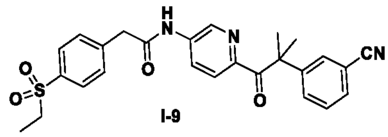
I-9 ,
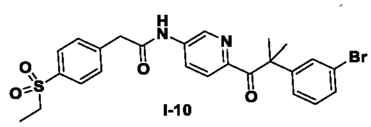
I-10 ,
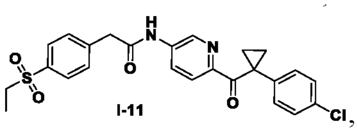
I-11 ,
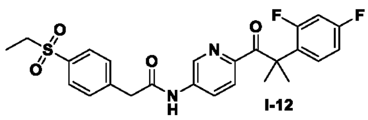
I-12 ,
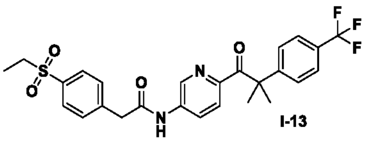
I-13 ,
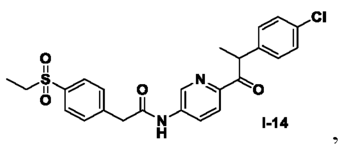
I-14 ,
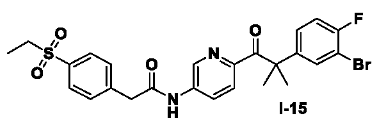
I-15 ,
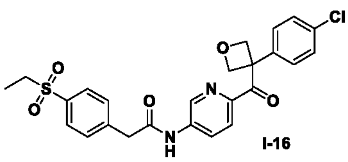
I-16 ,
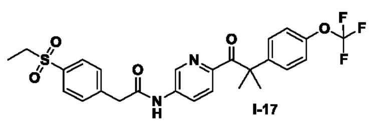
I-17 ,
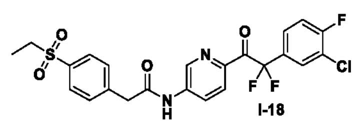
I-18 ,

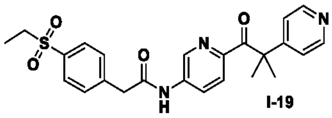
I-19 ,
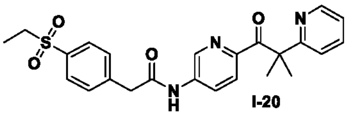
I-20 ,
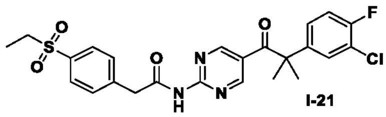
I-21 ,

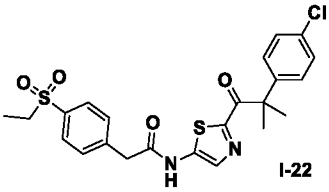
I-22 ,
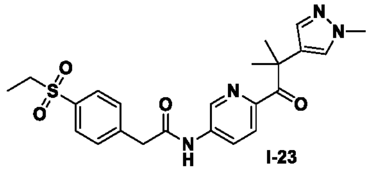
I-23 ,

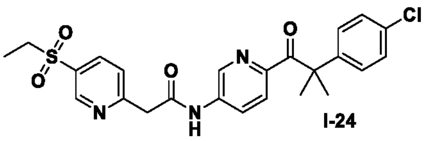
I-24 ,

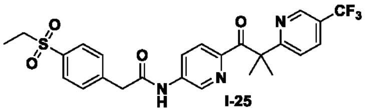
I-25 ,
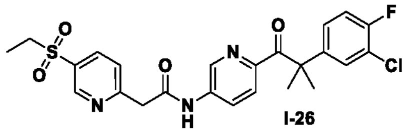
I-26 ,

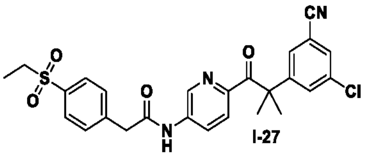
I-27 ,
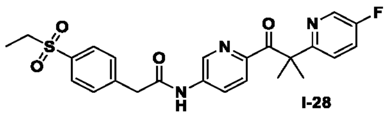
I-28 ,

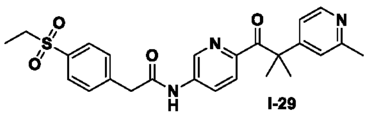
I-29 ,
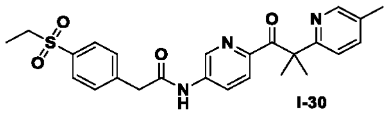
I-30 ,

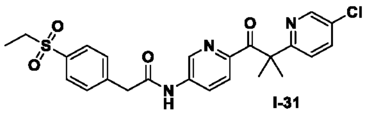
I-31 ,
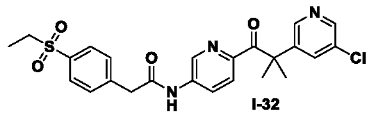
I-32 ,

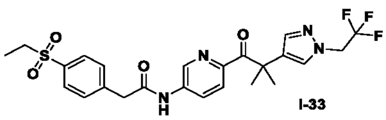
I-33 ,
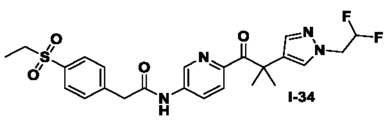
I-34 ,

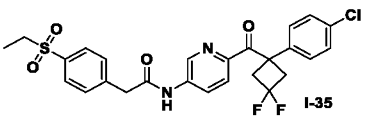
I-35 ,
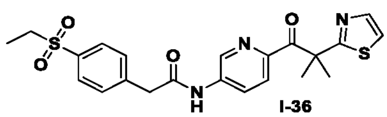
I-36 ,

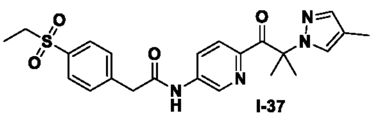
I-37 .
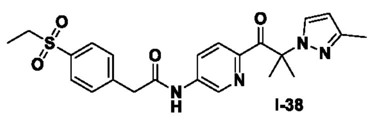
I-38

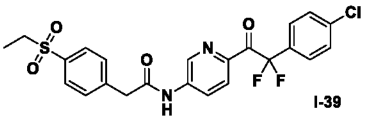
I-39,

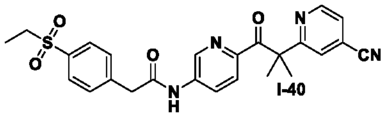
I-40,

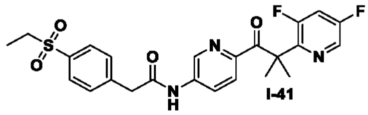
I-41,

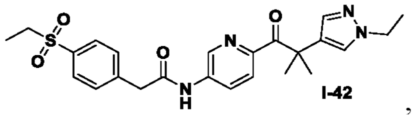
I-42,

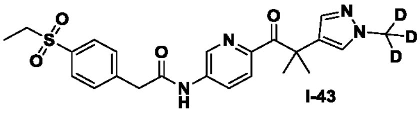
I-43,

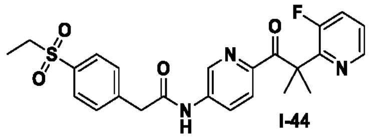
I-44,

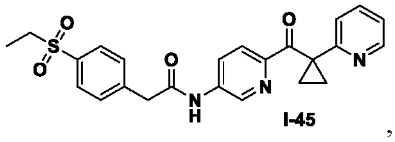
I-45,

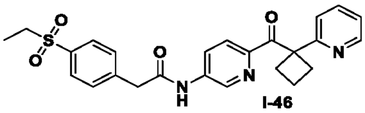
I-46,

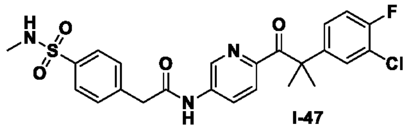
I-47,

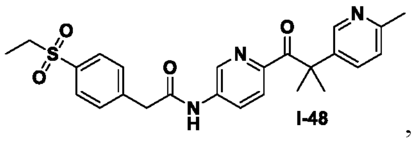
I-48,

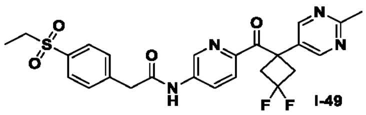
I-49,

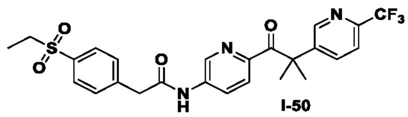
I-50,

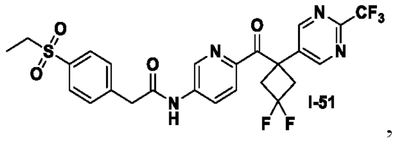
I-51,

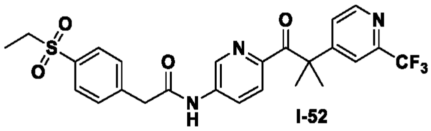
I-52,

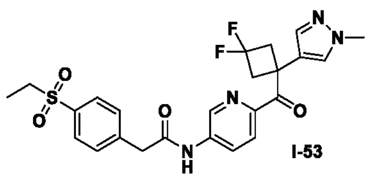
I-53,

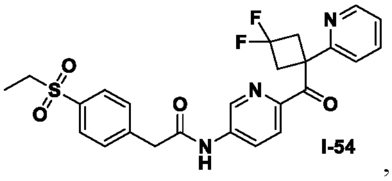
I-54 ,

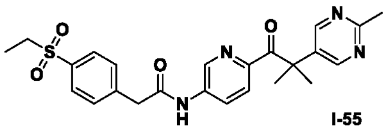
I-55 ,

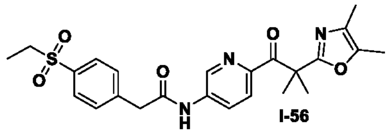
I-56 ,

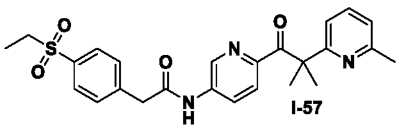
I-57 ,

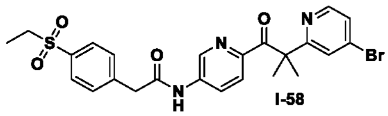
I-58 ,

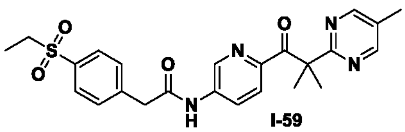
I-59 ,

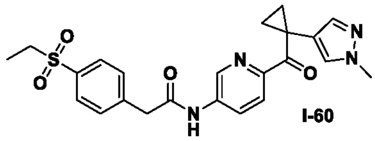
I-60 ,

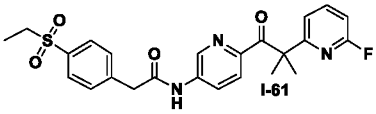
I-61 ,

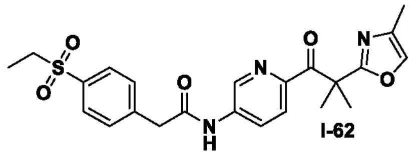
I-62 ,

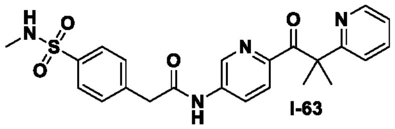
I-63 ,

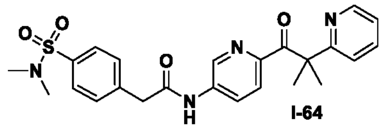
I-64 ,

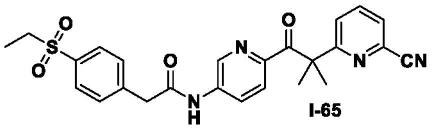
I-65 ,

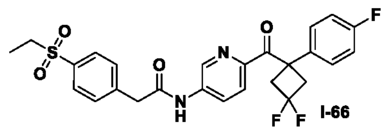
I-66 ,

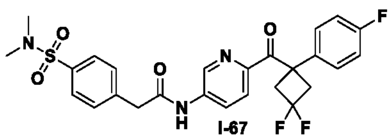
I-67 ,

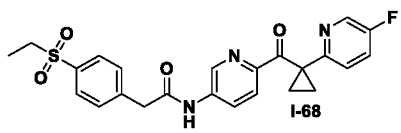
I-68 ,

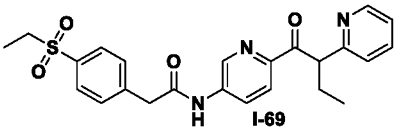
I-69 ,

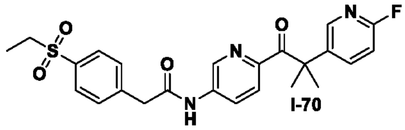
I-70 ,

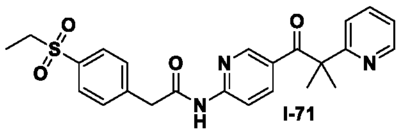
I-71,

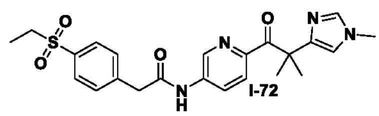
I-72,

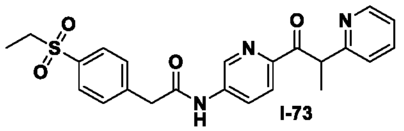
I-73,

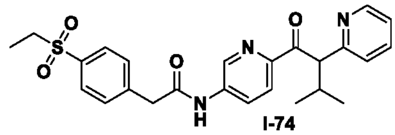
I-74,

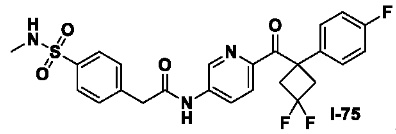
I-75,

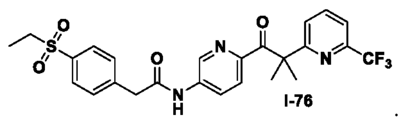
I-76.

The present invention also provides a method for preparing the compound represented by formula I, pharmaceutically acceptable salt thereof, solvate thereof, solvate of the pharmaceutically acceptable salt, metabolite thereof or prodrug thereof, wherein,

the method for preparing the compound represented by formula I includes the following step: in a solvent, the compound m or a salt of the compound m (the compound m') and the compound I-f are subjected to the condensation reaction as shown in the following schemes to obtain the compound represented by formula I;

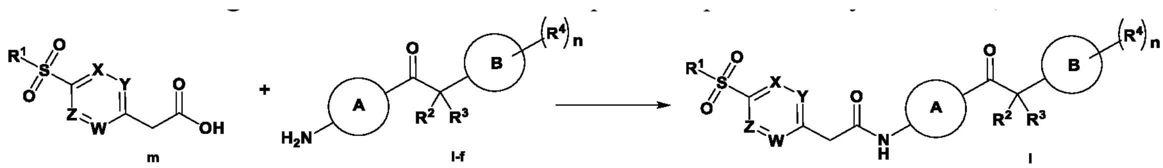

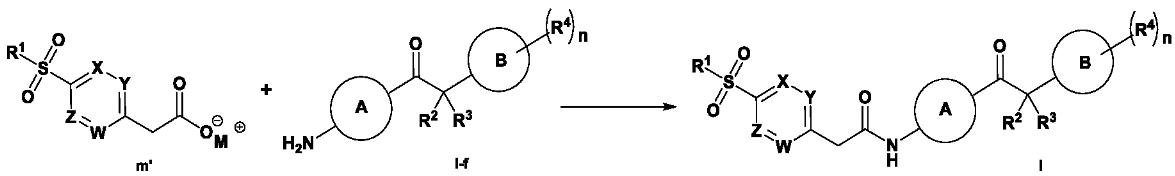

wherein, n, X, Y, Z, W, $R^1$, $R^2$, $R^3$, $R^4$, ring A and ring B are defined the same as above, and M represents an alkali metal such as Li, Na, and K.

The condensation reaction may be carried out under a condensation reaction condition known in the art, for example, it may be carried out under the action of a condensing agent under a basic condition, the base may be one or more selected from the group consisting of triethylamine (TEA), pyridine, and N,N-diisopropylethylamine (DIPEA), the condensing agent may be one or more selected from the group consisting of O-(benzotriazol-1-yl)-bis(dimethylamino)carbonium tetrafluoroborate (TBTU), O-(7-azabenzotriazol-1-yl)-bis(dimethylamino)carbonium hexafluorophosphate (HATU), propyl phosphoric anhydride (T3P), 1-(3-dimethylaminopropyl)-3-ethylcarbodiimide hydrochloride (EDCI), and 1-hydroxybenzotriazole (HOBT), and the solvent used in the condensation reaction may be one or more selected from the group consisting of dichloromethane (DCM) and N,N-dimethylformamide (DMF), and the reaction may be carried out at a temperature of -20 °C - 80 °C.

Further, the compound I-f may be obtained by deprotecting the compound I-e as shown in the following scheme:

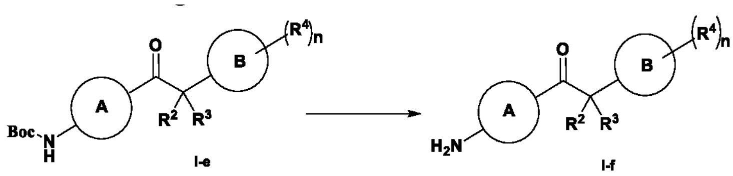

It can be understood that the above deprotection reaction may be carried out under a condition well known in the art, for example, it may be carried out under an acidic condition (hydrochloric acid/dioxane, trifluoroacetic acid, etc.) at a temperature of -20 °C - 50 °C. The solvent used in the deprotection reaction may be one or more selected from the group consisting of dichloromethane (DCM) and methanol (MeOH).

Further, the compound I-e may be obtained by oxidizing the compound I-d as shown in the following scheme:

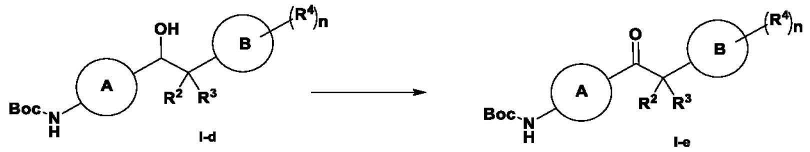

The oxidation reaction may be carried out under a condition well known in the art, for example, the oxidation reaction may be carried out using one or more oxidizing agents selected from the group consisting of pyridinium chlorochromate (PCC), manganese dioxide ($MnO_2$), 2-iodoxybenzoic acid (IBX), etc. at a temperature of 0 °C - 110 °C. The solvent used in the oxidation reaction may be one or more selected from the group consisting of dichloromethane (DCM) and ethyl acetate (EtOAc).

Alternatively, the compound I-e may be obtained by addition reaction of the compound p1, p2 or p3 with the compound n as shown in the following schemes:

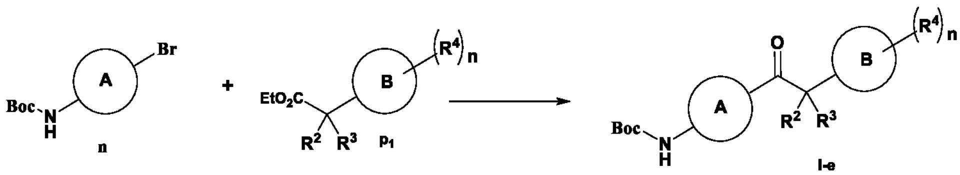

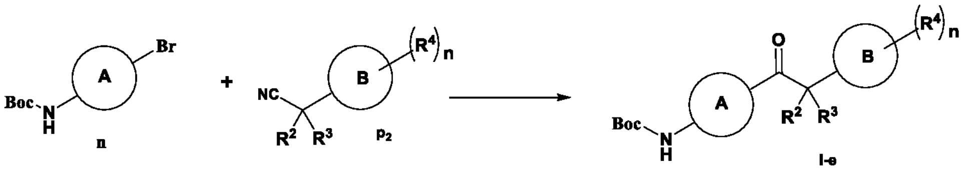

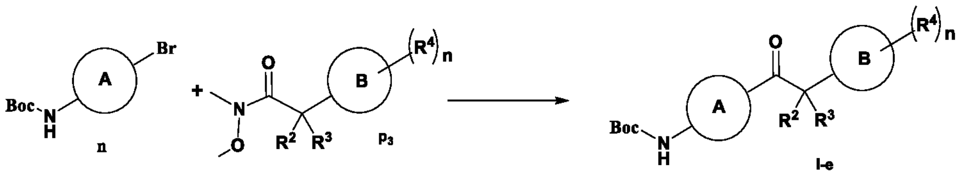

The addition reaction may be carried out under a condition well known in the art, for example, it may be carried out by using one or more alkyllithium reagents selected from the group consisting of butyllithium, lithium bis(trimethylsilyl)amide, etc. under an inert atmosphere at a reaction temperature of -78 °C - 0 °C. The solvent used in the addition reaction may be one or more selected from the group consisting of tetrahydrofuran (THF), diethyl ether, and n-hexane.

Further, the compound I-d may be obtained by addition reaction of the compound I-c and the compound n as shown in the following schemes:

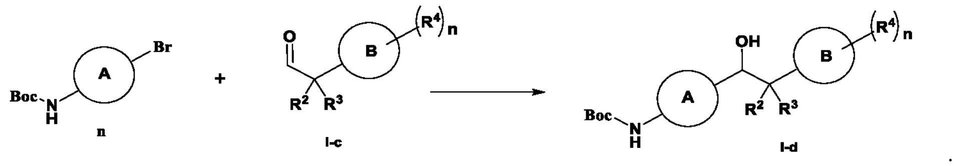

The addition reaction may be carried out under a condition well known in the art, for example, may be carried out by using one or more alkyllithium reagents selected from the group consisting of butyllithium, lithium bis(trimethylsilyl)amide (LiHMDS) and the like under an inert atmosphere at a reaction temperature of -78 °C - 0 °C. The solvent used in the addition reaction may be one or more selected from the group consisting of tetrahydrofuran (THF), diethyl ether, and n-hexane.

Further, the compound I-c may be obtained by reducing the compound I-b as shown in the following scheme:

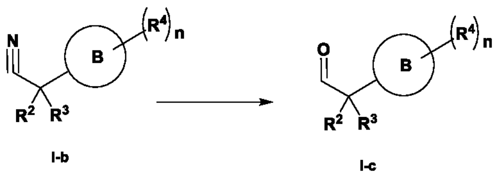

The reduction reaction may be carried out under a condition well known in the art, for example, it may be carried out under the action of one or more reducing agents selected from the group consisting of diisobutylaluminum hydride (DIBAL-H), lithium aluminum hydride ($LiAlH_4$), etc. at a reaction temperature of -78 °C - 0 °C. The solvent used in the reduction reaction may be selected from the group consisting of tetrahydrofuran (THF), dichloromethane (DCM), n-hexane, and diethyl ether.

Alternatively, the compound I-c may be obtained by subjecting the compound I-c' to an oxidation reaction as shown in the following scheme:

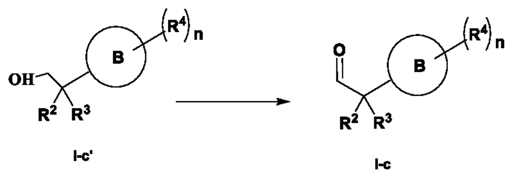

The oxidation reaction may be carried out under conditions well known in the art, for example, the oxidation reaction may be carried out by using one or more oxidizing agents selected from the group consisting of Dess-Martin, DMSO-$(COCl)_2$, etc. at a temperature of -78 ° C - 40 °C.

Further, the compound I-c' may be obtained by reducing the compound I-b' as shown in the following scheme:

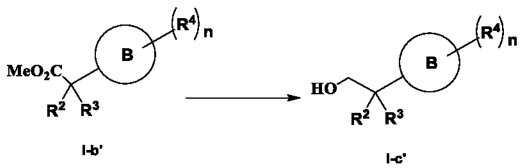

The reduction reaction may be carried out under a condition well known in the art, for example, the reduction reaction may be carried out by using one or more reducing agents selected from the group consisting of lithium aluminum hydride ($LiAlH_4$), diisobutyl aluminum hydride (DIBAL-H), etc. under the protection of an inert gas at a temperature of -78 ° C - 50 ° C; and the solvent used in the reduction reaction may be selected from the group consisting of tetrahydrofuran (THF), dichloromethane (DCM), n-hexane, and diethyl ether.

Alternatively, the compound I-c may be obtained by reducing the compound 1-b' as shown in the following scheme:

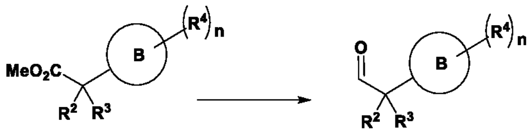

I-b' I-c

.

The reduction reaction may be carried out under a condition well known in the art, for example, it may be carried out at a reaction temperature of -78 °C - 0 °C under the action of a reducing agent selected from the group consisting of diisobutylaluminum hydride (DIBAL-H), etc. The solvent used in the reduction reaction may be selected from the group consisting of tetrahydrofuran (THF), dichloromethane (DCM), n-hexane, and ditheyl ether.

Further, the compound 1-b may be obtained by subjecting the compound I-a to a nucleophilic substitution reaction as shown in the following scheme

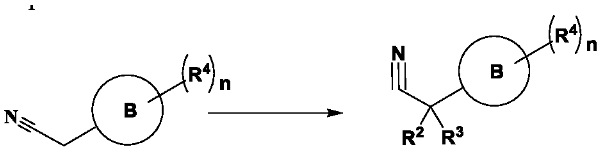

I-a I-b

For example, the compound I-a and a haloalkane are subjected to a nucleophilic substitution reaction in the presence of one or more reagents selected from the group consisting of sodium hydride, sodium tert-butoxide, potassium tert-butoxide, lithium bis(trimethylsilyl)amide, etc. at a temperature of -78 °C - 50 °C to obtain the compound 1-b; the solvent used in the nucleophilic substitution reaction may be one or more selected from the group consisting of N,N-dimethylformamide (DMF), and tetrahydrofuran (THF), and the example of the haloalkane may be selected from the group consisting of iodomethane and 1,2-dibromoethane.

Alternatively, the compound 1-b' may be obtained by subjecting the compound I-a' to a nucleophilic substitution reaction as shown in the following scheme:

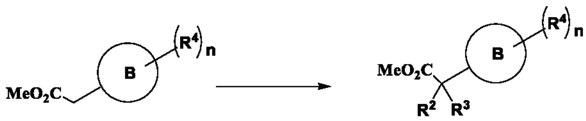

I-a' I-b'

.

For example, the compound I-a' and a haloalkane are subjected to a nucleophilic substitution reaction in the presence of one or more reagents selected from the group consisting of sodium hydride, sodium tert-butoxide, potassium tert-butoxide, lithium bis(trimethylsilyl)amide, etc. at a temperature of -78 °C - 50 °C to obtain the compound 1-b'; the solvent used in the nucleophilic substitution reaction may be one or more selected from the group consisting of tetrahydrofuran (THF) and N,N-dimethylformamide (DMF), and the example of the haloalkane may be selected from the group consisting of iodomethane and 1,2-dibromoethane.

In each of the above reactions, n, $R^1$, $R^2$, $R^3$, $R^4$, ring A and ring B are defined respectively the same as described above.

The present invention also provides a pharmaceutical composition, which comprises one or more selected from the group consisting of the compound represented by formula I, the pharmaceutically acceptable salt thereof, the solvate thereof, the solvate of the pharmaceutically acceptable salt, the metabolite thereof and the prodrug thereof, and optionally, a pharmaceutically acceptable carrier.

The present invention further provides a use of the compound represented by formula I, the pharmaceutically acceptable salt thereof, the solvate of, the solvate of the pharmaceutically acceptable salt, the metabolite thereof, the prodrug thereof, or the above pharmaceutical composition in the preparation of a RORγt protein receptor modulator.

The present invention further provides a use of the compound represented by formula I, the pharmaceutically acceptable salt thereof, the solvate thereof, the solvate of the pharmaceutically acceptable salt, the metabolite thereof, the prodrug thereof, or the above pharmaceutical composition in the preparation of a medicament for preventing or treating a disease associated with RORγt protein receptor.

The disease associated with RORγt protein receptor is preferably one or more selected from the group consisting

of psoriasis, multiple sclerosis (MS), rheumatoid arthritis (RA), inflammatory bowel disease (IBD), ankylosing spondylitis, systemic lupus erythematosus, Behcet's disease, and chronic obstructive pulmonary disease.

The present invention further provides a method for preventing and/or treating a disease, said method comprising administering to a subject in need of the treatment an effective amount of one or more selected from the group consisting of the compound represented by formula I, the pharmaceutically acceptable salt thereof, the solvate thereof, the solvate of the pharmaceutically acceptable salt, the metabolite thereof, and the prodrug thereof, or the above pharmaceutical composition; wherein the disease is one associated with the expression of the disease associated with RORyt protein receptor.

In the method, the disease associated with the expression of the disease associated with RORyt protein receptor is the same as above.

The term "pharmaceutically acceptable" means that salts, solvents, auxiliary materials, etc. are generally non-toxic, safe and suitable for use by patients. The "patient" is preferably a mammal, more preferably a human.

The term "pharmaceutically acceptable salt" refers to a salt prepared from the compound of the present invention with a relatively non-toxic, pharmaceutically acceptable acid or base. When the compound of the present invention contains a relatively acidic functional group, a base addition salt may be obtained by contacting the neutral form of such compound with a sufficient amount of a pharmaceutically acceptable base in a pure solution or in a suitable inert solvent. Pharmaceutically acceptable base addition salts include, but are not limited to, lithium salts, sodium salts, potassium salts, calcium salts, aluminum salts, magnesium salts, zinc salts, bismuth salts, ammonium salts, diethanolamine salts. When the compound of the present invention contains a relatively basic functional group, an acid addition salt may be obtained by contacting the neutral form of such compound with a sufficient amount of a pharmaceutically acceptable acid in a pure solution or in a suitable inert solvent. The pharmaceutically acceptable acid includes inorganic acids, including but not limited to, hydrochloric acid, hydrobromic acid, hydroiodic acid, nitric acid, carbonic acid, phosphoric acid, phosphorous acid, sulfuric acid and the like. The pharmaceutically acceptable acid includes organic acids, including but not limited to, acetic acid, propionic acid, oxalic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, salicylic acid, tartaric acid, methanesulfonic acid, isonicotinic acid, acidic citric acid, oleic acid, tannic acid, pantothenic acid, hydrogen tartrate, ascorbic acid, gentisic acid, fumaric acid, gluconic acid, sugar acid, formic acid, ethanesulfonic acid, pamoic acid (i.e. 4,4'-methylene-bis(3-hydroxy-2-naphthoic acid), amino acids (such as glutamic acid, arginine), etc. When the compound of the present invention contains relatively acidic and relatively basic functional groups, it can be converted into a base addition salt or an acid addition salt. For details, see Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

The term "solvate" refers to a substance formed by the compound of the present invention in combination with a stoichiometric or non-stoichiometric amount of a solvent. The solvent molecules in a solvate may exist in an ordered or non-ordered arrangement. The solvent includes but not limited to, water, methanol, ethanol and the like.

"Pharmaceutically acceptable salt" and "solvate" in the term "solvate of the pharmaceutically acceptable salt" are as described above and the term "solvate of the pharmaceutically acceptable salt" refers to a substance prepared from the compound of the present invention by firstly reacting with a relatively non-toxic, pharmaceutically acceptable acid or base and then combining with a stoichiometric or non-stoichiometric amount of a solvent. The "solvate of the pharmaceutically acceptable salt" includes, but is not limited to, hydrochloride monohydrate of the compound of the present invention.

The "compound", "pharmaceutically acceptable salt", "solvate" and "solvate of the pharmaceutically acceptable salt" may exist in crystal form or amorphous form. The term "crystal form" means that the ions or molecules therein are arranged strictly periodically in a three-dimensional space in a certain manner with regularity of periodic recurrence at a certain distance; due to the difference in the above periodic arrangement, there may be many crystal forms, that is, polymorphism. The term "amorphous" means that the ions or molecules therein are in a disorderly distribution state, that is, there is no periodic arrangement among ions or molecules.

The "compound", "pharmaceutically acceptable salt", "solvate" and "solvate of the pharmaceutically acceptable salt" may be present in a single stereoisomer or in the form of a mixture (such as a racemate) if they have stereoisomers. The term "stereoisomer" refers to cis-trans isomers or optical isomers. These stereoisomers may be separated, purified and enriched by asymmetric synthesis methods or chiral separation methods (including but not limited to thin layer chromatography, rotary chromatography, column chromatography, gas chromatography, high pressure liquid chromatography, etc.), and may also be obtained by chiral resolution through bond formation (chemical combination, etc.) or salt formation (physical combination, etc.) with other chiral compounds. The term "single stereoisomer" means that the mass content of one stereoisomer of the compound of the present invention is not less than 95% relative to all stereoisomers of the compound.

The "compound", "pharmaceutically acceptable salt", "solvate" and "solvate of the pharmaceutically acceptable salt" may be present in a single tautomer or in a mixture thereof, preferably in the form predominantly with the relatively

stable tautomer, if tautomers exist.

The atoms in the "compound", "pharmaceutically acceptable salt", "solvate" and "solvate of the pharmaceutically acceptable salt" may exist in their natural or unnatural abundance form. Taking hydrogen atom as an example, the form of its natural abundance means that about 99.985% thereof is protium, and about 0.015% is deuterium; the form of its unnatural abundance is, for example, about 95% thereof is deuterium. That is, one or more atoms in the "compound", "pharmaceutically acceptable salt", "solvate" and "solvate of the pharmaceutically acceptable salt" may be present in an unnatural abundance form.

When any variable (such as $R^{1-1-1}$) appears multiple times in the definition of a compound, the definition of the variable at each position has nothing to do with the definitions at other positions, and their definitions are independent of each other and do not affect each other. Therefore, if a certain group is substituted by 1, 2 or 3 $R^{1-1-1}$s, that is to say, the group may be substituted by up to 3 $R^{1-1-1}$s, and the definition of $R^{1-1-1}$ at this position is independent of the definition of $R^{1-1-1}$ at the remaining positions. Also, a combination of a substituents and/or a variable is permissible only if such combination results in a stable compound.

It should be understood that in the present application, the term "substituted" may indicate that one or more substituents are used for substitution, which may be determined according to the actual structure or environment.

The term "halogen" refers to fluorine, chlorine, bromine or iodine.

The term "alkyl" refers to a saturated linear or branched monovalent hydrocarbyl (such as a $C_1$-$C_6$ alkyl, such as a $C_1$-$C_4$ alkyl), usually having 3 to 12 carbon atoms. Examples of the alkyl include, but are not limited to, methyl, ethyl, 1-propyl, 2-propyl, 1-butyl, 2-methyl-1-butyl, 2-butyl, 2-methyl-2-propyl, 1-pentyl, 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, 1-heptyl and 1-octyl.

The term "cycloalkyl" refers to a saturated, non-aromatic cyclic hydrocarbyl (such as $C_3$-$C_6$ cycloalkyl), which usually has 3 to 20 carbon atoms, including monocyclic cycloalkyl and polycyclic cycloalkyl. Cycloalkyl contains 3 to 20, preferably 3 to 12, more preferably 3 to 6 carbon atoms.

Examples of monocyclic cycloalkyl include, but are not limited to, cyclopropyl, cyclobutyl, cyclopentyl, cyclohexyl, cycloheptyl, cyclooctyl, cyclononyl, cyclodecyl, cycloundecyl, and cyclododecyl.

Polycyclic cycloalkyl has a polycyclic (eg, bicyclic and tricyclic) cycloalkyl structure, including spiro-, fused- and bridged- cycloalkyls. Among them, "spirocycloalkyl" refers usually to a polycyclic group in which one carbon atom (referred to as the spiro atom) is shared between 5 to 20-membered monocyclic rings, it may contain one or more double bonds, but none of the rings has a fully conjugated $\pi$-electron system. Preferably the polycyclic cycloalkyl is 6- to 14-membered, more preferably 7- to 10-membered. According to the number of spiro atoms shared between the rings, the spirocycloalkyl may be divided into monospirocycloalkyl, bispirocycloalkyl or polyspirocycloalkyl, preferably the spirocycloalkyl is monospirocycloalkyl or bispirocycloalkyl. More preferably, the spirocycloalkyl is a 4-membered/4-membered, 4-membered/5-membered, 4-membered/6-membered, 5-membered/5-membered or 5-membered/6-membered monospirocycloalkyl. Examples of spirocycloalkyl include, but are not limited to,

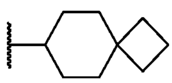, 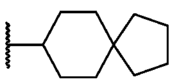,

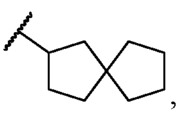, 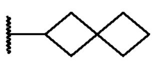, 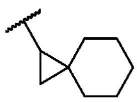.

"Fused cycloalkyl" means usually a 5- to 20-membered all-carbon polycyclic group, in which each ring in the system shares a pair of adjacent carbon atoms with other rings in the system, it may contain one or more double bonds, but none of the rings has a fully conjugated $\pi$-electron system. Preferably the fused cycloalkyl is 6- to 14-membered, more preferably 7- to 10-membered. According to the number of rings, the fused cycloalkyl may be divided into bicyclic, tricyclic, tetracyclic or polycyclic fused cycloalkyl, preferably the fused cycloalkyl is bicyclic or tricyclic, more preferably 5-membered/5-membered or 5-membered/6-membered bicycloalkyl. Examples of fused cycloalkyl include, but are not limited to,

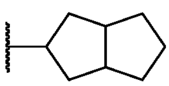, 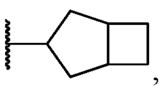, 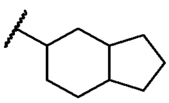, 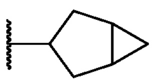, 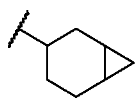,

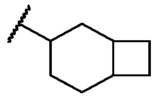, 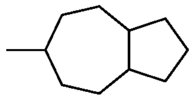.

"Bridged cycloalkyl" means usually a 5- to 20-membered all-carbon polycyclic group, in which any two rings share two carbon atoms not directly attached, it may contain one or more double bonds, but none of the rings has a fully conjugated π-electron system. Preferably the bridged cycloalkyl is 6- to 14-membered, more preferably 7- to 10-membered. According to the number of rings, the bridged cycloalkyl may be divided into bicyclic, tricyclic, tetracyclic or polycyclic bridged cycloalkyl, preferably the bridged cycloalkyl is bicyclic, tricyclic or tetracyclic, more preferably bicyclic or tricyclic. Examples of bridged cycloalkyl include, but are not limited to,

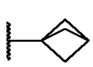, 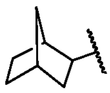, 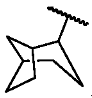, 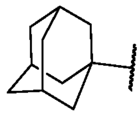.

The term "heterocycloalkyl" refers to a saturated ring group typically having 3 to 20 ring atoms, wherein at least one ring atom is a heteroatom independently selected from the group consisting of boron, silicon, oxygen, sulfur, selenium, nitrogen, and phosphorus, and the remaining ring atoms are C. The heterocycloalkyl may be a carbon group or a heteroatom group (i.e. it may be C-attached or N-attached, wherever it is possible). Examples of heterocycloalkyl include, but are not limited to, oxetanyl, pyrrolidinyl, tetrahydrofuranyl, tetrahydrothiophenyl, tetrahydropyranyl, tetrahydrothiopyranyl, piperidinyl, morpholinyl, 4-thiomorpholinyl, thioxanyl and piperazinyl. Fused ring moieties, spiro ring moieties and bridged ring moieties are also included within the scope of this definition. For example, a group derived from tetrahydropyrrole may be tetrahydropyrrol-1-yl (N-attached) or tetrahydropyrrol-3-yl (C-attached). For example, the heterocycloalkyl may be a 3- to 7-membered monocyclic ring (1 to 6 carbon atoms and 1 to 3 heteroatoms selected from the group consisting of N, O, P, B, Si, S and Se, wherein N, B, P or Se may be optionally substituted by one or more oxygen atoms to obtain a group such as NO, BOH, PO, $PO_2$, SeO; N may be optionally quaternized; S atom may be optionally substituted by one or more oxygen or nitrogen atoms to obtain a group such as SO, $SO_2$, $S(=O)(=NR^a)$, $S(=NR^b)$ or $S(=NR^c)_2$, and $R^a$, $R^b$ and $R^c$ are independently cyano, $C_1$-$C_7$ alkyl, $C_3$-$C_{14}$ cycloalkyl, "3- to 14-membered heterocycloalkyl in which heteroatom is one or more selected from the group consisting of boron, silicon, oxygen, sulfur, selenium, nitrogen, and phosphorus, and the number of heteroatoms is 1 to 4", "$C_1$-$C_7$ heteroaryl in which heteroatom is one or more selected from the group consisting of boron, silicon, oxygen, sulfur, selenium, nitrogen and phosphorus, and the number of heteroatoms is 1 to 4", $C_6$-$C_{10}$ aryl or $C_1$-$C_7$ alkoxy; meanwhile, $-CH_2-$ group may be optionally replaced by -C(=O)-, -C(=S)- or $-C(=NR^d)-$, $R^d$ is independently cyano, $C_1$-$C_7$ alkyl, $C_3$-$C_{14}$ cycloalkyl, "3- to 14-membered heterocycloalkyl in which heteroatom is one or more selected from the group consisting of boron, silicon, oxygen, sulfur, selenium, nitrogen and phosphorus, and the number of heteroatoms is 1 to 4", " $C_1$-$C_7$ heteroaryl in which heteroatom is one or more selected from the group consisting of boron, silicon, oxygen, sulfur, selenium, nitrogen and phosphorus, and the number of heteroatoms is 1 to 4", $C_6$-$C_{10}$ aryl or $C_1$-$C_7$ alkoxy; when the ring is a three-membered ring, there is only one heteroatom), or a bicyclic ring consisting of 7 to 10 atoms (4 to 9 carbon atoms and 1 to 3 heteroatoms selected from the group consisting of N, O, P, B, Si, and S, wherein N, S, B or P may be optionally substituted by one or more oxygen atoms to obtain a group such as NO, BOH, SO, $SO_2$, PO, $PO_2$, SeO, and the $-CH_2-$ group may optionally be replaced by -C(=O)-). Depending on the structure, the heterocyclyl may be a monovalent group or a divalent group, i.e., a sub-heterocyclyl.

The term "cycloalkenyl" refers to a monocyclic, partially unsaturated (containing 1 or 2 double bonds) non-aromatic cyclic hydrocarbyl, usually having 3 to 20 carbon atoms (e.g. $C_3$-$C_6$ cycloalkenyl).

The term "heterocycloalkenyl" refers to a monocyclic, partially unsaturated (e.g. containing 1 or 2 double bonds) non-aromatic cyclic hydrocarbyl, usually having 3 to 20 ring atoms, at least one of which is a heteroatom selected from the group consisting of boron, silicon, oxygen, sulfur, selenium, nitrogen and phosphorus, and the number of heteroatoms in the ring may be, for example, 1 to 4.

The term "saturated or unsaturated non-aromatic cyclic hydrocarbyl" refers to a monocyclic, saturated or unsaturated (for example containing 1 or 2 double bonds) and non-aromatic cyclic hydrocarbyl, usually having 3 to 20 carbon atoms (for example $C_3$-$C_{10}$ cyclohydrocarbyl), such as cycloalkyl, cycloalkenyl, such as cyclopentane, cyclopentene, cyclopentadiene, cyclohexane.

The term "saturated or unsaturated non-aromatic heterocyclyl" refers to a saturated or unsaturated (e.g., containing 1 or 2 double bonds) non-aromatic monocyclic or bicyclic ring, wherein at least one ring atom is a heteroatom selected from the group consisting of boron, silicon, oxygen, sulfur, selenium, nitrogen and phosphorus, and the number of heteroatoms in the ring may be, for example, 1 to 4, such as piperidinyl and piperazinyl.

The term "aryl" refers to any stable monocyclic or bicyclic carbocyclic ring with up to 14 atoms in each ring, and at least one ring is aromatic. Examples of the aforementioned aryl unit include phenyl, naphthyl, tetrahydronaphthyl, indanyl, biphenyl, phenanthrenyl, anthracenyl or acenaphthyl. It is understood that where the aryl substituent is a bicyclic substituent and one of the rings is non-aromatic, the attachment is through the aromatic ring.

The term "heteroaryl" refers to a stable monocyclic or bicyclic ring with up to 7 atoms in each ring, wherein at least one ring is aromatic and contains 1 to 4 heteroatoms selected from the group consisting of boron, silicon, oxygen, sulfur, selenium, nitrogen and phosphorus. Heteroaryl within the scope of this definition includes, but are not limited to, acridinyl, carbazolyl, cinnolinyl, quinoxalinyl, pyrazolyl, thiazolyl, indolyl, benzotriazolyl, furyl, thienyl, benzothienyl, benzofuryl, quinolinyl, isoquinolyl, oxazolyl, isoxazolyl, pyrazinyl, pyridazinyl, pyridyl, pyrimidinyl, pyrrolyl, tetrahydroquinolinyl. "Heteroaryl" should also be understood to include the N-oxide derivative of any nitrogen-containing heteroaryl. In cases where the heteroaryl substituent is a bicyclic substituent and one ring is non-aromatic or contains no heteroatom, it is understood that attachment is achieved through the aromatic ring. Heteroaromatic ring-fused-aromatic ring, bicyclic heteroaromatic ring systems may be formed in fused form. N, S, B, P or Se may be optionally substituted by one or more oxygen atoms to obtain a group such as NO, SO, $SO_2$, BOH, PO, $PO_2$, SeO, and N atom may be quaternized. The heteroaryl may be attached to the main structure at any heteroatom or carbon atom to form a stable compound. Depending on the structure, heteroaryl may be a monovalent group or a divalent group, i.e. heteroarylene.

The term "alkoxy" refers to an alkyl group attached through an oxygen bridge; said alkyl is defined as above.

The term "pharmaceutical auxiliary material" refers to excipient and additive used in the production of drugs and the preparation of prescriptions, and refers to all substances contained in a pharmaceutical preparation except for the active ingredient. Please refer to the Pharmacopoeia of the People's Republic of China (2015 Edition), Volume 4, or Handbook of Pharmaceutical Excipients (Raymond C Rowe, 2009 Sixth Edition).

The term "treatment" refers to therapeutic therapy. In relation to a specific disease or disorder, the treatment means: (1) amelioration of one or more biological manifestations of the disease or disorder, (2) interference with (a) one or more points in the biological cascade leading to or causing the disease or disorder or (b) one or more biological manifestations of the disease or disorder, (3) amelioration of one or more symptoms, affection or side effects associated with the disease or disorder, or one or more symptoms, affection or side effects associated with the disease or disorder or its treatment, or (4) slowing the development of the disease or disorder or one or more biological manifestations of the disease or disorder.

The term "prevention" refers to a reduction in the risk of acquiring or developing a disease or disorder.

The term "therapeutically effective amount" refers to an amount of a compound which, when administered to a patient, is sufficient to effectively treat a disease or disorder described herein. A "therapeutically effective amount" will vary depending on the compound, the disease or disorder and its severity, and the age of the patient to be treated, but can be adjusted as necessary by those skilled in the art.

The term "patient" refers to any animal that is about to or has received the administration of the compound or composition according to the embodiments of the present invention, preferably a mammal, and most preferably a human. The term "mammal" includes any mammal. Examples of mammals include, but are not limited to, cattle, horse, sheep, pig, cat, dog, mouse, rat, rabbit, guinea pig, monkey, human, etc., with humans being most preferred.

The term "active ingredient" refers to the active ingredient in the pharmaceutical composition or combination kit of the present invention, that is, the compound I (or the compound II), the pharmaceutically acceptable salt thereof, the solvate thereof, the solvate of the pharmaceutically acceptable salt, the metabolite thereof or the prodrug thereof, an anticancer drug, or the combination formed thereby.

On the basis of not violating common knowledge in the art, the preferred conditions may be combined arbitrarily to obtain preferred examples of the present invention.

The reagents and raw materials used in the present invention are all commercially available.

The positive and progressive effect of the present invention is that the compound of the present invention has inhibitory activity on RORyt, can effectively inhibit the RORyt protein receptor, thereby regulating the differentiation of Th17 cells and inhibiting the production of IL-17, and thus can treat the related autoimmune diseases mediated by RORγt.

## Embodiments

The present invention is further illustrated below by means of examples, but the present invention is not limited to the scope of the examples. The experimental methods that do not specify specific conditions in the following examples, may be selected according to conventional methods and conditions, or according to the product instructions.

The structures of all compounds of the present invention can be identified by nuclear magnetic resonance ($^1$HNMR) and/or mass spectrometry (MS).

$^1$H NMR chemical shifts ($\delta$) were reported in ppm ($10^{-6}$). NMR was performed on a Bruker AVANCE-400 spectrometer.

LC-MS was determined by Agilent 1200HPLC/6120 mass spectrometer.

HPLC was determined by Agilent 1260 high performance liquid chromatography. Specific conditions of HPLC: mobile phase A: water (0.1% formic acid), mobile phase B: acetonitrile; column time: 15min; column type: Xselect from Waters Company, 5um, 4.6×250mm.

The thin-layer silica gel plate is HSGF254 silica gel plate of LiangChen Silicon Material Co. Ltd. or Qingdao GF254 silica gel plate. Column chromatography generally uses 200-300 mesh silica gel of Yantai Huanghai as a carrier.

**Example 1**

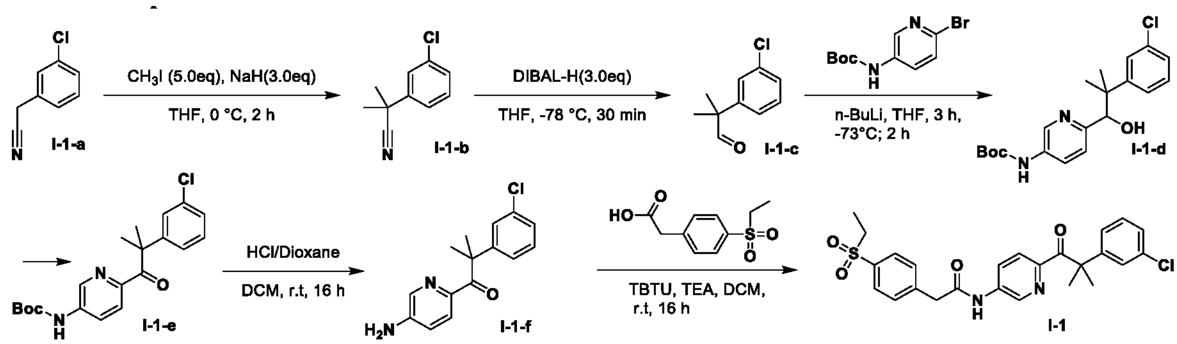


**Step 1:**

At 0°C, sodium hydride (3.96g, 99.0mmol) was dissolved in anhydrous tetrahydrofuran (THF, 100mL), added dropwise with a solution of 2-(3-chlorophenyl)acetonitrile (**I-1-a**) (5 g, 32.984 mmol) in anhydrous THF (60 mL), stirred for 1h, added with iodomethane (10.3 mL, 165 mmol) and stirred at 0 °C for another 2h. Thin layer chromatography (TLC, petroleum ether : ethyl acetate = 10:1) showed that the reaction was complete. The mixture was warmed to room temperature, quenched carefully with ice water, and extracted with ethyl acetate (100 mL×3). The combined extracts were washed with brine, dried over anhydrous sodium sulfate, filtered and concentrated to obtain 2-(3-chlorophenyl)-2-methyl-propionitrile (**I-1-b**) as a yellow oil (5.6 g, 31 mmol). $^1$H-NMR ($CDCl_3$): δ 7.47 (td, J = 2.0, 0.8 Hz, 1H), 7.44 - 7.34 (m, 2H), 7.34 - 7.32 (m, 1H), 1.74 (s, 6H).

**Step 2:**

At -78 °C, diisobutylaluminum hydride (DIBAL-H, 44 mL, 44 mmol, 1 mol/L) was slowly dropped into a solution of 2-(3-chlorophenyl)-2-methyl-propionitrile (**I-1-b**) (2.6 g, 14 mmol) in anhydrous THF (40 mL). After stirred at -78°C for 30min, the reaction mixture was warmed to room temperature and stirred for 16h. TLC (petroleum ether: ethyl acetate = 10:1) showed that the reaction was complete. The mixture was cooled in an ice bath, quenched slowly with 2N hydrochloric acid (HCl), and then extracted with ethyl acetate (100 mL×2). The combined extracts were dried over anhydrous sodium sulfate and concentrated to obtain 2-(3-chlorophenyl)-2-methyl-propanal (**I-1-c**) as a yellow oil (2.3g, 13mmol). $^1$H-NMR ($CDCl_3$): δ 9.51 (s, 1H), 7.37 - 7.29 (m, 3H), 7.18 (q, J = 1.6 Hz, 1H), 1.48 (s, 6H).

**Step 3:**

3-amino-6-bromopyridine (2 g, 11.560 mmol) and tert-butyl tert-butoxycarbonylcarbonate (2.78 g, 12.7 mmol) were dissolved in 10 mL of anhydrous THF. The reaction system was replaced with argon and cooled to 0 °C, and sodium [bis(trimethylsilyl)amide] (24 mL, 24 mmol, 1 mol/L) was added dropwise within 20min. After the addition, the temperature was raised to 30° C and the mixture was stirred for 3h. TLC (petroleum ether: ethyl acetate = 1:1) showed that the reaction was complete. The mixture was quenched with 50 mL of saturated $NH_4Cl$ and extracted with ethyl acetate (2 × 50 mL). The organic layer was washed with brine, dried over anhydrous sodium sulfate, filtered and concentrated, and the residue was purified by column chromatography (petroleum ether/ethyl acetate 0-50%) to obtain tert-butyl N-(6-bromo-3-pyridyl)carbamate (2.8 g, 10 mmol). $^1$H-NMR ($CDCl_3$): δ 8.26 (d, J = 2.9 Hz, 1H), 7.89 (d, J = 8.7 Hz, 1H), 7.42 (d, J = 8.7 Hz, 1H), 6.69 (s, 1H), 1.53 (s, 9H). LC-MS: m/z $(M+H)^+$= 275.0.

Tert-butyl N-(6-bromo-3-pyridyl)carbamate (2 g, 7.3225 mmol) was dissolved in anhydrous THF (10 mL), and the reaction system was replaced and protected by argon, and butyllithium (8.8 mL, 22 mmol, 2.5 mol/L) was injected therein through a syringe at -70°C. The mixture was stirred at -70°C for 2.5h. A solution of 2-(3-chlorophenyl)-2-methyl-propanal (**I-1-c**) (1.34 g, 7.34 mmol) in THF (10 mL) was added. After the addition, the mixture was stirred at -70 °C for

100min. TLC (petroleum ether: ethyl acetate = 5:1) showed disappearance of the starting material and formation of a new spot. The mixture was diluted with EtOAc (50 mL), washed with saturated $NH_4Cl$ (50 mL). The organic layer was washed with brine, dried over anhydrous sodium sulfate, filtered and concentrated, and the residue was purified by column chromatography (petroleum ether/ethyl acetate 0-50%) and concentrated to obtain tert-butyl N-[6-[2-[3-(3-chlorophenyl)-1-hydroxy-2-methyl-propyl]-3-pyridyl]carbamate (**I-1-d**) as a yellow solid (510 mg, 1.353 mmol). $^1$H-NMR ($CDCl_3$): δ 8.36 (d, J = 2.6 Hz, 1H), 7.70 (d, J = 8.5 Hz, 1H), 7.26 (d, J = 2.0 Hz, 1H), 7.23 (d, J = 1.9 Hz, 1H), 7.17 (dt, J = 6.9, 2.1 Hz, 1H), 6.50 (s, 1H), 6.40 (d, J = 8.6 Hz, 1H), 4.76 (d, J = 6.0 Hz, 1H), 4.45 (d, J = 6.7 Hz, 1H), 1.54 (s, 9H), 1.44 (s, 3H), 1.19 (s, 3H). LC-MS: m/z $(M+H)^+$= 377.1.

**Step 4:**

Tert-butyl N-[6-[2-[3-(3-chlorophenyl)-1-hydroxy-2-methyl-propyl]-3-pyridyl]carbamate (**I-1-d**) (260 mg, 0.6898 mmol) was dissolved in dichloromethane (DCM, 6 mL), added with pyridinium chlorochromate (PCC, 297 mg, 1.3778 mmol) and silica gel powder (500 mg), and stirred at 32°C for 3h. TLC (petroleum ether: ethyl acetate = 5:1) showed that the reaction was complete. The mixture was filtered. The filtrate was concentrated and purified by column chromatography (petroleum ether/ethyl acetate 0-50%), concentrated to obtain tert-butyl N-[6-[2-(3-chlorophenyl)-2-methyl-propionyl]-3-pyridyl]carbamate (**I-1-e**) as a white solid (120mg, 0.3201mmol). $^1$H-NMR ($CDCl_3$): δ 8.19 (d, J = 2.5 Hz, 1H), 8.03 - 7.92 (m, 2H), 7.31 (d, J = 2.1 Hz, 1H), 7.20 - 7.05 (m, 3H), 6.60 (s, 1H), 1.72 (s, 6H), 1.52 (s, 9H). LC-MS: m/z $(M+H)^+$= 375.1.

**Step 5:**

Tert-butyl N-[6-[2-[3-(3-chlorophenyl)-2-methyl-propionyl]-3-pyridyl]carbamate (**I-1-e**) (120 mg, 0.3201 mmol) was dissolved in DCM (3 mL), added with HCl/dioxane (4M, 1 mL), and stirred at 33 °C for 1h. TLC (diethyl ether: ethyl acetate = 5:1) showed the reaction was complete. The mixture was cooled in ice water, quenched with saturated sodium bicarbonate (NaHCOs), and extracted with DCM (20 mL×2). The organic layer was dried over anhydrous sodium sulfate, filtered and concentrated to obtain 1-(5-amino-2-pyridyl)-2-(3-chlorophenyl)-2-methyl-propan-1-one (**I-1-f**) as a pale yellow oil (80mg, 0.2912mmol). $^1$H-NMR ($CDCl_3$): δ 7.89 (d, J = 8.5 Hz, 1H), 7.75 (d, J = 2.8 Hz, 1H), 7.31 (t, J = 1.8 Hz, 1H), 7.18 - 7.06 (m, 3H), 6.89 (dd, J = 8.6, 2.8 Hz, 1H), 1.71 (s, 6H). LC-MS: m/z $(M+H)^+$= 275.1.

**Step 6:**

1-(5-amino-2-pyridyl)-2-(3-chlorophenyl)-2-methyl-propan-1-one (**I-1-f**) (60 mg, 0.2184 mmol) was dissolved in DCM (3mL), added wtih 2-(4-ethylsulfonylphenyl)acetic acid (57 mg, 0.24972 mmol), TBTU (80 mg, 0.2491 mmol) and triethylamine (66 mg, 0.65224 mmol). Under argon protection, the mixture was stirred at 33°C for 16h. TLC (petroleum ether: ethyl acetate = 3:1) showed that the reaction was complete. The reaction solution was concentrated, and the residue was purified by column chromatography (petroleum ether/ethyl acetate 0-50%) and concentrated to obtain N-[6-[2-(3-chlorophenyl)-2-methylpropionyl]-3-pyridyl]-2-(4-ethylsulfonylphenyl)acetamide (**I-1**) as a white solid (32mg, 0.06398mmol). $^1$H-NMR ($CDCl_3$): δ 8.35 (d, J = 2.5 Hz, 1H), 8.17 - 8.09 (m, 2H), 7.97 (d, J = 8.6 Hz, 1H), 7.83 - 7.77 (m, 2H), 7.49 - 7.41 (m, 2H), 7.30 (d, J = 2.1 Hz, 1H), 7.18 - 7.02 (m, 3H), 3.80 (s, 2H), 3.12 (q, J = 7.4 Hz, 2H), 1.71 (s, 6H), 1.28 (t, J = 7.4 Hz, 3H). LC-MS: m/z $(M+H)^+$= 485.1.

**Example 2**

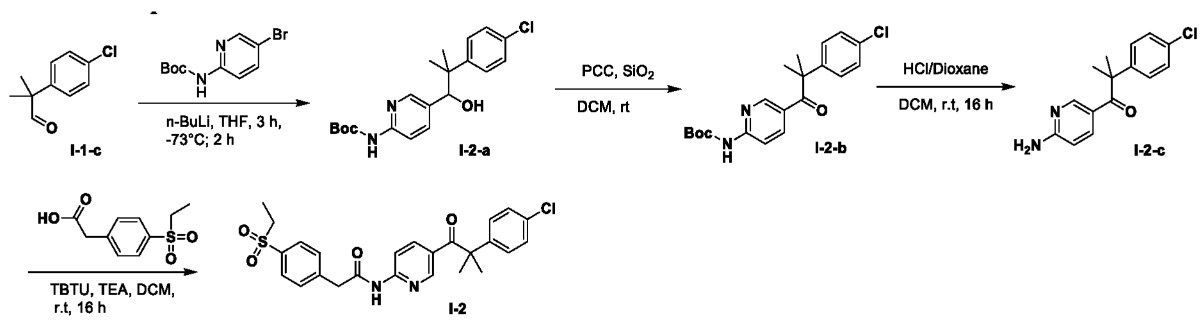


I-1-c I-2-a I-2-b I-2-c I-2

**Step 1:**

Tert-butyl N-(5-bromo-2-pyridyl)carbamate (0.5 g, 2 mmol) was dissolved in anhydrous THF (10 mL), added dropwise with butyllithium (1.8 mL, 4.5 mmol, 2.5 mol/L) at -70 °C under argon protection, and stirred at -70 °C for 2.5h. Then a solution of 2-(4-chlorophenyl)-2-methyl-propanal (**I-1-c**) (0.5 g, 3 mmol) in THF (10 mL) was added and stirred at -70 °C for 100min. TLC (petroleum ether: ethyl acetate = 5:1) showed the disappearance of the starting material and formation of a new spot. LCMS showed the product was formed. The mixture was quenched with saturated aqueous ammonium chloride (50 mL), and diluted with ethyl acetate (50 mL). The organic layer was washed with brine, dried over anhydrous sodium sulfate, filtered and concentrated, and the residue was purified by column chromatography (petroleum ether/ethyl acetate 0-50%) to obtain tert-butyl N-[5-[2-(4 -chlorophenyl)-1-hydroxy-2-methyl-propyl]-2-pyridyl]carbamate (**I-2-a**) as a white solid (230 mg, 0.6102 mmol). $^1$H-NMR ($CDCl_3$): 7.93 (s, 1H), 7.83 (d, J = 8.6 Hz, 1H), 7.42 (s, 1H), 7.36 (d, J = 9.0 Hz, 1H), 7.34 - 7.29 (m, 4H), 7.28 (s, 1H), 1.55 (d, J = 1.3 Hz, 9H), 1.32 (d, J = 5.5 Hz, 6H). LC-MS: m/z $(M+H)^+$= 377.1.

**Step 2:**

Tert-butyl N-[5-[2-[4-chlorophenyl)-1-hydroxy-2-methyl-propyl]-2-pyridyl]carbamate (I-2-a) (230 mg, 0.6102 mmol) was dissolved in DCM (4 mL), PCC (260 mg, 1.2062 mmol) and silica gel (150 mg) were added, and the mixture was stirred at 21 °C for 3h. TLC (petroleum ether: ethyl acetate = 5:1) showed that the reaction was complete. LCMS showed that the product was formed. The reaction product was filtered, the filtrate was concentrated and purified by column chromatography (petroleum ether/ethyl acetate 0-50%) to obtain tert-butyl N-[5-[2-[4-(4-chlorophenyl)-2-methyl-propionyl]-2-pyridyl]carbamate (**I-2-b**) as a white solid (120 mg, 0.3201 mmol). $^1$H-NMR ($CDCl_3$): δ 8.45 (dd, J = 2.3, 0.9 Hz, 1H), 7.82 - 7.75 (m, 3H), 7.37 - 7.31 (m, 2H), 7.26 - 7.20 (m, 2H), 1.61 (s, 6H), 1.53 (s, 9H).. LC-MS: m/z $(M+H)^+$= 375.1.

**Step 3:**

Tert-Butyl N-[5-[2-(4-chlorophenyl)-2-methyl-propionyl]-2-pyridyl]carbamate (**I-2-b**) (120 mg, 0.3201 mmol) was dissolved in dichloromethane (3 mL), added with 4M hydrochloric acid/dioxane (2 mL), and stirred at 20°C for 1 hr. TLC (petroleum ether: ethyl acetate = 5:1) showed that the reaction was complete. LCMS showed that the product was formed. The mixture was cooled in ice water, quenched with saturated aqueous $NaHCO_3$, extracted with dichloromethane (20 mL×2). The organic layer was dried over anhydrous sodium sulfate, filtered and concentrated to obtain 1-(6-amino-3-pyridyl)-2-(4-chlorophenyl)-2-methyl-propan-1-one (**I-2-c**) as a yellow oil (80 mg, 0.2912 mmol), which was used directly in the next step. LC-MS: m/z $(M+H)^+$ =275.1.

**Step 4:**

1-(6-amino-3-pyridyl)-2-(4-chlorophenyl)-2-methyl-propan-1-one (**I-2-c**) (80 mg, 0.2912 mmol) was dissolved in dichloromethane (3 mL), and 2-(4-ethylsulfonylphenyl)acetic acid (76 mg, 0.33295 mmol), TBTU (107 mg, 0.332 mmol) and triethylamine (88 mg, 0.86965 mmol) were added. The system was replaced with argon, and then stirred at 22 °C for 16h under the protection of argon. TLC (petroleum ether: ethyl acetate = 1:1) showed that the reaction was complete. LCMS showed that the product was formed. The mixture was concentrated and the residue was purified by column chromatography (petroleum ether/ethyl acetate 0-50%), concentrated to obtain N-[5-[2-(4-chlorophenyl)-2-methyl-propionyl]-2-pyridyl]-2-(4-ethylsulfonylphenyl)acet amide (**I-2**) as a white solid (30 mg, 0.06186 mmol). $^1$H-NMR ($CDCl_3$): δ 8.46 - 8.34 (m, 2H), 8.07 (d, J = 8.9 Hz, 1H), 7.94 - 7.87 (m, 2H), 7.83 (dd, J = 8.9, 2.4 Hz, 1H), 7.56 - 7.48 (m, 2H), 7.37 - 7.31 (m, 2H), 7.25 - 7.17 (m, 2H), 3.83 (s, 2H), 3.13 (q, J = 7.4 Hz, 2H), 1.60 (s, 6H), 1.30 (t, J = 7.4 Hz, 3H). LC-MS: m/z $(M+H)^+$= 485.1.

**Example 3**

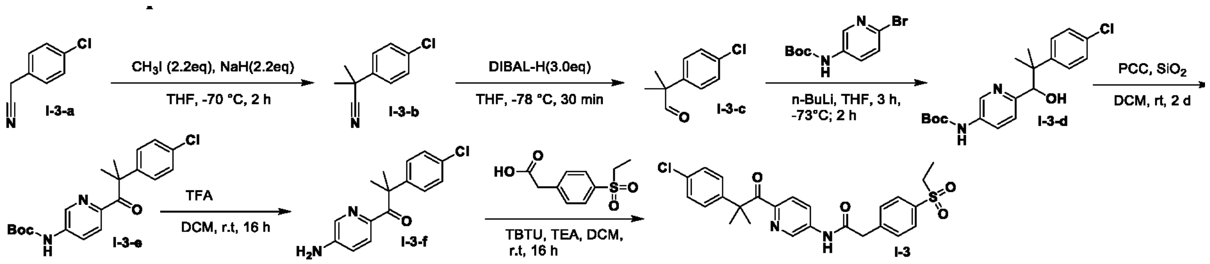


**Step 1:**

Sodium hydride (0.8g, 20 mmol) was added into anhydrous THF (20mL), and a solution of 2-(4-chlorophenyl)acetonitrile (**I-3-a**) (1g, 6.5967 mmol) in anhydrous THF (20 mL) was added dropwise at 0 °C. After stirred for 1h, the reaction mixture was added with iodomethane (2 mL, 32.1 mmol) and stirred at 0 °C for another 2h. TLC (petroleum ether: ethyl acetate = 10:1) showed that the reaction was complete. The reaction flask was raised to room temperature, quenched carefully with ice water, and then extracted with ethyl acetate (50 mL×3). The combined organic phases were washed with brine, dried over anhydrous sodium sulfate, filtered and concentrated to obtain 2-(4-chlorophenyl)-2-methylpropionitrile (**I-3-b**) as a pale yellow oil (1.1g, 6.1mmol). $^1$H-NMR ($CDCl_3$): δ 7.43 (d, J = 8.7 Hz, 2H), 7.38 (d, J = 8.8 Hz, 2H), 1.73 (s, 6H).

**Step 2:**

2-(4-chlorophenyl)-2-methylpropionitrile (**I-3-b**) (1.1 g, 6.1 mmol) was dissolved in anhydrous THF (20 mL), added wtih DIBAL-H (16 mL, 16 mmol) dropwise at -78 °C, stirred at -78 °C for 1h and then at room temperature for 16h. TLC (petroleum ether: ethyl acetate = 10:1) showed that the reaction was complete. The mixture was cooled in an ice bath, quenched slowly with 2N HCl, and then extracted with ethyl acetate (50 mL×2). The combined organic phases were dried over anhydrous sodium sulfate and concentrated to obtain 2-(4-chlorophenyl)-2-methyl-propanal (**I-3-c**) as a yellow oil (1g, 5.4750mmol). $^1$H-NMR ($CDCl_3$): δ 9.49 (s, 1H), 7.39 - 7.34 (m, 2H), 7.25 - 7.20 (m, 2H), 1.47 (s, 6H).

**Step 3:**

Tert-butyl N-(6-bromo-3-pyridyl)carbamate (0.5g, 2mmol) was dissolved in anhydrous THF (10mL), added dropwise with butyllithium (1.8 mL, 4.5 mmol, 2.5 mol/L) at -70 °C under argon protection, and stirred at -70 °C for 2.5h. Then a solution of 2-(4-chlorophenyl)-2-methyl-propanal (**I-3-c**) (0.5 g, 3 mmol) in THF (10 mL) was added and the mixture was stirred at -70° C for 100min. TLC (petroleum ether: ethyl acetate = 5:1) showed the disappearance of the starting material and formation of a new spot. LCMS showed that the product was formed. The mixture was diluted with ethyl acetate (50 mL) and quenched with saturated aqueous ammonium chloride (50 mL). The organic layer was washed with brine, dried over anhydrous sodium sulfate, filtered and concentrated, and the residue was purified by column chromatography (petroleum ether/ethyl acetate 0-40%) to obtain tert-butyl N-[6-[2-[4-(4-chlorophenyl)-1-hydroxy-2-methyl-propyl]-3-pyridyl]carbamate (**I-3-d**) as a pale yellow solid (170 mg, 0.4510 mmol). $^1$H-NMR ($CDCl_3$): δ 8.34 (d, J = 2.6 Hz, 1H), 7.70 (d, J = 8.3 Hz, 1H), 7.34 (d, J = 1.3 Hz, 1H), 7.27 (d, J = 8.0 Hz, 2H), 7.21 - 7.19 (m, 2H), 6.52 (s, 1H), 6.39 (d, J = 8.6 Hz, 1H), 1.54 (s, 9H), 1.44 (s, 3H), 1.19 (s, 3H). LC-MS: m/z $(M+H)^+$= 377.1.

**Step 4:**

Tert-butyl N-[6-[2-[4-(4-chlorophenyl)-1-hydroxy-2-methyl-propyl]-3-pyridyl]carbamate (**I-3-d**) (170 mg, 0.4510 mmol) was dissolved in DCM (4 mL), PCC (200 mg, 0.92782 mmol) and silica gel (150 mg) were added, and the mixture was stirred at 21 °C for 3h. TLC (petroleum ether: ethyl acetate = 5:1) showed that the reaction was complete. LCMS showed that the product was formed. The reaction mixture was filtered, and the filtrate was concentrated and purified by column chromatography (petroleum ether/ethyl acetate 0-50%) to obtain tert-butyl N-[6-[2-(4-chlorophenyl)-2-methylpropionyl]-3-pyridyl]carbamate (**I-3-e**) as a white solid (55mg, 0.1467mmol). $^1$H-NMR ($CDCl_3$): δ 8.19 (d, J = 2.5 Hz, 1H), 8.01 - 7.91 (m, 2H), 7.47 - 7.42 (m, 2H), 7.36 - 7.30 (m, 2H), 7.24 - 7.16 (m, 4H), 6.59 (s, 1H), 1.72 (s, 6H), 1.52 (s, 9H). LC-MS: m/z $(M+H)^+$= 375.1.

**Step 5:**

Tert-Butyl N-[6-[2-[4-(4-chlorophenyl)-2-methyl-propionyl]-3-pyridyl]carbamate (**I-3-e**) (55 mg, 0.1467 mmol)

was dissolved in dichloromethane (3 mL), added with trifluoroacetic acid (1 mL), and stirred at 22 °C for 1h. TLC (petroleum ether: ethyl acetate = 5:1) showed that the reaction was complete. LCMS showed that the product was formed. The mixture was cooled in ice water, quenched with saturated aqueous $NaHCO_3$, extracted with dichloromethane (20 mL×2). The organic layer was dried over anhydrous sodium sulfate, filtered and concentrated to obtain 1-(5-amino-2-pyridyl)-2-(4-chlorophenyl)-2-methyl-propane-1-one (**I-3-f**) as a pale yellow oil (40 mg, 0.1456 mmol), which was used directly in the next step. LC-MS: m/z $(M+H)^+$= 275.1.

**Step 6:**

1-(5-amino-2-pyridyl)-2-(4-chlorophenyl)-2-methyl-propan-1-one (**I-3-f**) (40 mg, 0.1456 mmol) was dissolved in dichloromethane (3 mL), 2-(4-ethylsulfonylphenyl)acetic acid (38 mg, 0.16648 mmol), TBTU (54 mg, 0.1682 mmol) and triethylamine (44 mg, 0.43483 mmol) were added. The system was replaced with argon, and then the mixture was stirred at 22 °C for 16h under the protection of argon. TLC (petroleum ether: ethyl acetate = 3:1) showed that the reaction was complete. LCMS showed that the product was formed. The mixture was concentrated, the residue was purified by column chromatography (petroleum ether/ethyl acetate 0-50%), and concentrated to obtain N-[6-[2-(4-chlorophenyl)-2-methylpropionyl]-3-pyridyl]-2-(4-ethylsulfonylphenyl)aceta mide (**I-3**) as a white solid (10mg, 0.02062mmol). $^1$H-NMR ($CDCl_3$): δ 8.31 (d, J = 2.5 Hz, 1H), 8.13 (dd, J = 8.7, 2.6 Hz, 1H), 7.97 (d, J = 8.7 Hz, 1H), 7.90 - 7.76 (m, 3H), 7.52 - 7.46 (m, 2H), 7.23 - 7.14 (m, 4H), 3.82 (s, 2H), 3.13 (q, J = 7.4 Hz, 2H), 1.71 (s, 6H), 1.30 (d, J = 7.4 Hz, 3H). LC-MS: m/z $(M+H)^+$= 485.1.

**Example 4**

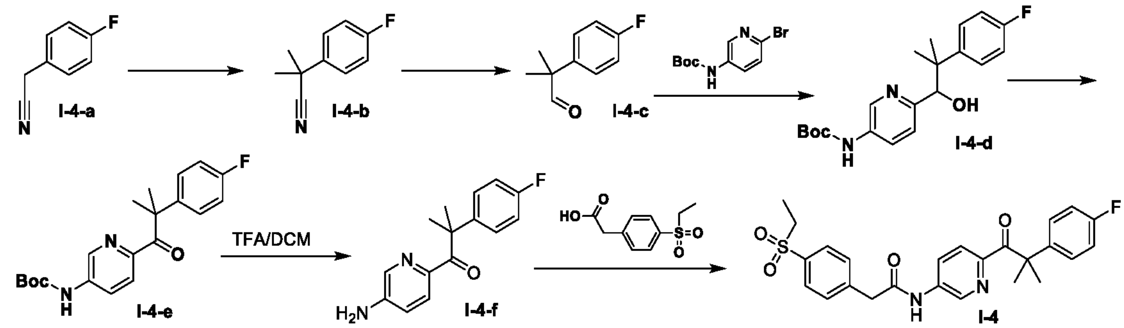


**Step 1:**

2-(4-fluorophenyl)acetonitrile (**I-4-a**) (4.2g, 31mmol) and iodomethane (13.2g, 93mmol) were added to 150ml of tetrahydrofuran, slowly added with sodium tert-butoxide (10.5g, 93.8mmol) under an ice-bath, and stirred overnight at room temperature. The reaction solution was quenched with saturated brine, extracted once with ethyl acetate, and the organic phase was washed twice with saturated brine, dried over anhydrous sodium sulfate, and concentrated to obtain 4.5 g of a product (**I-4-b**) as a colorless oil with a yield of 89%, which was used directly in the next step. $^1$H NMR (400 MHz, $CDCl_3$) δ 7.51 - 7.43 (m, 2H), 7.14 - 7.06 (m, 2H), 1.74 (s, 6H).

**Step 2:**

2-(4-fluorophenyl)-2-methylpropionitrile (**I-4-b**) (0.96g, 5.9mmol) was added into 20ml of tetrahydrofuran, slowly added with diisobutylaluminum hydride (18ml, 18mmol) at -78 °C, stirred at this temperature for 2h, slowly warmed to room temperature and stirred overnight. The reaction solution was quenched with 2 mol/L hydrochloric acid, extracted twice with ethyl acetate (2×30ml), dried over anhydrous sodium sulfate, concentrated and passed through a column (ethyl acetate: petroleum ether=0-10%) to obtain 0.75g of a colorless oil (**I-4-c**) with a yield of 77%. $^1$H NMR (400 MHz, $CDCl_3$): δ 9.49 (s, 1H), 7.27 (m, 2H), 7.12 - 7.06 (m, 2H), 1.48 (s, 6H).

**Step 3:**

Tert-butyl (6-bromopyridin-3-yl)carbamate (1.1g, 4mmol) was added to 30ml of anhydrous tetrahydrofuran, slowly added with n-butyllithium (5ml, 8mmol) at -78 °C, stirred at this temperature for 1h, slowly added with a solution

of 2-(4-fluorophenyl)-2-methylpropanal (**I-4-c**) (1.1 g, 4mmol) in 10ml of tetrahydrofuran, stirred at -78 °C for 1h, slowly warmed to room temperature and stirred overnight. The reaction solution was quenched with 10ml of water and extracted with ethyl acetate, and the organic phase was washed once with saturated brine, dried over anhydrous sodium sulfate, concentrated and passed through a column (ethyl acetate: petroleum ether=0-50%) to obtain 0.44g of a white solid (**I-4-d**) with a yield of 30%. LC-MS: m/z: $(M+H)^+$ =361, $^1H$ NMR (400 MHz, $CDCl_3$) δ 8.34 (d, *J* = 2.5 Hz, 1H), 7.68 (d, *J* = 7.7 Hz, 1H), 7.22 (dd, *J* = 8.9, 5.4 Hz, 2H), 6.99 (t, *J* = 8.7 Hz, 2H), 6.54 (s, 1H), 6.34 (d, *J* = 8.6 Hz, 1H), 4.74 (s, 1H), 4.44 (s, 1H), 1.53 (s, 9H), 1.45 (s, 3H), 1.20 (s, 3H).

**Step 4:**

Tert-butyl (6-(2-(4-(fluorophenyl)-1-hydroxy-2-methylpropyl)pyridin-3-yl)carbamate (**I-4-d**) (0.63g, 1.75mmol) and silica gel (4g) were added in 30ml of methylene dichloride, added with pyridinium chlorochromate (752mg, 3.5mmol), and stirred overnight at room temperature. The reaction solution was filtered, and the filtrate was contrated and passed through a column (ethyl acetate: petroleum ether = 0-30%) to obtain 0.24g of a white solid (**I-4-e**) with a yield of 30%. LC-MS: m/z: $(M+H)^+$ =359, $^1H$ NMR (400 MHz, $CDCl_3$) δ 8.20 (d, *J* = 2.3 Hz, 1H), 7.99 - 7.90 (m, 2H), 7.23 (dd, *J* = 8.8, 5.3 Hz, 2H), 6.93 (t, *J*= 8.8 Hz, 2H), 6.62 (s, 1H), 1.73 (s, 6H), 1.52 (s, 9H).

**Step 5:**

Tert-butyl (6-(2-(4-(fluorophenyl)-2-methylpropanoyl)pyridin-3-yl)carbamate **(I-4-e)** (0.25 g, 0.7 mmol) was added into 3ml of dichloromethane, added with 3ml of trifluoroacetic acid, and stirred at room temperature for 1h. The reaction solution was concentrated, added with 5ml of saturated sodium bicarbonate and extracted with dichloromethane (2×15ml), and the organic phases were combined, dried over anhydrous sodium sulfurate, concentrated and passed through a column (ethyl acetate: petroleum ether = 0-40%) to obtain 0.15 g of a white solid (**I-4-f**) with a yield of 83%. LC-MS: m/z: $(M+H)^+$ =259.

**Step 6:**

1-(5-aminopyridin-2-yl)-2-(4-fluorophenyl)-2-methylpropan-1-one (**I-4-f**) (0.15g, 0.58mmol), 2-(4-(ethylsulfonyl)phenyl)acetic acid (135mg, 0.59mmol) and triethylamine (0.28ml, 2mmol) were added to 20ml of dichloromethane, added with TBTU (210mg, 0.64mmol), and stirred at room temperature overnight. The reaction solution was concentrated and passed through a column (ethyl acetate: petroleum ether = 0-50%) to obtain 0.17 g of a white solid (**I-4**) with a yield of 62%. LC-MS: m/z: $(M+H)^+$ =469, $^1H$ NMR (400 MHz, $CDCl_3$) δ 8.37 (d, *J* = 2.4 Hz, 1H), 8.19 (s, 1H), 8.10 (dd, *J*= 8.7, 2.5 Hz, 1H), 7.88 (d, *J*= 8.7 Hz, 1H), 7.75 (d, *J*= 8.3 Hz, 2H), 7.43 (d, *J* = 8.3 Hz, 2H), 7.23 - 7.15 (m, 2H), 6.95 - 6.86 (m, 2H), 3.78 (s, 2H), 3.10 (q, *J* = 7.4 Hz, 2H), 1.70 (s, 6H), 1.26 (t, *J*= 7.4 Hz, 3H).

**Example 5**

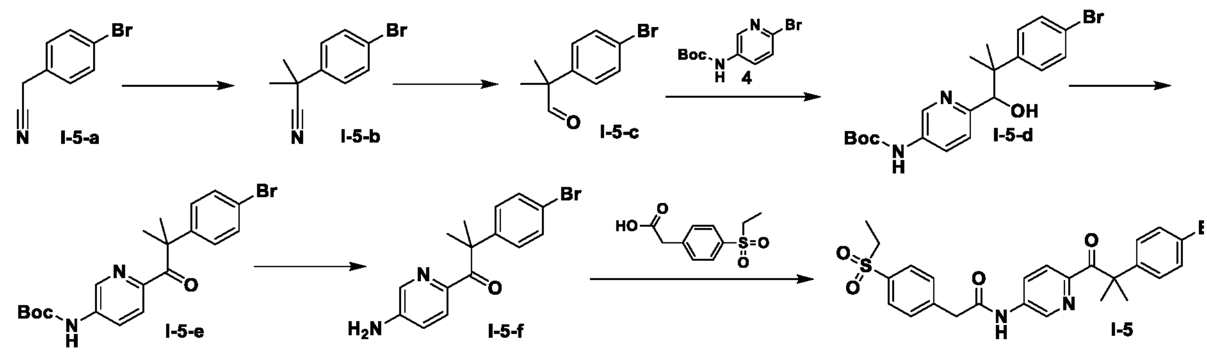
I-5-a I-5-b I-5-c 4 I-5-d I-5-e I-5-f I-5

**Step 1:**

2-(4-bromophenyl)acetonitrile (**I-5-a**) (4g, 20.4mmol) and iodomethane (8.6g, 61mmol) were added to 150ml of tetrahydrofuran, slowly added with sodium tert-butoxide (6.86g, 61.3mmol) in an ice bath, and stirred overnight at room temperature. The reaction solution was quenched with saturated brine and extracted once with ethyl acetate, and

the organic phase was washed twice with saturated brine, dried over anhydrous sodium sulfate and concentrated to obtain 4 g of a colorless oil (**I-5-b**) with a yield of 87%, which was used directly in the next step. $^1$H NMR (400 MHz, $CDCl_3$) δ 7.60 - 7.50 (m, 2H), 7.42 - 7.34 (m, 2H), 1.73 (s, 6H).

**Step 2:**

2-(4-bromophenyl)-2-methylpropionitrile (**I-5-b**) (1.8g, 8mmol) was added to 40ml of tetrahydrofuran, added slowly with diisobutylaluminum hydride (24ml, 24mmol) at -78 °C, stirred at this temperature for 2h and then at room temperature overnight. The reaction solution was quenched with 2mol/L hydrochloric acid, extracted twice with ethyl acetate (2×20ml), dried over anhydrous sodium sulfate, concentrated and passed through a column (ethyl acetate: petroleum ether = 0-10%) to obtain 1.6g of a colorless oil (**I-5-c**) with a yield of 88%. $^1$H NMR (400 MHz, $CDCl_3$) δ 9.50 (s, 1H), 7.57 - 7.48 (m, 2H), 7.21 - 7.11 (m, 2H), 1.47 (s, 6H).

**Step 3:**

Tert-butyl (6-bromopyridin-3-yl)carbamate (1.1g, 4mmol) was added to 30ml of anhydrous tetrahydrofuran, slowly added with n-butyllithium (5.8ml, 9.3mmol) at -78 °C, stirred at this temperature for 1 h, slowly added with a solution of 2-(4-bromophenyl)-2-methylpropanal (**I-5-c**) (0.92 g, 4.1 mmol) in anhydrous tetrahydrofuran (5 ml), stirred at -78 °C for 1 h, and slowly warmed to room temperature and stirred overnight. The reaction solution was quenched with 10ml of water and extracted with ethyl acetate, and the organic phase was washed once with saturated brine, dried over anhydrous sodium sulfate, concentrated and passed through a column (ethyl acetate: petroleum ether = 0-50%) to obtain 0.25g of a white solid (**I-5-d**) with a yield of 15%. LC-MS: m/z: $(M+H)^+$ = 421, $^1$H NMR (400 MHz, $CDCl_3$) δ 8.34 (d, *J* = 2.5 Hz, 1H), 7.70 (d, *J* = 7.8 Hz, 1H), 7.47 - 7.38 (m, 2H), 7.22 - 7.09 (m, 2H), 6.53 (s, 1H), 6.40 (d, *J*= 8.6 Hz, 1H), 4.74 (d, *J*= 6.6 Hz, 1H), 4.45 (d, *J*= 6.7 Hz, 1H), 1.54 (s, 9H), 1.43 (s, 3H), 1.18 (d, *J* = 5.8 Hz, 3H).

**Step 4:**

Tert-butyl (6-(2-(4-bromophenyl)-1-hydroxy-2-methylpropyl)pyridin-3-yl)carbamate (**I-5-d**) (0.3g, 0.83mmol) and manganese dioxide (2g, 23mmol) were added to 40ml of dichloromethane and the mixture was stirred overnight at reflux temperature. The reaction solution was filtered and concentrated to obtain 0.22 g of a white solid (**I-5-e**), which was directly used in the next step. The yield is 30%. LC-MS: m/z: $(M+H)^+$ =419, $^1$H NMR (400 MHz, $CDCl_3$) δ 8.19 (d, *J* = 2.5 Hz, 1H), 7.95 (dt, *J* = 8.7, 5.5 Hz, 2H), 7.39 - 7.33 (m, 2H), 7.18 - 7.10 (m, 2H), 6.60 (s, 1H), 1.72 (s, 6H), 1.52 (s, 9H).

**Step 5:**

Tert-butyl (6-(2-(4-bromophenyl)-2-methylpropanoyl)pyridin-3-yl)carbamate **(I-5-e)** (0.22g, 0.52mmol) was added into 3ml of dichloromethane, added wtih 3ml of trifluoroacetic acid, and stirred at room temperature for 1h. The reaction solution was concentrated, added with 5ml of saturated sodium bicarbonate, extracted with dichloromethane (2×20ml), dried over anhydrous sodium sulfate, concentrated and passed through a column (ethyl acetate: petroleum ether = 0-40%) to obtain 0.15 g of a white solid (**I-5-f**) with a yield of 89%. LC-MS: m/z: $(M+H)^+$ =259.

**Step 6:**

1-(5-aminopyridin-2-yl)-2-(4-bromophenyl)-2-methylpropan-1-one (**I-5-f**) (0.15 g, 0.47 mmol), 2-(4-(ethylsulfonyl)phenyl)acetic acid (110 mg, 0.48 mmol) and triethylamine (0.25 ml, 1.8 mmol) were added to 20 ml of dichloromethane, added wtih TBTU (170 mg, 0.53 mmol), and stirred overnight at room temperature. The reaction solution was concentrated and passed through a column (ethyl acetate: petroleum ether = 0-50%) to obtain 0.11 g of a white solid (**I-5**) with a yield of 44%. LC-MS: m/z: $(M+H)^+$ =529, $^1$H NMR (400 MHz, $CDCl_3$) δ 8.36 (d, *J*= 2.1 Hz, 1H), 8.13 (dd, *J*= 8.6, 2.2 Hz, 1H), 8.02 (s, 1H), 7.92 (d, *J* = 8.7 Hz, 1H), 7.79 (d, *J* = 8.2 Hz, 2H), 7.45 (d, *J* = 8.2 Hz, 2H), 7.34 (d, *J* = 8.5 Hz, 2H), 7.10 (d, *J* = 8.5 Hz, 2H), 3.80 (s, 2H), 3.10 (q, *J* = 7.4 Hz, 2H), 1.69 (s, 6H), 1.26 (t, *J*= 7.4 Hz, 3H).

**Example 6**

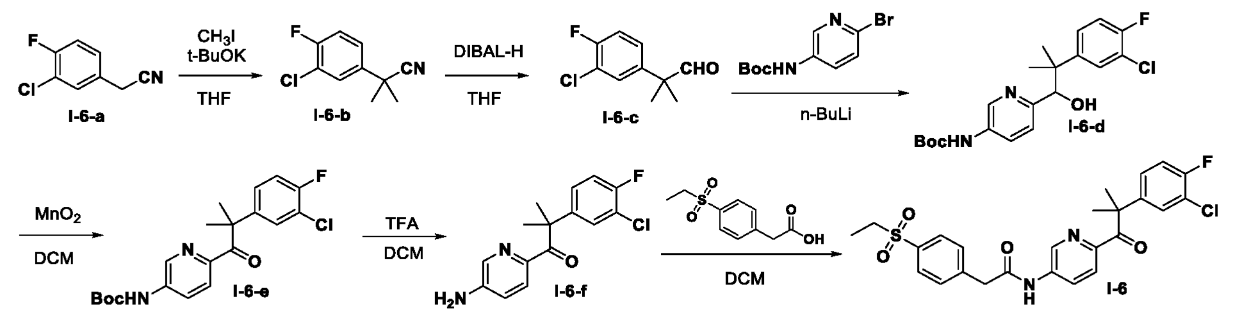


I-6-a I-6-b I-6-c I-6-d I-6-e I-6-f I-6

**Step 1:**

2-(3-chloro-4-fluorophenyl)acetonitrile (**I-6-a**) (1.0 g, 5.89 mmol) and iodomethane (1.1 mL, 17.7 mmol) were placed in anhydrous tetrahydrofuran (10 mL), cooled to 0°C under nitrogen protection, added with potassium tert-butoxide (2.0 g, 17.7 mmol), slowly raised to room temperature and stirred overnight. After the reaction was complete, water (10 mL) and ethyl acetate (10 mL) were added, the organic phase was separated, the aqueous phase was extracted with ethyl acetate (10 mL×2), the organic phases were combined, washed with saturated sodium chloride solution (10 mL×2) and water (10 mL), dried over anhydrous sodium sulfate, concentrated and purified by column chromatography (petroleum ether: ethyl acetate = 10:1) to obtain 2-(3-chloro-4-fluorophenyl)-2-methylpropionitrile (**I-6-b**) as a yellow oil (1.1 g). LC-MS: m/z: $(M+H)^+$= 197.3. $^1H$ NMR (400 MHz, $CDCl_3$) $\delta$ 7.52 (dd, J = 6.7, 2.5 Hz, 1H), 7.38 (ddd, J = 8.7, 4.3, 2.5 Hz, 1H), 7.18 (t, J = 8.6 Hz, 1H), 1.74 (s, 6H).

**Step 2:**

2-(3-chloro-4-fluorophenyl)-2-methylpropionitrile (**I-6-b**) (1.1 g, 5.56 mmol) was put in anhydrous tetrahydrofuran (10 mL), cooled to -78 °C under nitrogen protection, added with 1 mol/L diisobutylaluminum hydride (17 mL, 17.0 mmol), and slowly raised the temperature and stirred overnight. After the reaction was complete, the mixture was cooled to 0 °C and quenched with 2N HCl. Then water (10 mL) and ethyl acetate (10 mL) were added, the organic phase was separated, and the aqueous phase was extracted with ethyl acetate (10 mL×2). The organic phases were combined, washed with saturated sodium chloride solution (10 mL×2) and water (10 mL), dried over anhydrous sodium sulfate, concentrated and purified by column chromatography (petroleum ether: ethyl acetate = 10:1) to obtain 2-(3-chloro-4-fluorophenyl)-2-methylpropanal (**I -6-c**) as a yellow oil (930 mg). $^1H$ NMR (400 MHz, $CDCl_3$) $\delta$ 9.49 (s, 1H), 7.34 (dd, J = 7.0, 1.7 Hz, 1H), 7.19 - 7.11 (m, 2H), 1.48 (s, 7H).

**Step 3:**

Tert-butyl (6-bromopyridin-3-yl)carbamate (1.1 g, 4.03 mmol) was put in anhydrous tetrahydrofuran (10 mL), cooled to -78 °C, and added with n-butyllithium (5.5 mL, 8.85 mmol, 1.6 mol/L). When the reaction solution became turbid, it was stirred for 1h, added with 2-(3-chloro-4-fluorophenyl)-2-methylpropanal (**I-6-c**) (930 mg, 4.64 mmol), gradually raised to room temperature and stirred overnight. After the reaction was complete, water (10 mL) and ethyl acetate (10 mL) were added to separate the organic phase, and the aqueous phase was extracted with ethyl acetate (10 mL×2). The organic phases was combined, washed with saturated sodium chloride solution (10 mL×2) and water (10 mL), dried over anhydrous sodium sulfate, concentrated and purified by column chromatography (petroleum ether: ethyl acetate = 10:1) to obtain tert-butyl (6-(2-(3-(3-chloro-4-fluorophenyl)-1-hydroxyl-2-methylpropyl)pyridin-3-yl)carbamate (**I-6-d**) as a yellow oil (1.0 g). LC-MS: m/z: $(M+H)^+$= 395.1.

**Step 4:**

Tert-butyl (6-(2-(3-(3-chloro-4-fluorophenyl)-1-hydroxyl-2-methylpropyl)pyridin-3-yl)carbamate (**I-6-d**) (1.0 g, 2.54 mmol) was put in dichloromethane (10 mL), added with manganese dioxide (2.2 g, 25.3 mmol), and stirred at 50 °C for 48h. After the reaction was complete, the system was filtered, the filtrate was concentrated and the residue was purified by column chromatography (petroleum ether: ethyl acetate = 10:1) to obtain tert-butyl (6-(2-(3-(3-chloro-4-fluorophenyl)-2-methylpropionyl)pyridin-3-yl)carbamate (**I-6-e**) as a yellow solid (730 mg). LC-MS: m/z: $(M+H)^+$= 393.1.

**Step 5:**

Tert-butyl (6-(2-(3-(3-chloro-4-fluorophenyl)-2-methylpropionyl)pyridin-3-yl)carbamate (**I-6-e**) (730 mg, 1.86

mmol) was put in dichloromethane (5 mL), added with trifluoroacetic acid (5 mL), and stirred at room temperature for 2h. After the reaction is complete, the reaction mixture was concentrated, added with ethyl acetate (10 mL), washed with saturated sodium bicarbonate solution (10 mL×2) and water (10 mL), and dried over anhydrous sodium sulfate to obtain 1-(5-aminopyridin-2-yl)-2-(3-chloro-4-fluorophenyl)-2-methyl-propan-1-one (**I-6-f**) as a yellow solid (530 mg). LC-MS: m/z: $(M+H)^+$= 293.1.

**Step 6:**

1-(5-aminopyridin-2-yl)-2-(3-chloro-4-fluorophenyl)-2-methylpropan-1-one (**I-6-f**) (530 mg, 1.80 mmol) was put in dichloromethane (10 mL), added with 2-(4-(ethylsulfonyl)phenyl)acetic acid (472 mg, 2.07 mmol), TBTU (665 mg, 2.07 mmol) and triethylamine (0.78 mL) and stirred overnight. After the reaction is complete, water (10 mL) and dichloromethane (10 mL) were added therein to separate the organic phase, and the aqueous phase was extracted with dichloromethane (10 mL×2). The organic phases were combined, wasdhed with saturated sodium chloride solution (10 mL×2) and water (10 mL), dried over anhydrous sodium sulfate and concentrated, and the concentrate was purified by column chromatography (petroleum ether: ethyl acetate = 1:1) to obtain N-(6-(2-(3-chloro-4-fluorophenyl)-2-methylpropionyl)pyridin-3-yl)-2-(4-(ethylsulfonyl) phenyl)acetamide (**I- 6**) as a yellow solid (660 mg). LC-MS: m/z: $(M+H)^+$= 503.1. $^1$H NMR (400 MHz, $CDCl_3$) $\delta$ 8.36 (d, J = 2.4 Hz, 1H), 8.14 (dd, J = 8.7, 2.5 Hz, 1H), 7.99 (d, J = 8.7 Hz, 1H), 7.92 (s, 1H), 7.81 (d, J = 8.3 Hz, 2H), 7.47 (d, J = 8.2 Hz, 2H), 7.33 (dd, J = 7.0, 2.3 Hz, 1H), 7.06 (ddd, J = 8.5, 4.5, 2.3 Hz, 1H), 6.98 (t, J = 8.7 Hz, 1H), 3.81 (s, 2H), 3.13 (q, J = 7.4 Hz, 2H), 1.71 (s, 6H), 1.29 (t, J = 7.4 Hz, 4H).

**Example 7**

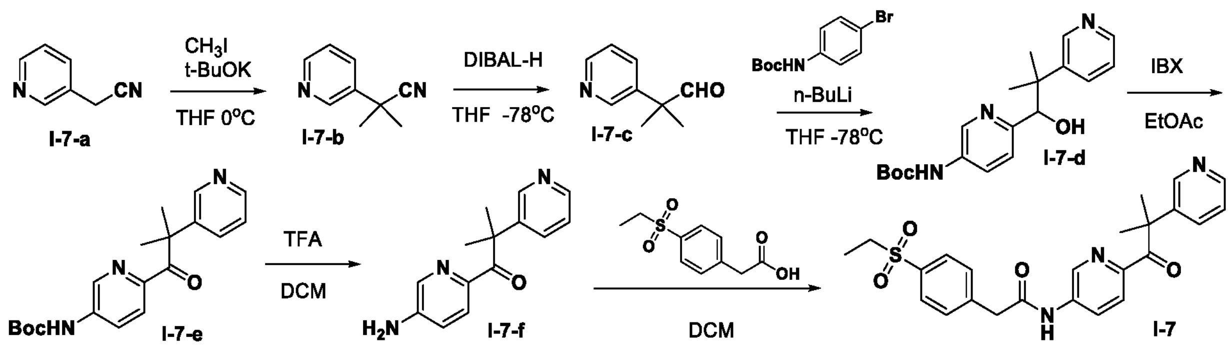


**Step 1:**

2-(pyridin-3-yl)acetonitrile (**I-7-a**) (500 mg, 4.23 mmol) and iodomethane (0.8 mL, 12.7 mmol) were put in anhydrous tetrahydrofuran (5 mL), cooled to 0 °C under nitrogen protection, added with potassium tert-butoxide (1.4 g, 12.5 mmol), gradually raised to room temperature and stirred overnight. After the reaction was complete, water (5 mL) and ethyl acetate (5 mL) were added to seperate the organic phase. The aqueous phase was extracted with ethyl acetate (5 mL×2). The organic phases were combined, washed with saturated sodium chloride solution (5 mL×2) and water (5 mL), dried over anhydrous sodium sulfate, concentrated and purified by column chromatography (petroleum ether: ethyl acetate = 1:1) to obtain 2-methyl-2-(pyridin-3-yl)propionitrile (**I-7-b**) as a yellow oil (480 mg). LC-MS: m/z: $(M+H)^+$= 147.1. $^1$H NMR (400 MHz, $CDCl_3$) $\delta$ 8.78 (d, J = 2.3 Hz, 1H), 8.61 (dd, J = 4.8, 1.5 Hz, 1H), 7.84 (ddd, J = 8.1, 2.5, 1.6 Hz, 1H), 7.37 (ddd, J = 8.1, 4.8, 0.6 Hz, 1H), 1.80 (s, 6H).

**Step 2:**

2-methyl-2-(pyridin-3-yl)propionitrile (**I-7-b**) (480 mg, 4.06 mmol) was put in anhydrous tetrahydrofuran (5 mL), cooled to -78 °C under nitrogen protection, added with 1 mol/L diisobutylaluminum hydride (9.9 mL, 9.9 mmol), gradually raised to room temperature and then stirred overnight. After the reaction was complete, the system was cooled to 0 °C and then added with water (5mL) and ethyl acetate (5 mL). The organic phase was separated and the aqueous phase was extracted with ethyl acetate (5 mL×2). The organic phases were combined, washed with saturated sodium chloride solution (5 mL×2) and water (5 mL), dried over anhydrous sodium sulfate, concentrated and purified by column chromatography (petroleum ether: ethyl acetate = 1:1) to obtain 2-methyl-2-(pyridin-3-yl) propanal (**I-7-c**) (170 mg) as a yellow oil. $^1$H NMR (400 MHz, $CDCl_3$) $\delta$ 9.57 (s, 1H), 8.65 - 8.49 (m, 2H), 7.62 (ddd, J = 8.0, 2.4, 1.7 Hz, 1H), 7.34 (ddd,

J = 8.0, 4.8, 0.6 Hz, 1H), 1.54 (s, 6H).

**Step 3:**

Tert-butyl (6-bromopyridin-3-yl)carbamate (289 mg, 1.06 mmol) was put in anhydrous tetrahydrofuran (3 mL), cooled to -78 °C under nitrogen protection, and added with n-butyllithium (1.4 mL, 2.24 mmol, 1.6 mol/L). After the reaction solution became turbid, it was stirred for 1h, added with 2-methyl-2-(pyridin-3-yl)propanal **(I-7-c)** (170 mg, 1.16 mmol), stirred overnight and gradually raised to room temperature. After the reaction was complete, water (5 mL) and ethyl acetate (5 mL) were added, the organic phase was separated and the aqueous phase was extracted with ethyl acetate (5 mL×2). The organic phases were combined, washed with saturated sodium chloride solution (5 mL×2) and water (5 mL), dried over anhydrous sodium sulfate, concentrated and purified by column chromatography (dichloromethane: methanol = 20:1) to obtain tert-butyl (6-(1-hydroxy-2-methyl-2-(pyridin-3-yl)propyl)pyridin-3-yl)carbamate (**I-7-d**) (175 mg) as a yellow solid. LC-MS: m/z: $(M+H)^+$= 344.2. $^1H$ NMR (400 MHz, $CDCl_3$) $\delta$ 8.48 (dt, J = 4.4, 3.3 Hz, 2H), 8.30 (d, J = 2.5 Hz, 1H), 7.84 - 7.74 (m, 1H), 7.67 (d, J = 8.2 Hz, 1H), 6.64 - 6.52 (m, 2H), 4.73 (s, 1H), 1.54 (s, 9H), 1.46 (s, 3H), 1.35 (s, 3H).

**Step 4:**

Tert-butyl (6-(1-hydroxy-2-methyl-2-(pyridin-3-yl)propyl)pyridin-3-yl)carbamate (**I-7-d**) (175 mg, 0.51 mmol) was put in ethyl acetate (5 mL), added with 2-iodoxybenzoic acid (416 mg, 1.48 mmol), and stirred overnight at 100°C. After the reaction was complete, the mixture was filtered and the filtrate was concentrated and purified by column chromatography (petroleum ether: ethyl acetate=1:1), to obtain tert-butyl (6-(2-methyl-2-(pyridin-3-yl)propionyl)pyridin-3-yl)carbamate (**I-7-e**) (170 mg) as a yellow solid. LC-MS: m/z: $(M+H)^+$= 342.2.

**Step 5:**

Tert-butyl (6-(2-methyl-2-(pyridin-3-yl)propanoyl)pyridin-3-yl)carbamate (**I-7-e**) (170 mg, 0.50 mmol) was put in dichloromethane (5 mL), added with trifluoroacetic acid (5 mL), and stirred at room temperature for 2h. After the reaction was complete, the mixture was concentrated, added with ethyl acetate (5 mL), washed with saturated sodium bicarbonate solution (5 mL×2) and water (5 mL), and dried over anhydrous sodium sulfate. 1-(5-aminopyridin-2-yl)-2-methyl-2-(pyridin-3-yl)propan-1-one (**I-7-f**) (120 mg) was obtained as a yellow solid. LC-MS: m/z: $(M+H)^+$= 242.1.

**Step 6:**

1-(5-aminopyridin-2-yl)-2-methyl-2-(pyridin-3-yl)propan-1-one (**I-7-f**) (120 mg, 0.50 mmol) was put in dichloromethane (5 mL), added with 2-(4-(ethylsulfonyl)phenyl)acetic acid (130 mg, 0.57 mmol), TBTU (184 mg, 0.57 mmol) and triethylamine (0.20 mL), and stirred overnight. After the reaction was complete, water (5 mL) and dichloromethane (5 mL) were added, the organic phase was separated, and the aqueous phase was extracted with dichloromethane (5 mL×2). The organic phases were combined, washed with saturated sodium chloride solution (5 mL×2) and water (5 mL), dried over anhydrous sodium sulfate, concentrated and purified by column chromatography (dichloromethane:methanol=20:1) to obtain 2-(4-(ethylsulfonyl)phenyl)-N-(6-(2-methyl-2-(pyridin-3-yl)propanoyl)pyridin-3-yl)acet amide (**I-7**) (28 mg) as a yellow solid. LC-MS: m/z: $(M+H)^+$= 452.2. $^1H$ NMR (400 MHz, MeOD) $\delta$ 8.55 - 8.39 (m, 2H), 8.32 (s, 1H), 8.16 (dd, J = 8.7, 2.5 Hz, 1H), 8.02 (d, J = 8.7 Hz, 1H), 7.88 (d, J = 8.3 Hz, 2H), 7.74 (d, J = 7.9 Hz, 1H), 7.60 (d, J = 8.3 Hz, 2H), 7.33 (dd, J = 7.7, 4.9 Hz, 1H), 3.86 (s, 2H), 3.21 (q, J = 7.4 Hz, 2H), 1.76 (s, 6H), 1.22 (t, J = 7.4 Hz, 3H).

**Example 8**

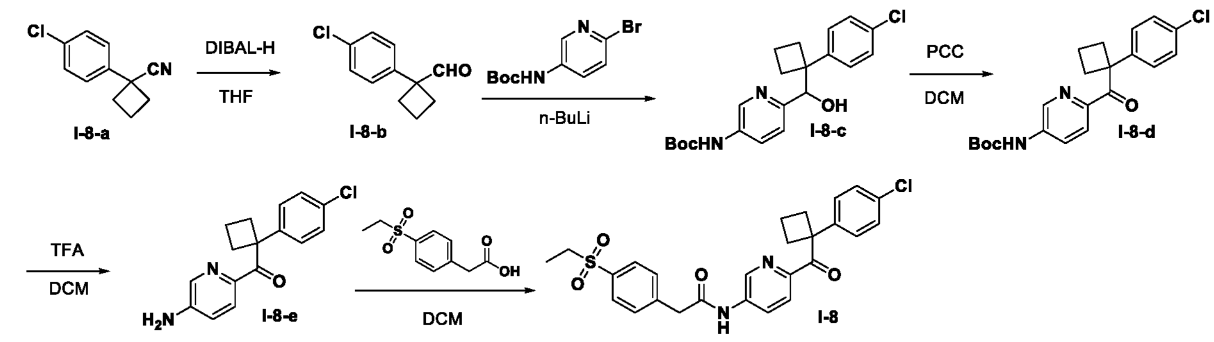

I-8-a I-8-b I-8-c I-8-d I-8-e I-8

**Step 1:**

1-(4-chlorophenyl)cyclobutane-1-carbonitrile (**I-8-a**) (1 g, 5.21 mmol) was put in anhydrous tetrahydrofuran (5 mL), coolded to -78 °C under nitrogen protection, added with 1 mol/L diisobutylaluminum hydride (7.8 mL, 7.83 mmol), gradually raised to room temperature and stirred overnight. After the reaction was complete, the reaction mixture was cooled to 0 °C and the reaction was quenched with 2N HCl. Then water (10 mL) and ethyl acetate (10 mL) were added, the organic phase was separated, and the aqueous phase was extracted with ethyl acetate (10 mL×2). The organic phases were combined, washed with saturated sodium chloride solution (10 mL×2) and water (10 mL), dried over anhydrous sodium sulfate, concentrated and purified by column chromatography (petroleum ether: ethyl acetate = 10:1) to obtain 1-(4-chlorophenyl)cyclobutane-1-carbaldehyde (**I-8- b**) (860 mg) as a yellow oil. LC-MS: m/z: $(M+H)^+$= 195.1.

**Step 2:**

Tert-butyl (6-bromopyridin-3-yl)carbamate (1.1 g, 4.02 mmol) was put in anhydrous tetrahydrofuran (10 mL), cooled to -78 °C under nitrogen protection and added with 1.6 mol/L n-butyllithium (5.3 mL, 8.42 mmol). After the reaction solution became turbid, it was stirred for 1h, added with 1-(4-chlorophenyl)cyclobutane-1-carbaldehyde (**I-8-b**) (860 mg, 4.41 mmol), gradually raised to room temperature and stirred overnight. After the reaction was complete, water (10 mL) and ethyl acetate (10 mL) were added, the organic phase was separated, and the aqueous phase was extracted with ethyl acetate (10 mL×2). The organic phases were combined, washed with saturated sodium chloride solution (10 mL×2) and water (10 mL), dried over anhydrous sodium sulfate, concentrated and purified by column chromatography (petroleum ether: ethyl acetate = 10:1) to obtain tert-butyl (6-(((1-(4-chlorophenyl)cyclobutyl)(hydroxy)methyl)pyridin-3-yl)carbamate (**I-8-c**) (700 mg) as a yellow oil. LC-MS: m/z: $(M+H)^+$= 389.1.

**Step 3:**

Tert-butyl (6-(((1-(4-chlorophenyl)cyclobutyl)(hydroxy)methyl)pyridin-3-yl)carbamate (**I-8-c**) (700 mg, 1.80 mmol) was put in dichloromethane (10 mL), added with PCC (776 mg, 3.60 mmol), and stirred at room temperature for 48h. After the reaction was complete, the mixture was filtered and the filtrate was concentrated and purified by column chromatography (petroleum ether: ethyl acetate=10:1) to obtain (6-(1-(4-(chlorophenyl)cyclobutane-1-carbonyl)pyridin-3-yl)carbamate (**I-8-d**) (280 mg), as a yellow solid. LC-MS: m/z: $(M+H)^+$= 387.1.

**Step 4:**

Tert-butyl (6-(1-(4-(chlorophenyl)cyclobutane-1-carbonyl)pyridin-3-yl)carbamate **(I-8-d)** (280 mg, 0.73 mmol) was put in dichloromethane (5 mL), added with trifluoroacetic acid (5 mL), and stirred at room temperature for 2h. After the reaction was complete, the mixture was concentrated, added with ethyl acetate (10 mL), washed with saturated sodium bicarbonate solution (10 mL×2) and water (10 mL), and dried over anhydrous sodium sulfate. (5-aminopyridin-2-yl)(1-(4-chlorophenyl)cyclobutyl)methanone (**I-8-e**) (240 mg) was obtained as a yellow solid. LC-MS: m/z: $(M+H)^+$= 293.1.

**Step 5:**

(5-aminopyridin-2-yl)(1-(4-chlorophenyl)cyclobutyl)methanone (**I-8-e**) (240 mg, 0.84 mmol) was put in dichloromethane (10 mL), added with 2-(4-(ethylsulfonyl)phenyl)acetic acid (220 mg, 0.96 mmol), TBTU (310 mg, 0.96 mmol) and triethylamine (0.40 mL), and stirred overnight. After the reaction was complete, water (10 mL) and dichloromethane (10 mL) were added, the organic phase was separated, and the aqueous phase was extracted with dichloromethane

(10 mL×2). The organic phases were combined, washed with saturated sodium chloride solution (10 mL×2) and water (10 mL), dried over anhydrous sodium sulfate, concentrated and purified by column chromatography (petroleum ether: ethyl acetate = 1:1) to obtain N-(6-(1-(4-chlorophenyl) cyclobutane-1-carbonyl)pyridin-3-yl)-2-(4-(ethylsulfonyl)phenyl)acetamide (**I-8**) (260 mg) as a yellow solid. LC-MS: m/z: $(M+H)^+$= 497.1. $^1H$ NMR (400 MHz, $CDCl_3$) $\delta$ 8.52 (d, J = 2.4 Hz, 1H), 8.08 (dd, J = 8.6, 2.5 Hz, 1H), 7.96 (d, J = 8.6 Hz, 1H), 7.86 (d, J = 8.3 Hz, 2H), 7.64 (s, 1H), 7.50 (d, J = 8.3 Hz, 2H), 7.46 - 7.38 (m, 2H), 7.26 - 7.18 (m, 2H), 3.84 (s, 2H), 3.14 (q, J = 7.4 Hz, 2H), 3.01 (ddd, J = 12.7, 9.1, 7.5 Hz, 2H), 2.65 (ddd, J = 12.8, 8.8, 5.6 Hz, 2H), 2.06 - 1.82 (m, 2H), 1.31 (t, J = 7.4 Hz, 3H).

**Example 9**

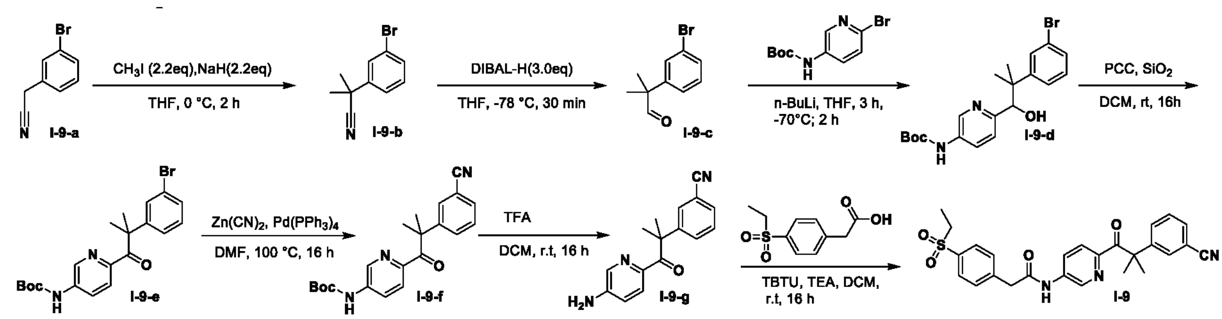


**Step 1:**

At 0°C, sodium hydride (3.06g, 76.5mmol) was dissolved in anhydrous THF (60 mL), and a solution of 2-(3-bromophenyl)acetonitrile (**I-9-a**) (5g, 25.505 mmol) in anhydrous THF (60 mL) was added dropwise in the system. After stirred for 1h, the reaction mixture was added with iodomethane (8.3 mL, 130 mmol) and stirred at 0 °C for another 2h. TLC (petroleum ether: ethyl acetate= 10:1) showed that the reaction was complete. The mixture was warmed to room temperature, quenched carefully with ice water, and extracted with ethyl acetate (100 mL×3). The combined extracts were washed with brine, dried over anhydrous sodium sulfate, filtered and concentrated to obtain 2-(3-bromophenyl)-2-methyl-propionitrile (**I-9-b**) (5.5 g, 25 mmol) as a yellow oil. $^1H$-NMR (MeOD): $\delta$ 7.70 (t, J = 1.9 Hz, 1H), 7.52 (dddd, J = 8.2, 5.3, 1.9, 1.0 Hz, 2H), 7.36 (t, J = 7.9 Hz, 1H), 1.74 (s, 6H). LC-MS: m/z $(M+H)^+$ = 222.9.

**Step 2:**

At -78 °C, DIBAL-H (100 mL, 100 mmol, 1 mol/L) was slowly added dropwise into a solution of 2-(3-bromophenyl)-2-methyl-propionitrile (**I-9-b**) (5 g, 22.311 mmol) in anhydrous THF (40 mL). After stirred at -78°C for 30min, the reaction mixture was raised to room temperature and stirred for 16h. TLC (petroleum ether: ethyl acetate = 10:1) showed that the reaction was complete. The mixture was cooled in an ice bath, quenched slowly with 2N HCl, and extracted with ethyl acetate (50 mL×2). The combined extracts were dried over anhydrous sodium sulfate and concentrated to obtain 2-(3-bromophenyl)-2-methyl-propanal (**I-9-c**) as a yellow oil (4.5 g, 20 mmol). $^1H$-NMR (MeOD): 9.50 (s, 1H), 7.57 (t, J = 1.9 Hz, 1H), 7.48 (q, J = 1.5 Hz, 1H), 7.36 (t, J = 7.9 Hz, 1H), 1.74 (sz, 1H), 1.45 (s, 6H).

**Step 3:**

Tert-butyl N-(6-bromo-3-pyridyl)carbamate (3 g, 10.984 mmol) was dissolved in anhydrous THF (100 mL). The reaction system was replaced with argon, and at -70°C, n-butyllithium (14 mL, 22 mmol, 1.6 mol/L) was injected via a syringe. The mixture was stirred at -70°C for 2.5 h, and added with a solution of 2-(3-bromophenyl)-2-methyl-propanal (**I-9-c**) (2.5 g, 11 mmol) in THF (20 mL). After the addition, the mixture was stirred at -70 °C for 100min. TLC (petroleum ether: ethyl acetate = 3:1) showed that the starting material disappeared and a new spot was formed. The mixture was diluted with ice water (100 mL), and extracted with ethyl acetate (100 mLx3). The organic layer was washed with brine, dried over anhydrous sodium sulfate, filtered and concentrated, and the residue was purified by column chromatography (Petroleum ether/ethyl acetate 0-50%) to obtain tert-butyl N-[6-[2-[3-(3-bromophenyl)-1-hydroxy-2-methyl-propyl]-3-pyridyl]carbamate (**I-9-d**) as a yellow solid (3.1 g, 7.4 mmol). $^1H$-NMR (DMSO-$d_6$): $\delta$ 9.48 (s, 1H), 8.47 (d, J = 2.5 Hz, 1H), 7.71 (dd, J = 8.6, 2.6 Hz, 1H), 7.44 (t, J = 1.9 Hz, 1H), 7.36 (ddd, J = 7.7, 2.0, 1.1 Hz, 1H), 7.29 - 7.25 (m, 1H), 7.21 (t, J = 7.8 Hz, 1H), 6.90 (d, J = 8.6 Hz, 1H), 5.32 (d, J = 5.0 Hz, 1H), 4.62 (d, J = 5.1 Hz, 1H), 1.48 (s, 9H), 1.23 (s, 3H),

1.20 (s, 3H). LC-MS: m/z $(M+H)^+$= 423.8.

**Step 4:**

Tert-butyl N-[6-[2-[3-(3-bromophenyl)-1-hydroxy-2-methyl-propyl]-3-pyridyl]carbamate (**I-9-d**) (1.6 g, 3.8 mmol) was dissolved in DCM (20 mL), PCC (1.64 g, 3.8 mmol) and silica gel powder (1 g) were added, and the mixture was stirred at 20°C for 16h. TLC (petroleum ether: ethyl acetate = 3:1) showed that the reaction was complete. The mixture was filtered. The filtrate was concentrated and purified by column chromatography (petroleum ether/ethyl acetate 0-20%) to obtain tert-butyl N-[6-[2-(3-bromophenyl)-2-methyl-propionyl]-3-pyridyl]carbamate (**I-9-e**) as a white solid (0.58 g, 1.4 mmol). $^1$H-NMR ($CDCl_3$): δ 8.20 (dd, J = 2.5, 0.7 Hz, 1H), 8.00 (d, J = 8.6 Hz, 1H), 7.95 (dd, J = 8.7, 2.5 Hz, 1H), 7.47 (t, J = 1.9 Hz, 1H), 7.27 (t, J = 1.7 Hz, 1H), 7.16 - 7.06 (m, 2H), 6.57 (s, 1H), 1.72 (s, 6H), 1.52 (s, 9H). LC-MS: m/z $(M+H)^+$= 420.8.

**Step 5:**

Tert-Butyl N-[6-[2-[3-(3-bromophenyl)-2-methyl-propionyl]-3-pyridyl]carbamate (**I-9-e**) (200 mg, 0.4770 mmol) was dissolved in anhydrous DMF (2 mL), and zinc(II) cyanide (90 mg, 0.76635 mmol) and tetrakis(triphenylphosphine) palladium(0) (70 mg, 0.060575 mmol) were added therein. The reaction vial was replaced with argon, and the mixture was stirred at 100°C for 16 h under the protection of argon. TLC (petroleum ether: ethyl acetate = 5:1) showed that a new spot was formed. The mixture was diluted with ethyl acetate (50 mL), and washed with water (30 mL×2). The organic layer was washed with brine, dried over anhydrous sodium sulfate, filtered and concentrated. The residue was purified by column chromatography (petroleum ether/ethyl acetate 0-30%) to obtain tert-butyl N-[6 -[2-(3-cyanophenyl)-2-methyl-propionyl]-3-pyridyl]carbamate **(I-9-f)** (100mg, 0.2737mmol) as a colorless oil. $^1$H-NMR ($CDCl_3$): δ 8.20 (d, J = 2.6 Hz, 1H), 8.02 (d, J = 8.6 Hz, 1H), 7.96 (dd, J = 8.7, 2.6 Hz, 1H), 7.63 (t, J = 1.7 Hz, 1H), 7.43 (ddt, J = 8.1, 3.5, 1.6 Hz, 2H), 7.35 - 7.29 (m, 1H), 6.79 (s, 1H), 1.73 (s, 6H), 1.51 (s, 9H). LC-MS: m/z $(M+H)^+$= 365.8.

**Step 6:**

Tert-butyl N-[6-[2-[3-(3-cyanophenyl)-2-methyl-propionyl]-3-pyridyl]carbamate **(I-9-f)** (100 mg, 0.2737 mmol) was dissolved in DCM (3 mL), added with TFA (1 mL), and stirred at 17 °C for 1h. TLC (petroleum ether: ethyl acetate = 5:1) showed that the reaction was complete. The mixture was cooled in ice water, quenched with saturated aqueous $NaHCO_3$, and extracted with DCM (20 mL×2). The organic layer was dried over anhydrous sodium sulfate, filtered and concentrated to obtain 3-[2-(5-amino-2-pyridyl)-1,1-dimethyl-2-oxoethyl]benzonitrile (**I-9-g**) (70mg, 0.2639mmol) as a pale yellow solid. $^1$H-NMR ($CDCl_3$): δ 7.89 (d, J = 8.5 Hz, 1H), 7.75 (d, J = 2.8 Hz, 1H), 7.31 (t, J = 1.8 Hz, 1H), 7.18 - 7.06 (m, 3H), 6.89 (dd, J = 8.6, 2.8 Hz, 1H), 1.71 (s, 6H). LC-MS: m/z $(M+H)^+$= 365.9.

**Step 7:**

3-[2-(5-amino-2-pyridyl)-1,1-dimethyl-2-oxoethyl]benzonitrile (**I-9-g**) (70 mg, 0.2639 mmol) was dissolved in DCM (3mL), added with 2-(4-ethylsulfonylphenyl)acetic acid (69 mg, 0.30229 mmol), TBTU (97 mg, 0.3021 mmol) and N,N-diethylethylamine (80 mg, 0.79059 mmol), and stirred at 16°C for 2h under argon protection. TLC (ethyl acetate) showed that the reaction was complete. The reaction solution was concentrated, and the residue was purified by column chromatography (petroleum ether/ethyl acetate 0-86%) to obtain N-[6-[2-(3-cyanophenyl)-2-methylpropionyl]-3-pyridyl]-2-(4-ethylsulfonylphenyl)aceta mide (**I-9**) (62 mg, 0.1304 mmol) as a white solid. $^1$H-NMR ($CDCl_3$): δ8.29 (d, J = 2.6 Hz, 1H), 8.18 (dd, J = 8.7, 2.6 Hz, 1H), 8.05 (d, J = 8.7 Hz, 1H), 7.90 - 7.84 (m, 2H), 7.81 (s, 1H), 7.64 (t, J = 1.7 Hz, 1H), 7.56 - 7.50 (m, 2H), 7.44 (ddt, J = 8.9, 7.6, 1.4 Hz, 2H), 7.33 (t, J = 7.8 Hz, 1H), 3.83 (s, 2H), 3.14 (q, J = 7.5 Hz, 2H), 1.74 (s, 6H), 1.30 (t, J = 7.5 Hz, 3H). LC-MS: m/z $(M+H)^+$= 475.9.

**Example 10**

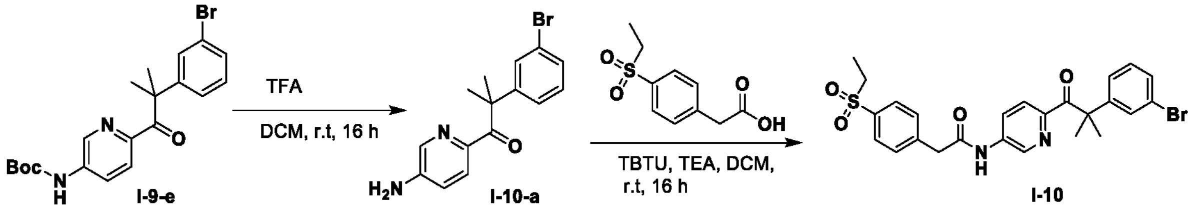


Step 1:

Tert-butyl N-[6-[2-[3-(3-bromophenyl)-2-methyl-propionyl]-3-pyridyl]carbamate (**I-9-e**) (100 mg, 0.2385 mmol) was dissolved in DCM (3 mL), added with TFA (1 mL), and stirred at 17 °C for 1h. TLC (petroleum ether: ethyl acetate = 5:1) showed that the reaction was complete. The mixture was cooled in ice water, quenched with saturated aqueous $NaHCO_3$ and extracted with DCM (20 mL×2). The organic layer was dried over anhydrous sodium sulfate, filtered and concentrated to obtain 1-(5-amino-2-pyridyl)-2-(3- bromophenyl)-2-methyl-propan-1-one (**I-10-a**) (70mg, 0.2193mmol) as a pale yellow solid. LC-MS: m/z $(M+H)^+$= 319.8.

Step 2:

1-(5-amino-2-pyridyl)-2-(3-bromophenyl)-2-methyl-propan-1-one (**I-10-a**) (70 mg, 0.2193 mmol) was dissolved in DCM (3mL), added with 2-(4-ethylsulfonylphenyl)acetic acid (60 mg, 0.26286 mmol), TBTU (80 mg, 0.2491 mmol) and N,N-diethylethylamine (66 mg, 0.65224 mmol), and stirred at 16°C for 2h under argon protection. TLC (ethyl acetate) showed that the reaction was complete. The reaction solution was concentrated, and the residue was purified by column chromatography (petroleum ether/ethyl acetate 0-76%) and concentrated, to obtain N-[6-[2-(3-bromophenyl)-2-methyl-propionyl]-3-pyridyl]-2-(4-ethylsulfonylphenyl)aceta mide (**I- 10**) (25 mg, 0.04722 mmol) as a white solid. $^1$H-NMR ($CDCl_3$): δ 8.34 (d, J = 2.6 Hz, 1H), 8.15 (dd, J = 8.7, 2.6 Hz, 1H), 8.02 (s, 1H), 7.98 (d, J = 8.7 Hz, 1H), 7.82 (d, J = 8.2 Hz, 2H), 7.51 - 7.44 (m, 3H), 7.28 - 7.25 (m, 1H), 7.15 - 7.06 (m, 2H), 3.81 (s, 2H), 3.13 (q, J = 7.4 Hz, 2H), 1.71 (s, 6H), 1.29 (t, J = 7.4 Hz, 3H). LC-MS: m/z $(M+H)^+$= 529.8.

## Example 11

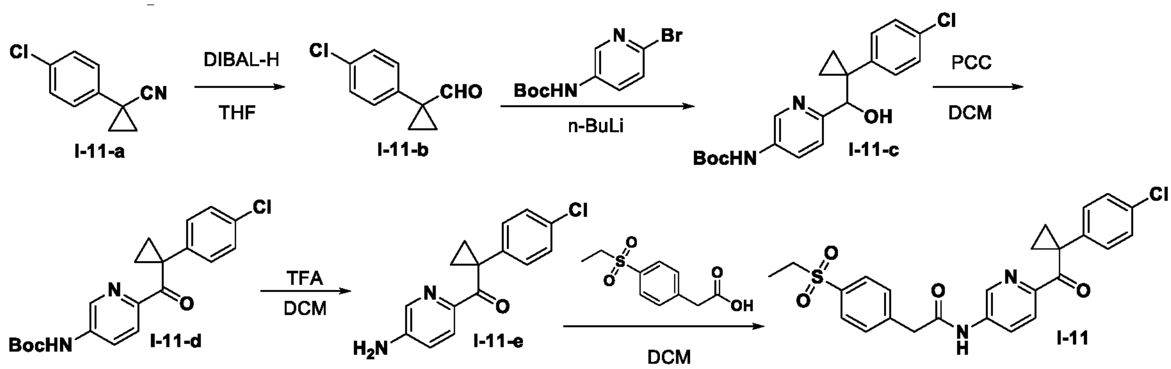


Step 1:

1-(4-chlorophenyl)cyclopropane-1-carbonitrile (**I-11-a**) (500 mg, 2.81 mmol) was put in anhydrous tetrahydrofuran (5 mL), cooled to -78 °C under nitrogen protection, added with 1 mol/L diisobutylaluminum hydride (8.4 mL, 8.44 mmol), gradually raised to room temperature and stirred overnight. After the reaction was complete, the reaction mixture was cooled to 0°C and quenched with 2N HCl. Water (10 mL) and ethyl acetate (10 mL) were added, the organic phase was separated, and the aqueous phase was extracted with ethyl acetate (10 mL×2). The organic phases were combined, washed with saturated sodium chloride solution (10 mL×2) and water (10 mL), dried over anhydrous sodium sulfate, concentrated and purified by column chromatography (petroleum ether: ethyl acetate = 10:1) to obtain 1-(4-chlorophenyl)cyclopropane-1-carbaldehyde (**I-11-b**) (510 mg) as a yellow oil. LC-MS: m/z: $(M+H)^+$= 181.1.

**Step 2:**

Tert-butyl (6-bromopyridin-3-yl)carbamate (687 mg, 2.51 mmol) was put in anhydrous tetrahydrofuran (10 mL), and added with n-butyllithium (3.3 mL, 5.28 mmol, 1.6 mol/L) under nitrogen protection. After the reaction solution became turbid, it was stirred for 1h, added with 1-(4-chlorophenyl)cyclopropane-1-carbaldehyde (**I-11-b**) (510 mg, 2.82 mmol), stirred overnight and gradually raised to room temperature. After the reaction was complete, water (10 mL) and ethyl acetate (10 mL) were added, the organic phase was separated, and the aqueous phase was extracted with ethyl acetate (10 mL×2). The organic phases were combined, washed with saturated sodium chloride solution (10 mL×2) and water (10 mL), dried over anhydrous sodium sulfate, concentrated and purified by column chromatography (petroleum ether: ethyl acetate = 10:1), to obtain tert-butyl (6-(((1-(4-chlorophenyl)cyclopropyl)(hydroxy)methyl)pyridin-3-yl)carbamate (**I-11-c**) (650 mg) as a yellow oil. LC-MS: m/z: $(M+H)^+$= 375.1.

**Step 3:**

Tert-butyl (6-(((1-(4-chlorophenyl)cyclopropyl)(hydroxy)methyl)pyridin-3-yl)carbamate (**I-11-c**) (650 mg, 1.74 mmol) was put in dichloromethane (10 mL), added wtih PCC (750 mg, 3.48 mmol), and stirred at room temperature for 48h. After the reaction was complete, the reaction mixture was filtered and the filtrate was contrated and purified by column chromatography (petroleum ether: ethyl acetate = 10:1), to obtain tert-butyl (6-(1-(4-(chlorophenyl)cyclopropane-1-carbonyl)pyridin-3-yl)carbamate (**I-11-d**) (70 mg) as a yellow solid. LC-MS: m/z: $(M+H)^+$= 373.1.

**Step 4:**

Tert-butyl (6-(1-(4-(chlorophenyl)cyclopropane-1-carbonyl)pyridin-3-yl)carbamate (**I-11-d**) (70 mg, 0.19 mmol) was put in dichloromethane (5 mL), added wtih trifluoroacetic acid (5 mL), and stirred at room temperature for 2h. After the reaction was complete, the reaction mixture was concentrated, added with ethyl acetate (10 mL), washed with saturated sodium bicarbonate solution (10 mL×2) and water (10 mL), and dried over anhydrous sodium sulfate to obtain (5-aminopyridin-2-yl)(1-(4-chlorophenyl)cyclopropyl)methanone (**I-11-e**) (53 mg) as a yellow solid. LC-MS: m/z: $(M+H)^+$= 273.1.

**Step 5:**

(5-aminopyridin-2-yl)(1-(4-chlorophenyl)cyclopropyl)methanone (**I-11-e**) (53 mg, 0.19 mmol) was put in dichloromethane (10 mL), added with 2-(4-(ethylsulfonyl)phenyl)acetic acid (51 mg, 0.22 mmol), TBTU (72 mg, 0.22 mmol) and triethylamine (0.08 mL), and stirred overnight. After the reaction was complete, water (10 mL) and dichloromethane (10 mL) were added, the organic phase was separated, and the aqueous phase was extracted with dichloromethane (10 mL×2). The organic phases were combined, washed with saturated sodium chloride solution (10 mL×2) and water (10 mL), dried over anhydrous sodium sulfate, concentrated and purified by column chromatography (petroleum ether: ethyl acetate = 1:1) to obtain N-(6-(1-(4-chlorophenyl)cyclopropane-1-carbonyl)pyridin-3-yl)-2-(4-(ethylsulfonyl)phenyl)acetamide (**I-11**) (260 mg) as a yellow solid. LC-MS: m/z: $(M+H)^+$= 483.1. $^1$H NMR (400 MHz, $CDCl_3$) $\delta$ 8.41 (d, J = 2.4 Hz, 1H), 8.15 (dd, J = 8.6, 2.3 Hz, 1H), 7.87 (d, J = 8.3 Hz, 2H), 7.81 - 7.72 (m, 2H), 7.51 (d, J = 8.2 Hz, 2H), 7.27 - 7.21 (m, 2H), 7.21 - 7.14 (m, 2H), 3.84 (s, 2H), 3.14 (q, J = 7.4 Hz, 2H), 1.84 (q, J = 4.2 Hz, 2H), 1.36 (q, J = 4.3 Hz, 2H), 1.31 (t, J = 5.1 Hz, 3H).

**Example 12**

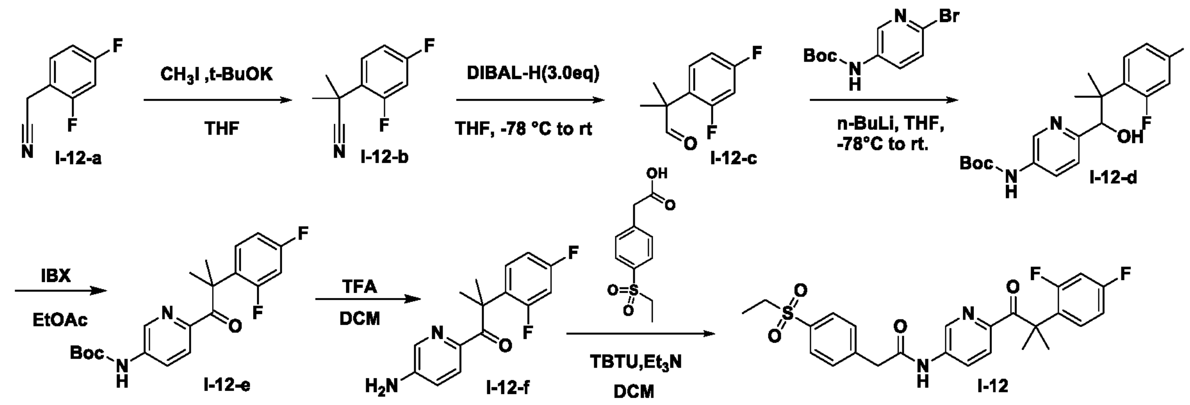


**Step 1:**

2,4-difluorophenylacetonitrile (**I-12-a**) (1.53 g, 9.99 mmol) was dissolved in dry tetrahydrofuran (30 mL), added with iodomethane (5.68 g, 40.0 mmol), cooled to 0 °C in ice bath, added with potassium tert-butoxide (3.36 g, 29.9 mmol) in batches, slowly raised to room temperature and stirred overnight. TLC showed that the reaction was complete. Water (20 mL) and ethyl acetate (20 mL) were added, the organic phase was separated, and the aqueous phase was extracted with ethyl acetate (20 mL×2). The organic phases were combined, washed with saturated sodium chloride solution (20 mL), dried over anhydrous sodium sulfate, concentrated and purified by column chromatography (petroleum ether: ethyl acetate = 10:1) to obtain 2-(2,4-difluorophenyl)-2-methylpropionitrile (**I-12-b**) (1.34 g) as a colorless liquid. $^1$H NMR (400 MHz, $CDCl_3$) $\delta$ 7.47 (ddd, J = 14.9, 7.5, 3.7 Hz, 1H), 6.93 - 6.83 (m, 2H), 1.79 (s, 6H).

**Step 2:**

2-(2,4-difluorophenyl)-2-methylpropionitrile (**I-12-b**) (614 mg, 3.39 mmol) was put in a three-neck flask, vacuumed and protected with argon. Then the reaction system was added with dry tetrahydrofuran (15 mL), cooled to -78 °C, and slowly added dropwise with DIBAL-H (10 mL, 1.0 mol/L). After the addition, the system was reacted at -78 °C for 3 h, and slowly stirred overnight at room temperature. TLC showed that the reaction was complet. The reaction system was cooled to 0 °C, and 2.0 M HCl solution was slowly added dropwise until the system pH=3, and the aqueous phase was extracted with ethyl acetate (20 mL×2). The organic phases were combined, washed with saturated sodium chloride solution (20 mL), dried over anhydrous sodium sulfate, concentrated and purified by column chromatography (petroleum ether: ethyl acetate = 10:1) to obtain 2-(2,4-difluorophenyl)-2-methylpropanal (**I-12-c**) (450 mg) as a colorless liquid. $^1$H NMR (400 MHz, $CDCl_3$) $\delta$ 9.61 (d, J = 4.3 Hz, 1H), 7.32 - 7.23 (m, 1H), 6.95 - 6.88 (m, 1H), 6.83 (ddd, J = 11.3, 8.7, 2.6 Hz, 1H), 1.44 (s, 6H).

**Step 3:**

2-bromo-5-Boc-aminopyridine (841 mg, 3.079 mmol) was put in a three-neck flask, vacuumed and protected with argon. The reaction system was added with dry tetrahydrofuran (15 mL), cooled to -78 °C, and slowly added with n-butyllithium (4.0 mL, 1.6 mol/L) dropwise. After the dropwise addition, the system was reacted at -78 °C for 3 h, slowly added with a solution of 2-(2,4-difluorophenyl)-2-methylpropanal (**I-12-c**) (567 mg, 3.078 mmol) in dry tetrahydrofuran (6 mL), reacted at -78 °C for 3 h after the dropwise addition, and slowly raised to room temperature overnight. TLC showed the reaction was complete, and the reaction system was cooled to 0 °C, added with saturated aqueous $NH_4Cl$ (10 mL), water (30 mL) and ethyl acetate (30 mL). The organic phase was separated, and the aqueous phase was extracted with ethyl acetate (20 mL×2). The organic phases were combined, washed with saturated sodium chloride solution (30 mL), dried over anhydrous sodium sulfate, concentrated and purified by column chromatography (petroleum ether: ethyl acetate = 2:1) to obtain tert-butyl (6-(2 -(2,4-difluorophenyl)-1-hydroxy-2-methylpropyl)pyridin-3-yl)carbamate (**I-12-d**) (356 mg) as a colorless liquid. LC-MS: m/z: $(M+H)^+$= 378.9.

**Step 4:**

Tert-butyl (6-(2-(2,4-difluorophenyl)-1-hydroxy-2-methylpropyl)pyridin-3-yl)carbamate (**I-12-d**) (356 mg, 0.79 mmol) was dissolved in ethyl acetate (20 mL), added with 2-iodoxybenzoic acid (IBX, 500 mg, 1.78 mmol), and heated to reflux overnight. TLC showed that the reaction was complete, and the reaction system was cooled to room temperature, and filtered off the insolubles in the system. The filtrate was rotary evaporated to dryness to obtain a crude tert-butyl

(6-(2-(2,4-difluorophenyl)-2-methylpropionyl)pyridin-3-yl)carbamate **(I-12-e)** (238 mg) as a pale yellow oil. LC-MS: m/z: $(M+H)^+$= 376.9.

**Step 5:**

The crude tert-butyl (6-(2-(2,4-difluorophenyl)-2-methylpropionyl)pyridin-3-yl)carbamate (**I-12-e**) (238 mg) in previous step was dissolved in dichloromethane (15 mL), added with trifluoroacetic acid (4 mL), and stirred overnight. TLC showed that the reaction was complete, and the reaction system was concentrated to obtain a crude 1-(5-aminopyridin-2-yl)-2-(2,4-difluorophenyl)-2-methylpropan-1-one (**I-12-f**) (246 mg) as a pale yellow oil. LC-MS: m/z: $(M+H)^+$=276.9.

**Step 6:**

The crude 1-(5-aminopyridin-2-yl)-2-(2,4-difluorophenyl)-2-methylpropan-1-one (**I-12-f**) (246 mg) in previous step, and 2-(4-(ethylsulfonyl)phenyl)acetic acid (290 mg, 1.27 mmol) were dissolved in dichloromethane (15 mL), added with triethylamine (260 mg, 2.57 mmol) and TBTU (410 mg, 1.27 mmol), and reacted at room temperature for 24h. TLC showed the reaction was complete. Water (20 mL) and dichloromethane (30 mL) were added to the reaction system, and the organic phase was separated, washed with water (20 mL×2) and saturated sodium chloride solution (30 mL), dried over anhydrous sodium sulfate, concentrated and purified by column chromatography (dichloromethane:methanol=40:1) to obtain N-(6-(2-(2,4-difluorophenyl)-2-methylpropionyl)pyridin-3-yl)-2-(4-(ethylsulfonyl)phen yl)acetamide (**I-12**) (185 mg) as a white solid. LC-MS: m/z: $(M+H)^+$= 486.8. $^1H$ NMR (400 MHz, $CDCl_3$) δ 8.23 (d, J = 2.3 Hz, 1H), 8.07 (dd, J = 8.7, 2.4 Hz, 1H), 8.00 (d, J = 8.6 Hz, 1H), 7.87 (d, J = 8.3 Hz, 2H), 7.53 - 7.39 (m, 4H), 6.86 (td, J = 8.1, 1.8 Hz, 1H), 6.51 (ddd, J = 11.3, 8.7, 2.6 Hz, 1H), 3.80 (s, 2H), 3.11 (q, J = 7.4 Hz, 2H), 1.69 (s, 6H), 1.28 (dd, J = 8.6, 6.3 Hz, 3H).

**Example 13**

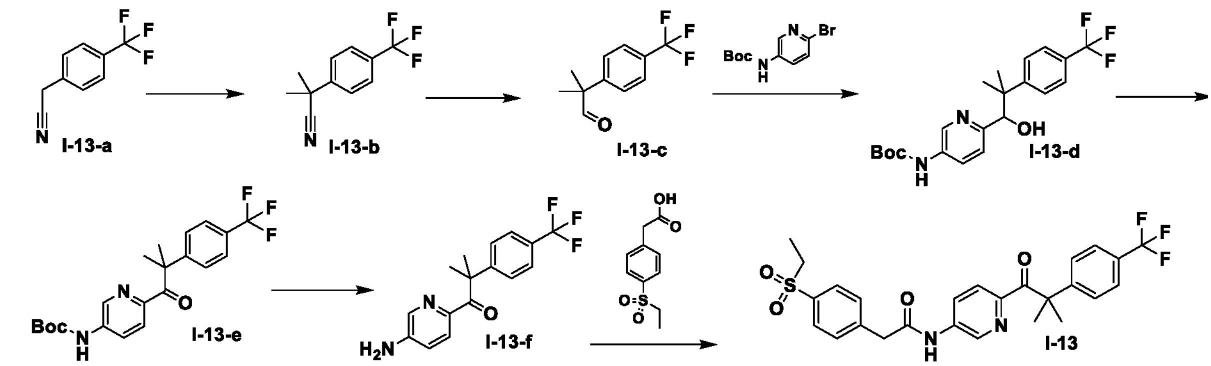

I-13-a I-13-b I-13-c I-13-d I-13-e I-13-f I-13

**Step 1:**

2-(4-(trifluoromethyl)phenyl)acetonitrile (**I-13-a**) (1.85g, 10mmol) and iodomethane (4.32g, 30.4mmol) were added to 80ml of tetrahydrofuran, slowly added with sodium tert-butoxide (3.36g, 30mmol) in an ice bath, and stirred overnight at room temperature. The reaction solution was quenched with saturated brine and extracted once with ethyl acetate, and the organic phase was washed twice with saturated brine, dried over anhydrous sodium sulfate, and concentrated to obtain 1.8g of a colorless oil (**I-13-b**) with a yield of 84%, which was directly used in the next step. $^1H$ NMR (400 MHz, $CDCl_3$) δ 7.69 (d, *J* = 8.4 Hz, 2H), 7.63 (d, *J* = 8.4 Hz, 2H), 1.78 (s, 6H).

**Step 2:**

2-methyl-2-(4-(trifluoromethyl)phenyl)propionitrile (**I-13-b**) (2.05g, 9.62mmol) was added to 50ml of tetrahydrofuran, slowly added with disobutyl aluminum hydride (29ml, 29mmol, 1 mol/L) at -78 °C, stirred at this temperature for 2h, and then at room temperature overnight. The reaction solution was quenched with 2mol/L hydrochloric acid, extracted twice with ethyl acetate (2×20ml), dried over anhydrous sodium sulfate, concentrated and passed through a column (ethyl acetate:petroleum ether=0-10%) to obtain 1.68g of a colorless oil (**I-13-c**) with a yield of 80.8%. $^1H$ NMR (400 MHz, $CDCl_3$) δ 9.55 (s, 1H), 7.68 - 7.65 (m, 2H), 7.42 - 7.29 (m, 2H), 1.52 (s, 6H).

**Step 3:**

Tert-butyl (6-bromopyridin-3-yl)carbamate (0.874g, 3.2mmol) was added to 10ml of anhydrous tetrahydrofuran, slowly added with n-butyllithium (4.2ml, 6.7mmol) at -78 °C and stirred at this temperature for 1h, and then slowly added with a solution of 2-methyl-2-(4-(trifluoromethyl)phenyl)propanal (**I-13-c**) (0.7g, 3.24mmol) in 10ml of tetrahydrofuran. The reaction solution was stirred at -78 °C for 1 h, slowly raised to room temperature and stirred overnight. The reaction liquid was quenched with 5 ml of saturated aqueous $NH_4Cl$, and extracted with ethyl acetate. The organic phase was washed once with saturated brine, dried over anhydrous sodium sulfate, concentrated and passed through a column (ethyl acetate:petroleum ether=0-50%) to obtain 0.64g of a white solid (**I-13-d**) with a yield of 48%. $^1H$ NMR (400 MHz, $CDCl_3$) δ 8.34 (d, *J* = 2.5 Hz, 1H), 7.72 (d, *J* = 7.6 Hz, 1H), 7.56 (d, *J* = 8.3 Hz, 2H), 7.40 (d, *J* = 8.2 Hz, 2H), 6.50 (s, 1H), 6.44 (d, *J* = 8.5 Hz, 1H), 4.79 (d, *J* = 6.4 Hz, 1H), 4.43 (d, *J* = 6.7 Hz, 1H), 1.59 (s, 4H), 1.54 (s, 9H), 1.46 (s, 2H).

**Step 4:**

Tert-butyl (6-(1-hydroxyl-2-methyl-2-(4-(trifluoromethyl)phenyl)propyl)pyridin-3-yl)carbamate (**I-13-d**)(0.63g, 1.53mmol) was added to 20ml of ethyl acetate, added with IBX (2g, 3.28mmol), and stirred overnight at reflux temperature. The reaction solution was filtered and concentrated to obtain 0.6g of a white solid (**I-13-e**) with a yield of 95%, which was directly used in the next step. LC-MS: m/z: $(M+H)^+$ =409.

**Step 5:**

Tert-butyl (6-(2-methyl-2-(4-(trifluoromethyl)phenyl)propionyl)pyridin-3-yl)carbamate (**I-13-e**) (600 mg, 1.47mmol) was added to 3ml of dichloromethane, added with 3ml of trifluoroacetic acid, and stirred at room temperature for 1h. The reaction solution was concentrated, added with 10ml of saturated sodium bicarbonate, extracted with dichloromethane (2×20ml), dried over anhydrous sodium sulfate, concentrated and passed through a column (ethyl acetate:petroleum ether=0-40%) to obtain 420 mg of a white solid (**I-13-f**) with a yield of 92%. LC-MS: m/z: $(M+H)^+$ =309.

**Step 6:**

1-(5-aminopyridin-2-yl)-2-(4-fluorophenyl)-2-methylpropane-1-one (**I-13-f**) (0.155g, 0.5mmol), 2-(4-(ethylsulfonyl)phenyl)acetic acid (116mg, 0.508mmol) and triethylamine (0.25ml, 1.8mmol) were added to 20ml of dichloromethane, added with TBTU (195mg, 0.6mmol), and stirred at room temperature overnight. The reaction solution was concentrated and passed through a column (ethyl acetate:petroleum ether=0-50%) to obtain 0.185 g of a white solid (**I-13**) with a yield of 71%. LC-MS: m/z: $(M+H)^+$ =519, $^1H$ NMR (400 MHz, $CDCl_3$) δ 8.37 (d, *J* = 2.2 Hz, 1H), 8.18 (dd, *J* = 8.7, 2.4 Hz, 1H), 7.99 (d, *J* = 8.7 Hz, 2H), 7.81 (d, *J* = 8.3 Hz, 2H), 7.49 (t, *J* = 8.9 Hz, 4H), 7.36 (d, *J* = 8.2 Hz, 2H), 3.82 (s, 2H), 3.12 (q, *J* = 7.4 Hz, 2H), 1.74 (s, 6H), 1.28 (t, *J* = 7.4 Hz, 3H).

**Example 14**

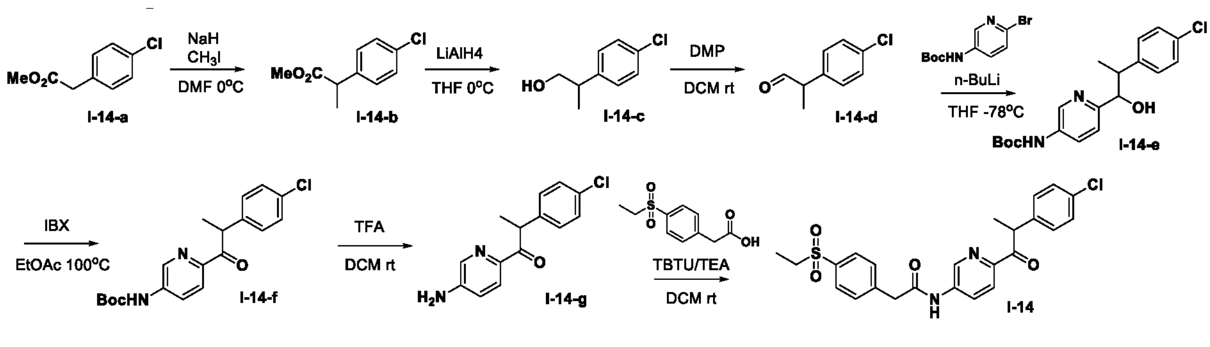


**Step 1:**

60% NaH (216 mg, 5.40 mmol) was put in anhydrous DMF (10 mL), cooled to 0 °C under nitrogen protection, added with methyl 2-(4-chlorophenyl)acetate (**I-14-a**) (1.0 g, 5.42 mmol), stirred for 5min at 0°C, added with iodomethane (769 mg, 5.42 mmol), stirred overnight and gradually raised to room temperature. After the reaction was complete, water

(10 mL) and ethyl acetate (10 mL) were added, the organic phase was separated, and the aqueous phase was extracted with ethyl acetate (10 mL×2). The organic phases were combined, washed with saturated sodium chloride solution (10 mL×2) and water (10 mL), dried over anhydrous sodium sulfate, concentrated and purified by column chromatography (petroleum ether: ethyl acetate = 10:1) to obtain methyl 2-(4-chlorophenyl)propionate (**I-14-b**) (870 mg) as a yellow oil. LC-MS: m/z: $(M+H)^+$= 199.1. $^1H$ NMR (400 MHz, $CDCl_3$) $\delta$ 7.31 (dd, J = 8.7, 2.1 Hz, 2H), 7.27 (t, J = 8.2 Hz, 2H), 3.72 (d, J = 7.2 Hz, 1H), 3.69 (s, 3H), 1.51 (d, J = 7.2 Hz, 3H).

**Step 2:**

1 mol/L lithium aluminum hydride (8.8 mL, 8.78 mmol) was put in anhydrous tetrahydrofuran (10 mL), cooled to 0 °C under nitrogen protection, added with ethyl 2-(4-chlorophenyl)propionate (**I-14-b**) (870 mg, 4.39 mmol), stirred overnight and gradually warmed to room temperature. After the reaction was complete, the reaction mixture was quenched with saturated ammonium chloride, and added with water (10 mL) and ethyl acetate (10 mL), and the organic phase was separated, the aqueous phase was extracted with ethyl acetate (10 mL×2). The organic phases were combined, washed with saturated sodium chloride solution (10 mL×2) and water (10 mL), dried over anhydrous sodium sulfate, concentrated and purified by column chromatography (petroleum ether: ethyl acetate = 10:1) to obtain 2-(4-chlorophenyl)propan-1-ol (**I-14-c**) (730 mg) as a yellow oil. $^1H$ NMR (400 MHz, $CDCl_3$) $\delta$ 7.32 (d, J = 8.6 Hz, 2H), 7.20 (d, J = 8.4 Hz, 2H), 3.70 (d, J = 7.1 Hz, 2H), 2.95 (dd, J = 13.8, 6.9 Hz, 1H), 1.28 (d, J = 7.0 Hz, 3H).

**Step 3:**

2-(4-chlorophenyl)propan-1-ol (**I-14-c**) (730 mL, 4.28 mmol) was put in dichloromethane (10 mL), cooled to 0 °C, added with Dess-Martin oxidizing agent (465 mg, 4.30 mmol), stirred overnight and gradually warmed to room temperature. After the reaction was complete, water (10 mL) and ethyl acetate (10 mL) were added, the organic phase was separated, and the aqueous phase was extracted with ethyl acetate (10 mL×2). The organic phases were combined, washed with saturated sodium chloride solution (10 mL×2) and water (10 mL), dried over anhydrous sodium sulfate, concentrated and purified by column chromatography (petroleum ether: ethyl acetate = 10:1) to obtain 2-(4-chlorophenyl)propanal (**I-14-d**) (505 mg) as a yellow oil. $^1H$ NMR (400 MHz, $CDCl_3$) $\delta$ 9.68 (d, J = 1.4 Hz, 1H), 7.40 - 7.35 (m, 2H), 7.22 - 7.12 (m, 2H), 3.64 (q, J = 6.8 Hz, 1H), 1.52 - 1.40 (m, 5H).

**Step 4:**

Tert-butyl (6-bromopyridin-3-yl)carbamate (743 mg, 2.72 mmol) was put in anhydrous tetrahydrofuran (10 mL), cooled to -78 °C under nitrogen protection, and added with n-butyllithium (3.6 mL, 5.71 mmol, 1.6 mol/L). After the reaction solution became turbid, it was stirred for 1h, added with 2-(4-chlorophenyl)propanal (**I-14-d**) (505 mg, 3.00 mmol), stirred overnight and gradually raised to room temperature. After the reaction was complete, water (10 mL) and ethyl acetate (10 mL) were added, the organic phase was separated, and the aqueous phase was extracted with ethyl acetate (10 mL×2). The organic phases were combined, washed with saturated sodium chloride solution (10 mL×2) and water (10 mL), dried over anhydrous sodium sulfate, concentrated and purified by column chromatography (petroleum ether: ethyl acetate = 10:1) to obtain tert-butyl (6-(2-(4-(chlorophenyl)-1-hydroxypropyl)pyridin-3-yl)carbamate (**I-14-e**) (260 mg) as a yellow oil. LC-MS: m/z: $(M+H)^+$= 363.1. $^1H$ NMR (400 MHz, $CDCl_3$) $\delta$ 8.45 (d, J = 2.5 Hz, 1H), 8.40 (d, J = 2.4 Hz, 1H), 8.30 (dd, J = 4.7, 1.3 Hz, 1H), 7.25 (d, J = 8.4 Hz, 2H), 7.14 (d, J = 8.4 Hz, 2H), 6.92 (d, J = 8.5 Hz, 1H), 4.75 (s, 1H), 3.13 - 3.04 (m, 1H), 1.55 (s, 7H), 1.25 (d, J = 7.1 Hz, 3H).

**Step 5:**

Tert-butyl (6-(2-(4-(chlorophenyl)-1-hydroxypropyl)pyridin-3-yl)carbamate (**I-14-e**) (260 mg, 0.72 mmol) was put in ethyl acetate (10 mL), added wtih 2-iodoxybenzoic acid (602 mg, 2.15 mmol), and stirred overnight at 100°C. After the reaction was complete, the reaction mixture was filtered, and the filtrate was concentrated and purified by column chromatography (petroleum ether: ethyl acetate = 10:1) to obtain tert-butyl (6-(2-(4-(chlorophenyl)propionyl)pyridin-3-yl)carbamate (**I-14-f**) (180 mg) as a yellow solid. LC-MS: m/z: $(M+H)^+$= 361.1.

**Step 6:**

Tert-butyl (6-(2-(4-(chlorophenyl)propionyl)pyridin-3-yl)carbamate (**I-14-f**) (180 mg, 1.86 mmol) was put in dichloromethane (5 mL), added with trifluoroacetic acid (5 mL) and stirred at room temperature for 2h. After the reaction was complete, the raction mixture was concentrated, added with ethyl acetate (10 mL), washed with saturated sodium bicarbonate solution (10 mL×2) and water (10 mL), and dried over anhydrous sodium sulfate. 1-(5-aminopyridin-2-yl)-2-(4-chlorophenyl)propan-1-one (**I-14-g**) (130 mg) was obtained as a yellow solid. LC-MS: m/z: $(M+H)^+$= 261.1. $^1$H NMR (400 MHz, $CDCl_3$) δ 8.05 (d, J = 2.6 Hz, 1H), 7.90 (d, J = 8.5 Hz, 1H), 7.39 - 7.31 (m, 2H), 7.27 - 7.21 (m, 2H), 6.96 (dd, J = 8.5, 2.8 Hz, 1H), 5.41 (q, J = 7.1 Hz, 1H), 1.52 (d, J = 7.1 Hz, 3H).

**Step 7:**

1-(5-aminopyridin-2-yl)-2-(4-chlorophenyl)propan-1-one (**I-14-g**) (130 mg, 0.50 mmol) was put in dichloromethane (10 mL), added with 2-(4-(ethylsulfonyl)phenyl)acetic acid (130 mg, 0.57 mmol), TBTU (184 mg, 0.57 mmol) and triethylamine (0.2 mL), and stirred overnight. After the reaction was complete, water (10 mL) and dichloromethane (10 mL) were added, the organic phase was separated, and the aqueous phase was extracted with dichloromethane (10 mL×2). The organic phases were combined, washed with saturated sodium chloride solution (10 mL×2) and water (10 mL), dried over anhydrous sodium sulfate, concentrated and purified by column chromatography (petroleum ether: ethyl acetate = 1:1) to obtain N-(6-(2-(4-chlorophenyl)propionyl)pyridin-3-yl)-2-(4-(ethylsulfonyl)phenyl)acetamide (**I-14**) (55 mg) as a yellow solid. LC-MS: m/z: $(M+H)^+$= 471.1. $^1$H NMR (400 MHz, DMSO-$d_6$) δ 10.84 (s, 1H), 8.87 (d, J = 2.2 Hz, 1H), 8.19 (dd, J = 8.6, 2.4 Hz, 1H), 7.97 (d, J = 8.6 Hz, 1H), 7.86 (d, J = 8.3 Hz, 2H), 7.61 (d, J = 8.2 Hz, 2H), 7.32 (s, 3H), 5.45 - 5.28 (m, 1H), 3.90 (s, 2H), 3.28 (d, J = 7.4 Hz, 2H), 1.41 (d, J = 7.0 Hz, 3H), 1.10 (t, J = 7.3 Hz, 3H).

**Example 19**

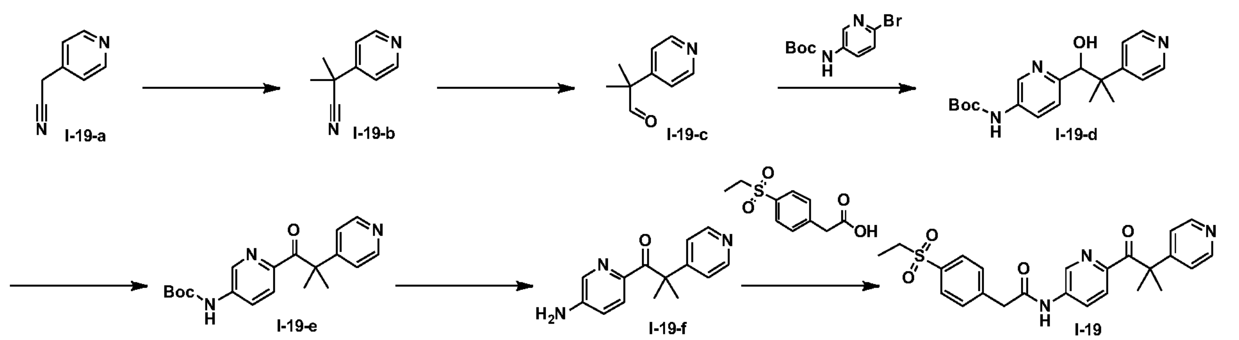

**Step 1:**

2-(pyridin-4-yl)acetonitrile (**I-19-a**) (0.9 g, 7.6 mmol) and iodomethane (3.5 g, 24.6 mmol) were added to 40 ml of tetrahydrofuran, slowly added with sodium tert-butoxide (2.7 g, 24.1 mmol) in an ice bath, and stirred overnight at room temperature. The reaction solution was quenched with saturated brine and extracted once with ethyl acetate. The organic phase was washed twice with saturated brine, dried over anhydrous sodium sulfate, concentrated and passed through a column (ethyl acetate:petroleum ether=0-70%) to obtain 1 g of 2-methyl-2-(pyridin-4-yl)propionitrile (**I-19-b**) as a brown oil with a yield of 90%. $^1$HNMR (400 MHz, $CDCl_3$) δ 8.66 (dd, J = 4.6, 1.6 Hz, 2 H), 7.42 (dd, J = 4.5, 1.7 Hz, 2 H), 1.74 (s, 6 H).

**Step 2:**

2-methyl-2-(pyridin-4-yl)propionitrile (**I-19-b**) (1.2 g, 8.2 mmol) was added to 40 ml of tetrahydrofuran, slowly added with diisobutyl aluminum hydride (20.5 ml, 20.5 mmol) at -78 °C, stirred at this temperature for 2 h and thenat room temperature overnight. The reaction solution was quenched with 2 mol/L hydrochloric acid, and extracted twice with ethyl acetate (3×30 ml). The organic phase was dried over anhydrous sodium sulfate, concentrated and separated by column chromatography (ethyl acetate:petroleum ether=0-60%) to obtain 0.25 g of 2-methyl-2-(pyridin-4-yl)propanal **(I-19-c)** as a brown oil with a yield of 20%. $^1$H NMR (400 MHz, $CDCl_3$) δ 9.55 (s, 1 H), 8.63 (dd, J = 4.6, 1.7 Hz, 2 H), 7.23 (dd, J = 4.6, 1.7 Hz, 2 H), 1.50 (s, 6 H).

**Step 3:**

Tert-butyl (6-bromopyridin-3-yl)carbamate (0.45 g, 1.6 mmol) was added to 6 ml of tetrahydrofuran, slowly added with n-butyllithium (2.1 ml, 3.4 mmol) -78 °C, stirred at this temperature for 1 h, slowly added with a solution of 2-methyl-2-(pyridin-4-yl)propanal **(I-19-c)** (0.24 g, 1.6 mmol) in 5 ml tetrahydrofuran, stirred at -78°C for 1 h, slowly raised

to room temperature and stirred overnight. The reaction solution was quenched with 5 ml of saturated aqueous $NH_4Cl$, and extracted with ethyl acetate. The organic phase was washed once with saturated brine, dried over anhydrous sodium sulfate, concentrated and passed through a column (dichloromethane:methanol=0-10%) to obtain 0.24 g of a white solid (**I-19-d**) with a yield of 42%. LC-MS: m/z: $(M+H)^+$=344.

**Step 4:**

Tert-butyl (6-(1-hydroxy-2-methyl-2-(pyridin-4-yl)propyl)pyridin-3-yl)carbamate (**I-19-d**) (0.24 g, 0.7 mmol) was added to 20 ml of ethyl acetate, added with IBX (0.9 g, 1.5 mmol), and stirred overnight at reflux temperature. The reaction solution was filtered, concentrated and passed through a column (dichloromethane:methanol=0-10%) to obtain 0.2 g of a white solid (**I-19-e**) with a yield of 83%. LC-MS: m/z: $(M+H)^+$ =342.

**Step 5:**

Tert-butyl (6-(2-methyl-2-(pyridin-4-yl)propionyl)pyridin-3-yl)carbamate (**I-19-e**) (200 mg, 0.58 mmol) was added to 3 ml of dichloromethane, added with 3 ml of trifluoroacetic acid, and stirred at room temperature for 1h. The reaction solution was concentrated, added with 10 ml of saturated sodium bicarbonate, extracted with dichloromethane (2×20 ml), dried over anhydrous sodium sulfate, concentrated and passed through a column (ethyl acetate:petroleum ether = 0-40%) to obtain 85 mg of a white solid (**I-19-f**) with a yield of 60%. LC-MS: m/z: $(M+H)^+$ =242.

**Step 6:**

1-(5-aminopyridin-2-yl)-2-methyl-2-(pyridin-4-yl)propan-1-one (85 mg, 0.35 mmol) (**I-19-f**), 2-(4-(ethylsulfonyl)phenyl)acetic acid (90 mg, 0.39 mmol) and triethylamine (0.25 ml, 1.8 mmol) were added to 20 ml of dichloromethane, added with TBTU (125 mg, 0.39 mmol), and stirred overnight at room temperature. The reaction solution was concentrated and passed through a column (dichloromethane:methanol=0-10%) to obtain 42 mg of a white solid (**I-19**) with a yield of 26%. LC-MS: m/z: $(M+H)^+$ =452, $^1H$ NMR (400 MHz, DMSO-$d_6$) δ 10.71 (s, 1 H), 8.48 (d, J = 2.3 Hz, 1 H), 8.41 (dd, J = 4.7, 1.4 Hz, 2 H), 8.14 (dd, J = 8.7, 2.5 Hz, 1 H), 8.00 (d, J = 8.7 Hz, 1 H), 7.83 (d, J = 8.3 Hz, 2 H), 7.58 (d, J = 8.3 Hz, 2 H), 7.18 (dd, J = 4.6, 1.6 Hz, 2 H), 3.85 (s, 2 H), 3.27 (q, J = 7.3 Hz, 2 H), 1.63 (s, 6 H), 1.09 (t, J = 7.4 Hz, 3 H).

**Example 20**

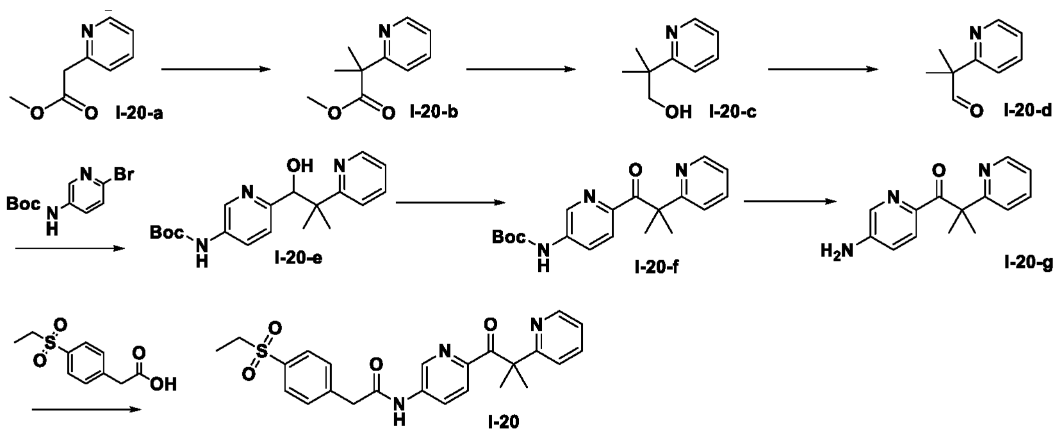
I-20-a
I-20-b
I-20-c
I-20-d
I-20-e
I-20-f
I-20-g
I-20

**Step 1:**

Methyl 2-(pyridin-2-yl)acetate (**I-20-a**) (3.05 g, 20.2 mmol) and iodomethane (11.5 g, 81 mmol) were added to 20 ml of tetrahydrofuran, slowly added with a solution of lithium bistrimethylsilylamide in tetrahydrofuran (50 ml, 50 mmol, 1 mol/L) in an ice bath, and stirred overnight at room temperature. The reaction solution was quenched with saturated brine, and extracted twice with ethyl acetate, and the organic phase was washed twice with saturated brine, dried over anhydrous sodium sulfate, concentrated and separated by column chromatography (ethyl acetate:petroleum ether = 0-30%) to obtain 3.3 g of a brown oil (**I-20-b**) with a yield of 91%. LC-MS: m/z: $(M+H)^+$ =180, $^1H$ NMR (400 MHz, $CDCl_3$) δ 8.57 (ddd, J = 4.8, 1.8, 0.9 Hz, 1 H), 7.66 (td, J = 7.8, 1.9 Hz, 1 H), 7.30 (dt, J = 8.0, 1.0 Hz, 1 H), 7.16 (ddd, J = 7.5,

4.8, 1.1 Hz, 1 H), 3.70 (d, J = 2.8 Hz, 3 H), 1.63 (s, 6H).

**Step 2:**

Methyl 2-methyl-2-(pyridin-2-yl)propionate (**I-20-b**) (4.4 g, 25 mmol) was added into 25 ml of tetrahydrofuran, slowly added with a solution of lithium aluminum tetrahydride in tetrahydrofuran (27 ml, 27 mmol, 1 mol/L) at 0 °C, and stirred at this temperature for 1h. The reaction solution was quenched with saturated aqueous ammonium chloride, extracted twice with ethyl acetate (3×30 ml), dried over anhydrous sodium sulfate and concentrated to obtain 3.4 g of a colorless oil (**I-20-c**) with a yield of 92%. LC-MS: m/z: $(M+H)^+$ =152.

**Step 3:**

2-methyl-2-(pyridin-2-yl)propan-1-ol (**I-20-c**) (3 g, 19.8 mmol) was added to 160 ml of dichloromethane, added with Dess-Martin oxidant (17 g, 40 mmol) in an ice bath, and stirred at room temperature for 1h. The reaction solution was quenched with saturated aqueous sodium bicarbonate and 80 mmol of aqueous sodium thiosulfate, and the aqueous phase was extracted twice with dichloromethane. The combined organic phase was dried over anhydrous sodium sulfate and passed through a column (ethyl acetate: petroleum ether = 0-50%) to obtain 2.6 g of a colorless oil (**I-20-d**) with a yield of 88%. LC-MS: m/z: $(M+H)^+$ =150. $^1$H NMR (400 MHz, $CDCl_3$) δ 9.79 (s, 1 H), 8.62 (ddd, J = 4.8, 1.8, 0.9 Hz, 1 H), 7.72 (td, J = 7.8, 1.9 Hz, 1 H), 7.31 (dt, J = 8.0, 1.0 Hz, 1 H), 7.21 (ddd, J = 7.5, 4.9, 1.1 Hz, 1 H), 1.52 (d, J = 2.1 Hz, 7 H).

**Step 4:**

Tert-butyl (6-bromopyridin-3-yl)carbamate (2.73 g, 10 mmol) was added to 30 ml of tetrahydrofuran, slowly added with n-butyllithium (13.2 ml, 21 mmol) at -78 °C, stirred at this temperature for 0.5 h, slowly added with a solution of 2-methyl-2-(pyridin-2-yl)propanal (**I-20-d**) (1.49 g, 10 mmol) in 5 ml tetrahydrofuran. After stirred at -78 °C for 1 h, the reaction solution was quenched with 20 ml of saturated aqueous $NH_4Cl$ and extracted with ethyl acetate. The organic phase was washed once with saturated brine, dried over anhydrous sodium sulfate, concentrated and passed through a column (dichloromethane:methanol = 0-10%), to obtain 1.6 g of a white solid (**I-20-e**) with a yield of 47%. LC-MS: m/z: $(M+H)^+$ =344.

**Step 5:**

Tert-butyl (6-(1-hydroxy-2-methyl-2-(pyridin-2-yl)propyl)pyridin-3-yl)carbamate (**I-20-e**) (2.6 g, 7.6 mmol) was added to 100 ml of ethyl acetate, added with IBX (4.2 g, 15 mmol), and stirred overnight at reflux temperature. The reaction solution was filtered, concentrated and passed through a column (ethyl acetate: petroleum ether=0-50%) to obtain 2.53 g of a white solid (**I-20-f**) with a yield of 98%. LC-MS: m/z: $(M+H)^+$ =342.

**Step 6:**

Tert-butyl (6-(2-methyl-2-(pyridin-2-yl)propionyl)pyridin-3-yl)carbamate (**I-20-f**) (2.53 g, 7.41 mmol) was added to 3 ml of dichloromethane, added with 3 ml of trifluoroacetic acid, and stirred at room temperature for 1h. The reaction solution was concentrated, added with 10 ml of saturated sodium bicarbonate, extracted with dichloromethane (2×20 ml), dried over anhydrous sodium sulfate, concentrated and passed through a column (ethyl acetate:petroleum ether=0-40%) to obtain 1.73 g of a white solid (**I-20-g**) with a yield of 96%. LC-MS: m/z: $(M+H)^+$=242.

**Step 7:**

1-(5-aminopyridin-2-yl)-2-methyl-2-(pyridin-2-yl)propan-1-one (**I-20-g**) (1.66 g, 6.88 mmol), 2-(4-(ethylsulfonyl)phenyl)acetic acid (2 g, 8.76 mmol) and N,N-diisopropylethylamine (4.7 ml, 27 mmol) were added to 120 ml of dichloromethane, added with a 50% solution of T3P (9 g, 14 mmol) in ethyl acetate, and stirred at room temperature for 0.5h. The reaction solution was concentrated and passed through a column (dichloromethane:methanol=0-15%) to obtain 2.9 g of 2-(4-(ethylsulfonyl)phenyl)-N-(6-(2-methyl-2-(pyridin-2-yl)propanoyl)pyridin-3-yl)acet amide (I-20) as a white solid with a yield of 93%. LC-MS: m/z: $(M+H)^+$ =452, $^1$H NMR (400 MHz, $CDCl_3$) δ 9.80 (s, 1 H), 8.47 (d, *J* = 2.4 Hz, 1 H), 8.28 (dd, *J* = 4.9, 0.9 Hz, 1 H), 7.88 (d, *J* = 8.7 Hz, 1 H), 7.83 (d, *J* = 8.3 Hz, 2 H), 7.69 (td, *J* = 7.8, 1.8 Hz, 1 H), 7.61 (dd, *J* = 8.7, 2.4 Hz, 1 H), 7.53 (d, *J* = 8.3 Hz, 2 H), 7.34 (d, *J* = 8.0 Hz, 1 H), 7.16 - 7.08 (m, 1 H), 3.88 (s, 2 H), 3.10 (q, *J* = 7.4 Hz, 2 H), 1.73 (s, 6 H), 1.27 (t, *J* = 7.4 Hz, 3 H).

## Example 23

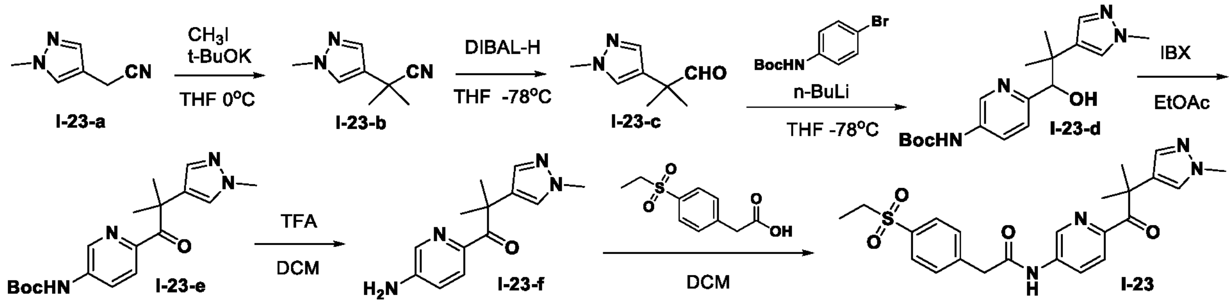


### Step 1:

2-(1-methyl-1H-pyrazol-4-yl)acetonitrile (500 mg, 4.13 mmol) (**I-23-a**), and iodomethane (0.8 mL, 12.7 mmol) were put in anhydrous THF (5 mL), cooled to 0 °C under nitrogen protection, added with potassium tert-butoxide (1.4 g, 12.5 mmol), stirred overnight and gradually raised to room temperature. After the reaction was complete, water (5 mL) and ethyl acetate (5 mL) were added, the organic phase was separated, and the aqueous phase was extracted with ethyl acetate (5 mL×2). The organic phases were combined, washed with saturated sodium chloride solution (5 mL×2) and water (5 mL), dried over anhydrous sodium sulfate, concentrated and purified by column chromatography (petroleum ether: ethyl acetate = 1:1) to obtain 2-methyl-2-(1-methyl-1H-pyrazol-4-yl)propionitrile (**I-23-b**) (460 mg) as a yellow oil. LC-MS: m/z: $(M+H)^+$= 150.1. $^1H$ NMR (400 MHz, $CDCl_3$) $\delta$7.47 (s, 1 H), 7.39 (s, 1 H), 3.91 (s, 3 H), 1.69 (s, 6H).

### Step 2:

2-methyl-2-(1-methyl-1H-pyrazol-4-yl)propionitrile (**I-23-b**) (460 mg, 3.08 mmol) was put in anhydrous tetrahydrofuran (5 mL), cooled to -78 °C under nitrogen protection, added with diisobutylaluminum hydride (9.2 mL, 9.2 mmol, 1 mol/L), stirred overnight and gradually raised to room temperature. After the reaction was complete, the reaction mixture was cooled to 0 °C, and water (5 mL) and ethyl acetate (5 mL) were added. The organic phase was separated, and the aqueous phase was extracted with ethyl acetate (5 mL×2). The organic phases were combined, washed with saturated sodium chloride solution (5 mL×2) and water (5 mL mL), dried over anhydrous sodium sulfate, concentrated and purified by column chromatography (petroleum ether: ethyl acetate = 1:1) to obtain 2-methyl-2-(1-methyl-1H-pyrazole-4-yl)propanal (**I-23-c**) (470 mg), as a yellow oil. LC-MS: m/z: $(M+H)^+$= 153.1. $^1H$ NMR (400 MHz, $CDCl_3$) $\delta$9.44 (s, 1 H), 7.40 (s, 1 H), 7.35 (s, 1 H), 3.91 (s, 3 H), 1.42 (s, 6 H).

### Step 3:

Tert-butyl (6-bromopyridin-3-yl)carbamate (762 mg, 2.79 mmol) was put in anhydrous tetrahydrofuran (3 mL), cooled to -78 °C under nitrogen protection, and added with n-butyllithium (3.6 mL, 5.86 mmol, 1.6 mol/L). After the reaction solution became turbid, it was stirred for 1h, added with 2-methyl-2-(1-methyl-1H-pyrazol-4-yl)propanal (**I-23 -c**) (470 mg, 3.07 mmol), stirred overnight and gradually warmed to room temperature. After the reaction was complete, water (5 mL) and ethyl acetate (5 mL) were added, the organic phase was separated, and the aqueous phase was extracted with ethyl acetate (5 mL×2). The organic phases were combined, washed with saturated sodium chloride solution (5 mL×2) and water (5 mL), dried over anhydrous sodium sulfate, concentrated and purified by column chromatography (dichloromethane: methanol=20:1) to obtain tert-butyl (6-(1-hydroxyl-2-methyl-2-(1-methyl-1H-pyrazol-4-yl)propyl)pyridin-3-yl)carbamate (**I-23-d**) (126 mg) as a yellow solid. LC-MS: m/z: $(M+H)^+$= 347.3.

### Step 4:

Tert-butyl (6-(1-hydroxyl-2-methyl-2-(1-methyl-1H-pyrazol-4-yl)propyl)pyridin-3-yl)carbamate (**I-23-d**) (126 mg, 0.36 mmol) was put in ethyl acetate (5 mL), added with 2-iodoxybenzoic acid (306 mg, 1.09 mmol), and stirred at 100 °C overnight. After the reaction was complete, the reaction mixture was filtered, and the filtrate was concentrated and purified by column chromatography (petroleum ether: ethyl acetate = 1:1) to obtain tert-butyl (6-(2-methyl-2-(1-methyl-1H-pyrazol-4-yl)propionyl)pyridin-3-yl)carbamate (**I-23-e**) (106 mg) as a yellow solid. LC-MS: m/z: $(M+H)^+$= 345.3. $^1H$ NMR (400 MHz, $CDCl_3$) $\delta$8.40 (d, J = 2.3 Hz, 1 H), 7.93 (dt, J = 15.8, 5.5 Hz, 2 H), 7.46 (s, 1 H), 7.27 (s, 1 H), 6.70 (s,

1 H), 3.84 (s, 3 H), 1.77 (s, 6 H), 1.54 (s, 9 H).

**Step 5:**

Tert-butyl (6-(2-methyl-2-(1-methyl-1H-pyrazol-4-yl)propionyl)pyridin-3-yl)carbamate (**I-23-e**) (106 mg, 0.31 mmol) was put in dichloromethane (5 mL), added with trifluoroacetic acid (5 mL) and stirred at room temperature for 2h. After the reaction was complete, the reaction mixture was concentrated, added with ethyl acetate (5 mL), washed with saturated sodium bicarbonate solution (5 mL×2) and water (5 mL), and dried over anhydrous sodium sulfate. 1-(5-aminopyridin-2-yl)-2-methyl-2-(1-methyl-1H-pyrazol-4-yl)propan-1-one (**I-23-f**) (80 mg) was obtained as a yellow solid. LC-MS: m/z: $(M+H)^+$= 245.2. $^1$H NMR (400 MHz, $CDCl_3$) $\delta$ 7.96 (d, J = 2.8 Hz, 1 H), 7.82 (d, J = 8.6 Hz, 1 H), 7.47 (s, 1 H), 7.26 (s, 1 H), 6.91 (dd, J = 8.6, 2.8 Hz, 1 H), 3.84 (s, 3 H), 1.76 (s, 6 H).

**Step 6:**

1-(5-aminopyridin-2-yl)-2-methyl-2-(1-methyl-1H-pyrazol-4-yl)propan-1-one (**I-23-f**) (80 mg, 0.32 mmol) was put in dichloromethane (5 mL), added with 2-(4-(ethylsulfonyl)phenyl)acetic acid (86 mg, 0.37 mmol), TBTU (121 mg, 0.37 mmol), and triethylamine (0.14 mL), and stirred overnight. After the reaction was complete, water (5 mL) and dichloromethane (5 mL) were added, the organic phase was separated, and the aqueous phase was extracted with dichloromethane (5 mL×2). The organic phases were combined, washed with saturated sodium chloride solution (5 mL×2) and water (5 mL), dried over anhydrous sodium sulfate, concentrated and purified by column chromatography (dichloromethane:methanol=20:1) to obtain 2-(4-(ethylsulfonyl)phenyl)-N-(6-(2-methyl-2-(1-methyl-1H-pyrazol-4-yl)propionyl)pyr idin-3-yl)acetamide (**I-23**) (98 mg) as a yellow solid. LC-MS: m/z: $(M+H)^+$= 455.2. $^1$H NMR (400 MHz, $CDCl_3$) $\delta$ 8.76 (s, 1 H), 8.55 (d, J = 2.0 Hz, 1 H), 8.22 (dd, J = 8.7, 2.0 Hz, 1 H), 7.88 (d, J = 8.3 Hz, 3 H), 7.54 (d, J = 8.2 Hz, 2 H), 7.49 (s, 1 H), 7.26 (s, 1 H), 3.88 (s, 2H), 3.83 (s, 3 H), 3.14 (q, J = 7.4 Hz, 2 H), 1.75 (s, 6 H), 1.31 (t, J = 7.4 Hz, 3 H).

**Example 24**

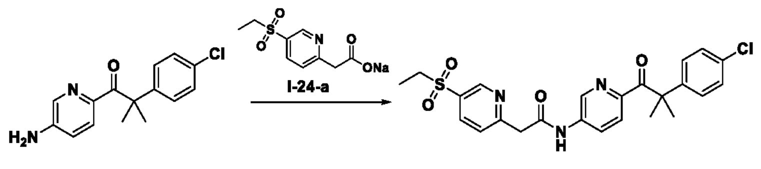


I-3-f I-24

**Step 1:**

1-(5-aminopyridin-2-yl)-2-(4-chlorophenyl)-2-methylpropan-1-one (**I-3-f**) (40 mg, 0.14 mmol), sodium 2-(5-(ethylsulfonyl)pyridin-2-yl)acetate (**I-24-a**) (40 mg, 0.16 mmol) and HATU (80 mg, 0.21 mmol) were added to 3 ml of pyridine, and stirred at room temperature overnight. The reaction solution was concentrated, and separated by thin layer chromatography {methanol:(ethyl acetate:dichloromethane=5:10)=1:15} to obtain 15 mg of N-(6-(2-(4-chlorophenyl)-2-methylpropionyl)pyridin-3-yl)-2-(5-(ethylsulfonyl)pyridin-2 -yl)acetamide (**I-24**) as a white solid with a yield of 21%. LC-MS: m/z: $(M+H)^+$ =486, $^1$H NMR (400 MHz, $CDCl_3$) δ 9.75 (s, 1 H), 9.09 (s, 1 H), 8.39 (d, J = 1.7 Hz, 1 H), 8.25 - 8.15 (m, 2 H), 7.99 (d, J = 8.6 Hz, 1 H), 7.55 (d, J = 8.1 Hz, 1 H), 7.25 - 7.15 (m, 4 H), 4.02 (s, 2 H), 3.19 (q, J = 7.4 Hz, 2 H), 1.72 (s, 6 H), 1.35 (t, J = 7.4 Hz, 3 H).

**Example 25**

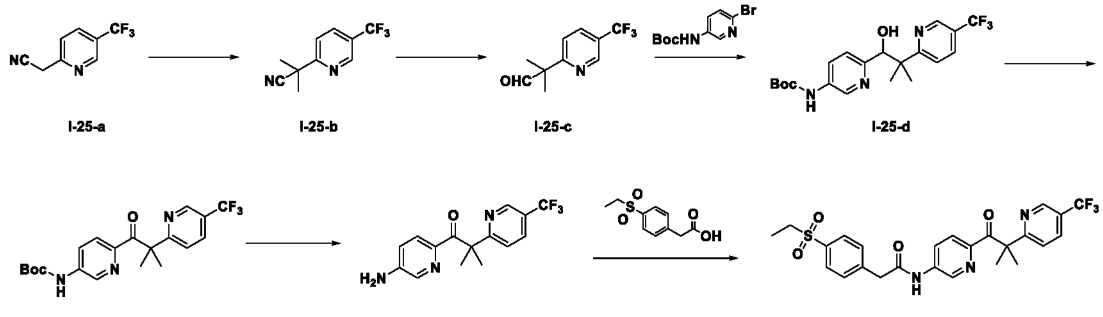

I-25-a I-25-b I-25-c I-25-d

I-25-e I-25-f I-25

**Step 1:**

2-(5-(trifluoromethyl)pyridin-2-yl)acetonitrile (**I-25-a**) (1 g, 5.37 mmol) and iodomethane (2.29 g, 16.12 mmol) were dissolved in 20 ml of THF, cooled to 0°C, added with sodium tert-butoxide (1.55 g, 16.12 mmol), heated to 15 °C and stirred for 16h. The reaction solution was quenched with saturated brine and extracted with ethyl acetate (20 mL), and the organic phase was washed with saturated brine, dried over anhydrous sodium sulfate, filtered and concentrated to obtain 1 g of a brown oil (**I-25-b**) with a yield of 96.55%. $^1$H NMR (400 MHz, $CDCl_3$) $\delta$ 8.94 - 8.86 (m, 1 H), 8.01 (dd, J = 8.3, 2.3 Hz, 1 H), 7.78 (d, J = 8.3 Hz,1 H), 1.81 (s, 6 H).

**Step 2:**

2-methyl-2-(5-(trifluoromethyl)pyridin-2-yl)propionitrile (**I-25-b**) (1 g, 4.67 mmol) was dissolved in 10 ml of tetrahydrofuran, cooled to -78 °C, slowly added dropwise with 12.7 mL of a solution of diisobutylaluminum hydride in toluene (1 mol/L) under the protection of nitrogen. The reaction solution was stirred at -78 °C for 1 h, then heated to 15 °C and stirred for 16h. The reaction solution was quenched with 1M aqueous hydrochloric acid and extracted with ethyl acetate (20 mL$\times$2). The combined organic layers were dried over anhydrous sodium sulfate, filtered and concentrated to obtain a crude, which was purified by column chromatography (ethyl acetate: petroleum ether = 0-60%) to obtain 100 mg of a yellow oil (**I-25-c**) with a yield of 9.8%. LC-MS: m/z: $(M+H)^+$ =218.0.

**Step 3:**

Tert-butyl N-(6-bromo-3-pyridyl)carbamate (125 mg, 0.46 mmol) was dissolved in 3 ml of anhydrous tetrahydrofuran. The system was replaced with nitrogen three times and cooled to -78 °C, and a solution (0.48 mL, 2.5 M) of n-butyllithium in n-hexane was slowly added. The reaction solution was stirred for 1h, and added with 2-methyl-2-(5-(trifluoromethyl)pyridin-2-yl)propanal (**I-25-c**) (100 mg, 0.46 mmol). The reaction solution was stirred at -78 °C for 2h, then warmed to 15 °C and stirred for 16h. The reaction solution was quenched with saturated aqueous ammonium chloride and extracted with ethyl acetate (20 mL$\times$2). The organic phase was dried over anhydrous sodium sulfate, filtered and concentrated, and purified by column chromatography (ethyl acetate:petroleum ether=0-60%) to obtain 30 mg of a white solid (**I-25-d**) with a yield of 15.8%. LC-MS: m/z: $(M+H)^+$ =412.0.

**Step 4:**

Tert-butyl (6-(1-hydroxyl-2-methyl-2-(5-(trifluoromethyl)pyridin-2-yl)propyl)pyridin-3-yl)carbam ate (**I-25-d**) (30 mg, 0.073 mmol) was dissolved in 5 ml of ethyl acetate, added with 31 mg of 2-iodoxybenzoic acid, refluxed and stirred for 2h. The reaction solution was filtered and concentrated to obtain 28 mg of a white solid (**I-25-e**) with a yield of 93.79%. LC-MS: m/z: $(M+H)^+$= 410.0.

**Step 5:**

Tert-butyl (6-(2-methyl-2-(thiazol-2-yl)propionyl)pyridin-3-yl)carbamate (**I-25-e**) (28 mg, 0.068 mmol) was dissolved in 2 ml of dichloromethane, added with 2 ml of trifluoroacetic acid, stirred at 15 °C for 1h, and concentrated. The residue was dissolved in 5 mL of dichloromethane, and neutralized with saturated aqueous ammonium bicarbonate till pH=9. The organic phase was dried over anhydrous sodium sulfate, filtered, and the filtrate was concentrated to obtain 20 mg of a white solid (**I-25-f**) with a yield of 94.55%. LC-MS: m/z: $(M+H)^+$=310.0.

**Step 6:**

1-(5-aminopyridin-2-yl)-2-methyl-2-(5-(trifluoromethyl)pyridin-2-yl)propan-1-one (**I-25-f**) (20 mg, 0.065 mmol) and 2-(4-ethylsulfonylphenyl)acetic acid (15 mg, 0.065 mmol) were dissolved in 2 ml of dichloromethane, added with 17 mg of N,N-diisopropylethylamine and a solution of 1-propylphosphoric anhydride(62 mg) in ethyl acetate (50%) successively, and stirred at 15 °C for 16h. After the reaction was complete, the system was concentrated, and the residue was purified by thin-layer chromatography (dichloromethane: methanol=10:1) to obtain 6 mg of 2-(4-(ethylsulfonyl)phenyl)-N-(6-(2-methyl-2-(5-(trifluoromethyl)pyridin-2-yl)propiony l)pyridin-3-yl)acetamide (**I-25**) as an off-white solid with a yield of 17.86%. LC-MS: m/z: $(M+H)^+$ = 520, $^1$H NMR (400 MHz, $CDCl_3$) δ 8.57 (s, 1 H), 8.34 (s, 1 H), 8.15 (s, 1 H), 8.05 (s, 2 H), 7.90 (dd, J = 8.4, 2.0Hz, 1 H), 7.83 (d, J = 8.1 Hz, 2 H), 7.51 (dd, J = 14.0, 8.3 Hz, 3 H), 3.83 (s, 2 H), 3.12 (q, J = 7.4 Hz, 2 H),1.75 (s, 6 H), 1.31 - 1.27 (m, 3 H).

**Example 26**

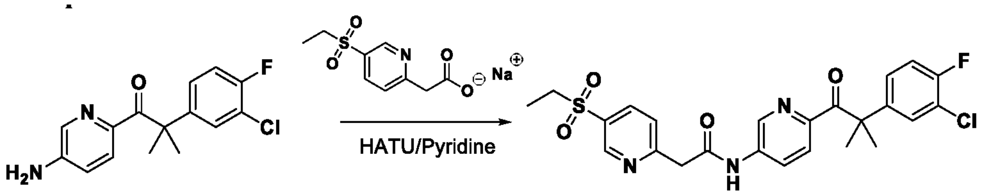


I-6-f I-26

1-(5-aminopyridin-2-yl)-2-(3-chloro-4-fluorophenyl)-2-methylpropan-1-one (**I-6-f**) (85 mg, 0.290 mmol) was dissolved in pyrine (5 mL), and sodium 2-(5-(ethylsulfonyl)pyridin-2-yl)acetate (43 mg, 0.171 mmol), 2-(7-aza benzotriazole)-N,N,N',N'-tetramethyluronium hexafluorophosphate (HATU) (220 mg, 0.579 mmol) were added successively, and the reaction system was stirred overnight at room temperature. After the reaction was complete, the reaction solution was concentrated to obtain a crude, which was purified by column chromatography (dichloromethane:methanol=30:1) to obtain N-(6-(2-(3-chloro-4-fluorophenyl)-2-methylpropionyl)pyridin-3-yl)-2-(5-(ethylsulfonyl) pyridin-2-yl)acetamide (**I-26**) (29 mg) as a yellow solid. LC-MS: m/z: $(M+H)^+$= 504.1. $^1$H NMR (400 MHz, MeOD) δ 8.96 (d, J = 1.9 Hz, 1 H), 8.49 (d, J = 2.4 Hz, 1 H), 8.26 (dd, J = 8.2, 2.3 Hz, 1 H), 8.15 (dd, J = 8.7, 2.6 Hz, 1 H), 7.97 (d, J = 8.7 Hz, 1 H), 7.68 (d, J = 8.1 Hz, 1 H), 7.33 (dd, J = 7.0, 2.2 Hz, 1 H), 7.13 (ddd, J = 7.0, 4.7, 2.4 Hz, 1 H), 7.07 (t, J = 8.9 Hz, 1 H), 4.07 (s, 2 H), 3.30 - 3.25 (m, 2 H), 1.69 (s, 6 H), 1.25 (dd, J = 7.3, 5.2 Hz, 3 H).

**Example 27**

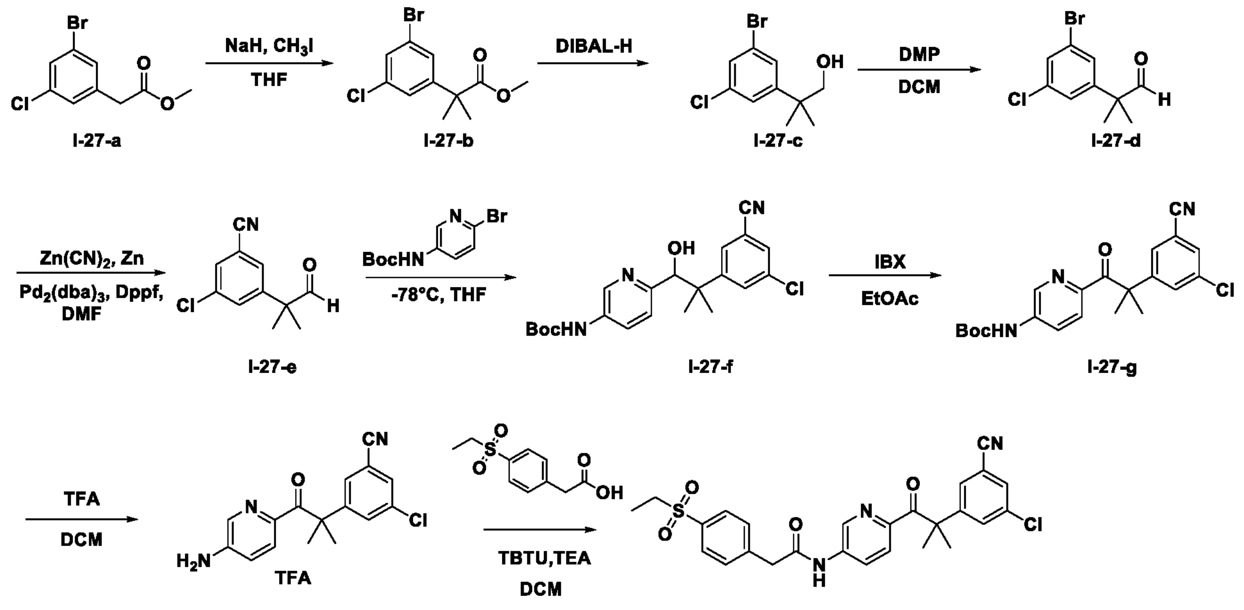


I-27-a I-27-b I-27-c I-27-d I-27-e I-27-f I-27-g I-27-h I-27

**Step 1:**

Methyl 2-(3-bromo-5-chlorophenyl)acetate (**I-27-a**) (6.90 g, 26.18 mmol) was dissolved in dry THF (100 mL), cooled to 0 °C, added with NaH (60%, 3.14 g, 78.55 mmol) in batches, stirred at room temperature for 30min, added with $CH_3I$ (11.15 g, 78.55 mmol), and reacted at room temperature for 3h. After the reaction was complete, the reaction mixture was quenched with saturated ammonium chloride solution (20 mL), and the aqueous phase was extracted with ethyl acetate (100 mL×3). The organic phases were combined, washed with water (120 mL×1) and saturated sodium chloride solution (120 mL×2), dried over anhydrous sodium sulfate, concentrated and purified by column chromatography(dichloromethane: petroleum ether = 40% to 70%) to obtin methyl 2-(3-bromo-5-chlorophenyl)-2-methylpropionate (**I-27-b**) (4.20 g, 14.40 mmol) as a colorless liquid.

**Step 2:**

At -78 °C, into a solution of methyl 2-(3-bromo-5-chlorophenyl)-2-methylpropionate (**I-27-b**) (3.44 g, 11.80 mmol) in anhydrous THF (65 mL)), diisobutylaluminum hydride (DIBAL-H, 1.5 M in toluene, 27.53 ml, 41.29 mmol) was slowly added dropwise. After stirred at -78 °C for 30min, the reaction mixture was slowly raised to room temperature and stirred for 16h. After the reaction was complete, the mixture was cooled in an ice bath, quenched slowly with 2 N HCl, and extracted with ethyl acetate (100 mL×2). The combined extracts were dried over anhydrous sodium sulfate, concentrated, and purified by column chromatography (dichloromethane:methanol=15:1) to obtain 2-(3-bromo-5-chlorophenyl)-2-methylpropyl-1-ol (**I-27-c**) (3.05 g, 11.57 mmol) as a colorless liquid.

**Step 3:**

2-(3-bromo-5-chlorophenyl)-2-methylpropyl-1-ol (**I-27-c**) (2.51 g, 9.52 mmol) was dissolved in DCM (120 ml), and Dess-Martin oxidant (4.85 g, 11.43 mmol) was added in batches in an ice bath. After the addition, the raction system was raised to room temperature and stirred for 4h. After the reaction was complete, the raction system was cooled in an ice bath, and a mixed solution of sodium bicarbonate and sodium thiosulfate was slowly added dropwise to the system. After the addition, the system was stirred until it became clear. The organic phase was separated, and the aqueous phase was extracted with dichloromethane (100 mL×3). The organic phases were combined, washed with water (120 mL×1) and saturated sodium chloride solution (120 mL×2), dried over anhydrous sodium sulfate, concentrated and purified by column chromatography (dichloromethane: petroleum ether = 20% to 40%) to obtain 2-(3-bromo-5-chlorophenyl)-2-methylpropanal (**I-27-d**) (1.75 g, white solid).

**Step 4:**

2-(3-bromo-5-chlorophenyl)-2-methylpropanal (**I-27-d**) (2.00 g, 7.65 mmol) was dissolved in DMF (60 ml), followed by addition of $Zn(CN)_2$ (5.39 g, 45.88 mmol), Zn powder (499.96 mg, 7.65 mmol), DPPF (847.88 mg, 1.53 mmol) and $Pd_2(dba)_3$ (700 mg, 0.76 mmol). The reaction system was protected by nitrogen, raised to 100 °C and reacted for 4h. After the reaction was complete, the reaction solution was cooled to room temperature, added with water (500 ml), and extracted with ethyl acetate (200 mL×3). The organic phases were combined, washed with saturated sodium chloride solution (200 mL×2), dried over anhydrous sodium sulfate, concentrated and purified by column chromatography (dichloromethane: petroleum ether = 60% to 90%) to obtain 2-(3-cyano-5-chlorophenyl)-2-methylpropanal (**I-27-e**) (710 mg) as a yellow solid. $^1$H NMR (400 MHz, $CDCl_3$) δ 9.49 (s, 1 H), 7.57 (t, J = 1.6 Hz, 1 H), 7.49 (t, J = 1.9 Hz, 1 H), 7.47 (t, J = 1.6 Hz, 1 H), 1.49 (s, 6 H).

**Step 5:**

Tert-butyl (6-bromopyridin-3-yl)carbamate (273 mg, 1.0 mmol) was put in anhydrous tetrahydrofuran (5 mL), cooled to -78 °C under nitrogen protection, and added with n-butyllithium (1.31 mL, 2.1 mmol, 1.6 mol/L). After the reaction solution became turbid, it was stirred for 1h, added with a solution of 2-(3-cyano-5-chlorophenyl)-2-methylpropanal (**I-27-e**) (210 mg, 1.01 mmol) in tetrahydrofuran (3 mL), reacted at -78 °C for 2h and slowly warmed to room temperature overnight. After the reaction was complete, the reaction solution was quenched with saturated sodium bicarbonate solution (10 mL), and the aqueous phase was extracted with ethyl acetate (30 mL×3). The organic phases were combined, washed with water (20 mL×1) and saturated sodium chloride solution (20 mL×2), dried over anhydrous sodium sulfate, concentrated and purified by column chromatography (dichloromethane:methanol=25:1) to obtain tert-butyl (6-(2-(3-chloro-5-cyanophenyl)-1-hydroxyl-2-methylpropyl)pyridin-3-yl)carbamate (**I-27-f**) (248 mg) as a white solid. LCMS (ESI, m/z):$[M+H]^+$= 402.1.

**Step 6:**

Tert-butyl (6-(2-(3-chloro-5-cyanophenyl)-1-hydroxy-2-methylpropionyl)pyridin-3-yl)carbamate (**I-27-f)** (213 mg, 0.53 mmol) was put in ethyl acetate (25 mL), added with 2-iodoxybenzoic acid (450 mg, 1.61 mmol) and stirred overnight at 100 °C. After the reaction was complete, the reaction mixture was filtered, and the filtrate was concentrated and purified by column chromatography (petroleum ether: ethyl acetate=10:1) to obtain tert-butyl (6-(2-(3-chloro-5-cyanophenyl)-2-methylpropionyl)pyridin-3-yl)carbamate (**I-27-g**) (190 mg) as a yellow solid. LC-MS: m/z: $(M+H)^+$= 400.1.

**Step 7:**

Tert-butyl (6-(2-(3-chloro-5-cyanophenyl)-2-methylpropanoyl)pyridin-3-yl)carbamate (**I-27-g**) (44 mg) was put in dichloromethane (5 mL), added with trifluoroacetic acid (2 mL) and stirred at room temperature for 5h. After the reaction was complete, the raction mixture was concentrated to obtain a crude 3-(1-(5-aminopyridin-2-yl)-2-methyl-1-carbonylpropyl-2-yl)-5-chlorobenzonitrile (**I-27-h**) (60 mg) as a yellow oil, which was directly used in the next step. LC-MS: m/z: $(M+H)^+$= 300.1.

**Step 8:**

The crude 3-(1-(5-aminopyridin-2-yl)-2-methyl-1-carbonylpropyl-2-yl)-5-chlorobenzonitrile **(I-27-h)** (60 mg) in previous step was dissolved in dry dichloromethane (10 mL), added with 2-(4-(ethylsulfonyl)phenyl)acetic acid (31 mg, 0.136 mmol), TBTU (46 mg, 0.143 mmol), triethylamine (64 mg, 0.632 mmol), and stirred overnight. After the reaction was complete, water (5 mL) and dichloromethane (5 mL) were added, the organic phase was separated, and the aqueous phase was extracted with dichloromethane (10 mL×2). The organic phases were combined, washed with saturated sodium chloride solution (5 mL×2) and water (5 mL), dried over anhydrous sodium sulfate, concentrated and purified by column chromatography (dichloromethane:methanol=40:1), to obtain N-(6-(2-(3-chloro-5-cyanophenyl)-2-methylpropionyl)pyridin-3-yl)-2-(4-(ethylsulfonyl) phenyl) acetamide (**I-27**) as a white solid. LC-MS: m/z: $(M+H)^+$= 510.1. $^1$H NMR (400 MHz, $CDCl_3$) δ 8.43 (s, 1 H), 8.23 (d, J = 8.1 Hz, 2 H), 8.01 (d, J = 8.3 Hz, 1 H), 7.83 (d, J = 7.9 Hz, 2 H), 7.51 (d, J = 7.9 Hz, 2 H), 7.47-7.36 (m, 3 H), 3.84 (s, 2 H), 3.12 (q, J = 7.4 Hz, 2 H), 1.70 (s, 6 H), 1.28 (t, J = 7.4 Hz, 3 H).

**Example 32**

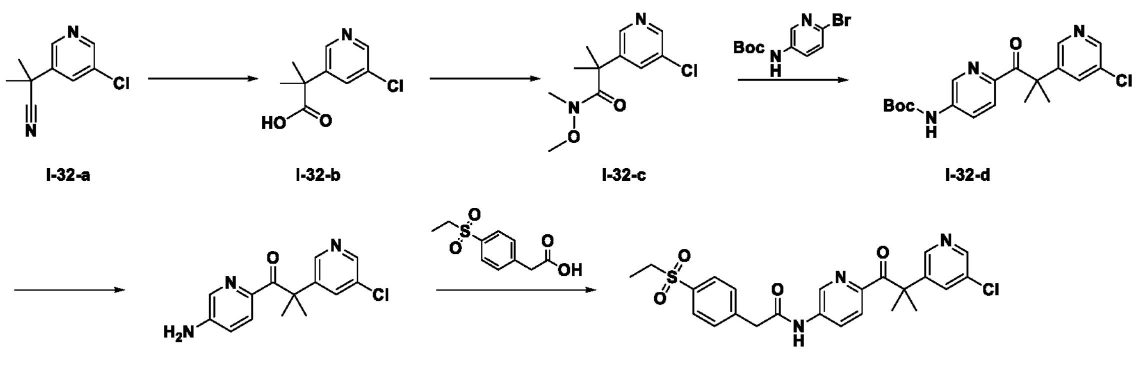

I-32-a I-32-b I-32-c I-32-d

I-32-e I-32

**Step 1:**

2-(5-chloropyridin-3-yl)-2-methylpropionitrile (**I-32-a**) (362 mg, 2 mmol) was added to 10 ml of concentrated hydrochloric acid, and stirred overnight at 110 °C. The reaction solution was concentrated to obtain 380 mg of 2-(5-chloropyridin-3-yl)-2-methylpropionic acid (**I-32-b**) as a white solid with a yield of 95%. LC-MS: m/z: $(M+H)^+$ =200.

**Step 2:**

2-(5-chloropyridin-3-yl)-2-methylpropionic acid (**I-32-b**) (0.38 g, 1.9 mmol), N-methoxymethylamine hydrochloride (300 mg, 3 mmol) and N,N-diisopropylethylamine (1 ml, 5.75 mmol) were added to 12 ml of dichloromethane, added with HATU (1.1 g, 2.9 mmol), and stirred overnight at 30 °C. The reaction solution was concentrated and passed through a column (ethyl acetate:petroleum ether=0-50%) to obtain 400 mg of a white solid (**I-32-c**) with a yield of 87%. LC-MS: m/z: $(M+H)^+$ =243.

**Step 3:**

Tert-butyl (6-bromopyridin-3-yl)carbamate (0.273 g, 1 mmol) was added to 3 ml of tetrahydrofuran, added slowly with n-butyllithium (1.32 ml, 2.1 mmol) at -78 °C, and stirred at this temperature for 1h. A solution of 2-(5-chloropyridin-3-yl)-N-methoxy-N,2-dimethylpropanamide (**I-32-c**) (0.20 g, 0.82 mmol) in 5ml of tetrahydrofuran was slowly added, and the reaction solution was stirred at -78 °C for 1 h and then slowly warmed to room temperature. The reaction solution was quenched with saturated aqueous $NH_4Cl$ and extracted with ethyl acetate, and the organic phase was washed once with saturated brine, dried over anhydrous sodium sulfate, concentrated and passed through a column (ethyl acetate:petroleum ether = 0-50%) to obtain 75 mg of a white solid (**I-32-d**) with a yield of 20%. LC-MS: m/z: $(M+H)^+$ = 376.

**Step 4:**

Tert-butyl (6-(2-(5-chloropyridin-3-yl)-2-methylpropanoyl)pyridin-3-yl)carbamate (**I-32-d**) (150 mg, 0.4 mmol) was added to 3 ml of dichloromethane, added with 3 ml of trifluoroacetic acid, and stirred at room temperature for 1h. The reaction solution was concentrated, added with 5 ml of saturated aqueous sodium carbonate, and extracted with dichloromethane (2×20 ml). The organic phase was dried over anhydrous sodium sulfate and concentrated to obtain 100 mg of a white solid (**I-32-e**) with a yield of 90%. LC-MS: m/z: $(M+H)^+$ =276.

**Step 5:**

1-(5-aminopyridin-2-yl)-2-(5-chloropyridin-3-yl)-2-methylpropan-1-one (**I-32-e**) (90 mg, 0.33 mmol), 2-(4-(ethylsulfonyl)phenyl)acetic acid (80 mg, 0.35 mmol) and N,N-diisopropylethylamine (0.3 ml, 2 mmol) were added to 20 ml of dichloromethane, added with TBTU (170 mg, 0.53 mmol), and stirred overnight at room temperature. The reaction solution was concentrated and passed through a column (ethyl acetate: petroleum ether = 0-100%) to obtain 90 mg of N-(6-(2-(5-chloropyridin-3-yl)-2-methylpropionyl)pyridin-3-yl)-2-(4-(ethylsulfonyl)phenyl)acetami de (**I-32**) as a white solid with a yield of 56%. LC-MS: m/z: $(M+H)^+$ =486, $^1$H NMR (400 MHz, DMSO-$d_6$) δ 10.70 (s, 1 H), 8.52 (d, J = 2.2 Hz, 1 H), 8.41 (d, J = 2.3 Hz, 1 H), 8.31 (d, J = 2.0 Hz, 1 H), 8.14 (dd, J = 8.7, 2.5 Hz, 1 H), 8.01 (d, J = 8.7 Hz, 1 H), 7.87 - 7.81 (m, 2 H), 7.78 (t, J = 2.2 Hz, 1 H), 7.59 (d, J = 8.4 Hz, 2 H), 3.85 (s, 2 H), 3.27 (q, J = 7.4 Hz, 2 H), 1.69 (s, 6 H), 1.10 (t, J = 7.4 Hz, 3 H).

**Example 33**

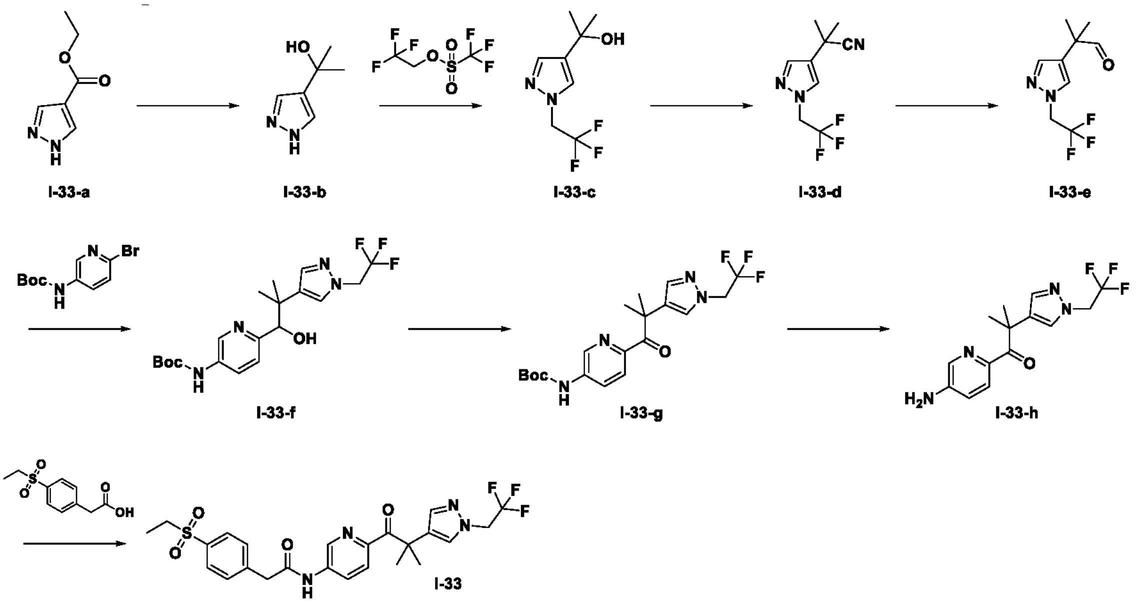

I-33-a
I-33-b
I-33-c
I-33-d
I-33-e
I-33-f
I-33-g
I-33-h
I-33

**Step 1:**

Ethyl 1H-pyrazole-4-carbonate (**I-33-a**) (7 g, 50 mmol) was added into 100 ml of tetrahydrofuran, added with a solution of methylmagnesium chloride in tetrahydrofuran solution (68 ml, 204 mmol, 3 mol/L) in an ice bath, reacted in the ice bath for 1 h and then raised to room temperature overnight. The reaction solution was quenched with saturated aqueous ammonium chloride and extracted with ethyl acetate. The organic phase was washed with saturated brine, dried over anhydrous sodium sulfate and concentrated to obtain a crude, which was washed with a mixed solution of ethyl acetate and petroleum ether (ethyl acetate: petroleum ether = 2:1) to obtain 3.6 g of a white solid (**I-33-b**) with a yield of 57%. LC-MS: m/z: $(M+H)^+$ =127. $^1H$ NMR (400 MHz, DMSO-$d_6$) $\delta$ 12.48 (s, 1 H), 7.46 (s, 2 H), 4.70 (s, 1 H), 1.40 (s, 6 H).

**Step 2:**

2-(1H-pyrazol-4-yl)propan-2-ol (**I-33-b**) (1.26g, 10 mmol), 2,2,2-trifluoroethyl triflate (3.1 g, 13 mmol) and potassium carbonate were added to 60 ml of acetonitrile, and stirred overnight at 95 °C. The reaction mixture was filtered, concentrated and passed through a column (methanol:dichloromethane=0-10%) to obtain 1.65 g of a white solid (**I-33-c**) with a yield of 79%. LC-MS: m/z: $(M+H)^+$ =209.

**Step 3:**

To a suspension of indium bromide (354 mg, 1 mmol) in dichloromethane (40 mL), was added trimethylsilyl cyanide (1.5 g, 15 mmol). To this mixture, a solution of 2-(1-(2,2,2-trifluoroethyl)-1H-pyrazol-4-yl)propan-2-ol (**I-33-c**) (1 g, 4.8 mmol) in dichloromethane was added dropwise at room temperature. The resulting mixture was stirred overnight at room temperature. Saturated aqueous sodium bicarbonate was added and the mixture was filtered through a celite pad. The filtrate was partitioned between saturated aqueous sodium bicarbonate and dichloromethane, and the aqueous layer was extracted with ethyl acetate again. The combined organic layers were dried over anhydrous sodium sulfate, filtered and concentrated to obtain a crude, which was purified by column chromatography (dichloromethane:methanol=0-5%) to obtain 0.7 g of a colorless oil (**I-33-d**) with a yield of 70%. LC-MS: m/z: $(M+H)^+$ =218.

**Step 4:**

2-methyl-2-(1-(2,2,2-trifluoroethyl)-1H-pyrazol-4-yl)propionitrile (**I-33-d**) (0.87 g, 4 mmol) was added into 20 ml of tetrahydrofuran, added slowly with diisobutylaluminum hydride (10 ml, 10 mmol) at -78 °C, stirred at this temperature

for 2 h and then at room temperature overnight. The reaction solution was quenched with 2 mol/L hydrochloric acid and extracted twice with ethyl acetate (2×30ml), and the organic phase was dried over anhydrous sodium sulfate and concentrated to obtain 0.65 g of a colorless oil (**I-33-e**) with a yield of 74%, which was directly used in the next step. LC-MS: m/z: $(M+H)^+$ =221.

**Step 5:**

Tert-butyl (6-bromopyridin-3-yl)carbamate (0.82 g, 7 mmol) was added to 10 ml of tetrahydrofuran, added slowly with n-butyllithium (4 ml, 6.4 mmol) at -78 °C, stirred at this temperature for 1 h, slowly added with a solution of 2-methyl-2-(1-(2,2,2-trifluoroethyl)-1H-pyrazol-4-yl)propanal (**I-33-e**) (0.62 g, 2.8 mmol) in 5 ml of tetrahydrofuran, stirred at -78 °C for 2 h and then slowly warmed to room temperature. The reaction solution was quenched with 20 ml of saturated aqueous $NH_4Cl$ and extracted with ethyl acetate, and the organic phase was washed once with saturated brine, dried over anhydrous sodium sulfate, concentrated and passed through a column (ethyl acetate: petroleum ether = 0-60%) to obtain 0.48 g of a white solid (**I-33-f**) with a yield of 39%. LC-MS: m/z: $(M+H)^+$ =415. $^1H$ NMR (400 MHz, $CDCl_3$) δ 8.34 (d, J = 2.5 Hz, 1 H), 7.76 (d, J = 7.7 Hz, 1 H), 7.33 (s, 1 H), 7.13 (s, 1 H), 6.75 - 6.58 (m, 2 H), 4.64 (q, J = 8.4 Hz, 2 H), 4.55 (s, 1 H), 1.54 (s, 9 H), 1.33 (s, 3 H), 1.22 (s, 3 H).

**Step 6:**

Tert-butyl (6-(1-hydroxyl-2-methyl-2-(1-(2,2,2-trifluoroethyl)-1H-pyrazol-4-yl)propyl)pyridin-3-yl )carbamate (**I-33-f**) (0.15 g, 0.36 mmol) was added to 20 ml of ethyl acetate, added with IBX (0.41 g, 0.67 mmol), and stirred overnight at reflux temperature. The reaction solution was filtered, concentrated and passed through a column (ethyl acetate: petroleum ether=0-50%) to obtain 120 mg of a white solid (**I-33-g**) with a yield of 80%. LC-MS: m/z: $(M+H)^+$ =413.

**Step 7:**

Tert-butyl (6-(2-methyl-2-(1-(2,2,2-trifluoroethyl)-1H-pyrazol-4-yl)propionyl)pyridin-3-yl)carbam ate (**I-33-g**) (180 mg, 0.43 mmol) was added to 3 ml of dichloromethane, added with 3 ml of trifluoroacetic acid, and stirred at room temperature for 1h. The reaction solution was concentrated, added with 10 ml of saturated aqueous sodium bicarbonate, and extracted with dichloromethane (2×20 ml). The organic phase was dried over anhydrous sodium sulfate, concentrated and passed through a column (ethyl acetate: petroleum ether = 0-40%) to obtain 120 mg of a white solid (**I-33-h**) with a yield of 88%. LC-MS: m/z: $(M+H)^+$ =313.

**Step 8:**

1-(5-aminopyridin-2-yl)-2-methyl-2-(1-(2,2,2-trifluoroethyl)-1H-pyrazol-4-yl)prop ane-1-one (**I-33-h**) (120 mg, 0.38 mmol), 2-(4-(ethylsulfonyl)phenyl)acetic acid (100 mg, 0.44 mmol) and triethylamine (0.25 ml, 1.8 mmol) were added into 20 ml of dichloromethane, added with TBTU (200 mg, 0.62 mmol), and stirred overnight at room temperature. The reaction solution was concentrated and passed through a column (ethyl acetate: petroleum ether=0-80%) to obtain 70 mg of 2-(4-(ethylsulfonyl)phenyl)-N-(6-(2-methyl-2-(1-(2,2,2-trifluoroethyl)-1H-pyrazol-4-yl) propionyl)pyridin-3-yl)acetamide (**I-33**) as a white solid with a yield of 34%. LC-MS: m/z: $(M+H)^+$ =523, $^1H$ NMR (400 MHz, $CDCl_3$) δ 8.54 (d, J = 2.4 Hz, 1 H), 8.43 (s, 1 H), 8.14 (dd, J = 8.7, 2.4 Hz, 1 H), 7.87 (d, J = 8.7 Hz, 1 H), 7.82 (d, J = 8.2 Hz, 2 H), 7.54 (s, 1 H), 7.49 (d, J = 8.2 Hz, 2 H), 7.42 (s, 1 H), 4.62 (q, J = 8.4 Hz, 2 H), 3.84 (s, 2 H), 3.14 (q, J = 7.4 Hz, 2 H), 1.76 (s, 6 H), 1.30 (t, J = 7.4 Hz, 3 H).

**Example 35**

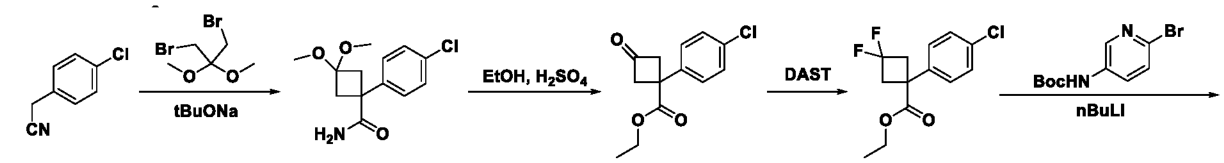


I-35-a I-35-b I-35-c I-35-d

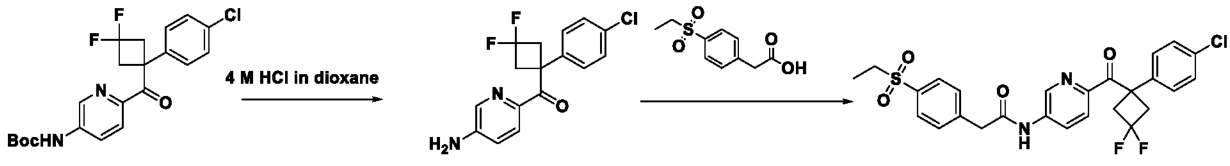


I-35-e I-35-f I-35

**Step 1:**

2-(4-chlorophenyl)acetonitrile (**I-35-a**) (20 g, 132 mmol) was dissolved in 200 mL of DMSO, and 1,3-dibromo-2,2-dimethoxy propane (34.9 g, 133.25 mmol) and sodium tert-butoxide (27.89 g, 290.25 mmol) were added successively. The reaction mixture was heated to 125 °C and stirred for 1h, then slowly cooled to room temperature and stirred for 20h. TLC showed that the reaction was complete, and the reaction system was added with water, and extracted with ethyl acetate (3×500 mL). The organic phases were combined, concentrated to obtain a crude, which was separated by column chromatography (ethyl acetate: petroleum ether = 40% to 60%) to obtain 1-(4-chlorophenyl)-3,3-dimethoxycyclobutane-1-carboxamide (**I-35-b**) (12.5 g, 46.34 mmol, yield 35%) as a yellow solid. LCMS (ESI, m/z):$[M-H]^-$= 268.1.

**Step 2:**

1-(4-chlorophenyl)-3,3-dimethoxycyclobutane-1-carboxamide (**I-35-b**) (12.5 g, 46.34 mmol) was dissolved in ethanol (160 mL), added slowy with concentrated $H_2SO_4$ (40 mL) under stirring, and heated to reflux for 2h. The reaction system was cooled to 0 °C, diluted with 100 mL of water and stirred for 30min. TLC showed that the reaction was complete, and the reaction system was extracted with ethyl acetate (3×500 mL). The organic phases were combined, concentrated to obtain a crude, which was separated by column chromatography (ethyl acetate: petroleum ether = 5% to 10%) to obtain ethyl 1-(4-chlorophenyl)-3-oxocyclobutane-1-carboxylate (**I-35-c**) (5.4 g, 21.37 mmol, yield 46%) as a yellow oil. $^1H$ NMR(400 MHz, $CDCl_3$) δ 7.42 - 7.22 (m, 4 H), 4.16 (q, *J* = 7.1 Hz, 2 H), 3.96 - 3.84 (m, 2 H), 3.59 - 3.47 (m, 2 H), 1.20 (t, *J* = 7.1 Hz, 3 H).

**Step 3:**

Ethyl 1-(4-chlorophenyl)-3-oxocyclobutane-1-carboxylate (**I-35-c**) (5.4 g, 21.37 mmol) was dissolved in dry dichloromethane (100 mL), and diethylaminosulfur trifluoride (DAST, 4.13 g, 25.64 mmol) was added at room temperature. The reaction system was stirred at this temperature for 16h. TLC showed that the reaction was complete, and the reaction system was quenched with saturated aqueous $NaHCO_3$ (100 mL), and extracted with ethyl acetate (3×300 mL). The organic phases were combined and concentrated to obtain a crude, which was separated by column chromatography (ethyl acetate: petroleum ether = 0%-10%) to obtain ethyl 1-(4-chlorophenyl)-3,3-difluorocyclobutane-1-carboxylate (**I-35-d**) (3.2 g, 11.63 mmol, yield 54%) as a yellow oil. $^1H$ NMR(400 MHz, $CDCl_3$) δ 7.39 - 7.32 (m, 2 H), 7.29 - 7.19 (m, 2 H), 4.13 (q, *J* = 7.1 Hz, 2 H), 3.57 - 3.35 (m, 2 H), 3.11 - 2.90 (m, 2 H), 1.19 (t, *J* = 7.1 Hz, 3 H).

**Step 4:**

Tert-butyl (6-bromopyridin-3-yl)carbamate (1.79 g, 6.55 mmol) was dissolved in dry tetrahydrofuran (5 mL), cooled to -78 °C, slowly added dropwise with n-BuLi (2.5 M in n-Hexane, 5.5 mL, 13.75 mmol), and stirred at this temperature for 1h. Then a solution of ethyl 1-(4-chlorophenyl)-3,3-difluorocyclobutane-1-carboxylate (**I-35-d**) (1.5 g, 5.45 mmol) in tetrahydrofuran (5 mL) was added dropwise to the system at -78 °C, and after the addition, the system was stirred for 2h while the tempertature was kept. LCMS showed that the reaction was complete, and the reaction system was quenched with saturated $NaHCO_3$ (30 mL), and extracted with ethyl acetate (3×50 mL). The organic phases were combined and concentrated to obtain a crude, which was separated by column chromatography (methanol: dichloromethane=0%-10%) to obtain tert-butyl (6-(1-(4-chlorophenyl)-3,3-difluorocyclobutane-1-carbonyl)pyridin-3-yl)carbamate (**I-35-e**) (480 mg, 1.14 mmol, yield 20%) as a yellow oil. LCMS (ESI, m/z):$[M+H]^+$= 423.2.

**Step 5:**

Tert-butyl 6-(1-(4-chlorophenyl)-3,3-difluorocyclobutane-1-carbonyl)pyridin-3-yl)carbamate (**I-35-e**) (475 mg, 1.12 mmol) was dissolved in a solution of HCl in dioxane (4 mol/L, 5.00 mL), and stirred at room temperature for 1.5h. LCMS showed that the reaction was complete, and the reaction system was concentrated and purified by reverse phase preparative chromatography (C18 silica gel; ACN:$H_2O$ (containing 3% $NH_3 \cdot H_2O$), 15% to 30%) to obtain (5-aminopyridin-2-yl)(1-(4-chlorophenyl)-3,3-difluorocyclobutyl)methanone (**I-35-f**) as a yellow solid (219 mg, 0.68 mmol, 60% yield). LCMS (ESI, m/z):$[M+H]^+$= 323.00. $^1H$ NMR (300 MHz, $CDCl_3$) $\delta$ 8.05 - 7.83 (m, 2 H), 7.54 - 7.38 (m, 2 H), 7.28 - 7.19 (m, 2 H), 6.91 (dd, *J* = 8.5, 2.8 Hz, 1 H), 4.13 (s, 2 H), 3.75 - 3.52 (m, 2 H), 3.41 - 3.10 (m, 2 H).

**Step 6:**

(5-aminopyridin-2-yl)(1-(4-chlorophenyl)-3,3-difluorocyclobutyl)methanone (**I-35-f**) (33 mg, 0.1 mmol), 2-(4-(ethylsulfonyl)phenyl)acetic acid (25 mg, 0.1 mmol) and N,N-diisopropylethylamine (0.2 ml, 1 mmol) were added to 5 ml of N,N-dimethyl formamide, added with a 50% solution of T3P (140 mg, 0.22 mmol) in ethyl acetate , and stirred overnight at 40 °C. The reaction solution was washed with water, dried over anhydrous sodium sulfate, concentrated, separated and purified by thin layer chromatography (methanol: (dichloromethane:ethyl acetate=18:2)=1:20) to obtain 40 mg of a white solid (**I-35**) with a yield of 73%. LC-MS: m/z: $(M+H)^+$ = 533, $^1H$ NMR (400 MHz, DMSO-$d_6$) $\delta$ 10.81 (s, 1 H), 8.78 (d, J = 2.3 Hz, 1 H), 8.13 (dd, J = 8.7, 2.5 Hz, 1 H), 7.98 (d, J = 8.7 Hz, 1 H), 7.85 (d, J = 8.3 Hz, 2 H), 7.60 (d, J = 8.3 Hz, 2 H), 7.50 (d, J = 8.6 Hz, 2 H), 7.36 (d, J = 8.6 Hz, 2 H), 3.88 (s, 2 H), 3.57 (q, J = 14.7 Hz, 2 H), 3.33 - 3.22 (m, 4 H), 1.10 (t, J = 7.4 Hz, 3 H).

**Example 36**

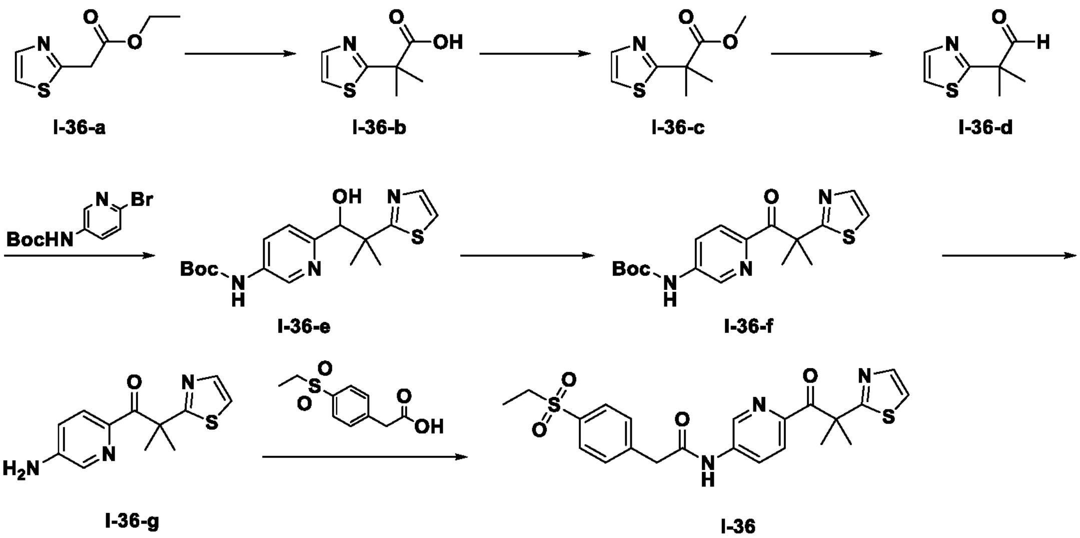

I-36-a I-36-b I-36-c I-36-d

I-36-e I-36-f

I-36-g I-36

**Step** 1:

Ethyl 2-thiazol-2-ylacetate (**I-36-a**) (900 mg, 5.25 mmol) was dissolved in 20 ml of tetrahydrofuran, cooled to 0 °C, and added with sodium hydride (841 mg, 60%, 21.026 mmol). The reaction solution was stirred for 1h, and iodomethane (4.48 g, 31.53 mmol) was added. The reaction solution was warmed to 15 °C and stirred for 16h. Then the reacton solution was quenched with 1 N hydrochloric acid till pH=6, and extracted with ethyl acetate (0 mLx2). The aqueous phase was concentrated to obtain a crude, which was dissolved in methanol (30 mL) and filtered. The filtrate was concentrated to obtain 800 mg of a brown oil (**I-36-b**) with a yield of 88.89%. LC-MS: m/z: $(M+H)^+$ =172.0.

**Step 2:**

2-methyl-2-(thiazol-2-yl)propionic acid (**I-36-b**) (800 mg, 4.67 mmol) was dissolved in 5 ml of methanol, cooled to 0 °C, slowly added dropwise with 5 mL of thionyl chloride, heated to 15 °C and stirred for 16h. The reaction solution

was concentrated, the residue was dissolved in 15 mL of water, and basified with saturated aqueous sodium bicarbonate to pH = 8, and the solution was extracted with ethyl acetate (20 mL×2). The organic phase was dried with anhydrous sodium sulfate, filtered and concentrated to obtain 420 mg of a brown oil (**I-36-c**) with a yield of 48.5%. LC-MS: m/z: $(M+H)^+$ =186.0.

**Step 3:**

Methyl 2-methyl-2-thiazol-2-ylpropionate (**I-36-c**) (800 mg, 4.67 mmol) was dissolved in 10 ml of tetrahydrofuran, cooled to -78 °C, slowly added dropwise with 3.6 mL of a solution of diisobutylaluminum hydride in toluene (1 M) under nitrogen protection, and stirred at -78 °C for 1h. The reaction solution was quenched with 20 mL of saturated aqueous ammonium chloride, and extracted with ethyl acetate (20 mL×2). The combined organic layers were dried over anhydrous sodium sulfate, filtered and concentrated to obtain a crude, which was purified by column chromatography (ethyl acetate:petroleum ether = 0 - 60%) to obtain 240 mg of a white solid (**I-36-d**) with a yield of 68.19%. LC-MS: m/z: $(M+H)^+$ = 156.0.

**Step 4:**

Tert-butyl N-(6-bromo-3-pyridyl)carbamate (422 mg, 1.55 mmol) was dissolved in 4 ml of tetrahydrofuran, replaced with nitrogen three times, cooled to -78 °C, and slowly added with a 2.5 M solution of n-butyllithium in n-hexane (2.1 mL). The reaction solution was stirred for 1h, and 2-methyl-2-thiazol-2-yl-propanal (**I-36-d**) (240 mg, 1.55 mmol) was added. The reaction solution was stirred at -78 °C for 2h, then warmed to 15 °C and stirred for 16h. The reaction solution was quenched with saturated aqueous ammonium chloride and extracted with EtOAc (20 mL×2). The organic phase was dried over anhydrous sodium sulfate, filtered, concentrated, and purified by column chromatography (ethyl acetate: petroleum ether = 0-60%) to obtain 150 mg of a white solid (**I-36-e**) with a yield of 27.8%. LC-MS: m/z: $(M+H)^+$ = 350.0.

**Step 5:**

Tert-butyl (6-(1-hydroxy-2-methyl-2-(thiazol-2-yl)propyl)pyridin-3-yl)carbamate (**I-36-e**) (140 mg, 0.40 mmol) was dissolved in 5 ml of ethyl acetate, and 168 mg of 2-iodoxybenzoic acid was added. The reaction solution was refluxed and stirred for 16h. The reaction solution was filtered and concentrated to obtain 120 mg of a white solid **(I-36-f)** with a yield of 86.21%. LC-MS: m/z: $(M+H)^+$ =348.0.

**Step 6:**

Tert-butyl (6-(2-methyl-2-(thiazol-2-yl)propionyl)pyridin-3-yl)carbamate (**I-36-f**) (110 mg, 0.30 mmol) was dissolved in 5 ml of dichloromethane, and 5 ml of trifluoroacetic acid was added. The reaction solution was stirred at 15 °C for 1h. Then the reaction solution was concentrated, and the residue was dissolved in 5 mL of dichloromethane, and neutralized with saturated aqueous ammonium bicarbonate to pH = 9. The organic phase was dried over anhydrous sodium sulfate and filtered, and the filtrate was concentrated to obtain 75 mg of a white solid (**I-36-g**) with a yield of 95.8%. LC-MS: m/z: $(M+H)^+$ = 28.0.

**Step** 7:

1-(5-aminopyridin-2-yl)-2-methyl-2-(thiazol-2-yl)propan-1-one (**I-36-g**) (75 mg, 0.30 mmol) and 2-(4-ethylsulfonylphenyl)acetic acid (69 mg, 0.30 mmol) were dissolved in 2 ml of dichloromethane, and 78 mg of N,N-diisopropylethylamine and 289 mg of 1-propylphosphoric anhydride (50% ethyl acetate solution) were added successively. The reaction solution was stirred at 15 °C for 16h. The reaction solution was concentrated, and the residue was purified by thin-layer chromatography (dichloromethane:methanol=10:1) to obtain 50 mg of an off-white solid (**I-36**) with a yield of 36.03%. LC-MS: m/z: $(M+H)^+$=458, $^1$H NMR (400 MHz, $CDCl_3$) δ 9.23 (s, 1 H), 8.63 (s, 1 H), 7.90 (d, J = 8.0 Hz, 2 H), 7.79 (d, J = 8.6 Hz,1 H), 7.60 (d, J = 8.3 Hz, 3 H), 7.37 (d, J = 3.2 Hz, 1 H), 7.15 (d, J = 8.4 Hz, 1 H), 3.88 (s, 2 H), 3.13 (q, J =7.4 Hz, 2 H), 1.85 (d, J = 16.9 Hz, 6 H), 1.30 (t, J = 7.4 Hz, 3 H).

**Example 37**

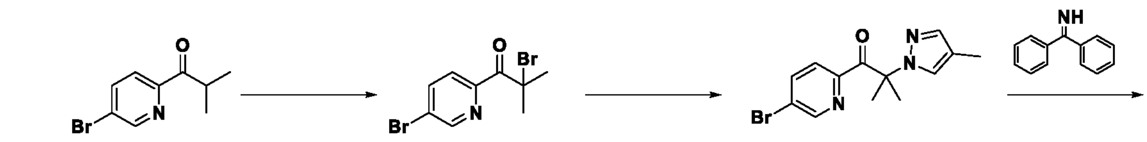

I-37-a I-37-b I-37-c

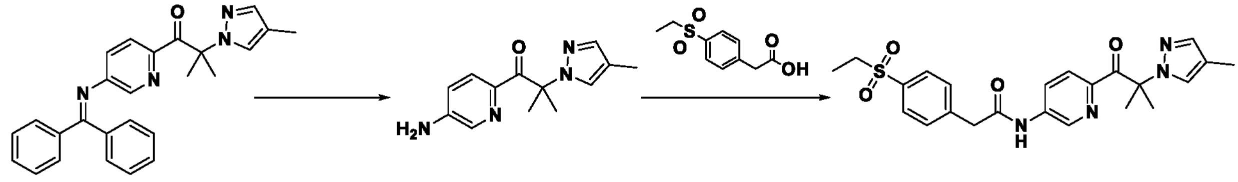

I-37-d I-37-e I-37

**Step** 1:

1-(5-bromo-2-pyridyl)-2-methyl-propan-1-one (**I-37-a**) (5 g, 21.9 mmol) was dissolved in 50 ml acetic acid, and liquid bromine (10.5 g, 65.76 mmol) was added at room temperature. The reaction solution was heated to 105 °C and stirred for 16h. The reaction solution was concentrated to obtain a brown oil, which was purified by column chromatography (methanol: dichloromethane = 0-3%) to obtain 6 g of a brown oil **(I-37-b)** with a yield of 89.5%. LC-MS: m/z: $(M+H)^+$ =306.0.

**Step 2:**

2-Bromo-1-(5-bromopyridin-2-yl)-2-methylpropan-1-one **(I-37-b)** (600 mg, 1.95 mmol) was dissolved in 20 mL of N,N-dimethylformamide, and 4-methyl-1H-pyrazole (161 mg, 1.95 mmol) and cesium carbonate (1.27 g, 3.90 mmol) were added at room temperature. The reaction solution was heated to 80 °C, stirred for 16 h and concentrated, and the residue was dissolved in 20 mL of ethyl acetate. The organic phase was washed with saturated brine (20 mL×2), dried over anhydrous sodium sulfate, filtered and concentrated to obtain 500 mg of a brown oil (**I-37-c**) with a yield of 83%. LC-MS: m/z: $(M+H)^+$= 308.0.

**Step 3:**

1-(5-Bromo-2-pyridyl)-2-methyl-2-(4-methylpyrazol-1-yl)propan-1-one (**I-37-c**) (550 mg, 1.78 mmol) and diphenylmethylamine (388 mg, 2.14 mmol) were dissolved in 15 ml of dioxane, and tris[dibenzylideneacetone]dipalladium (82 mg, 0.089 mmol), 4,5-bisphenyl phosphino-9,9-dimethylxanthene (103 mg, 0.18 mmol) and cesium carbonate (1.16 g, 3.57 mmol) were added. The reaction liquid was replaced with nitrogen three times, heated to 95 °C and stirred for 16h. The reaction liquid was filtered and concentrated, and the residue was purified by column chromatography (methanol: dichloromethane = 0-6%) to obtain 500 mg of an off-white solid (**I-37-d**) with a yield of 68.9%. LC-MS: m/z: $(M+H)^+$ =409.0.

**Step 4:**

(1-[5-(benzimido)-2-pyridyl]-2-methyl-2-(4-methylpyrazol-1-yl)propan-1-one (**I-37-d**) (180 mg, 0.74 mmol) was dissolved in 10 ml of methanol, and 1 mL of 1 N hydrochloric acid was added. The reaction solution was stirred at 15 °C for 10min. Then the reaction solution was extracted with 20 mL of ethyl acetate, and the organic phase was washed with saturated aqueous sodium bicarbonate (20 mL×2), dried with anhydrous sodium sulfate, filtered and concentrated to obtain a residue, which was purified by column chromatography (dichloromethane: methanol = 0-10%) to obtain 180 mg of an off-white solid (**I-37-e**) with a yield of 85.99%. LC-MS: m/z: $(M+H)^+$ =245.0.

**Step 5:**

1-(5-amino-2-pyridyl)-2-methyl-2-(4-methylpyrazolyl-1-yl)propan-1-one (**I-37-e**) (80 mg, 0.33 mmol) and 2-(4-ethylsulfonylphenyl)acetic acid (75 mg, 0.33 mmol) were dissolved in 3 ml of dichloromethane, followed by the addition of 85 mg of N,N-diisopropylethylamine and 313 mg of 1-propylphosphoric anhydride (50% ethyl acetate solution). The reaction solution was stirred at 15 °C for 16h and concentrated, and the residue was purified by thin-layer chromatography (dichloromethane: methanol = 10:1) to obtain 95 mg of an off-white solid (**I-37**) with a yield of 65.83%. LC-MS: m/z: $(M+H)^+$=455. $^1H$ NMR (400 MHz, $CDCl_3$) δ9 .68 (s, 1 H), 8.45 (d, J = 2.2 Hz, 1 H), 7.85 (d, J = 8.3 Hz, 2 H), 7.71 (dd,

J= 8.6, 2.2 Hz, 1 H), 7.61 - 7.55 (m, 3 H), 7.43 (s, 1 H), 7.29 (s, 1 H), 3.88 (s, 2 H), 3.12 (q, J = 7.4 Hz, 2 H),2.12 (s, 3 H), 1.45 (s, 3 H), 1.29 (t, J = 7.4 Hz, 3 H), 1.24 (s, 3 H).

**Example 38**

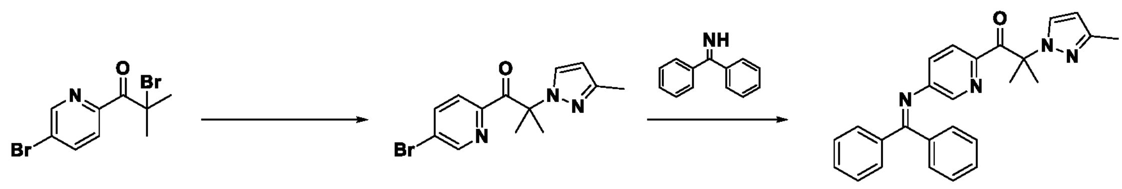

I-38-a I-38-b I-38-c

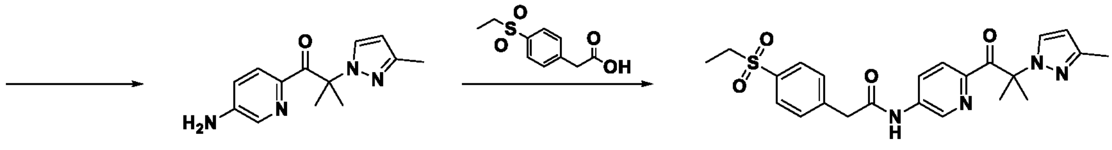

I-38-d I-38

**Step** 1:

2-Bromo-1-(5-bromopyridin-2-yl)-2-methylpropan-1-one (**I-38-a**) (1 g, 3.26 mmol) was dissolved in 10 mL of N,N-dimethylformamide, 5-methyl-1H-pyrazole (321 mg, 3.91 mmol) and cesium carbonate (2.12 g, 6.52 mmol) were added at room temperature. The reaction solution was heated to 80 °C, stirred for 16 h and concentrated, and the residue was dissolved in 20 mL of ethyl acetate. The organic phase was washed with saturated brine (20 mL×2), dried over anhydrous sodium sulfate, filtered and concentrated to obtain 740 mg of a brown oil (**I-38-b**) with a yield of 73.7%. LC-MS: m/z: $(M+H)^+$ = 308.0.

**Step 2:**

1-(5-Bromo-2-pyridyl)-2-methyl-2-(3 -methylpyrazol-1-yl)propan-1-one (**I-38-b**) (740 mg, 2.40 mmol) and diphenylmethylamine (522 mg, 2.88 mmol) were dissolved in 15 ml of dioxane, and tris[dibenzylideneacetone]dipalladium (220 mg, 0.24 mmol), 4,5-bisdiphenyl phosphino-9,9-dimethylxanthene (277 mg, 0.48 mmol) and cesium carbonate (1.56 g, 4.80 mmol) were added. The reaction liquid was replaced with nitrogen three times, heated to 95 °C, stirred for 16 h and filtered and the filtrate was concentrated to obtain a residue, which was purified by column chromatography (methanol: dichloromethane = 0-6%) to obtain 800 mg of an off-white solid (**I-38-c**) with a yield of 81.56%. LC-MS: m/z: $(M+H)^+$ = 409.0.

**Step 3:**

1-[5-(Benzimido)-2-pyridyl]-2-methyl-2-(3-methylpyrazol-1-yl)propane-1-one **(I-38-c)** (600 mg, 1.47 mmol) was dissolved in 10 ml of methanol, and 1 mL of 1 N hydrochloric acid was added. The reaction solution was stirred at 15 °C for 10min and extracted with 20 mL of ethyl acetate, and the organic phase was wased with saturated aqueous sodium bicarbonate (10 mL), dried over anhydrous sodium sulfate, filtered and concentrated to obtain a residue, which was purified by column chromatography (dichloromethane: methanol=0-10%) to obtain 50 mg of an off-white solid (**I-38-d**) with a yield of 13.93%. LC-MS: m/z: $(M+H)^+$ =245.0.

**Step 4:**

1-(5-aminopyridin-2-yl)-2-methyl-2-(3-methyl-1H-pyrazol-1-yl)propane-1-one (**I-38-d**) (40 mg, 0.16 mmol) and 2-(4-ethylsulfonylphenyl)acetic acid (38 mg, 0.17 mmol) were dissolved in 3 ml of dichloromethane, and 64 mg of N,N-diisopropylethylamine and 158 mg 1-propylphosphoric anhydride (50% ethyl acetate solution) were added successively. The reaction solution was stirred at 15 °C for 16h, and concentrated, and then the residue was purified by thin-layer chromatography (dichloromethane: methanol=10:1) to obtain 15 mg of an off-white solid (**I-38**) with a yield of 20%. LC-MS: m/z: $(M+H)^+$= 455. $^1H$ NMR (400 MHz, $CDCl_3$) δ 9.52 (s, 1 H), 8.39 (s, 1 H), 7.86 (d, J = 6.0 Hz, 2 H), 7.77 (s, 1 H),

7.69 (s, 1 H), 7.58 (s, 2 H), 7.31 (d, J = 7.8 Hz, 1 H), 6.12 (s, 1 H), 3.91 (s, 2 H), 3.10 (dd, J = 14.6, 7.3 Hz, 2 H), 2.25 (s, 3 H), 1.46 (s, 3 H), 1.27 (t, J = 7.4 Hz, 3 H), 1.24(s, 3H).

**Example 39**

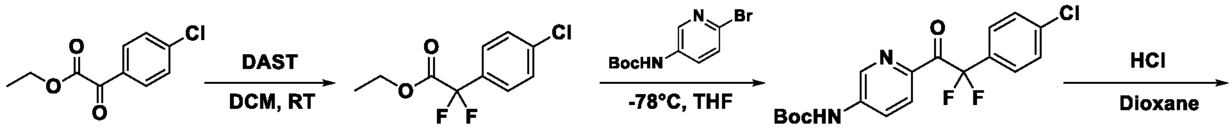


I-39-a I-39-b I-39-c

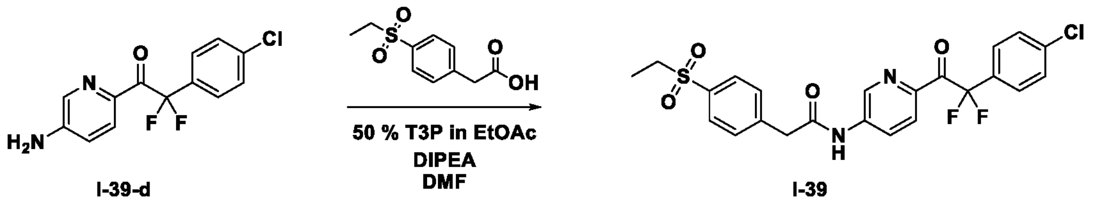


I-39-d I-39

**Step** 1:

Ethyl 2-(4-chlorophenyl)-2-oxalate (**I-39-a**) (10 g, 47.03 mmol) was dissolved in dry dichloromethane (100 mL), added slowly with DAST (8.34 g, 51.73 mmol) and stirred at room temperature for 16h. After the reaction was complete, the reaction mixture was quenched with saturated sodium bicarbonate solution (100 mL), the organic phase was separated, and the aqueous phase was extracted with ethyl acetate (300 mL×2). The organic phases were combined, washed with saturated sodium chloride solution (100 mL×2) and water (100 mL), dried over anhydrous sodium sulfate, concentrated and purified by column chromatography (petroleum ether: ethyl acetate = 10:1) to obtain ethyl 2-(4-chlorophenyl)-2,2-difluoroacetate (**I-39-b**) (7.3 g) as a yellow oil. $^1$H NMR (400 MHz, $CDCl_3$) δ 7.59 - 7.52 (m, 2 H), 7.47 - 7.41 (m, 2 H), 4.30 (q, J = 7.1 Hz, 2H), 1.38 - 1.22 (m, 3 H).

**Step 2:**

Tert-butyl (6-bromopyridin-3-yl)carbamate (1.0 g, 3.66 mmol) was put in anhydrous tetrahydrofuran (5 mL), cooled to -78 °C under nitrogen protection, and added with 2.5 mol/L n-butyllithium (3.08 mL, 7.69 mmol). After the reaction solution became turbid, it was stirred for 1h, added with a solution of ethyl 2-(4-chlorophenyl)-2,2-difluoroacetate (**I-39-b**) (688 mg, 2.93 mmol) in tetrahydrofuran (3 mL), and reacted at -78 °C for 2h. After the reaction was complete, the reaction solution was quenched with saturated sodium bicarbonate solution (10 mL), and the aqueous phase was extracted with ethyl acetate (30 mL×3). The organic phases were combined, washed with water (20 mL×1) and saturated sodium chloride solution (20 mL×2), dried over anhydrous sodium sulfate, concentrated and purified by column chromatography (dichloromethane: methanol = 25: 1) to obtain tert-butyl (6-(2-(4-chlorophenyl)-2,2-difluoroacetyl)pyridin-3-yl)carbamate (**I-39-c**) (250 mg) as a yellow oil. LCMS (ESI, m/z):$[M+H]^+$= 383.1.

**Step 3:**

Tert-butyl (6-(2-(4-chlorophenyl)-2,2-difluoroacetyl)pyridin-3-yl)carbamate (**I-39-c**) (250 mg) and HCl (4 M in dioxane, 5.00 mL) was mixed and stirred at room temperature for 1.5h. LCMS showed that the reacton was complete, and the reaction system was concentrated and purified by reverse phase preparative chromatography (C18 silica gel; ACN: $H_2O$ (containing 3% $NH_3 \cdot H_2O$), 15% to 30%) to obtain 1-(5-aminopyridin-2-yl)-2-(4-chlorophenyl)-2,2-difluoroethane-1-one (**I-39-d**) (110 mg) as a yellow solid. LCMS (ESI, m/z):$[M+H]^+$= 283.00, $^1$H NMR (300 MHz, DMSO-$d_6$) δ 7.92 - 7.79 (m, 2 H), 7.64 (d, *J* = 8.3 Hz, 2 H), 7.55 (d, *J* = 8.3 Hz, 2 H), 6.93 (dd, *J* = 8.7, 2.8 Hz, 1 H), 6.72 (s, 2 H).

**Step 4:**

1-(5-aminopyridin-2-yl)-2-(4-chlorophenyl)-2,2-difluoroethane-1-one (**I-39-d**) (16.3 mg, 0.058 mmol) was dissolved in DMF (5 mL), 2-(4-ethylsulfonylphenyl)acetic acid (16.1 mg, 0.071 mmol), diisopropylethylamine (116 mg, 0.898

mmol), and T3P (50% in ethyl acetae) (136 mg, 0.21372 mmol) were added sequentially, and the reaction system was stirred at room temperature for 16h. After the reaction was complete, the reaction system was quenched with saturated sodium bicarbonate solution (10 mL), and the aqueous phase was extracted with ethyl acetate (30 mL). The organic phases were combined, washed with water (20 mL) and saturated sodium chloride solution (20 mL), dried over anhydrous sodium sulfate, concentrated and purified by column chromatography (dichloromethane: methanol = 40:1) to obtain N-(6-(2-(4-chlorophenyl)-2,2-difluoroacetyl)pyridin-3-yl)-2-(4-(ethylsulfonyl)phenyl)ac etamide (**I- 39**) (9.6 mg) as a pale yellow solid. LCMS (ESI, m/z):$[M+H_2O+H]^+$= 511.1, $^1H$ NMR (400 MHz, $CDCl_3$) $^1H$ NMR (400 MHz, $CDCl_3$) δ 8.65 (s, 1H), 8.29 (d, J = 8.4 Hz, 1H), 8.19 - 8.02 (m, 2H), 7.83 (d, J = 7.6 Hz, 2H), 7.68 (d, J = 8.3 Hz, 2H), 7.50 (d, J = 7.5 Hz, 2H), 7.40 (d, J = 8.2 Hz, 2H), 3.87 (s, 2H), 3.15 (q, J = 7.3 Hz, 2H), 1.31 (t, J = 7.3 Hz, 1H).

### Example 40

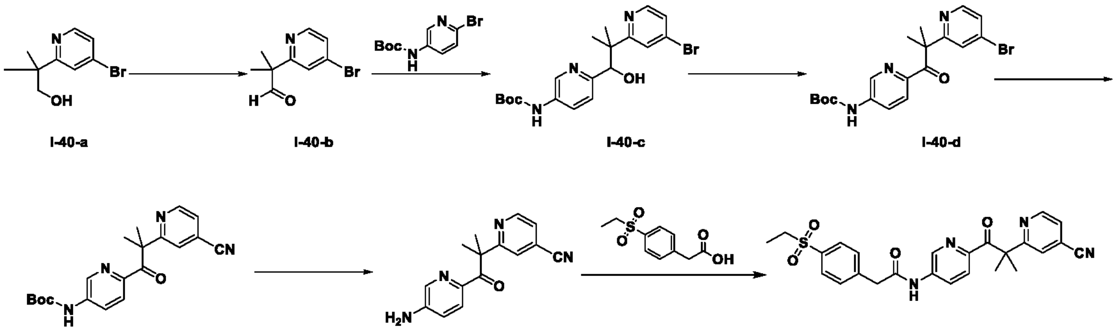

I-40-a I-40-b I-40-c I-40-d

I-40-e I-40-f I-40

**Step 1:**

Under nitrogen protection, a solution of oxalyl chloride (111 mg, 0.874 mmol) in dichloromethane (4 mL) was put in a 25 mL three-neck flask and cooled to -78 °C, and a solution of DMSO (102 mg, 1.311 mmol) in dichloromethane (2 mL) was added dropwise. After the addition, the reaction mixture was stirred for 30min, slowly added dropwise with a solution of 2-(4-bromopyridin-2-yl)-2-methylpropan-1-ol (**I-40-a**) (100 mg, 0.437 mmol) in dichloromethane (2 mL), stirred at -78 °C for 45min, then added with triethylamine (264 mg, 2.614 mmol), stirred for 1h and slowly raised to room temperature. After the reaction was complete, ethyl acetate (60 mL) was added to the system, and the organic phase was washed with water (20 mL×1) and saturated sodium chloride solution (20 mL×2), dried over anhydrous sodium sulfate, concentrated and purified by column chromatography (petroleum ether: ethyl acetate =3:1) to obtain 2-(4-bromopyridin-2-yl)-2-methylpropanal (**I-40-b**) (45 mg) as a yellow oil. LCMS (ESI, m/z):$[M+H]^+$= 228.0, 230.0.

**Step 2:**

Tert-butyl (6-bromopyridin-3-yl)carbamate (92 mg, 0.338 mmol) was added to 3 ml of tetrahydrofuran, slowly added with n-butyllithium (0.43 ml, 0.676 mmol) at -78 °C, and stirred at this temperature for 1h. Then a solution of 2-(4-bromopyridin-2-yl)-2-methylpropanal (**I-40-b**) (70 mg, 0.307 mmol) in 3 ml of tetrahydrofuran was slowly added, and the reaction solution was stirred at -78 °C for 1 h and then slowly raised to room temperature overnight. The reaction solution was quenched with saturated aqueous $NH_4Cl$ and extracted with ethyl acetate. The organic phase was washed once with saturated brine, dried over anhydrous sodium sulfate, concentrated and passed through a column (ethyl acetate: petroleum ether = 0-50%) to obtain 40 mg of tert-butyl (6-(2-(4-bromopyridin-2-yl)-1-hydroxy-2-methylpropyl)pyridin-3-yl)carbamate (**I-40-c**) as a white solid. LC-MS: m/z: $(M+H)^+$ =422.1, 424.1.

**Step 3:**

Tert-butyl (6-(2-(4-bromopyridin-2-yl)-1-hydroxy-2-methylpropyl)pyridin-3-yl)carbamate (**I-40-c**) (40 mg, 0.095 mmol) was dissolved in 5 ml of ethyl acetate, 100 mg of 2-iodoxybenzoic acid was added, and the reaction solution was refluxed and stirred for 16h. The reaction solution was filtered and concentrated to obtain 34 mg of tert-butyl (6-(2-(4-bromopyridin-2-yl)-2-methylpropionyl)pyridin-3-yl)carbamate (**I-40-d**) as a white solid. LC-MS: m/z: $(M+H)^+$ =420.1, 422.1.

**Step 4:**

34 mg of tert-butyl (6-(2-(4-bromopyridin-2-yl)-2-methylpropionyl)pyridin-3-yl)carbamate (**I-40-d**) was dissolved in dry DMF (10 mL), added with zinc cyanide (14 mg, 0.119 mmol), tris(dibenzylideneacetone)dipalladium ($Pd_2(dba)_3$, 3.7 mg, 0.0081 mmol) and 1,1'-bis(diphenylphosphino)ferrocene (dppf, 9 mg, 0.016 mmol), heated to 100 °C under argon protection and stirred for 4h. After the reaction was complete, the system was cooled to room temperature, and ethyl acetate (40 mL) was added. The organic phase was washed with saturated sodium chloride solution (20 mL×3) and water (20 mL×1), dried over anhydrous sodium sulfate, concentrated and purified by column chromatography (petroleum ether: ethyl acetate = 3:1) to obtain 12 mg of tert-butyl (6-(2-(4-cyanopyridin-2-yl)-2-methylpropionyl)pyridin-3-yl)carbamate (**I-40-e**) as a yellow oil. LC-MS: m/z: $(M+H)^+$ =367.1.

**Step 5:**

12 mg of tert-butyl (6-(2-(4-cyanopyridin-2-yl)-2-methylpropionyl)pyridin-3-yl)carbamate (**I-40-e**) was dissolved in 5 ml of dichloromethane, and 3 ml of trifluoroacetic acid was added. The reaction solution was stirred at 15 °C for 4h, and concentrated to obtain 15 mg of a crude 2-(1-(5-aminopyridin-2-yl)-2-methyl-1-oxo-propan-2-yl)isonicotinonitrile (**I-40-f**) as a yellow oil. LC-MS: m/z: $(M+H)^+$ = 267.1.

**Step 6:**

15 mg of the crude 2-(1-(5-aminopyridin-2-yl)-2-methyl-1-oxopropan-2-yl)isonicotinonitrile (**I-40-f**) in previous step, 2-(4-(ethylsulfonyl)phenyl)acetic acid (30 mg, 0.131 mmol) and triethylamine (60 mg, 0.59 mmol) were added to 10 ml of dichloromethane, added with TBTU (62 mg, 0.193 mmol), and stirred overnight at room temperature. The reaction solution was concentrated and passed through a column (ethyl acetate: petroleum ether = 0-100%) to obtain 6 mg of N-(6-(2-(4-cyanopyridin-2-yl)-2-methylpropionyl)pyridin-3-yl)-2-(4-(ethylsulfonyl)phe nyl)acetamide (**I-40**) as a white solid. LC-MS: m/z: $(M+H)^+$ =477.1, $^1H$ NMR (400 MHz, MeOD) δ 8.38 (dd, J = 5.0, 0.8 Hz, 1H), 8.36 (d, J = 2.2 Hz, 1H), 8.12 (dd, J = 8.7, 2.5 Hz, 1H), 8.03 (d, J = 8.6 Hz, 1H), 7.87 (dd, J = 8.5, 1.8 Hz, 3H), 7.59 (d, J = 8.4 Hz, 2H), 7.42 (dd, J = 5.0, 1.4 Hz, 1H), 3.84 (s, 2H), 3.19 (q, J = 7.4 Hz, 2H), 1.71 (s, 6H), 1.21 (t, J = 7.4 Hz, 3H).

**Example 41**

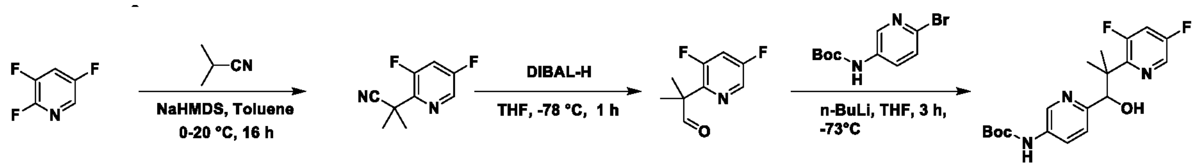


I-41-a I-41-b I-41-c I-41-d

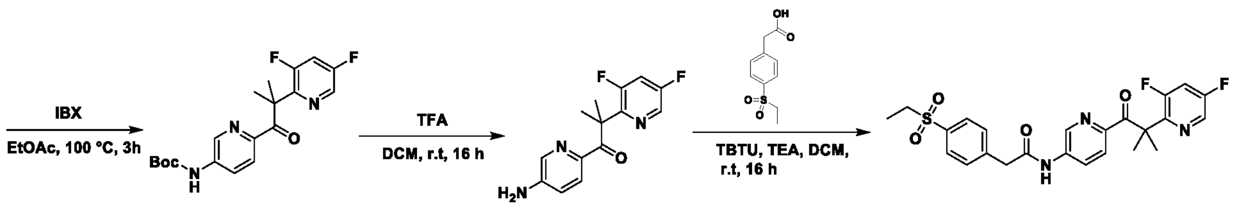


I-41-e I-41-f I-41

**Step** 1:

2,3,5-trifluoropyridine (3 g, 22.545 mmol) (**I-41-a**) and 2-methylpropionitrile (3.12 g, 45.2 mmol) were dissolved in toluene (20 mL), cooled to 0 °C, added dropwise with NaHMDS solution (11 mL, 22 mmol, 2 mol/L), and stirred at 20 °C for 16h. TLC (petroleum ether: ethyl acetate=10:1) showed that the product was formed. The system was concentrated to dryness and redissolved in ethyl acetate. The organic phase was quenched with saturated ammonium chloride, washed with water and brine, dried over magnesium sulfate and concentrated to dryness. The residue was purified by column chromatography (petroleum ether/ethyl acetate 0~100%) to obtain the targeted 2-(3,5-difluoro-2-pyridyl)-2-methyl-propionitrile (1.9 g, 10 mmol, yield 46%) (**I-41-b**) as an oil. $^1H$ NMR ($CDCl_3$):δ 8.32 (dd, J = 2.3, 0.8 Hz, 1H), 7.29

(ddd, J = 10.3, 7.8, 2.4 Hz, 1H), 1.81 (d, J = 0.9 Hz, 6H). LC-MS: m/z$(M+H)^+$ = 183.1.

**Step 2:**

2-(3,5-difluoro-2-pyridyl)-2-methyl-propionitrile (1.8 g, 9.9 mmol) (**I-41-b**) was dissolved in tetrahydrofuran (20 mL), replaced with argon and cooled to -78 °C, DIBAL-H (30 mL, 30 mmol, 1 mol/L) was added slowly, and the mixture was stirred at 33 °C for 16h. TLC (petroleum ether:ethyl acetate=10:1) showed that the reaction was complete. LCMS showed that the product was formed. The mixture was quenched with 1 N hydrochloric acid, and extracted with ethyl acetate (30 mL×2). The organic layer was washed with brine, dried over anhydrous sodium sulfate, filtered and concentrated to obtain 2-(3,5-difluoro-2-pyridyl)-2-methyl-propanal (680 mg, 3.6723 mmol, 37% yield) (**I-41-c**) as a pink oil. $^1$H-NMR ($CDCl_3$) δ 9.73 (d, J = 2.6 Hz, 1H), 8.34 (d, J = 2.4 Hz, 1H), 7.23 (ddd, J = 10.4, 8.0, 2.4 Hz, 1H), 1.53 (d, J = 1.0 Hz, 6H). LC-MS(ESI: m/z): 186.0$[M+H]^+$

**Step 3:**

Tert-butyl N-(6-bromo-3-pyridyl)carbamate (650 mg, 2.3798 mmol) was dissolved in tetrahydrofuran (6 mL), cooled to -78 °C, added slowly with n-butyllithium (3.2 mL, 1.6 mol/L), and stirred at -78 °C for 30min. 2-(3,5-difluoro-2-pyridyl)-2-methyl-propanal (660 mg, 3.5643 mmol) (**I -41-c**) was added, and the mixture was stirred at 30°C for 16h. LCMS showed the product was formed. TLC (petroleum ether: ethyl acetate = 10: 1) showed the formation of a new spot. The mixture was cooled in an ice water, quenched with saturated ammonium chloride, and extracted with ethyl acetate (50 ml×2). The organic layer was washed with brine, dried over anhydrous sodium sulfate, filtered and concentrated. The residue was purified by column chromatography ($SiO_2$: 10 g, eluent: Petroleum ether/ethyl acetate = 0-80%) to obtain tert-butyl N-[6-[2-(3,5-difluoro-2-pyridyl)-1-hydroxy-2-methyl-propyl]-3-pyridyl]carbamate **(I-41-d)** (270 mg, 0.7116 mmol, 29.90% yield) as a white solid. $^1$H-NMR ($CDCl_3$):δ 8.42 (d, J = 2.6 Hz, 1H), 8.29 (dd, J = 2.3, 1.1 Hz, 1H), 7.93 (d, J = 8.0 Hz, 1H), 7.19 (ddd, J = 11.5, 8.1, 2.5 Hz, 1H), 6.94 (d, J = 8.6 Hz, 1H), 6.70 (s, 1H), 5.26 (s, 1H), 1.55 (s, 9H), 1.43 (d, J = 2.2 Hz, 3H), 1.31 (d, J = 1.8 Hz, 3H). LC-MS: m/z $(M+H)^+$= 380.2.

**Step 4:**

Tert-butyl N-[6-[2-(3,5-difluoro-2-pyridyl)-1-hydroxy-2-methyl-propyl]-3-pyridyl]carbamate **(I-41-d)** (200 mg, 0.527 mmol) was dissolved in ethyl acetate (2 mL), added with 2-iodoxybenzoic acid (740 mg, 2.643 mmol), and stirred at 100 °C for 2h. LCMS showed the product was formed. TLC (petroleum ether:ethyl acetate=1:1) showed that the reaction was complete. The mixture was filtered, and the filtrate was concentrated to obtain tert-butyl N-[6-[2-(3,5-difluoro-2-pyridyl)-2-methyl-propionyl]-3-pyridyl]carbamate (**I-41-e**) (180 mg, 0.4769 mmol, 90.48% yield) as a white solid. $^1$H-NMR ($CDCl_3$): δ 8.30 (d, J = 2.4 Hz, 1H), 8.14 - 8.07 (m, 2H), 8.00 (dd, J = 8.6, 2.7 Hz, 1H), 6.97 (ddd, J = 10.5, 8.1, 2.4 Hz, 1H), 6.73 (s, 1H), 1.73 (d, J = 0.9 Hz, 6H), 1.52 (s, 9H). LC-MS: m/z$(M+H)^+$ = 378.1.

**Step 5:**

Tert-butyl N-[6-[2-(3,5-difluoro-2-pyridyl)-2-methyl-propionyl]-3-pyridyl]carbamate (**I-41-e**) (160 mg, 0.424 mmol) was dissolved in dichloromethane (4 mL), added with trifluoroacetic acid (2 mL), and stirred at 26 °C for 16h. LCMS showed the product was formed. The mixture was quenched with saturated sodium bicarbonate and extracted with dichloromethane (20 mL×2). The organic layer was washed with brine, dried over anhydrous sodium sulfate, filtered and concentrated to obtain 1-(5-amino-2-pyridyl)-2-(3,5-difluoro-2-pyridyl)-2-methyl-propan-1-one (**I-41-f**) (110 mg, 0.397 mmol, 93.57% yield) as a white solid. $^1$H-NMR ($CDCl_3$): δ 8.33 (d, J = 2.3 Hz, 1H), 7.95 (d, J = 8.3 Hz, 1H), 7.68 (s, 1H), 7.05 - 6.83 (m, 2H), 1.72 (s, 6H). LC-MS: m/z$(M+H)^+$= 278.1.

**Step 6:**

Tert-butyl N-[6-[2-(3,5-difluoro-2-pyridyl)-2-methyl-propionyl]-3-pyridyl]carbamate (**I-41-f**) (110 mg, 0.397 mmol), 2-(4-ethylsulfonylphenyl)acetic acid (104 mg, 0.456 mmol) and N,N-diisopropylethylamine (120 mg, 1.186 mmol,) were dissolved in dichloromethane (5 mL), added with 2-(1H-benzotriazole-1-yl)-1,1,3,3-tetramethyluronium tetrafluoroborate (TBTU) (146 mg, 0.455 mmol), and mixed and stirred at 30 °C for 16h. LCMS showed the product was formed. The mixture was concentrated and the residue was purified by column chromatography ($SiO_2$: 10 g, eluent: petroleum ether/ethyl acetate=0-80%) to obtain N-[6-[2-(3,5-difluoro-2-pyridyl)-2-methyl-propionyl]-3-pyridyl]-2-(4-ethylsulfonylphen yl)acetamide (**I-41**) (40 mg, 0.082 mmol, 20.68% yield) as a white solid. $^1$H-NMR ($CDCl_3$): δ 8.36 - 8.20 (m, 3H), 8.08 (d, J = 8.6 Hz, 1H), 7.92 - 7.83 (m, 2H), 7.53 (d, J = 8.2 Hz, 2H), 6.99 (ddd, J = 10.4, 8.0, 2.4 Hz, 1H), 3.86 (s, 2H), 3.14 (q, J = 7.4 Hz, 2H), 1.75 - 1.71 (m, 6H), 1.31 (t, J = 7.4 Hz, 3H). LC-MS: m/z $(M+H)^+$= 488.2.

**Example 42**

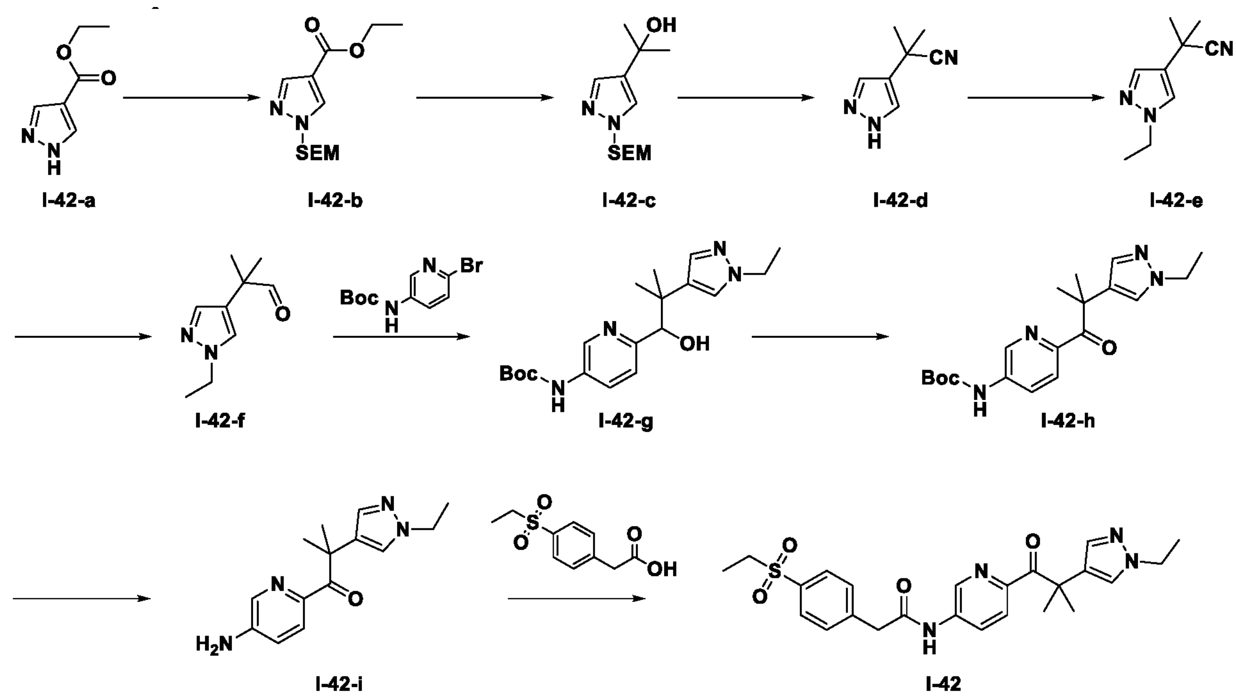

I-42-a I-42-b I-42-c I-42-d I-42-e I-42-f I-42-g I-42-h I-42-i I-42

**Step** 1:

Ethyl 1H-pyrazole-4-carboxylate (**I-42-a**) (2.8 g, 20 mmol) was dissolved in 45 ml of tetrahydrofuran, added with sodium hydride (1 g, 25 mmol, 60%) under an ice bath, stirred for 10min, added with 2-(trimethylsilyl)ethoxymethyl chloride (5 g, 30 mmol), and stirred at room temperature overnight. The reaction solution was added with 30 ml of saturated aqueous ammonium chloride and extracted with ethyl acetate twice. The organic phase was dried over anhydrous sodium sulfate and concentrated to obtain a crude, which was passed through a column (ethyl acetate:petroleum ether=0-30%) to obtain 5.1g of ethyl 1-((2-(trimethylsilyl)ethoxy)methyl)-1H-pyrazole-4-carboxylate **(I-42-b)** as a colorless liquid with a yield of 94%. LC-MS: m/z: $(M+H)^+$ =271. $^1H$ NMR (400 MHz, $CDCl_3$) δ 8.08 (s, 1H), 7.95 (s, 1H), 5.45 (s, 2H), 4.32 (q, J = 7.1 Hz, 2H), 3.64 - 3.54 (m, 2H), 1.37 (t, J = 7.1 Hz, 3H), 0.97 - 0.90 (m, 2H), 0.03 - -0.03 (m, 9H).

**Step 2:**

Ethyl 1-((2-(trimethylsilyl)ethoxy)methyl)-1H-pyrazole-4-carboxylate **(I-42-b)** (4.6 g, 17 mmol) was added to 30 ml of tetrahydrofuran, added with a solution of methylmagnesium chloride in tetrahydrofuran (17 ml, 51 mmol, 3 mol/L) in an ice bath, reacted for 1 h in the ice bath and then raised to room temperature overnight. The reaction solution was quenched with saturated aqueous ammonium chloride, and extracted with ethyl acetate. The organic phase was washed with saturated brine, dried over anhydrous sodium sulfate and concentrated to obtain 4.1g of a crude 2-(1-((2-(trimethylsilyl)ethoxy)methyl)-1H-pyrazol-4-yl)propan-2-ol (**I-42-c**) as a colorless liquid, which was directly used in the next step, with a yield of 94%. LC-MS: m/z: $(M+H)^+$ =257.

**Step 3:**

2-(1-((2-(trimethylsilyl)ethoxy)methyl)-1H-pyrazol-4-yl)propan-2-ol (4.2 g, 16.4 mmol) (**I-42-c**), and trimethylsilyl cyanide (9 g, 90.9 mmol) were added to 50 ml of dichloromethane, added with a solution of stannic tetrachloride in dichloromethane (16.5 ml, 16.5 mmol, 1 mol/L), and stirred at room temperature overnight. Then 35 mmol of aqueous potassium fluoride and 20 ml of saturated saline were added, and the reaction solution was stirred for 0.5h, filtered with diatomaceous earth. The organic layer was dried over anhydrous sodium sulfate and concentrated to obtain a crude, which was washed with a mixture of ethyl acetate and petroleum ether (ethyl acetate: petroleum ether=1:1), to obtain 1 g of 2-methyl-2-(1H-pyrazol-4-yl)propionitrile (**I-42-d**) as a white solid with a yield of 47%. LC-MS: m/z: $(M+H)^+$ =136.

$^1$H NMR (400 MHz, $CDCl_3$) δ 7.62 (dd, J = 17.9, 2.2 Hz, 2H), 1.72 (d, J = 2.3 Hz, 6H).

**Step 4:**

2-methyl-2-(1H-pyrazol-4-yl)propionitrile (**I-42-d**) (700 mg, 5.2 mmol) was added to 15 ml of tetrahydrofuran, added with sodium hydride (220 mg, 5.5 mmol) in an ice bath, stirred for 20min, added with iodomethane (1.6 g, 10.3 mmol), and stirred overnight at room temperature. The reaction solution was quenched with 10 ml of saturated aqueous $NH_4Cl$, and extracted with ethyl acetate. The organic phase was washed once with saturated brine, dried over anhydrous sodium sulfate, concentrated and passed through a column (methanol: dichloromethane=0-10%) to obtain 0.3 g of 2-(1-ethyl-1H-pyrazol-4-yl)-2-methylpropionitrile (**I-42-e**) as a white solid with a yield of 40%. LC-MS: m/z: $(M+H)^+$ =164.

**Step 5:**

2-(1-ethyl-1H-pyrazol-4-yl)-2-methylpropionitrile (**I-42-e**) (0.33 g, 2 mmol) was added to 5 ml of tetrahydrofuran, slowly added with diisobutylaluminum hydride (5 ml, 5 mmol) at -78 °C, stirred at this temperature for 2 h and then at room temperature overnight. The reaction solution was quenched with 2 mol/L hydrochloric acid and extracted twice with ethyl acetate (2×20 ml). The organic phase was dried over anhydrous sodium sulfate, and concentrated to obtain 0.25 g of 2-(1-ethyl-1H-pyrazol-4-yl)-2-methylpropanal (**I-42-f**) as a colorless oil with a yield of 74%, which was used directly in the next step. LC-MS: m/z: $(M+H)^+$ =167.

**Step 6:**

Tert-butyl (6-bromopyridin-3-yl)carbamate (0.54 g, 2 mmol) was added to 6 ml of tetrahydrofuran, slowly added with n-butyllithium (2.6 ml, 4.2 mmol) at -78 °C, stirred at this temperature for 1 h, slowly added with a solution of 2-(1-ethyl-1H-pyrazol-4-yl)-2-methylpropanal (0.3 g, 1.8 mmol) (**I-42-f**) in 5 ml of tetrahydrofuran, stirred at -78 °C for 2 h and slowly warmed to room temperature. The reaction solution was quenched with 20 ml of saturated aqueous $NH_4Cl$ and extracted with ethyl acetate, and the organic phase was washed once with saturated brine, dried over anhydrous sodium sulfate, concentrated, and passed through a column (ethyl acetate: petroleum ether=0-60%) to obtain 50 mg of tert-butyl (6-(2-(1-ethyl-1H-pyrazol-4-yl)-1-hydroxyl-2-methylpropyl)pyridin-3-yl)carbamate (**I-42-g**) as a white solid with a yield of 7.6%. LC-MS: m/z: $(M+H)^+$ =361.

**Step** 7:

Tert-butyl (6-(2-(1-ethyl-1H-pyrazol-4-yl)-1-hydroxyl-2-methylpropyl)pyridin-3-yl)carbamate (**I-42-g**) (50 mg, 0.14 mmol) was added to 20 ml of ethyl acetate, added with IBX (0.14 g, 0.48 mmol), and stirred overnight at reflux temperature. The reaction solution was filtered, concentrated, and separated by thin layer chromatography (ethyl acetate: petroleum ether = 2:1) to obtain 35 mg of tert-butyl (6-(2-(1-ethyl-1H-pyrazol-4-yl)-2-methylpropionyl)pyridin-3-yl)carbamate (**I-42-h**) as a white solid with a yield of 70%. LC-MS: m/z: $(M+H)^+$ =359.

**Step 8:**

Tert-butyl (6-(2-(1-ethyl-1H-pyrazol-4-yl)-2-methylpropionyl)pyridin-3-yl)carbamate (**I-42-h**) (35 mg, 0.1 mmol) was added to 3 ml of dichloromethane, added with 3 ml of trifluoroacetic acid, and stirred at room temperature for 1h. The reaction solution was concentrated, added with 10 ml of saturated aqueous sodium bicarbonate and extracted with dichloromethane (2×20 ml). The organic phase was dried over anhydrous sodium sulfate and concentrated to obtain 20 mg of 1-(5-aminopyridin-2-yl)-2-(1-ethyl-1H-pyrazol-4-yl)-2-methylpropan-1-one (**I-42-i**) as a off-white solid with a yield of 79%. LC-MS: m/z: $(M+H)^+$ =259.

**Step 9:**

1-(5-aminopyridin-2-yl)-2-(1-ethyl-1H-pyrazol-4-yl)-2-methylpropan-1-one (**I-42-i**) (20 mg, 0.08 mmol), 2-(4-(ethylsulfonyl)phenyl)acetic acid (25 mg, 0.1 mmol) and triethylamine (0.1 ml, 0.7 mmol) were added to 10 ml of dichloromethane, added with TBTU (35 mg, 0.11 mmol), and stirred overnight at room temperature. The reaction solution was concentrated and separated by thin layer chromatography (ethyl acetate: petroleum ether=3:1) to obtain 20 mg of N-(6-(2-(1-ethyl-1H-pyrazol-4-yl)-2-methylpropionyl)pyridin-3-yl)-2-(4-(ethylsulfonyl) phenyl)acetamide (**I-42**) as a white solid with a yield of 55%. LC-MS: m/z: $(M +H)^+$ =469, $^1$H NMR (400 MHz, $CDCl_3$) δ 8.99 (s, 1H), 8.55 (s, 1H), 8.22 (d, J = 8.6 Hz, 1H), 7.86 (dd, J = 8.5, 2.4 Hz, 3H), 7.57-7.49 (m, 3H), 7.31 (s, 1H), 4.10 (q, J = 7.3 Hz, 2H), 3.88 (s, 2H), 3.14 (q, J = 7.4 Hz, 2H), 1.75 (s, 6H), 1.44 (t, J = 7.3 Hz, 3H), 1.30 (td, J = 7.4, 3.2 Hz, 4H).

**Example 43**

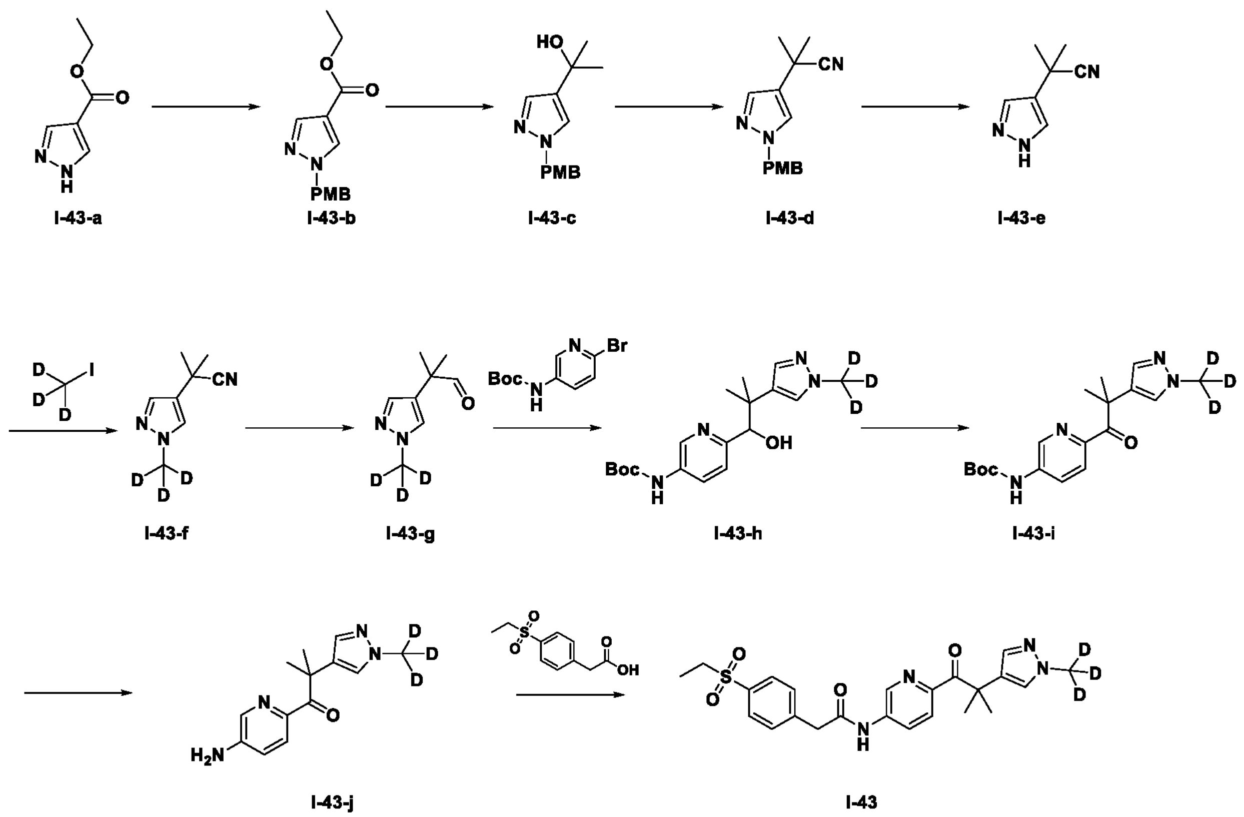

I-43-a I-43-b I-43-c I-43-d I-43-e

I-43-f I-43-g I-43-h I-43-i

I-43-j I-43

**Step 1:**

Ethyl 1H-pyrazole-4-carboxylate (**I-43-a**) (4.2 g, 30 mmol), 4-methoxybenzyl chloride (5.45 g, 34.8 mmol) and potassium carbonate (8.4 g, 61 mmol) were added into 60 ml of acetonitrile, and stirred overnight under refluxing. The reaction solution was filtered and concentrated to obtain a crude, which was passed through a column (ethyl acetate: petroleum ether=0-50%) to obtain 7.5 g of ethyl 1-(4-methoxybenzyl)-1H-pyrazole-4-carboxylate (**I-43-b**) as a colorless liquid with a yield of 96%. LC-MS: m/z: $(M+H)^+$ =261.

**Step 2:**

Ethyl 1-(4-methoxybenzyl)-1H-pyrazole-4-carboxylate (5.2 g, 20 mmol) (**I-43-b**) was added into 45 ml of tetrahydrofuran, added with a solution of methylmagnesium chloride in tetrahydrofuran (27 ml, 81 mmol, 3 mol/L) in an ice bath, reacted in the ice bath for 1 h and then raised to room temperature overnight. The reaction solution was quenched with saturated aqueous ammonium chloride and extracted with ethyl acetate, and the organic phase was washed with saturated brine, dried over anhydrous sodium sulfate and concentrated to obtain 4.5 g of a crude 2-(1-(4-methoxybenzyl)-1H-pyrazol-4-yl)propan-2-ol (**I-43-c**) as a colorless liquid, which was used directly in the next step, with a yield of 91%. LC-MS: m/z: $(M+H)^+$ =247.

**Step 3:**

2-(1-(4-methoxybenzyl)-1H-pyrazol-4-yl)propan-2-ol (**I-43-c**) (2.4 g, 9.7 mmol) and trimethylsilyl cyanide (5 g, 50 mmol) were added to 20 ml of dichloromethane, added with a solution of stannic tetrachloride in dichloromethane (10 ml, 10 mmol, 1 mol/L) in an ice bath under nitrogen protection, and stirred at room temperature overnight. The reaction solution was added with 20 mmol of aqueous potassium fluoride and 20 ml of saturated brine, stirred for 0.5h, and filterred with diatomaceous earth. The organic layer was dried over anhydrous sodium sulfate and concentrated to obtain a crude, which was passed through a column (ethyl acetate: petroleum ether = 0-50%) to obtain 1.3 g of 2-(1-(4-methoxybenzyl)-1H-pyrazol-4-yl)-2-methylpropionitrile (**I-43-d**) as a white solid with a yield of 52%. LC-MS: m/z: $(M+H)^+$

=256, $^{1}H$ NMR (400 MHz, $CDCl_3$) δ 7.51 (d, J = 0.6 Hz, 1H), 7.35 (d, J = 0.5 Hz, 1H), 7.25 - 7.20 (m, 2H), 6.95 - 6.89 (m, 2H), 5.22 (s, 2H), 3.83 (s, 3H), 1.67 (s, 6H).

**Step 4:**

2-(1-(4-methoxybenzyl)-1H-pyrazol-4-yl)-2-methylpropionitrile (**I-43-d**) (0.8 g, 3.1 mmol) was added to 8 ml of trifluoroacetic acid, and stirred overnight at 100°C. The reaction solution was concentrated, added with 5 ml of water and 5 ml of saturated aqueous sodium bicarbonate, and extracted twice with dichloromethane. The organic phase was dried, concentrated and passed through a column (methanol: dichloromethane =0-10%), to obtain 0.25 g of 2-methyl-2-(1H-pyrazol-4-yl)propionitrile (**I-43-e**) as a white solid with a yield of 59%. LC-MS: m/z: $(M+H)^+$ =136.

**Step 5:**

2-methyl-2-(1H-pyrazol-4-yl)propionitrile (**I-43-e**) (200 mg, 1.48 mol), iodomethane-d3 (240 mg, 1.65 mmol) and potassium carbonate (420 mg, 3 mmol) were added to 5 ml of N,N-dimethylformamide, and stirred overnight at 60 °C. The reaction solution was filtered, concentrated and passed through a column (methanol:dichloromethane=0-10%) to obtain 0.2 g of 2-methyl-2-(1-(methyl-d3)-1H-pyrazol-4-yl)propionitrile (**I-43-f**) as a white solid with a yield of 88%. LC-MS: m/z: $(M+H)^+$ =153.

**Step 6:**

2-Methyl-2-(1-(methyl-d3)-1H-pyrazol-4-yl)propionitrile (**I-43-f**) (0.30 g, 1.97 mmol) was added to 8 ml of THF, slowly added with diisobutylaluminum hydride (5 ml, 5 mmol) at -78 °C, stirred at this temperature for 2 h and then at room temperature overnight. The reaction solution was quenched with 2 mol/L hydrochloric acid, and extracted twice with ethyl acetate (2×20 ml). The organic phase was dried over anhydrous sodium sulfate, concentrated and passed through a column (methanol:dichloromethane=0-10%) to obtain 0.13 g of 2-methyl-2-(1-(methyl-d3)-1H-pyrazol-4-yl)propanal (**I-43-g**) as a colorless oil with a yield of 42%. LC-MS: m/z: $(M+H)^+$ =156.

**Step 7:**

Tert-butyl (6-bromopyridin-3-yl)carbamate (0.3 g, 1.1 mmol) was added to 3 ml of tetrahydrofuran, added slowly with n-butyllithium (1.5 ml, 2.4 mmol) at -78 °C, stirred at this temperature for 1 h, slowly added with a solution of 2-methyl-2-(1-(methyl-d3)-1H-pyrazol-4-yl)propanal (**I-43-g**) (0.13 g, 0.84 mmol) in 5 ml of tetrahydrofuran, stirred at -78 °C for 2 h and slowly warmed to room temperature. The reaction solution was quenched with 20 ml of saturated aqueous $NH_4Cl$, and extracted with ethyl acetate. The organic phase was washed with saturated brine once, dried over anhydrous sodium sulfate, concentrated and passed through a column (ethyl acetate: petroleum ether=0-100%) to obtain 120 mg of tert-butyl (6-(1-hydroxyl-2-methyl-2-(1-(methyl-d3)-1H-pyrazol-4-yl)propyl)pyridin-3-yl)carbam ate (**I-43-h**) as a white solid with a yield of 41%. LC-MS: m/z: $(M+H)^+$ =350.

**Step 8:**

Tert-butyl (6-(1-hydroxyl-2-methyl-2-(1-(methyl-d3)-1H-pyrazol-4-yl)propyl)pyridin-3-yl)carbam ate (**I-43-h**) (120 mg, 0.34 mmol) and IBX (0.2 g, 0.68 mmol) were added to 15 ml of ethyl acetate and stirred overnight at reflux temperature. The reaction solution was filtered, concentrated and separated by thin-layer chromatography (ethyl acetate: petroleum ether=2:1) to obtain 82 mg of tert-butyl (6-(2-methyl-2-(1-(methyl-d3)-1H-pyrazol-4-yl)propionyl)pyridin-3-yl)carbamate **(I-43-i)** as a white solid with a yield of 67%. LC-MS: m/z: $(M+H)^+$ =348.

**Step 9:**

Tert-butyl (6-(2-methyl-2-(1-(methyl-d3)-1H-pyrazol-4-yl)propionyl)pyridin-3-yl)carbamate **(I-43-i)** (82 mg, 0.24 mmol) was added to 3 ml of dichloromethane, added with 3 ml of trifluoroacetic acid, and stirred at room temperature for 1h. The reaction solution was concentrated, added with 10 ml of saturated sodium bicarbonate, extracted with dichloromethane (2×20 ml), dried over anhydrous sodium sulfate and concentrated to obtain 55 mg of 1-(5-aminopyridin-2-yl)-2-methyl-2-(1-(methyl-d3)-1H-pyrazol-4-yl)propan-1-one **(I-43-j)** as a off-white solid with a yield of 94%. LC-MS: m/z: $(M+H)^+$ =248.

**Tenth step:**

1-(5-aminopyridin-2-yl)-2-methyl-2-(1-(methyl-d3)-1H-pyrazol-4-yl)propan-1-one **(I-43-j)** (55 mg, 0.22 mmol), 2-(4-(ethylsulfonyl)phenyl)acetic acid (60 mg, 0.26 mmol) and triethylamine (0.1 ml, 0.7 mmol) were added to 10 ml of dichloromethane, added with TBTU (90 mg, 0.28 mmol), and stirred overnight at room temperature. The reaction solution was concentrated and passed through a column (ethyl acetate: petroleum ether=0-100%) to obtain 30 mg of 2-(4-(ethylsulfonyl)phenyl)-N-(6-(2-methyl-2-(1-(methyl-D3)-1H-pyrazol-4-yl)propion yl)pyridin-3-yl)acetamide (**I-43**) as a white solid with a yield of 29%. LC-MS: m/z: $(M+H)^+$ =458, $^1H$ NMR (400 MHz, $CDCl_3$) $\delta$ 9.03 (s, 1H), 8.50 (d, J = 2.3 Hz, 1H), 8.17 (dd, J = 8.7, 2.3 Hz, 1H), 7.83 (dd, J = 8.3, 7.4 Hz, 3H), 7.48 (dd, J = 8.8, 4.3 Hz, 3H), 7.24 (d, J = 0.5 Hz, 1H), 3.83 (s, 2H), 3.12 (q, J = 7.4 Hz, 2H), 1.72 (s, 6H), 1.28 (t, J = 7.4 Hz, 3H).

**Example 44**

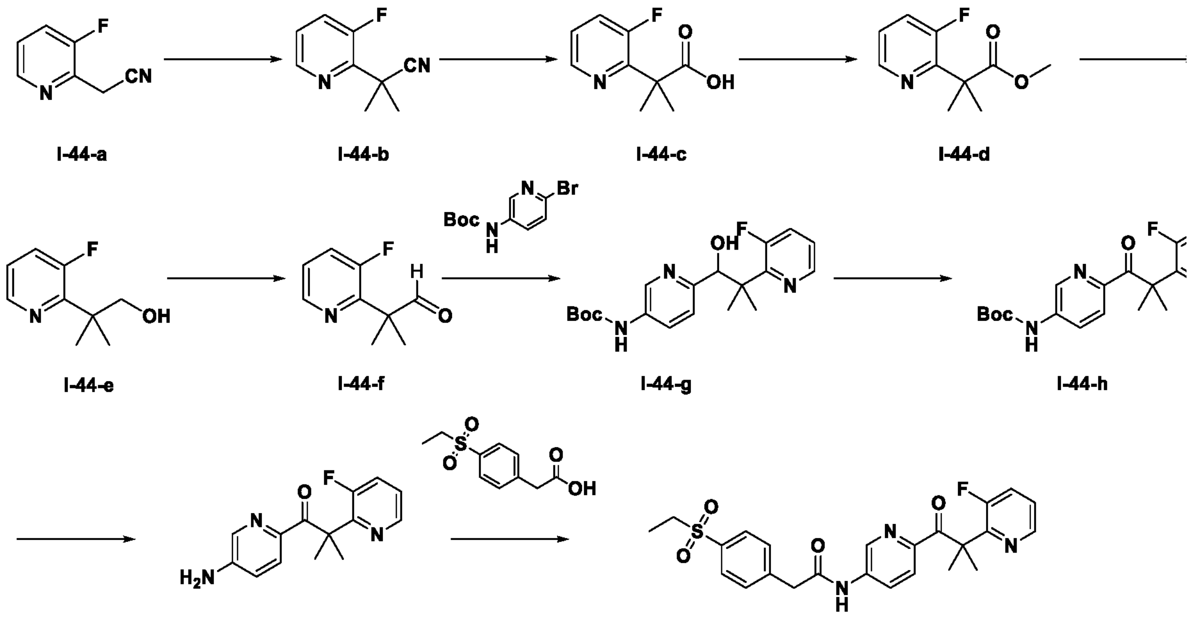

I-44-a I-44-b I-44-c I-44-d

I-44-e I-44-f I-44-g I-44-h

I-44-i I-44

**Step 1:**

2-(3-fluoropyridin-2-yl)acetonitrile (**I-44-a**) (820 mg, 6 mmol) was added into 20 ml of tetrahydrofuran, slowly added with a solution of lithium bistrimethylsilylamide in tetrahydrofuran (12.6 ml, 12.6 mmol, 1 mol/L) in an ice bath, stirred for 10min, added with iodomethane (2.5 g, 18 mmol) in the ice bath, and stirred overnight at room temperature. The reaction solution was quenched with saturated brine, and extracted twice with ethyl acetate. The organic phase was washed twice with saturated brine, dried over anhydrous sodium sulfate, concentrated, and passed through a column (ethyl acetate: petroleum ether=0-20%) to obtain 900 mg of 2-(3-fluoropyridin-2-yl)-2-methylpropionitrile (**I-44-b**) as a colorless oil with a yield of 91%. LC-MS: m/z: $(M+H)^+$ =165.

**Step 2:**

2-(3-fluoropyridin-2-yl)-2-methylpropionitrile (**I-44-b**) (900 mg, 5.48 mmol) was added into 6 ml of concentrated hydrochloric acid and stirred overnight at 105 °C. The reaction mixture was concentrated to obtain 1 g of 2-(3-fluoropyridin-2-yl)-2-methylpropionic acid (**I-44-c**) as a white solid, which was directly used in the next step, with a yield of 99%. LC-MS: m/z: $(M+H)^+$ =184.

**Step 3:**

2-(3-fluoropyridin-2-yl)-2-methylpropionic acid (**I-44-c**) (1 g, 5.46 mmol) was added into 30 ml of methanol, slowly added with thionyl chloride (3 ml) in an ice bath, and stirred overnight under refluxing. The reaction mixture was

concentrated, neutralized with saturated aqueous sodium bicarbonate, and extracted three times with ethyl acetate. The organic phase was dried over anhydrous sodium sulfate, and concentrated to obtain 1 g of methyl 2-(3-fluoropyridin-2-yl)-2-methylpropionate **(I-44-d)** as a colorless oil with a yield of 92%. LC-MS: m/z: $(M+H)^+$=198.

**Step 4:**

Methyl 2-(3-fluoropyridin-2-yl)-2-methylpropionate **(I-44-d)** (400 mg, 2 mmol) was added into 10 ml of tetrahydrofuran, slowly added with a solution of lithium aluminum tetrahydride in tetrahydrofuran (2.2 ml, 2.2 mmol, 1 mol/L) at 0 °C, and stirred at this temperature for 1h. The reaction solution was quenched with saturated aqueous ammonium chloride, and extracted twice with ethyl acetate (2×10 ml). The organic phase was dried over anhydrous sodium sulfate and concentrated to obtain 0.32 g of 2-(3-fluoropyridin-2-yl)-2-methylpropan-1-ol (**I-44-e**) as a colorless oil with a yield of 93%. LC-MS: m/z: $(M+H)^+$ =170.

**Step 5:**

2-(3-fluoropyridin-2-yl)-2-methylpropan-1-ol (**I-44-e**) (400 mg, 2.36 mmol) was added into 50 ml of dichloromethane, added with Dess-Martin oxidant (2 g, 4.7 mmol) in an ice bath, and stirred at room temperature for 1h. The reaction solution was quenched with saturated aqueous sodium bicarbonate and 5 mmol of aqueous sodium thiosulfate, and the aqueous phase was extracted twice with dichloromethane. The combined organic phase was dried over anhydrous sodium sulfate and passed through a column (ethyl acetate: petroleum ether=0-50%) to obtain 240 mg 2-(3-fluoropyridin-2-yl)-2-methylpropanal (**I-44-f**) as a colorless oil with a yield of 60%. LC-MS: m/z: $(M+H)^+$ =168.

**Step 6:**

Tert-butyl (6-bromopyridin-3-yl)carbamate (410 mg, 1.5 mmol) was added to 4 ml of tetrahydrofuran, slowly added with n-butyllithium (2 ml, 3.2 mmol) at -78 °C, stirred at this temperature for 1 h, and slowly added with a solution of 2-(3-fluoropyridin-2-yl)-2-methylpropanal (**I-44-f)** (240 mg, 1.4 mmol) in 5 ml of THF. After stirred at -78 °C for 1 h, the reaction solution was quenched with 10 ml of saturated aqueous $NH_4Cl$, and extracted with ethyl acetate. The organic phase was washed once with saturated brine, dried over anhydrous sodium sulfate, concentrated and passed through a column (dichloromethane: methyl alcohol=0-10%) to obtain 270 mg of tert-butyl (6-(2-(3-fluoropyridin-2-yl)-1-hydroxyl-2-methylpropyl)pyridin-3-yl)carbamate (**I-44-g**) as a white solid with a yield of 52%. LC-MS: m/z: $(M+H)^+$ =362.

**Step 7:**

Tert-butyl (6-(2-(3-fluoropyridin-2-yl)-1-hydroxy-2-methylpropyl)pyridin-3-yl)carbamate (**I-44-g**) (270 mg, 0.75 mmol) was added to 20 ml of ethyl acetate, added with IBX (410 mg, 1.46 mmol), and stirred overnight at reflux temperature. The reaction solution was filtered, concentrated and passed through a column (ethyl acetate:petroleum ether=0-50%) to obtain 200 mg of tert-butyl (6-(2-(3-fluoropyridin-2-yl)-2-methylpropionyl)pyridin-3-yl)carbamate (**I-44-h**) as a white solid with a yield of 74%. LC-MS: m/z: $(M+H)^+$ =360.

**Step 8:**

Tert-butyl (6-(2-(3-fluoropyridin-2-yl)-2-methylpropanoyl)pyridin-3-yl)carbamate **(I-44-h)** (200 mg, 0.55 mmol) was added to 3 ml of dichloromethane, added with 3 ml of trifluoroacetic acid, and stirred at room temperature for 1h. The reaction solution was concentrated, added with 10 ml of saturated aqueous sodium carbonate, and extracted with dichloromethane (2×20 ml). The organic phase was dried over anhydrous sodium sulfate and concentrated to obtain 120 mg of 1-(5-aminopyridin-2-yl)-2-(3-fluoropyridin-2-yl)-2-methylpropan-1-one (**I-44-i**) as a off-white solid with a yield of 83%. LC-MS: m/z: $(M+H)^+$ =260.

**Step 9:**

1-(5-aminopyridin-2-yl)-2-(3-fluoropyridin-2-yl)-2-methylpropan-1-one (**I-44-i**) (120 mg, 0.46 mmol), 2-(4-(ethylsulfonyl)phenyl)acetic acid (130 mg, 0.57 mmol) and triethylamine (0.4 ml, 3 mmol) were added to 15 ml of dichloromethane, added with a 50% solution of $T_3P$ (700 mg, 110 mmol) in ethyl acetate , and stirred at room temperature for 1h. The reaction solution was concentrated and passed through a column (dichloromethane: methanol = 0-10%) to obtain 150 mg of 2-(4-(ethylsulfonyl)phenyl)-N-(6-(2-(3-fluoropyridin-2-yl)-2-methylpropionyl)pyridin-3 -yl)acetamide (**I-44**) as a white solid with a yield of 69%. LC-MS: m/z: $(M+H)^+$ =470, $^1$H NMR (400 MHz, $CDCl_3$) δ 9.87 (s, 1H), 8.52 (dd, *J* = 8.7, 2.4 Hz, 1H), 8.23 (dd, *J* = 3.3, 1.4 Hz, 1H), 8.20 (d, *J* = 2.3 Hz, 1H), 8.10 (d, *J* = 8.7 Hz, 1H), 7.89 - 7.80 (m, 2H),

7.51 (d, $J$ = 8.3 Hz, 2H), 7.25 - 7.10 (m, 2H), 3.90 (s, 2H), 3.10 (q, $J$ = 7.4 Hz, 2H), 1.75 (s, 6H), 1.27 (t, $J$ = 7.4 Hz, 3H).

Example 45

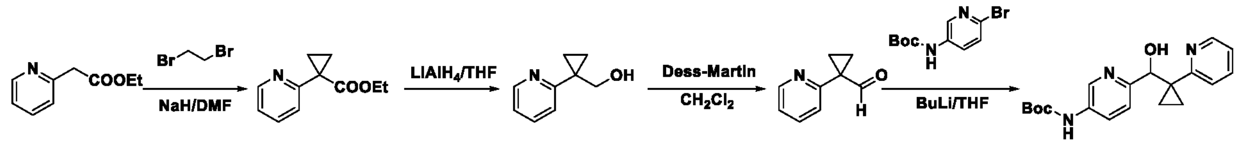


I-45-a I-45-b I-45-c I-45-d I-45-e

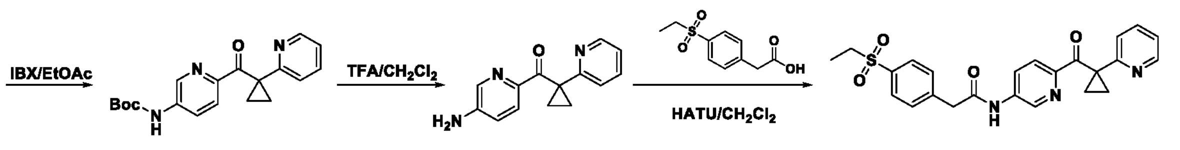


I-45-f I-45-g I-45

**Step 1:**

Ethyl 2-(2-pyridyl)acetate (**I-45-a**) (5.0 g, 30 mmol) was dissolved in N,N-dimethylformamide (30 mL), cooled to 0 °C, added with sodium hydride (1.8 g, 76 mmol) under nitrogen protection, and stirred at 0 °C for 0.5h. Then 1,2-dibromoethane (11.0 g, 61 mmol) was added. After the addition, the reaction solution was stirred at room temperature for 2h, quenched with water and extracted with ethyl acetate (50 mL×3). The organic phase was washed with saturated brine, dried over anhydrous sodium sulfate, filtered, and evaporated to dryness to obtain a crude, which was separated by silica gel column chromatography (petroleum ether/ethyl acetate=0~50%) to obtain targeted ethyl 1-(pyridin-2-yl)cyclopropane-1-carboxylate (**I-45-b**) (2.3 g, 40%). LC-MS: 192.1[M+H]$^+$.

**Step 2:**

Ethyl 1-(pyridin-2-yl)cyclopropane-1-carboxylate (**I-45-b**) (2.3 g, 12 mmol) was dissolved in tetrahydrofuran (20 mL) under nitrogen protection, cooled to 0°C, and added with a solution of lithium aluminum hydride in tetrahydrofuran (14 mL, 1.0 mol/L). After the addition, the reaction solution was stirred at room temperature for 16h, quenched with saturated aqueous ammonium chloride (50 mL) and extracted with ethyl acetate (50 mL×3). The organic phase was washed with saturated brine, dried over anhydrous sodium sulfate, filtered, and evaporated to dryness to obtain a crude, which was separated by silica gel column chromatography (petroleum ether/acetic acid Ethyl ester = 0-50%) to obtain the targeted (1-(pyridin-2-yl)cyclopropyl)methanol (**I-45-c**) (1.35 g, 75%). LC-MS: 150.1[M+H]$^+$.

**Step 3:**

(1-(pyridin-2-yl)cyclopropyl)methanol (**I-45-c**) (1.35 g, 9.05 mmol) was dissolved in dichloromethane (15 mL), added with Dess-Martin oxidant (4.22 g, 9.95 mmol), and stirred at room temperature for 2h. The reaction solution was filtered, and the filtrate was evaporated to dryness to obtain a crude, which was separated by silica gel column chromatography (petroleum ether/ethyl acetate=0~50%) to obtain the targeted 1-(2-pyridyl)cyclopropane carbaldehyde (**I-45-d**) (650 mg, 48.8%). LC-MS: 148.1[M+H]$^+$.

**Step 4:**

Tert-butyl (6-bromopyridin-3-yl)carbamate (1.27 g, 4.64 mmol) was dissolved in tetrahydrofuran (13 mL) under nitrogen protection, cooled to -78 °C, added with a solution of n-butyllithium in tetrahydrofuran (5.8 mL, 1.6 mol/L) while keeping the temperature below -78 °C during the dropwise addition, and stirred at -78 °C for 0.5h. Then 1-(2-pyridyl)cyclopropanecarbaldehyde (**I-45-d**) (650 mg, 4.42 mmol) was added, and the reaction solution was stirred at room temperature for 16h. The reaction solution was quenched with saturated aqueous ammonium chloride (50 ml), and extracted with ethyl acetate (50 mL×3). The organic phase was washed with saturated brine, dried over anhydrous sodium sulfate, filtered, and evaporated to dryness to obtain a crude, which was separated by silica gel column chromatography (petroleum ether/ethyl acetate = 0~50%) to obtain the targeted tert-butyl 6-(hydroxy(1-(pyridin-2-yl)cyclopropyl)methyl)pyridin-3-yl)carbamate (**I-45-e**) (520 mg, 34.5%). LC-MS: 356.2[M+H]$^+$.

**Step 5:**

Tert-butyl (6-(hydroxy(1-(pyridin-2-yl)cyclopropyl)methyl)pyridin-3-yl)carbamate (**I-45-e**) (0.52 g, 1.52 mmol) was dissolved in ethyl acetate (40 mL), added with 2-iodobenzoic acid (0.64 g, 2.29 mmol), heated to reflux and stirred to react overnight. The reaction solution was filtered, and the filtrate was evaporated to dryness to obtain a crude, which was separated by silica gel column chromatography (petroleum ether/ethyl acetate=0~50%) to obtain the targeted tert-butyl (6-(1-(pyridin-2-yl)cyclopropane-1-carbonyl)pyridin-3-yl)carbamate (**1-45-f**) (480 mg, 92.9%). LC-MS: 340.1[M+H]$^+$.

**Step 6:**

Tert-butyl (6-(1-(pyridin-2-yl)cyclopropane-1-carbonyl)pyridin-3-yl)carbamate (**1-45-f**) (0.48 g, 1.41 mmol) was dissolved in dichloromethane (20 mL), added with trifluoroacetic acid (20 mL), and stirred overnight. The reaction solution was evaporated to dryness to obtain a crude (5-aminopyridin-2-yl)(1-(pyridin-2-yl)cyclopropyl)methanone (**I-45-g**) (320 mg, 94.6%), which is the targeted compound. LC-MS: 240.1[M+H]$^+$.

**Step 7:**

(5-aminopyridin-2-yl)(1-(pyridin-2-yl)cyclopropyl)methanone (**I-45-g**) (100 mg, 0.42 mmol) was dissolved in dichloromethane (20 mL), and 2-(4-(ethylsulfonyl)phenyl)acetic acid (105 mg, 0.46 mmol), N-ethyl-N-isopropyl-2-propanamine (108 mg, 0.84 mmol) and 1-propylphosphoric anhydride (199 mg, 0.63 mmol) were successively added. The reaction solution was stirred overnight at room temperature, and evaporated to dryness to obtain a crude, which was separated by silica gel column chromatography (dichloromethane/methanol=0~5%) to obtain the targeted 2-(4-(ethylsulfonyl)phenyl)-N-(6-(1-(pyridin-2-yl)cyclopropane-1-carbonyl)pyridin-3-yl )acetamide (**I-45**)(52 mg, 27.7%). LC-MS: 450.1[M+H]$^+$. $^1$H NMR (400 MHz, DMSO-$d_6$) δ 10.68 (s, 1H), 8.49 (d, J = 2.3 Hz, 1H), 8.30-8.23 (m, 1H), 8.16 (dd, J =8.6, 2.4 Hz, 1H), 7.92 (d, J = 8.6 Hz, 1H), 7.84 (d, J = 8.3 Hz, 2H), 7.61 (dd, J = 12.7, 4.9 Hz, 3H), 7.09(dd, J = 7.5, 4.0 Hz, 2H), 3.85 (s, 2H), 3.28 (q, J = 7.4 Hz, 2H), 1.53 (qd,J = 8.3, 5.6 Hz, 4H), 1.10 (t, J =7.3 Hz, 3H).

**Example 46**

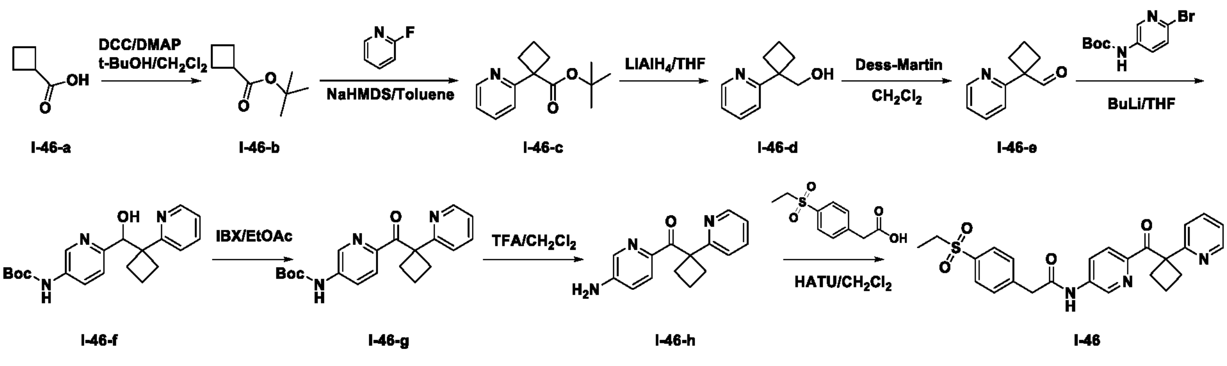

I-46-a I-46-b I-46-c I-46-d I-46-e
I-46-f I-46-g I-46-h I-46

**Step 1:**

Cyclobutanecarboxylic acid (**I-46-a**) (5.0 g, 50.0 mmol) was dissolved in dichloromethane (40 mL), added with dicyclohexylcarbodiimide (11.0 g, 55.5 mmol), 4-dimethylaminopyridine (0.61 g, 5.0 mmol) and tert-butanol (7.4 g, 100.0 mmol), and stirred at room temperature for 16h. The reaction solution was concentrated and separated by silica gel column chromatography (petroleum ether/ethyl acetate = 0-10%) to obtain the targeted tert-butyl cyclobutylcarboxylate (**I-46-b**) (6.0 g, 77%). $^1$H NMR (400 MHz, $CDCl_3$) δ 3.03 (d, J = 0.6 Hz, 1H), 2.29-2.08 (m, 4H), 1.91 (d, J = 8.4 Hz, 2H), 1.45(s, 10 H).

**Step 2:**

Tert-butyl cyclobutylcarboxylate (**I-46-b**) (1.6 g, 10.0 mmol) and 2-fluoropyridine (1.0 g, 10.0 mmol) were dissolved in toluene (20 mL), added with a solution of sodium bis(trimethylsilyl)amide in tetrahydrofuran (11.0 mL, 1.0 mol/L) under nitrogen protection, and stirred at room temperature for 16h. The reaction solution was quenched with saturated aqueous ammonium chloride (100 mL) and extracted with ethyl acetate (50 mL×3). The organic phase was washed with saturated brine, dried over anhydrous sodium sulfate, filtered, and evaporated to dryness to obtain a crude, which was separated by silica gel column chromatography (petroleum ether/ethyl acetate = 0~50%) to obtain the targeted tert-butyl 1-(pyridin-2-yl)cyclobutane-1-carboxylate (**I-46-c**) (1.55 g, 65%). LC-MS: 234.1$[M+H]^+$.

**Step 3:**

Tert-butyl 1-(pyridin-2-yl)cyclobutane-1-carboxylate (**I-46-c**) (1.4 g, 6.0 mmol) was dissolved in tetrahydrofuran (20 mL) under nitrogen protection, cooled to 0 °C, and added with a solution of lithium aluminum hydride in tetrahydrofuran (7.2 mL, 1.0 mol/L). After the addition, the reaction solution was stirred at room temperature for 16h, quenched with saturated aqueous ammonium chloride (50 mL) and extracted with ethyl acetate (50 mL×3). The organic phase was washed with saturated brine, dried over anhydrous sodium sulfate, filtered, and evaporated to dryness to obtain a crude, which was separated by silica gel column chromatography (petroleum ether/ Ethyl acetate=0~50%) to obtain the targeted (1-(pyridin-2-yl)cyclobutyl)methanol (**I-46-d**) (0.9 g, 90%). LC-MS: 164.1$[M+H]^+$.

**Step 4:**

(1-(pyridin-2-yl)cyclobutyl)methanol (**I-46-d**) (0.9 g, 5.51 mmol) was dissolved in dichloromethane (15 mL), added with Dess-Martin oxidant (2.57 g, 6.07 mmol), and stirred at room temperature for 2h. The reaction solution was filtered, and the filtrate was evaporated to dryness to obtain a crude, which was separated by silica gel column chromatography (petroleum ether/ethyl acetate=0~50%) to obtain the targeted 1-(pyridin-2-yl)cyclobutane-1-carbaldehyde (**I-46-e**) (550 mg, 61.9%). LC-MS: 162.1$[M+H]^+$.

**Step 5:**

Tert-butyl (6-bromopyridin-3-yl)carbamate (0.98 g, 3.58 mmol) was dissolved in tetrahydrofuran (10 mL) under nitrogen protection, cooled to -78 °C, added with a solution of n-butyllithium in tetrahydrofuran (4.5 mL, 1.6 mol/L) while keeping the temperature below -78 °C during the dropwise addition, and stirred at -78 °C for 0.5h. Then 1-(pyridin-2-yl)cyclobutane-1-carbaldehyde (**I-46-e**) (550 mg, 3.41 mmol) was added, and the reaction solution was stirred at room temperature for 16h, quenched with saturated aqueous ammonium chloride (50 ml) and extracted with ethyl acetate (50 mL×3). The organic phase was washed with saturated brine, dried over anhydrous sodium sulfate, filtered, and evaporated to dryness to obtain a crude, which was separated by silica gel column chromatography (petroleum ether/ethyl acetate = 0~50%) to obtain the targeted tert-butyl (6-(hydroxyl(1-(pyridin-2-yl)cyclobutyl)methyl)pyridin-3-yl)carbamate (**I-46-f**) (750 mg, 61.9%). LC-MS: 356.2[M+H]+.

**Step 6:**

Tert-butyl (6-(hydroxy(1-(pyridin-2-yl)cyclobutyl)methyl)pyridin-3-yl)carbamate (**I-46-f**) (0.75 g, 2.11 mmol) was dissolved in ethyl acetate (40 mL), added with 2-iodobenzoic acid (0.89 g, 3.17 mmol), heated to reflux and stirred to react overnight. The reaction solution was filtered, and the filtrate was evaporated to dryness to obtain a crude, which was separated by silica gel column chromatography (petroleum ether/ethyl acetate=0~50%) to obtain the targeted tert-butyl (6-(1-(pyridin-2-yl)cyclobutane-1-carbonyl)pyridin-3-yl)carbamate (**I-46-g**) (620 mg, 83.1%). LC-MS: 354.2$[M+H]^+$.

**Step 7:**

Tert-butyl (6-(1-(pyridin-2-yl)cyclobutane-1-carbonyl)pyridin-3-yl)carbamate (**I-46-g**) (0.62 g, 1.75 mmol) was dissolved in dichloromethane (20 mL), added with trifluoroacetic acid (20 mL), and stirred overnight. The reaction solution was evaporated to dryness to obtain a crude (5-aminopyridin-2-yl)(1-(pyridin-2-yl)cyclobutyl)methanone (**I-46-h**) (320 mg, 72.0%). LC-MS: 254.2$[M+H]^+$.

**Step 8:**

(5-aminopyridin-2-yl)(1-(pyridin-2-yl)cyclobutyl)methanone (**I-46-h**) (100 mg, 0.39 mmol) was dissolved in

dichloromethane (20 mL), added with 2-(4-(ethylsulfonyl)phenyl)acetic acid (99 mg, 0.43 mmol), N-ethyl-N-isopropyl-2-propanamine (102 mg, 0.79 mmol) and 1-propylphosphoric anhydride (188 mg, 0.59 mmol) successively, and stirred overnight at room temperature. The reaction solution was evaporated to dryness to obtain a crude, which was separated by silica gel column chromatography (dichloromethane/methanol=0~5%) to obtain the targeted 2-(4-(ethylsulfonyl)phenyl)-N-(6-(1-(pyridin-2-yl)cyclobutane-1-carbonyl)pyridin-3-yl) acetamide (**I-46**)(62 mg, 33.9%). LC-MS: 464.1[M+H]$^+$. $^1$H NMR (400 MHz, DMSO-$d_6$) δ 10.66 (s, 1H), 8.51 (s, 1H), 8.32 (s, 1H), 8.12 (d, J = 7.6 Hz, 1H), 8.00 (s,1H), 7.91-7.71 (m, 3H), 7.59 (s, 3H), 7.13 (s, 1H), 3.84 (s, 2H), 3.27 (d, J = 6.6 Hz, 2H), 2.79 (s, 2H),2.56 (s, 2H), 2.06 (s, 1H), 1.86 (s, 1H), 1.09 (s, 3H).

## Example 47

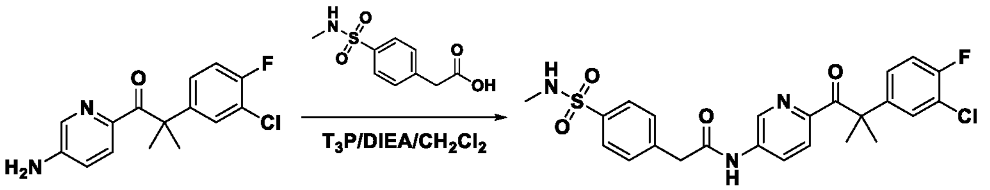


I-6-f I-47

**Step 1:**

1-(5-aminopyridin-2-yl)-2-(3-chloro-4-fluorophenyl)-2-methylpropan-1-one (**I-6-f**) (80 mg, 0.27 mmol) was dissolved in dichloromethane (20 mL), added with 2-(4-(N-methylsulfamoyl)phenyl)acetic acid (69 mg, 0.30 mmol), N-ethyl-N-isopropyl-2-propylamine (71 mg, 0.57 mmol) and 1-propylphosphoric anhydride (130 mg, 0.41 mmol) successively, and stirred overnight at room temperature. The reaction solution was evaporated to dryness to obtain a crude, which was separated by silica gel column chromatography (dichloromethane / methanol = 0~5%) to obtain the targeted N-(6-(2-(3-chloro-4-fluorophenyl)-2-methylpropionyl)pyridin-3-yl)-2-(4-(Methylsulfon yl)phenyl)acetamide (**I-47**) (28 mg, 20.3%). LC-MS: 504.1[M+H]$^+$. $^1$H NMR (400 MHz, DMSO-$d_6$) δ 10.69 (s, 1H), 8.53 (d, J = 2.2 Hz, 1H), 8.13 (dd, J = 8.7, 2.3 Hz, 1H),7.98 (d, J = 8.7 Hz, 1H), 7.73 (d, J = 8.1 Hz, 2H), 7.53 (d, J = 8.1 Hz, 2H), 7.41 (t, J = 4.8 Hz, 2H), 7.26 (t,J = 8.9 Hz, 1H), 7.18-7.07 (m, 1H), 3.82 (s, 2H), 2.41 (d, J = 4.9 Hz, 3H), 1.65 (s, 6H).

## Example 48

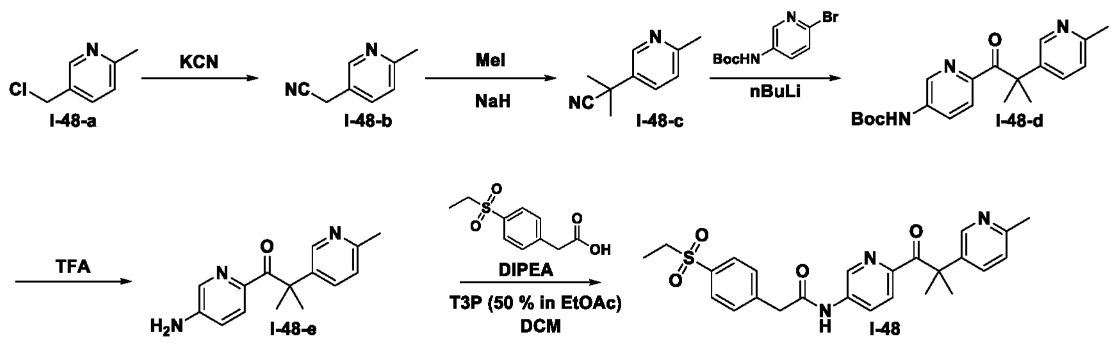


I-48-a I-48-b I-48-c I-48-d I-48-e I-48

**Step 1:**

5-(Chloromethyl)-2-methylpyridine (**I-48-a**) (5 g, 35.311 mmol) was dissolved in MeOH (66 mL) and water (33 mL), and KCN (3.45 g, 52.966 mmol) was added in portions at room temperature. The reaction system was heated to 70 °C and stirred for 18h. After the reaction was complete, the system was cooled to room temperature, quenched with saturated aqueous $FeSO_4$, and extracted with ethyl acetate (3 × 100 mL). The organic phases were combined, dried over anhydrous $Na_2SO_4$, filtered and concentrated, and the concentrate was separated by column chromatography, using dichloromethane/ethyl acetate (4:1), to obtain 2-(6-methylpyridin-3-yl)acetonitrile (**I-48-b**) (1.1 g, 24%) as a yellow

oil. LCMS: (ESI, m/z): $[M+H]^+$= 133.1.

**Step 2:**

2-(6-methylpyridin-3-yl)acetonitrile (**I-48-b**) (1.1 g, 8.323 mmol) was dissolved in dry tetrahydrofuran (10 mL) and cooled to 0 °C, and sodium hydride (0.83 g, 20.808 mmol, 60%) was added in batches. The reaction system was raised to room temperature and stirred for 0.5 h, then added with iodomethane (3.54 g, 24.969 mmol), and stirred at room temperature for 1h. LCMS showed that the raw material disappeared, and the reaction system was cooled to 0 °C, and quenched with saturated aqueous $NH_4Cl$ at this temperature. The organic phase was separated, and the aqueous phase was extracted with ethyl acetate (2 × 50 mL). The organic phases were combined, dried over anhydrous $Na_2SO_4$ and concentrated, and the resulting residue was purified by column chromatography (petroleum ether/ethyl acetate=3:1), to obtain 2-methyl-2-(6-methylpyridin-3-yl)propionitrile (**I-48-c**) (1.05 g, 79%) as a bright yellow oil. LCMS: (ESI, m/z): $[M+H]^+$= 161.1.

**Step 3:**

Tert-butyl (6-bromopyridin-3-yl)carbamate (2.68 g, 9.829 mmol) was added to 30 ml of tetrahydrofuran, added with n-butyllithium (2.5 M in n-hexane, 7.9 mL, 19.659 mmol) at -78 °C, stirred at this temperature for 1 h, added dropwise with a solution of 2-methyl-2-(6-methylpyridin-3-yl)propionitrile (**I-48-c**) (1.05 g, 6.553 mmol) in THF (2 mL), and stirred for 1h. The reaction system was quenched with saturated aqueous ammonium chloride at -78 °C, and extracted with ethyl acetate (2 × 50 mL). The organic phases were combined, dried over anhydrous $Na_2SO_4$, and concentrated, and the obtained residue was purified by reverse phase column chromatography (C18 silica gel; mobile phase A: acetonitrile; mobile phase B; water (10 mmol/L $NH_4HCO_3$), the volume ratio of A: 20% to 70%) to obtain tert-butyl N-{6-[2-methyl-2-(6-methylpyridin-3-yl)propionyl]pyridin-3-yl}carbamate (520 mg, 23%) as a white solid. LCMS:(ESI, m/z): $[M+H]^+$= 356.2.

**Step 4:**

Tert-butyl N-{6-[2-methyl-2-(6-methylpyridin-3-yl)propionyl]pyridin-3-yl}carbamate (**I-48-d**) (520 mg, 1.463 mmol) was added to 6 ml of dichloromethane, added with 3 ml of trifluoroacetic acid, and stirred at room temperature for 1h. The reaction solution was concentrated, added with 10 ml of saturated aqueous sodium bicarbonate, and extracted with dichloromethane (2×20 ml). The organic phase was dried over anhydrous sodium sulfate and concentrated to obtain a crude as a yellow oil, which was purified by reverse phase column chromatography (C18 silica gel; mobile phase A: acetonitrile, mobile phase B: water (0.1% $NH_3 \cdot H_2O$), the volume ratio of A: 10% to 60%) to obtain 1-(5-aminopyridin-2-yl)-2-methyl-2-(6-methylpyridin-3-yl)propan-1-one (**I-48-e**) (217 mg, yield 58%) as a white solid. LCMS: (ESI, m/z): $[M+H]^+$ = 256.2, H-NMR: $^1H$ NMR (300 MHz, DMSO-$d_6$) $\delta$ 8.23 (m, 1H), 7.74 (m, 1H), 7.59 (m, 1H), 7.46 (m, 1H), 7.11 (m, 1H), 6.86 (m, 1H), 6.14 (s, 2H), 2.38 (s, 3H), 1.62 (s, 6H).

**Step 5:**

1-(5-aminopyridin-2-yl)-2-methyl-2-(6-methylpyridin-3-yl)propan-1-one (**I-48-e**) (50 mg, 0.196 mmol) obtained in the previous step, 2-(4-(ethylsulfonyl)phenyl)acetic acid (67 mg, 0.294 mmol) and N,N-diisopropylethylamine (103 mg, 0.797 mmol) were added to dichloromethane (5 ml), added with T3P (50% in ethyl acetate, 249 mg, 0.391 mmol), and stirred at room temperature for 18h. The reaction solution was concentrated and separated by column chromatography (dichloromethane:methanol=25:1) to obtain 23 mg of 2-(4-(ethylsulfonyl)phenyl)-N-(6-(2-methyl-2-(6-methylpyridin-3-yl)propionyl)pyridin-3-yl)acetamide (**I-48**) as a white solid with a yield of 23%. LCMS: (ESI, m/z): $[M+H]^+$ = 466.1. $^1H$ NMR (400 MHz, $CDCl_3$) $\delta$ 11.07 (s, 1H), 8.55 (s, 1H), 8.50 - 8.38 (m, 1H), 8.11 (d, J = 1.6 Hz, 1H), 8.00 (d, J = 8.7 Hz, 1H), 7.80 (d, J = 8.0 Hz, 2H), 7.45 (d, J = 8.0 Hz, 2H), 7.38 (d, J = 6.6 Hz, 1H), 7.05 (d, J = 8.1 Hz, 1H), 3.82 (s, 2H), 3.08 (q, J = 7.4 Hz, 2H), 2.44 (s, 3H), 1.73 (s, 6H), 1.25 (t, J = 7.4 Hz, 3H).

**Example 54**

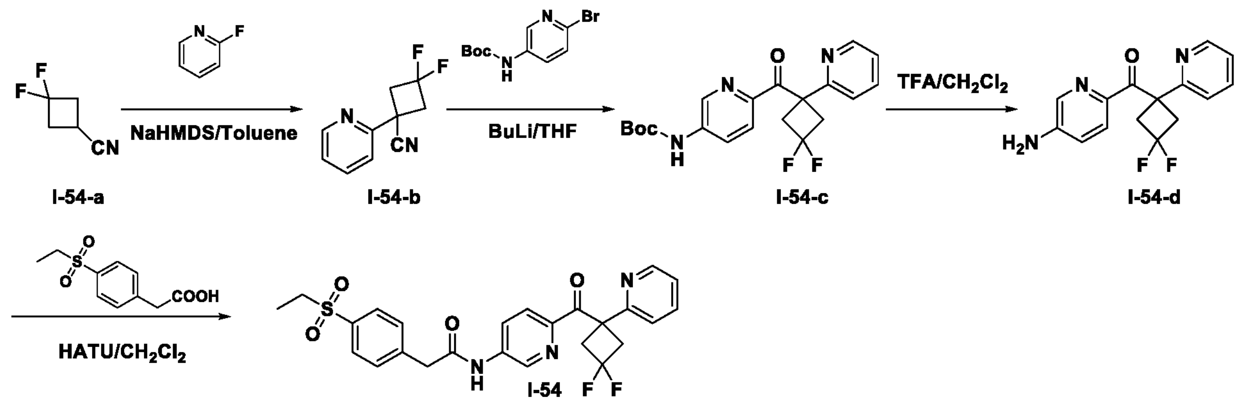


I-54-a I-54-b I-54-c I-54-d I-54

**Step 1:**

3,3-Difluorocyclobutane-1-carbonitrile (**I-54-a**) (0.9 g, 8.0 mmol) and 2-fluoropyridine (0.7 g, 8.0 mmol) were dissolved in toluene (20 mL), added with a solution of sodium bis(trimethylsilyl)amide in tetrahydrofuran (8.0 mL, 1.0 mol/L) under nitrogen protection, and stirred at room temperature for 16h. The reaction solution was quenched with saturated aqueous ammonium chloride (100 mL) and extracted with ethyl acetate (50 mL×3). The organic phase was washed with saturated brine, dried over anhydrous sodium sulfate, filtered, and evaporated to dryness to obtain a crude, which was separated by silica gel column chromatography (petroleum ether /ethyl acetate=0~50%) to obtain the targeted 3,3-difluoro-1-(pyridin-2-yl)cyclobutane-1-carbonitrile (**I-54-b**) (1.12 g, 80%). LC-MS: 195.1$[M+H]^+$.

**Step 2:**

Tert-butyl (6-bromopyridin-3-yl)carbamate (0.59 g, 2.16 mmol) was dissolved in tetrahydrofuran (10 mL) under nitrogen protection, cooled to -78 °C, added with a solution of n-butyllithium in tetrahydrofuran (4.3 mL, 1.6 mol/L) while keeping the temperature below -78 °C during the dropwise addition, and stirred at -78 °C for 0.5h. Then 3,3-difluoro-1-(pyridin-2-yl)cyclobutane-1-carbonitrile (**I-54-b**) (400 mg, 2.06 mmol) was added, and the reaction solution was stirred at room temperature for 16h. Then the reactio solution was quenched with saturated aqueous ammonium chloride (50 ml) and extracted with ethyl acetate (50 mL×3). The organic phase was washed with saturated brine, dried over anhydrous sodium sulfate, filtered, and evaporated to dryness to obtain a crude, which was purified by silica gel column chromatography (petroleum ether /ethyl acetate=0~50%) to obtain the targeted tert-butyl (6-(3,3-difluoro-1-(pyridin-2-yl)cyclobutane-1-carbonyl)pyridin-3-yl)carbamate (**I-54-c**) (320 mg, 39.9%). LC-MS: 390.1$[M+H]^+$.

**Step 3:**

Tert-butyl (6-(3,3-difluoro-1-(pyridin-2-yl)cyclobutane-1-carbonyl)pyridin-3-yl)carbamate (**I-54-c**) (362 mg, 0.91 mmol) was dissolved in dichloromethane (20 mL), added with trifluoroacetic acid (20 mL), and stirred overnight. The reaction solution was evaporated to dryness to obtain a crude ((5-aminopyridin-2-yl)(3,3-difluoro-1-(pyridin-2-yl)cyclobutyl)methanone (**I-54-d**) (220 mg, 81.8%), which is the target compound. LC-MS: 290.1$[M+H]^+$.

**Step 4:**

((5-aminopyridin-2-yl)(3,3-difluoro-1-(pyridin-2-yl)cyclobutyl)methanone (**I-54-d**) (120 mg, 0.41 mmol) was dissolved in dichloromethane (20 mL), added with 2-(4-(ethylsulfonyl)phenyl)acetic acid (104 mg, 0.46 mmol), N-ethyl-N-isopropyl-2-propylamine (107 mg, 0.83 mmol) and 1-propylphosphoric anhydride (198 mg, 0.62 mmol) successively, and stirred overnight at room temperature. The reaction solution was evaporated to dryness to obtain a crude, which was searated by silica gel column chromatography (dichloromethane/methanol = 0~5%) to obtain the targeted N-(6-(3,3-difluoro-1-(pyridin-2-yl)cyclobutane-1-carbonyl)pyridin-3-yl)-2-(4-(ethyl sulfonyl)phenyl)acetamide (**I-54**) (42 mg, 20.3%). LC-MS: 499.9$[M+H]^+$. $^1H$ NMR (400 MHz, DMSO-$d_6$) δ10.75 (s, 1H), 8.59 (d, J = 2.1 Hz, 1H), 8.40 (dd, J = 4.8, 0.9 Hz, 1H),8.16 (dd, J = 8.7, 2.5 Hz, 1H), 8.06 (d, J = 8.7 Hz, 1H), 7.84 (d, J = 8.3 Hz, 2H), 7.76 (td, J = 7.8, 1.8 Hz,1H), 7.60-7.55 (m, 3H), 7.20 (ddd, J = 7.5, 4.8, 0.9 Hz, 1H), 3.86 (s, 2H), 3.57-3.33 (m, 4H), 3.27 (t, J= 7.3 Hz, 2H), 1.10 (t, J = 7.4 Hz, 3H).

## Example 56

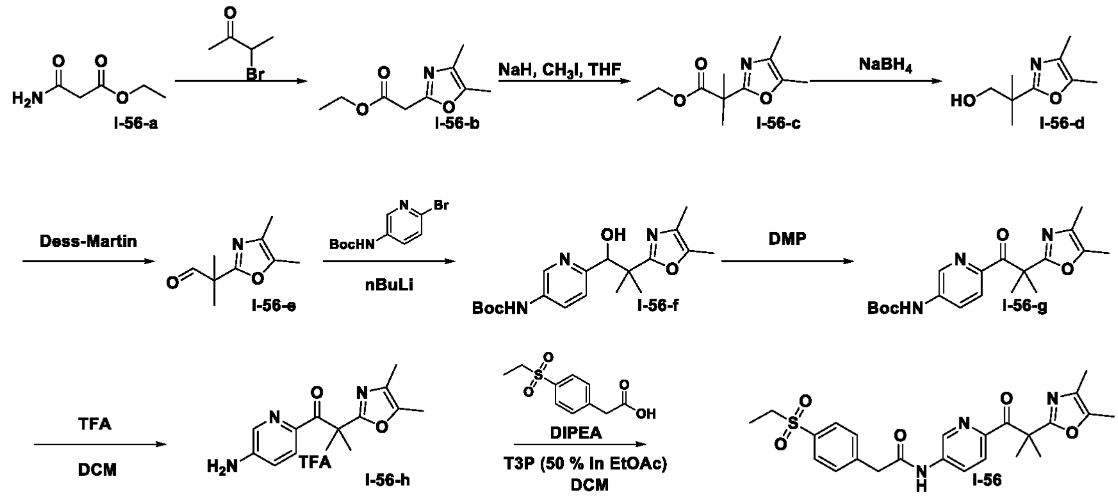


**Step 1:**

Ethyl 2-carbamoylacetate (**I-56-a**) (22 g, 167.771 mmol) and 3-bromo-2-butanone (25.33 g, 167.771 mmol) were mixed and stirred, and heated to 130 °C to react for 3h. LCMS showed that reaction was complete, and the reaction system was concentrated and purified by reverse phase column chromatography (C18 silica gel; mobile phase: A: acetonitrile; B: water (0.1% trifluoroacetic acid), the volume ratio of A: 25% to 45%) to obtain ethyl 2-(4,5-dimethyl-1,3-oxazol-2-yl)acetate (**I-56-b**) (1.6 g, 5%) as a yellow oil. LCMS: (ESI, m/z): $[M+H]^+$= 184.1.

**Step 2:**

Ethyl 2-(4,5-dimethyl-1,3-oxazol-2-yl)acetate (**I-56-b**) (1.6 g) was dissolved in dry THF (20 mL) under nitrogen protection. The reaction system was cooled to 0 °C, and sodium hydride (60%, 0.42 g, 17.466 mmol) and iodomethane (3.10 g, 21.833 mmol) were added. The reaction system was stirred at 0 °C for 1h, and quenched with saturated aqueous $NH_4Cl$ at this temperature. The organic phase was separated, and the aqueous phase was extracted with ethyl acetate (2 × 50 mL). The organic phases were combined, dried over anhydrous $Na_2SO_4$, and concentrated, and the resulted residue was purified by column chromatography (petroleum ether/ethyl acetate = 2:1) to obtain ethyl 2-(4,5-dimethyl-1,3-oxazol-2-yl)-2-methylpropanoate (**I-56-c**) (1.1 g, 60%)I as a colorless oil. LCMS: $[M+H]^+$= 212.1.

**Step 3:**

Ethyl 2-(4,5-dimethyl-1,3-oxazol-2-yl)-2-methylpropanoate (**I-56-c**) (920 mg, 4.355 mmol) was dissolved in EtOH (10 mL), added with sodium borohydride (823.71 mg, 21.775 mmol) in portions at room temperature, raised to 70 °C and stirred for 3h. The reaction system was cooled to 0 °C, quenched with saturated aqueous $NH_4Cl$ at this temperature, concentrated and purified by column chromatography (petroleum ether/ethyl acetate=1:1) to obtain 2-(4,5-dimethyl-1,3-oxazol-2-yl)-2-methylpropane-1-ol (**I-56-d**) (600 mg, 81%) as a yellow oil. LCMS: $[M+H]^+$=170.2.

**Step 4:**

2-(4,5-dimethyl-1,3-oxazol-2-yl)-2-methylpropan-1-ol (**I-56-d**) (600 mg, 3.546 mmol) was dissolved in dichloromethane (10 mL), and Dess-Martin oxidant (1.65 g, 3.901 mmol) was added at room temperature. After stirred at room temperature for 4h, the reaction solution was quenched with saturated aqueous sodium bicarbonate (10 mL) and aqueous sodium thiosulfate (8 mmol), and the aqueous phase was extracted twice with dichloromethane. The combined organic phase was dried over anhydrous sodium sulfate and passed through a column (ethyl acetate:petroleum ethet=1:1) to obtain 2-(4,5-dimethyl-1,3-oxazol-2-yl)-2-methylpropanal (**I-56-e**) (380 mg, yield 64%) as a yellow oil. LCMS: (ESI, m/z): $[M+H]^+$ = 168.1.

**Step 5:**

Tert-butyl (6-bromopyridin-3-yl)carbamate (744.86 mg, 2.728 mmol) was added to 10 ml of tetrahydrofuran, slowly added with n-butyllithium (2 M in tetrahydrofuran, 8.183 mmol) at -78 °C, stirred at this temperature for 1 h, slowly added with a solution of 2-(4,5-dimethyl-1,3-oxazol-2-yl)-2-methylpropanal (**I-56-e**) (380 mg, 2.273 mmol) in tetrahydrofuran (5 ml), and stirred at -78 °C for 2h. The reaction solution was quenched with saturated aqueous $NH_4Cl$ (5 ml) and extracted with ethyl acetate. The organic phase was washed once with saturated brine, dried over anhydrous sodium sulfate, concentrated and passed through a column (ethyl acetate:petroleum ether=0-100%) to obtain tert-butyl N-{6-[2-(4,5-dimethyl-1,3-oxazol-2-yl)-1-hydroxy-2-methylpropyl]pyridin-3-yl}carbam ate (**I-56-f**) (210 mg, yield 26%) as a yellow oil. LCMS: (ESI, m/z): $[M+H]^+$= 362.4.

**Step 6:**

tert-butyl N-{6-[2-(4,5-dimethyl-1,3-oxazol-2-yl)-1-hydroxy-2-methylpropyl]pyridin-3-yl}carbam ate (**I-56-f**) (210 mg, 0.581 mmol) was added to 10 ml of dichloromethane, added with Dess-Martin oxidant (DMP, 492.86 mg, 1.162 mmol) in an ice bath, and stirred at room temperature for 1h. The reaction solution was quenched with saturated aqueous sodium bicarbonate and aqueous sodium thiosulfate (5 mmol), and the aqueous phase was extracted twice with dichloromethane. The combined organic phase was dried over anhydrous sodium sulfate and passed through a column (ethyl acetate:petroleum ether = 0-50%) to obtain tert-butyl N-{6-[2-(4,5-dimethyl-1,3-oxazol-2-yl)-2-methylpropionyl]pyridin-3-yl}carbamate (**I-56-g**) (142 mg, yield 68%) as a white solid. LCMS:(ESI, m/z): $[M+H]^+$ = 360.2. H-NMR: $^1H$ NMR (300 MHz, deuterated chloroform) $\delta$ 8.62 (s, 1H), 8.32 (m, 1H), 8.21 (m, 1H), 8.05 (m, 1H), 2.07 (s, 3H), 1.99 (s, 3H), 1.72 (s, 6H), 1.52 (s, 9H).

**Step 7:**

Tert-butyl N-{6-[2-(4,5-dimethyl-1,3-oxazol-2-yl)-2-methylpropionyl]pyridin-3-yl}carbamate (**I-56-g**) (60 mg, 0.167 mmol) was added to 6 ml of dichloromethane, added with 3 ml of trifluoroacetic acid (TFA), and stirred at room temperature for 1h. The reaction solution was concentrated, added with 10 ml of saturated aqueous sodium bicarbonate, and extracted with dichloromethane (2×20 ml). The organic phase was dried over anhydrous sodium sulfate and concentrated to obtain 93 mg of a crude 1-(5-aminopyridin-2-yl)-2-(4,5-dimethyloxazol-2-yl)-2-methylpropyl-1-one trifluoroacetate (**I-56-h**) as a yellow oil. LC-MS: m/z: $(M+H)^+$=260.2.

**Step 8:**

The crude 1-(5-aminopyridin-2-yl)-2-(4,5-dimethyloxazol-2-yl)-2-methylpropyl-1-one trifluoroacetate (**I-56-h**) (93 mg) in previous step, 2-(4-(ethylsulfonyl)phenyl)acetic acid (40 mg, 0.17 mmol) and N,N-diisopropylethylamine (0.1 ml, 0.6 mmol) were added into dichloromethane (5 ml), added with T3P (50% in ethyl acetate, 130 mg, 0.20 mmol), and stirred at room temperature for 4h. The reaction solution was concentrated and separated by thin layer chromatography (cichloromethane:methanol=20:1) to obtain 10 mg of N-(6-(2-(4,5-dimethyloxazol-2-yl)-2-methylpropionyl)pyridin-3-yl)-2-(4-(ethylsulfonyl) phenyl)acetamide (**I-56**) as a white solid. LC-MS: m/z: $(M+H)^+$ =470.1, $^1H$ NMR (400 MHz, $CDCl_3$) $\delta$ 11.83 - 11.44 (m, 1H), 8.64 - 8.54 (m, 1H), 8.54 - 8.45 (m, 1H), 8.13 - 8.02 (m, 1H), 7.95 - 7.81 (m, 2H), 7.65 - 7.44 (m, 2H), 3.97 (s, 2H), 3.11 (q, J = 7.4 Hz, 2H), 2.07 (s, 3H), 1.88 (s, 3H), 1.72 (s, 3H), 1.28 (t, J = 7.4 Hz, 3H).

**Example 58**

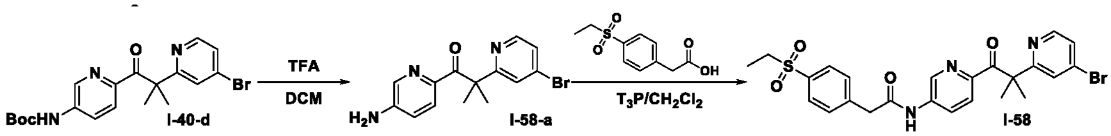


**Step 1:**

Tert-butyl (6-(2-(4-bromopyridin-2-yl)-2-methylpropanoyl)pyridin-3-yl)carbamate (**I-40-d**) (100 mg, 0.24 mmol) was dissolved in dichloromethane (20 mL), added with trifluoroacetic acid (20 mL), and stirred overnight. The reaction solution was evaporated to dryness to obtain a crude 1-(5-aminopyridin-2-yl)-2-(4-bromopyridin-2-yl)-2-methylpropan-1-one (**I-58-a**) (75 mg, 98.5%). LC-MS: 320.0 [M+H]$^+$.

**Step 2:**

(1-(5-aminopyridin-2-yl)-2-(4-bromopyridin-2-yl)-2-methylpropan-1-one (**I-58-a**) (75 mg, 0.24 mmol) was dissolved in dichloromethane (20 mL), added with 2-(4-(ethylsulfonyl)phenyl)acetic acid (59 mg, 0.26 mmol), N-ethyl-N-isopropyl-2-propylamine (61 mg, 0.47 mmol) and 1-propylphosphoric anhydride (111 mg, 0.35 mmol) successively, and stirred overnight at room temperature. The reaction solution was evaporated to dryness to obtain a crude, which was separated by silica gel column chromatography (dichloromethane/methanol = 0~5%) to obtain the targeted N-(6-(2-(4-bromopyridin-2-yl)-2-methylpropionyl)pyridin-3-yl)-2-(4-(ethylsulfonyl)phe nyl)acetamide (**I-58**) (62 mg, 49.9%). LC-MS: 530.0[M+H]$^+$. 1H NMR (400 MHz, DMSO-$d_6$) δ 10.67 (s, 1H), 8.51 - 8.35 (m, 2H), 8.11 (d, J = 7.8 Hz, 1H), 8.06 - 7.96(m, 2H), 7.84 (d, J = 7.5 Hz, 2H), 7.59 (d, J = 6.1 Hz, 3H), 3.84 (s, 2H), 3.27 (d, J = 7.1 Hz, 2H), 1.65 (s,6H), 1.10 (t, J = 6.8 Hz, 3H).

**Example 59**

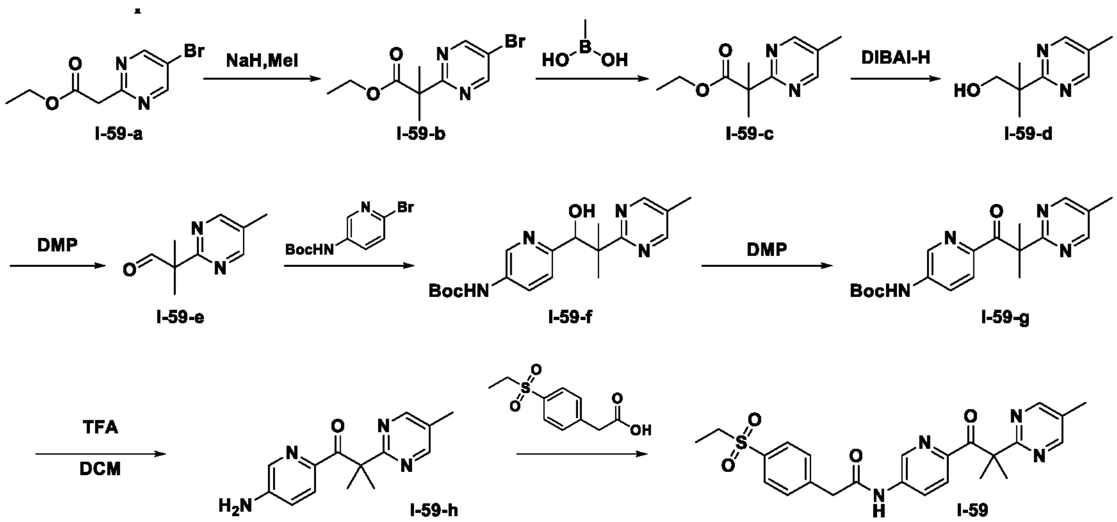


**Step 1:**

Ethyl 2-(5-bromopyrimidin-2-yl)acetate (**I-59-a**) (10 g, 40.804 mmol) was dissolved in dry tetrahydrofuran (80 mL), added with NaH (4.90 g, 122.412 mmol, 60%) in batches in an ice bath under nitrogen protection, and stirred at this temperature for 1h. At 0 °C, iodomethane (14.48 g, 102.010 mmol) was added dropwise to the reaction solution, and after the addition, the reaction solution was raised to room temperature and stirred for 1h. LCMS showed that the reaction was complete, and the reaction solution was cooled to 0 °C, quenched with saturated aqueous ammonium chloride (100 mL), added with water (50 mL) and extracted with ethyl acetate (3 × 100 mL). The organic phases were combined, dried over anhydrous $Na_2SO_4$, and filtered, and the filtrate was concentrated and separated by column chromatography (PE:EA=10:1) to obtain ethyl 2-(5-bromopyrimidin-2-yl)-2-methylpropionate (**I-59-b**) (8 g, 72%) as a yellow oil. LCMS: (ESI, m/z): [M+H]$^+$= 272.7.

**Step 2:**

Ethyl 2-(5-bromopyrimidin-2-yl)-2-methylpropanoate (**I-59-b**) (5 g, 18.306 mmol) and methylboronic acid (1.31 g, 21.967 mmol) were dissolved in 1,4-dioxane (50 mL), added with tris(dibenzylideneacetone)dipalladium(0) (0.34 g, 0.366 mmol), tri-tert-butylphosphine tetrafluoroborate (0.21 g, 0.732 mmol) and cesium carbonate (17.95 g, 54.918 mmol), raised to 100 °C and stirred overnight under nitrogen protection. LCMS showed that the reaction was complete. The reaction system was concentrated and the concentrate was separated by column chromatography (PE:EA=5:1) to obtain ethyl 2-methyl-2-(5-methylpyrimidin-2-yl)propionate (**I-59-c**) (2.1 g, 55%) as a yellow oil. LCMS: (ESI, m/z): [M+H]$^+$= 209.05.

**Step 3:**

Under nitrogen protection, ethyl 2-methyl-2-(5-methylpyrimidin-2-yl)propionate (**I-59-c**) (2.1 g, 10.812 mmol) was dissolved in dry THF (20 mL), cooled to -78 °C, and slowly added dropwise with diisobutylaluminum hydride (1 M in toluene, 21.565 mL, 21.656 mmol). After the addition, the reaction was carried out at this temperature for 1h. LCMS showed that the reaction was complete, and the reaction mixture was quenched with saturated aqueous ammonium chloride (50 mL), added with water (50 mL), and extracted with ethyl acetate (3×100 mL). The organic phases were combined, dried over anhydrous $Na_2SO_4$ and filtered, and the filtrate was concentrated and separated by column chromatography (PE: EA=1:2) to obtain 2-methyl-2-(5-methylpyrimidin-2-yl)propan-1-ol (**I-59-d**) (870 mg, 48%) as a yellow oil. LCMS: (ESI, m/z): $[M+H]^+$= 167.00.

**Step 4:**

2-methyl-2-(5-methylpyrimidin-2-yl)propan-1-ol (**I-59-d**) (870 mg, 5.234 mmol) was added to 20 ml of dichloromethane, added with Dess-Martin oxidant (4.44 g, 10.468 mmol) in an ice bath, and stirred at room temperature for 1h. LCMS showed that the reaction was complete, and the reaction solution was quenched with saturated aqueous sodium bicarbonate and 25 mmol of aqueous sodium thiosulfate, and the aqueous phase was extracted twice with dichloromethane. The combined organic phase was dried over anhydrous sodium sulfate and passed through a column (petroleum ether:ethyl acetate=5:1) to obtain 2-methyl-2-(5-methylpyrimidin-2-yl)propanal (**I-59-e**) (410 mg, 48%) as a yellow oil. LCMS:(ESI, m/z): $[M+H]^+$=165.1.

**Step 5:**

Tert-butyl (6-bromopyridin-3-yl)carbamate (818.35 mg, 2.996 mmol) was added into 10 ml of anhydrous tetrahydrofuran, slowly added with n-butyllithium (2.5 M in n-hexane, 3.00 mL, 7.491 mmol), stirred at this temperature for 1 h, slowly added with a solution of 2-methyl-2-(5-methylpyrimidin-2-yl)propanal (**I-59-e**) (410 mg, 2.497 mmol) in tetrahydrofuran (5 ml), and stirred at -78 °C for 2h. The reaction solution was quenched with saturated aqueous $NH_4Cl$ (20 ml), and extracted with ethyl acetate (3x50 mL). The organic phase was washed once with saturated brine, dried over anhydrous sodium sulfate and concentrated, and the residue was separated by reverse phase column chromatography (C18 silica gel; mobile phase A: acetonitrile, mobile phase B: water (0.1% $NH_3 \cdot H_2O$), the volume ratio of A: 10% to 50%) to obtain tert-butyl (6-(1-hydroxy-2-methyl-2-(5-methylpyrimidin-2-yl)propyl)pyridin-3-yl)carbamate (**I-59-f**) (320 mg, 7%) as a yellow solid. LCMS: (ESI, m/z): $[M+H]^+$= 359.2.

**Step 6:**

Tert-butyl (6-(1-hydroxyl-2-methyl-2-(5-methylpyrimidin-2-yl)propyl)pyridin-3-yl)carbamate (**1-59-f**) (380 mg, 1.060 mmol) was added to 10 ml of dichloromethane, added with Dess-Martin oxidant (899.30 mg, 2.120 mmol) in an ice bath, and stirred at room temperature for 1h. LCMS showed that the reaction was complete, and the reaction solution was quenched with saturated saturated aqueous sodium bicarbonate and 5 mmol of aqueous sodium thiosulfate, and the aqueous phase was extracted with dichloromethane (3×20 mL). The combined organic phase was dried over anhydrous sodium sulfate and passed through a column (dichloromethane:methanol=10:1), to obtain tert-butyl N-{6-[2-methyl-2-(5-methylpyrimidin-2-yl)propionyl]pyridin-3-yl}carbamate (**I-59-g**) as a yellow solid (210.9 mg, 56%). LCMS: (ESI, m/z): $[M+H]^+$= 357.15. $^1$H NMR (400 MHz, DMSO-$d_6$) $\delta$ 9.83 (s, 1H), 8.46 (d, $J$ = 0.9 Hz, 2H), 8.17 (t, $J$ = 1.6 Hz, 1H), 7.98 (d, $J$ = 1.5 Hz, 2H), 2.20 (s, 3H), 1.59 (s, 6H), 1.46 (s, 9H).

**Step 7:**

Tert-butyl N-{6-[2-methyl-2-(5-methylpyrimidin-2-yl)propionyl]pyridin-3-yl}carbamate (**I-59-g**) (50 mg, 0.14 mmol) was added to 2 ml of dichloromethane, added with 2 ml of trifluoroacetic acid, and stirred at room temperature for 1h. The reaction solution was concentrated, added with 5 ml of saturated sodium bicarbonate, and extracted with dichloromethane (2×20 ml). The organic phase was dried over anhydrous sodium sulfate and concentrated to obtain 35 mg of 1-(5-aminopyridin-2-yl)-2-methyl-2-(5-methylpyrimidin-2-yl)propan-1-one (**I-59-h**) as a white solid, which was directly used in the next step, with a yield of 97%. LC-MS: m/z: $(M+H)^+$=257.

**Step 8:**

1-(5-aminopyridin-2-yl)-2-methyl-2-(5-methylpyrimidin-2-yl)propan-1-one (35 mg, 0.137 mmol) (**I-59-h**), 2-(4-(ethylsulfonyl)phenyl)acetic acid (42 mg, 0.184 mmol) and N,N-diisopropylethylamine (0.5 ml, 3 mmol) were added

to 10 ml of dichloromethane, added with a 50% solution of $T_3P$ (170 mg, 0.267 mmol) in ethyl acetate, and stirred at room temperature for 1h. The reaction solution was concentrated and passed through a column (ethyl acetate:petroleum ether=0-100%) to obtain 43 mg of 2-(4-(ethylsulfonyl)phenyl)-N-(6-(2-methyl-2-(5-methylpyrimidin-2-yl)propionyl)pyridin-3-yl)acetamide (**I-59**) as a white solid with a yield of 67%. LC-MS: m/z: $(M+H)^+$ =467, $^1$HNMR (400 MHz, $CDCl_3$) δ 9.10 (s, 1H), 8.37 (s, 2H), 8.33 - 8.24 (m, 2H), 8.13 (d, J = 9.2 Hz, 1H), 7.87 (d, J = 8.3 Hz, 2H), 7.53 (d, J = 8.2 Hz, 2H), 3.89 (s, 2H), 3.13 (q, J = 7.4 Hz, 2H), 2.23 (s, 3H), 1.75 (s, 6H), 1.30 (t, J = 7.4 Hz, 3H).

**Example 60**

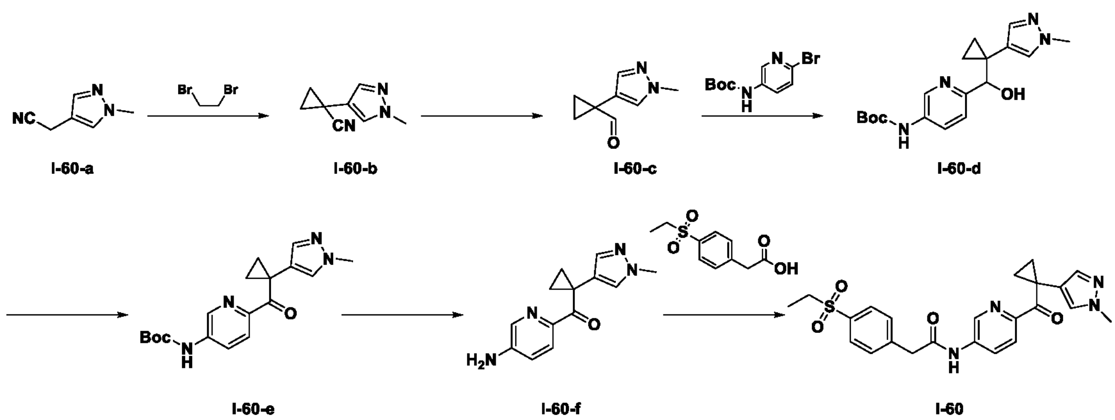

I-60-a I-60-b I-60-c I-60-d I-60-e I-60-f I-60

**Step 1:**

2-(1-methyl-1H-pyrazol-4-yl)acetonitrile (485 mg, 4 mmol) (**I-60-a**) was added into 15 ml of tetrahydrofuran, slowly added with a solution of lithium diisopropylamide in tetrahydrofuran (4.6 ml, 9.2 mmol, 2 mol/L) in an ice bath, and stirred at 0 °C for 1h. Then 1,2-dibromoethane (0.9 g, 4.8 mmol) was added, and the reaction mixtue was stirred at 0 °C for 1 h and then at room temperature overnight. The reaction solution was quenched with 10 ml of water and extracted twice with ethyl acetate. The organic phase was washed twice with saturated brine, dried over anhydrous sodium sulfate, concentrated, and passed through a column (methanol:dichloromethane=0-5%) to obtain 1-(1-methyl-1H-pyrazol-4-yl)cyclopropane-1-carbonitrile as a brown oil (240 mg, yield 40%). LC-MS: m/z: $(M+H)^+$ = 148.

**Step 2:**

1-(1-methyl-1H-pyrazol-4-yl)cyclopropane-1-carbonitrile (240 mg, 1.6 mmol) **(I-60-b)** was added into 5 ml of tetrahydrofuran, added with diisobutylaluminum hydride (4.1 mL, 4.1 mmol) at -78 °C, stirred at this temperature for 2h and then at room temperature overnight. The reaction solution was quenched with 2 mol/L aqueous hydrochloric acid, and the aqueous phase was extracted three times with ethyl acetate. The combined organic phases were dried over anhydrous sodium sulfate to obtain 60 mg of 1-(1-methyl-1H-pyrazol-4-yl)cyclopropane-1-carbaldehyde (**I-60-c**) as a colorless oil, which was used directly in the next step. LC-MS: m/z: $(M+H)^+$ =151.

**Step 3:**

Tert-butyl (6-bromopyridin-3-yl)carbamate (160 mg, 0.59 mmol) was added to 5 ml of tetrahydrofuran, slowly added with n-butyllithium (0.8 ml, 1.3 mmol) at -78 °C, stirred at this temperature for 1h, slowly added with a solution of 1-(1-methyl-1H-pyrazol-4-yl)cyclopropane-1-carbaldehyde (**I-60-c**) (60 mg, 0.4 mmol) in tetrahydrofuran (5 ml), and stirred at -78 °C for 2h. The reaction solution was quenched with saturated aqueous $NH_4Cl$ (5 ml), and extracted with ethyl acetate. The organic phase was washed once with saturated brine, dried over anhydrous sodium sulfate, concentrated and passed through a column (ethyl acetate:petroleum ether=0-100%) to obtain 50 mg of tert-butyl (6-(hydroxyl(1-(1-methyl-1H-pyrazol-4-yl)cyclopropyl)methyl)pyridin-3-yl)carbamate (**I-60-d**) as a white solid, with a yield of 36%. LC-MS: m/z: $(M+H)^+$=345.

**Step 4:**

Tert-butyl (6-(hydroxy(1-(1-methyl-1H-pyrazol-4-yl)cyclopropyl)methyl)pyridin-3-yl)carbamate (**I-60-d**)(50 mg, 0.14 mmol) was added to 5 ml of ethyl acetate, added with IBX (80 mg, 0.28 mmol), and stirred overnight at reflux temperature. The reaction solution was filtered and concentrated to obtain 45 mg of tert-butyl (6-(1-(1-methyl-1H-pyrazol-4-yl)cyclopropane-1-carbonyl)pyridin-3-yl)carbamate (**I-60-e**) as a white solid, which was used directly in the next step, with a yield of 90%. LC-MS: m/z: $(M+H)^+$ =343.

**Step 5:**

Tert-butyl (6-(1-(1-methyl-1H-pyrazol-4-yl)cyclopropane-1-carbonyl)pyridin-3-yl)carbamate (**I-60-e**) (45 mg, 0.13 mmol) was added to 1.5 ml of dichloromethane, added with 1.5 ml of trifluoroacetic acid, and stirred at room temperature for 1h. The reaction solution was concentrated, added with 5 ml of saturated sodium bicarbonate, and extracted with dichloromethane (2×15ml). The organic phase was dried over anhydrous sodium sulfate and concentrated to obtain 30 mg of (5-aminopyridin-2-yl)(1-(1-methyl-1H-pyrazol-4-yl)cyclopropyl)methanone (**I-60-f**) as an off-white solid, which was directly used in the next step, with a yield of 94%. LC-MS: m/z: $(M+H)^+$ =243.

**Step 6:**

(5-Aminopyridin-2-yl)(1-(1-methyl-1H-pyrazol-4-yl)cyclopropyl)methanone (30 mg, 0.12 mmol) (**I-60-f**), 2-(4-(ethylsulfonyl)phenyl)acetic acid (40 mg, 0.17 mmol) and N,N-diisopropylethylamine (0.1 ml, 0.6 mmol) were added to 10 ml of dichloromethane, added with TBTU (60 mg, 0.18 mmol) and stirred at room temperature for 4h. The reaction solution was concentrated and separated by thin layer chromatography {methanol:(dichloromethane:ethyl acetate=12:3)=1:10} to obtain 17 mg of 2-(4-(ethylsulfonyl)phenyl)-N-(6-(1-(1-methyl-1H-pyrazol-4-yl)cyclopropane-1-carbon yl)pyridin-3-yl)acetamide (**I-60**) as a white solid with a yield of 30%. LC-MS: m/z: $(M+H)^+$=453, $^1H$ NMR (400 MHz, $CDCl_3$) δ 9.14 (s, 1H), 8.52 (d, *J* = 2.2 Hz, 1H), 8.27 (dd, *J* = 8.6, 2.4 Hz, 1H), 7.82 (d, *J* = 8.3 Hz, 2H), 7.77 (d, *J* = 8.6 Hz, 1H), 7.51 (d, *J* = 8.3 Hz, 2H), 7.36 (s, 1H), 7.24 (s, 1H), 3.86 (s, 2H), 3.78 (s, 3H), 3.12 (q, *J* = 7.4 Hz, 2H), 1.77 (q, *J* = 3.9 Hz, 2H), 1.31 - 1.23 (m, 5H).

**Example 63**

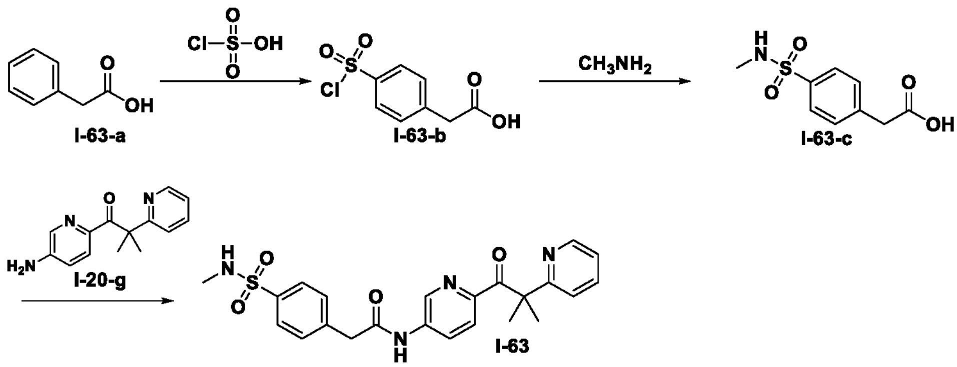

I-63-a I-63-b I-63-c I-20-g I-63

**Step 1:**

2-Phenylacetic acid (**I-63-a**) (5.00 g, 36.72 mmol) and chlorosulfonic acid (35 ml) were mixed and stirred at 0 °C for 1h, slowly raised to room temperature and stirred for 12h. The reaction mixture was quenched with ice water, and a solid precipitated out. The system was filtered, and the filter cake was vacuum-dried to obtain a crude, which was recrystallized twice in chloroform to obtain 2-(4-(chlorosulfonyl)phenyl)acetic acid (**I-63-b**) (1.30 g, 5.54 mmol, yield 15%) as a white solid. LCMS: (ESI, m/z): $[M+H]^+$= 235.25.

**Step 2:**

2-(4-(chlorosulfonyl)phenyl)acetic acid (**I-63-b**) (3.30 g, 14.06 mmol) was dissolved in methanol (30 ml), added with a methylamine solution (31% in methanol, 1.41 g, 14.06 mmol), raised to room temperature and stirred for 2h. The

reaction system was adjusted to pH 10 with an aqueous NaOH (2 N) and concentrated under reduced pressure, and the residue was dissolved in water and adjusted with a HCl solution (6 N) to pH 1. After stirred for 1h, the system was concentrated and the residue was purified by reverse phase column chromatography (C18 silica gel, A: acetonitrile; B: water (0.1% trifluoroacetic acid), the ratio of A: 15% to 35%) to obtain 2-(4-(N-methylsulfamoyl)phenyl)acetic acid (**I-63-c**) (660.00 mg, 2.88 mmol, 20% yield) as a white solid. LCMS: (ESI, m/z): $[M+1]^+$= 230.10. $^1H$ NMR (400 MHz, $CD_3Cl$) δ 7.83 (d, J = 8.3 Hz, 2H), 7.46 (d, J = 8.2 Hz, 2H), 4.39 (s, 1H), 3.75 (s, 2H), 2.68 (s, 3H).

**Step 3:**

1-(5-aminopyridin-2-yl)-2-methyl-2-(pyridin-2-yl)propan-1-one (55 mg, 0.22 mmol) (**I-20-g**), 2-(4-(N-methylsulfamoyl)phenyl)acetic acid (**I-63-c**) (50 mg, 0.22 mmol) and N,N-diisopropylethylamine (0.3 ml, 2 mmol) were added to 20 ml of dichloromethane, added with a 50% solution of $T_3P$ (200 mg, 0.3 mmol) in ethyl acetate, and stirred at room temperature for 1h. The reaction solution was washed with saturated aqueous sodium bicarbonate, concentrated and passed through a column (ethyl acetate:dichloromethane = 0-15%) to obtain 30 mg of N-(6-(2-methyl-2-(pyridin-2-yl)propionyl)pyridin-3-yl)-2-(4-(N-methylsulfamoyl)pheny l)acetamide (**I-63**) as a white solid with a yield of 30%. LC-MS: m/z: $(M+H)^+$=453, $^1$HNMR (400 MHz, DMSO-$d_6$) δ 10.59 (s, 1H), 8.39 (d, J = 2.3 Hz, 1H), 8.23 - 8.16 (m, 1H), 8.08 (dd, J = 8.7, 2.5 Hz, 1H), 7.97 (d, J = 8.7 Hz, 1H), 7.73 (ddd, J = 10.0, 6.0, 2.0 Hz, 3H), 7.51 (d, J = 8.3 Hz, 2H), 7.47 (d, J = 8.0 Hz, 1H), 7.40 (q, J = 5.0 Hz, 1H), 7.08 (ddd, J = 7.4, 4.8, 0.9 Hz, 1H), 3.79 (s, 2H), 2.40 (d, J = 5.0 Hz, 3H), 1.62 (s, 6H).

**Example 64**

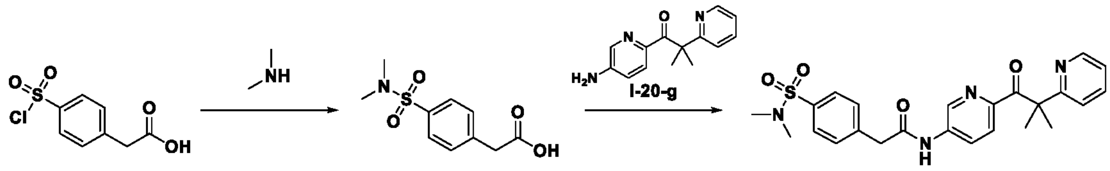


I-63-b I-64-a I-64

**Step 1:**

4-(Chlorosulfonyl)phenyl]acetic acid (2.5 g, 10.654 mmol) was dissolved in dry THF (30 mL), added with dimethylamine (1 M in tetrahydrofuran, 11.72 mL, 11.719 mmol) at room temperature, and stirred overnight. LCMS showed that the reaction was complete. The system was concentrated under reduced pressure, and the concentrate was purified by reverse phase column chromatography (C18 silica gel, mobile phase A: acetonitrile; mobile phase B: water (0.1% trifluoroacetic acid), the volume ratio of A: 10% to 30%) to obtain [4-(dimethylsulfamoyl)phenyl]acetic acid (525.4 mg, 20% yield) as a yellow solid. LCMS: (ESI, m/z): $[M+1]^+$= 243.9. $^1H$ NMR (300 MHz, $CDCl_3$) δ 7.75 (d, *J* = 8.3 Hz, 2H), 7.47 (d, *J* = 8.2 Hz, 2H), 3.75 (s, 2H), 2.71 (s, 6H).

**Step 2:**

1-(5-aminopyridin-2-yl)-2-methyl-2-(pyridin-2-yl)propan-1-one (55 mg, 0.22 mmol) (**I-20-g**), [4-(dimethylsulfamoyl)phenyl]acetic acid (**I-64-a**) (46 mg, 0.19 mmol) and N,N-diisopropylethylamine (108 mg, 0.84 mmol) were added to 10 ml of dichloromethane, added with a 50% solution of $T_3P$ (240 mg, 0.38 mmol) in ethyl acetate, and stirred at room temperature for 1h. The reaction solution was washed with saturated aqueous sodium bicarbonate, concentrated and passed through a column (ethyl acetate: dichloromethane = 0-15%) to obtain 45 mg of 2-(4-(N,N-dimethylsulfamoyl)phenyl)-N-(6-(2-methyl-2-(pyridin-2-yl)propionyl)pyridi n-3-yl)acetamide (**I-64**) as a white solid with a yield of 51%. LCMS: (ESI, m/z): $[M+1]^+$ = 467.2. $^1H$ NMR (400 MHz, $CDCl_3$) δ 9.05 (s, 1H), 8.42 (s, 1H), 8.31 (d, J = 4.3 Hz, 1H), 7.92 (d, J = 8.6 Hz, 1H), 7.79 (d, J = 8.8 Hz, 1H), 7.72 (dd, J = 12.2, 7.9 Hz, 3H), 7.52 (d, J = 8.1 Hz, 2H), 7.37 (d, J = 8.1 Hz, 1H), 7.16 - 7.09 (m, 1H), 3.86 (s, 2H), 2.70 (s, 6H), 1.74 (s, 6H).

**Example 65**

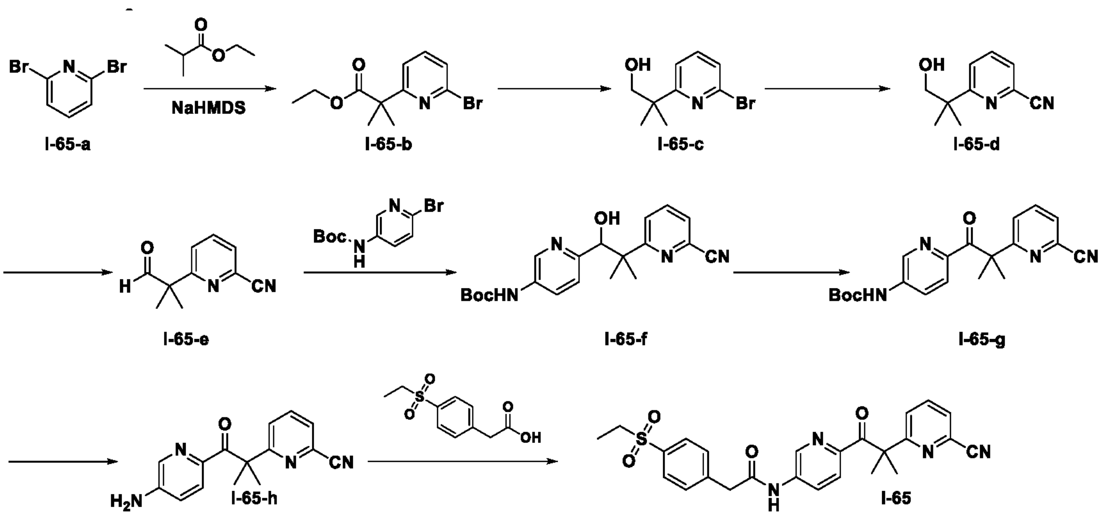


I-65-a I-65-b I-65-c I-65-d

I-65-e I-65-f I-65-g

I-65-h I-65

**Step 1:**

Ethyl isobutyrate (5.0 g, 43.0 mmol) and 2,6-dibromopyridine (**I-65-a**) (11.0 g, 47.0 mmol) were dissolved in tetrahydrofuran (50 mL), added with a solution of sodium bis(trimethylsilyl)amide in tetrahydrofuran (52.0 mL, 1.0 mol/L) under nitrogen protection, and stirred at room temperature for 16h. The reaction solution was quenched with saturated aqueous ammonium chloride (100 mL), and extracted with ethyl acetate (100 mL×3). The organic phase was washed with saturated brine, dried over anhydrous sodium sulfate, filtered, and evaporated to dryness to obtain a crude, which was separated by silica gel column chromatography (petroleum ether/ethyl acetate = 0~50%) to obtain the targeted ethyl 2-(6-bromopyridin-2-yl)-2-methylpropanoic acid (**I-65-b**) (5.5 g, 47%). LC-MS: 272.1[M+H]$^+$.

**Step 2:**

Ethyl 2-(6-bromopyridin-2-yl)-2-methylpropanoic acid (**I-65-b**) (0.59 g, 2.16 mmol) was dissolved in tetrahydrofuran (10 mL) under nitrogen protection, cooled to -78°C, added with a solution of diisobutylaluminum hydride in tetrahydrofuran (5.5 mL, 1.0 mol/L) while keeping the temperature below -70°C during the dropwise addition, and stirred at -78°C for 1h. Then the reaction solution was quenched with saturated aqueous ammonium chloride (20 ml) and filtered to remove the solid, and the filtrate was extracted with ethyl acetate (50 mL×3). The organic phase was washed with saturated brine, dried over anhydrous sodium sulfate, filtered, and evaporated to dryness to obtain a crude, which was purified by silica gel column chromatography (petroleum ether/ethyl acetate=0~50%) to obtain the targeted 2-(6-bromopyridin-2-yl)-2-methylpropane-1-ol (**I-65-c**) (410 mg, 48.0%). LC-MS: 230.0 [M+H]$^+$.

**Step 3:**

2-(6-bromopyridin-2-yl)-2-methylpropan-1-ol (**I-65-c**) (410 mg, 1.78 mmol) was dissolved in N,N-dimethylformamide (20 mL), added with zinc cyanide (314 mg, 2.67 mmol) and tetrakis(triphenylphosphine) palladium (206 mg, 0.18 mmol) under nitrogen protection, heated to 80 °C and stirred for 2h. The reaction solution was evaporated to dryness to obtain a crude, which was purified by silica gel column chromatography (petroleum ether/ethyl acetate=0~50%) to obtain the targeted 6-(1-hydroxy-2-methylpropan-2-yl)picolinonitrile (**I-65-d**) (280 mg, 89.2%). LC-MS: 177.1[M+H]$^+$.

**Step 4:**

6-(1-hydroxy-2-methylpropan-2-yl)picolinonitrile (**I-65-d**) (280 mg, 1.59 mmol) was dissolved in dichloromethane (20 mL), added with Dess-Martin oxidant (741 mg, 1.75 mmol), and stirred at room temperature for 2h. The reaction solution was evaporated to dryness to obtain a crude, which was separated by silica gel column chromatography (dichloromethane/methanol=0~5%) to obtain the targeted 6-(2-methyl-1-oxypropan-2-yl)picolinonitrile (**I-65-e**) (140 mg, 50.6%). LC-MS: 175.1 [M+H]$^+$.

**Step 5:**

Tert-butyl (6-bromopyridin-3-yl)carbamate (1.28 g, 4.70 mmol) was dissolved in tetrahydrofuran (12 mL) under

nitrogen protection, cooled to -78°C, added with a solution of n-butyllithium in tetrahydrofuran (9.4 mL, 1.6 mol/L) while keeping the temperature below -70°C during the dropwise addition, and stirred at -78°C for 0.5h. Then the reaction solution was added with 6-(2-methyl-1-oxypropan-2-yl)picolinonitrile (**I-65-e**) (780 mg, 4.48 mmol), and stirred at room temperature for 16h. The reaction solution was quenched with saturated aqueous ammonium chloride (50 ml) and extracted with ethyl acetate (50 mL×3). The organic phase was washed with saturated brine, dried over anhydrous sodium sulfate, filtered, and evaporated to dryness to obtain a crude, which was purified by silica gel column chromatography (petroleum ether/ethyl acetate = 0~50%) to obtain the targeted tert-butyl (6-(2-(6-cyanopyridin-2-yl)-1-hydroxyl-2-methylpropyl)pyridin-3-yl)carbamate (**I-65-f**) (580 mg, 35.2%). LC-MS: 369.2 $[M+H]^+$.

**Step 6:**

Tert-butyl (6-(2-(6-cyanopyridin-2-yl)-1-hydroxyl-2-methylpropyl)pyridin-3-yl)carbamate (**I-65-f**) (0.58 g, 1.57 mmol) was dissolved in ethyl acetate (40 mL), added with 2-iodobenzoic acid (IBX, 0.66 g, 2.37 mmol), heated to reflux and stirred to react overnight. The reaction solution was filtered, and the filtrate was evaporated to dryness to obtain a crude, which was purified by silica gel column chromatography (petroleum ether/ethyl acetate=0~50%) to obtain the targeted (6-(2-(6-cyanopyridin-2-yl)-2-methylpropanyl)pyridin-3-yl)carbamate (**I-65-g**) (420 mg, 72.8%). LC-MS: 367.2$[M+H]^+$.

**Step 7:**

Tert-butyl (6-(2-(6-cyanopyridin-2-yl)-2-methylpropanyl)pyridin-3-yl)carbamate (**I-65-g**) (0.42 g, 1.15 mmol) was dissolved in dichloromethane (20 mL), added with trifluoroacetic acid (20 mL), and stirred overnight. After the reaction was complete, the reaction solution was evaporated to dryness to obtain a crude 1-(5-aminopyridin-2-yl)-2-methyl-2-(pyridin-2-yl)propan-1-one (**I-65-h**) (250 mg, 81.9%). LC-MS: 267.1$[M+H]^+$.

**Step 8:**

The product 1-(5-aminopyridin-2-yl)-2-methyl-2-(pyridin-2-yl)propan-1-one (**I-65-h**) (250 mg) in previous step was dissolved in dichloromethane (20 mL), added with 2-(4-(ethylsulfonyl)phenyl)acetic acid (236 mg, 0.65 mmol), N-ethyl-N-isopropyl-2-propylamine (485 mg, 3.76 mmol) and 1-propylphosphoric anhydride (448 mg, 1.41 mmol), and stirred overnight at room temperature. The reaction solution was evaporated to dryness to obtain a crude, which was purified by silica gel column chromatography (dichloromethane/methanol = 0~5%) to obtain the targeted N-(6-(2-(6-cyanopyridin-2-yl)-2-methylpropanyl)pyridin-3-yl)-2-(4-(ethylsulfonyl)phen yl)acetamide (156 mg, 34.9%). LC-MS: 467.2$[M+H]^+$. $^1$H NMR (400 MHz, DMSO-$d_6$) δ 10.70 (s, 1H), 8.42 (s, 1H), 8.03 (d, J = 9.0 Hz, 3H), 7.89-7.74 (m, 4H),7.58 (d, J = 7.0 Hz, 2H), 3.84 (s, 2H), 3.27 (d, J = 6.9 Hz, 2H), 1.63 (s, 6H), 1.10 (d, J = 6.6 Hz, 3H).

**Example 68**

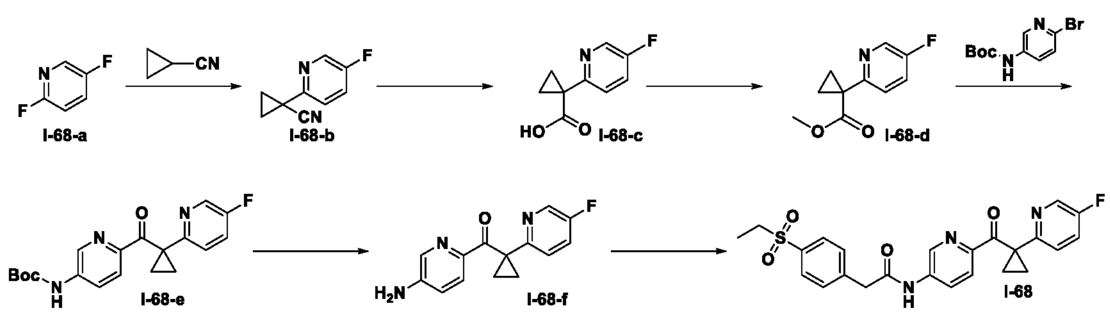

I-68-a
I-68-b
I-68-c
I-68-d
I-68-e
I-68-f
I-68

**Step 1:**

Cyclopropanecarbonitrile (3.36 g, 50.1 mmol) was added into 50 ml of tetrahydrofuran, slowly added with a solution of sodium bistrimethylsilylamide in tetrahydrofuran (25.5 ml, 51 mmol, 2 mol/L) in an ice bath, and stirred at 0 °C for 20min. Then a solution of 2,5-difluoropyridine (**I-68-a**) (5.8 g, 50 mmol) in 15 ml of tetrahydrofuran was added. The reaction mixture was slowly warmed to room temperature, stirred for 1.5 h, and quenched with saturated aqueous ammonium chloride, and the organic phase was washed twice with saturated brine, dried over anhydrous sodium sulfate,

concentrated, and passed through a column (ethyl acetate: petroleum ether=0-10%) to obtain 4.5 g of 1-(5-fluoropyridin-2-yl)cyclopropane-1-carbonitrile **(I-68-b)** as a white solid with a yield of 55%. LC-MS: m/z: $(M+H)^+$ =163, $^1$H-NMR (400 MHz, $CDCl_3$) δ 8.32 (d, J = 2.8 Hz, 1H), 7.72 (dd, J = 8.7, 4.1 Hz, 1H), 7.42 (td, J = 8.3, 2.9 Hz, 1H), 1.79 (td, J = 5.8, 2.1 Hz, 2H), 1.73 (td, J = 5.7, 2.1 Hz, 2H).

**Step 2:**

1-(5-fluoropyridin-2-yl)cyclopropane-1-carbonitrile (600 mg, 3.7 mmol) (**I-68-b**) was added to 6 ml of ethanol, added with 6 ml of a 25% sodium hydroxide solution, and stirred overnight at 100 °C. The reaction solution was adjusted to pH=5-6 with 6 mol/L and 1 mol/L hydrochloric acid, and extracted three times with ethyl acetate (3×15mL). The organic phase was dried over anhydrous sodium sulfate and concentrated to obtain 600 mg of a crude 1-(5-fluoropyridin-2-yl)cyclopropane-1-carboxylic acid (**I-68-c**) as a brown solid, which was directly used in the next step, with a yield of 89%. LC-MS: m/z: $(M+H)^+$ =182.

**Step 3:**

1-(5-fluoropyridin-2-yl)cyclopropane-1-carboxylic acid (I-68-c) (550 mg, 3 mmol) was added into 20 ml of methanol, slowly added with thionyl chloride (3.3 ml) in an ice batch, and refluxed and stirred overnight. The reaction mixture was concentrated, neutralized with saturated sodium bicarbonate solution, and extracted three times with ethyl acetate. The organic phase was dried over anhydrous sodium sulfate, and concentrated to obtain 560 mg of methyl 1-(5-fluoropyridin-2-yl)cyclopropane-1-carboxylate (**I-68-d**) as a colorless oil with a yield of 94%. LC-MS: m/z: $(M+H)^+$ =196.

**Step 4:**

Tert-butyl (6-bromopyridin-3-yl)carbamate (273 mg, 1 mmol) was added to 4 ml of tetrahydrofuran, slowly added with n-butyllithium (1.25 ml, 2 mmol) at -78 °C, stirred at this temperature for 40min, slowly added with a solution of methyl 1-(5-fluoropyridin-2-yl)cyclopropane-1-carboxylate (**I-68-d**) (196 mg, 1 mmol) in 3 ml of tetrahydrofuran and stirred at -78 °C for 2h. The reaction solution was quenched with 4 ml of saturated aqueous $NH_4Cl$, and extracted with ethyl acetate. The organic phase was washed once with saturated brine, dried over anhydrous sodium sulfate, concentrated and passed through a column (ethyl acetate: petroleum ether = 0-100%) to obtain 110 mg of tert-butyl (6-(1-(5-fluoropyridin-2-yl))cyclopropane-1-carbonyl)pyridin-3-yl)carbamate (**I-68-e**) as a white solid with a yield of 30%. LC-MS: m/z: $(M+H)^+$ =358.

**Step 5:**

Tert-butyl (6-(1-(5-fluoropyridin-2-yl))cyclopropane-1-carbonyl)pyridin-3-yl)carbamate (**I-68-e**) (100 mg, 0.28 mmol) was added to 3 ml of dichloromethane, added with 3 ml of trifluoroacetic acid, and stirred at room temperature for 1h. The reaction solution was concentrated, added with 6 ml of saturated aqueous sodium carbonate, and extracted with dichloromethane (2×20ml). The organic phase was dried over anhydrous sodium sulfate and concentrated to obtain 60 mg of (5-aminopyridin-2-yl)(1-(5-fluoropyridin-2-yl)cyclopropyl)methanone (**I-68-f**) as a white solid, which was directly used in the next step, with a yield of 83%. LC-MS: m/z: $(M+H)^+$ =258.

**Step 6:**

(5-aminopyridin-2-yl)(1-(5-fluoropyridin-2-yl)cyclopropyl)methanone (60 mg, 0.23 mmol), 2-(4-(ethylsulfonyl)phenyl)acetic acid (60 mg, 0.26 mmol) and N,N-diisopropylethylamine (0.2 ml, 1 mmol) were added to 8 ml of dichloromethane, added with a 50% solution of $T_3P$ (0.3 g, 0.47 mmol) in ethyl acetate , and stirred at room temperature for 1h. The reaction solution was washed with saturated sodium bicarbonate and concentrated, and separated by thin layer chromatography (ethyl acetate: petroleum ether=3:1) to obtain 50 mg of 2-(4-(ethylsulfonyl)phenyl)-N-(6-(1-(5-fluoropyridin-2-yl)cyclopropane-1-carbonyl)pyri din-3-yl)acetamide (**I-68**) as a white solid with a yield of 45%. LC-MS: m/z: $(M+H)^+$ = 468, $^1$H NMR (400 MHz, DMSO-$d_6$) δ 10.68 (s, 1H), 8.48 (d, J = 2.3 Hz, 1H), 8.26 (d, J = 3.0 Hz, 1H), 8.15 (dd, J = 8.6, 2.5 Hz, 1H), 7.92 (d, J = 8.6 Hz, 1H), 7.84 (d, J = 8.3 Hz, 2H), 7.63 - 7.50 (m, 3H), 7.22 (dd, J = 8.8, 4.2 Hz, 1H), 3.85 (s, 2H), 3.27 (q, J = 7.4 Hz, 2H), 1.62 - 1.53 (m, 2H), 1.53 - 1.45 (m, 2H), 1.09 (t, J = 7.4 Hz, 3H).

**Example 71**

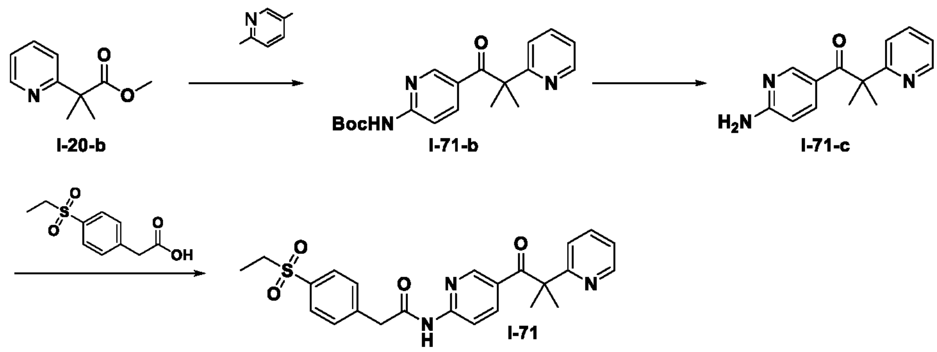

I-20-b I-71-b I-71-c

I-71

**Step 1:**

Tert-butyl (5-bromopyridin-2-yl)carbamate (1 g, 3.66 mmol) was dissolved in 10 ml of tetrahydrofuran, replaced with nitrogen three times and cooled to -78 °C, slowly dropwise added with 5 mL of n-butyllithium (2.5 M in n-hexane), stirred for 1h, added with methyl 2-methyl-2-(2-pyridyl)propionate (**I-20-b**) (656 mg, 3.66 mmol), stirred at -78 °C for 2h, then warmed up to 15 °C and stirred for 16h. The reaction solution was quenched with saturated aqueous ammonium chloride and extracted with EtOAc (30 mL×2). The organic phase was dried over anhydrous sodium sulfate, filtered, concentrated, and purified by column chromatography (ethyl acetate: petroleum ether = 0-60%) to obtain 300 mg of tert-butyl (5-(2-methyl-2-(pyridin-2-yl)propionyl)pyridin-2-yl)carbamate (**I-71-b**) as a white solid with a yield of 24%. LC-MS: m/z: $(M+H)^+$ =342.0.

**Step 2:**

Tert-butyl (5-(2-methyl-2-(pyridin-2-yl)propionyl)pyridin-2-yl)carbamate (**I-71-b**) (270 mg, 0.79 mmol) was dissolved in 2 ml of dichloromethane, added with 2 ml of trifluoroacetic acid, stirred at 15 °C for 1h and concentrated. The residue was dissolved in 5 mL of dichloromethane, and neutralized with saturated aqueous ammonium bicarbonate to pH = 9. The organic phase was dried over anhydrous sodium sulfate, filtered and concentrated to obtain 150 mg of 1-(6-aminopyridin-3-yl)-2-methyl-2-(pyridin-2-yl)propan-1-one (**I-71-c**) as a white solid with a yield of 78.6%. LC-MS: m/z: $(M+H)^+$ =242.0.

**Step 3:**

1-(6-aminopyridin-3-yl)-2-methyl-2-(pyridin-2-yl)propan-1-one (**I-71-c**) (100 mg, 0.41 mmol) and 2-(4-ethylsulfonylphenyl)acetic acid (94.6 mg, 0.41 mmol) were dissolved in 3 ml of N,N-dimethylformamide, successively added with 107 mg of N,N-diisopropylethylamine and 396 mg of a 50% solution of 1-propylphosphoric anhydride in ethyl acetate, microwaved at 120 °C for 3h, and concentrated. The residue was purified by preparative HPLC to obtain 50 mg of 2-(4-(ethylsulfonyl)phenyl)-N-(5-(2-methyl-2-(pyridin-2-yl)propionyl)pyridin-2-yl)acet amide (**I-71**) as a white solid with a yield of 26.7%. LC-MS: m/z: $(M+H)^+$ = 452, $^1H$ NMR (400 MHz, $CDCl_3$) δ 8.55 (dd, *J* = 4.9, 0.9 Hz, 1H), 8.40 (d, *J* = 1.9 Hz, 1H), 8.25 (s, 1H), 8.08(d, *J* = 8.8 Hz, 1H), 7.95 - 7.89 (m, 2H), 7.84 (dd, *J* = 8.9, 2.3 Hz, 1H), 7.71 (td, *J* = 7.7, 1.8 Hz, 1H), 7.54(d, *J* = 8.3 Hz, 2H), 7.33 (d, *J* = 8.0 Hz, 1H), 7.20 (ddd, *J* = 7.5, 4.9, 1.0 Hz, 1H), 3.84 (s, 2H), 3.13 (q, *J* =7.4 Hz, 2H), 1.69 (s, 6H), 1.31 (t, *J* = 7.4 Hz, 3H).

**Example 72**

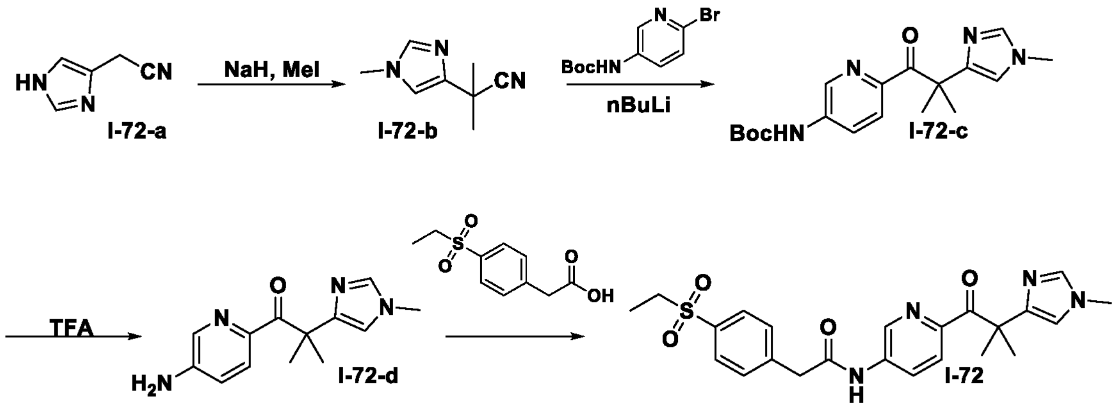


**Step 1:**

2-(1H-imidazol-4-yl)acetonitrile (**I-72-a**) (5 g, 46.678 mmol) was dissolved in dry tetrahydrofuran (100 mL), cooled to 0 °C and added with sodium hydride (6.53 g, 163.373 mmol, 60%) in batches. The system was raised to room temperature and stirred for 0.5 h, and added with iodomethane (43.07 g, 303.407 mmol). After the addition, the reaction was carried out at room temperature for 1h. Then the reaction system was cooled to 0 °C again, quenched with ice water at this temperature, and extracted with ethyl acetate (2×100 mL). The organic phases were combined, dried over anhydrous $Na_2SO_4$ and filtered, and the filtrate was rotary evaporated to dryness and purified by column chromatography with an eluent of (petroleum ether: ethyl acetate=1:1) to obtain 2-methyl-2-(1-methylimidazol-4-yl)propionitrile (**I-72-b**) (1.2 g, 18% yield) as a yellow oil. LCMS: $[M+H]^+$= 150.3.

**Step 2:**

Tert-butyl (6-bromopyridin-3-yl)carbamate (3.30 g, 12.064 mmol) was added to 12 ml of tetrahydrofuran, slowly added with n-butyllithium (2.5 M in n-hexane, 9.65 mL, 24.129 mmol) at -78 °C, stirred at this temperature for 1 h, added dropwise with a solution of 2-methyl-2-(1-methylimidazol-4-yl) propionitrile (**I-72-b**) (1.2 g, 8.043 mmol) in THF (5 mL), and stirred for 1h. The reaction system was quenched with saturated aqueous ammonium chloride at -78 °C, and extracted with ethyl acetate (2×50 mL). The organic phases were combined, dried over anhydrous $Na_2SO_4$, and concentrated. The obtained residue was purified by reverse phase column chromatography (C18 silica gel; mobile phase A: acetonitrile; mobile phase B: water (0.1% $NH_3 \cdot H_2O$); the volume ratio of A: 20% to 75%) to obtain tert-butyl N-{6-[2-methyl-2-(1-methylimidazol-4-yl)propionyl]pyridin-3-yl}carbamate (**I-72-c**) (114 mg, 5%) as a white solid. LCMS:(ESI, m/z): $[M+H]^+$= 345.1.

**Step 3:**

Tert-butyl N-{6-[2-methyl-2-(1-methylimidazol-4-yl)propionyl]pyridin-3-yl}carbamate (**I-72-c**) (114 mg, 0.330 mmol) was added to 6 ml of dichloromethane, added with 3 ml of trifluoroacetic acid, and stirred at room temperature for 1h. The reaction solution was concentrated, added with 10 ml of saturated aqueous sodium bicarbonate, and extracted with dichloromethane (2×20 ml). The organic phase was dried over anhydrous sodium sulfate and concentrated to obtain a crude as a yellow oil, which was purified by reverse phase column chromatography (C18 silica gel; mobile phase A: acetonitrile, mobile phase B: water (0.1% $NH_3 \cdot H_2O$), the volume ratio of A: 20% to 70%) to obtain 1-(5-aminopyridin-2-yl)-2-methyl-2-(1 -methylimidazol-4-yl)propan-1-one (**I-72-d**) (217 mg, yield 58%) as a white solid. LCMS: (ESI, m/z): $[M+H]^+$ = 245.05, $^1$H NMR (300 MHz, DMSO-$d_6$) δ 7.70 (m, 1H), 7.61 (m, 1H), 7.26 (m, 1H), 6.90 - 6.78 (m, 2H), 6.00 (s, 2H), 3.56 (s, 3H), 1.56 (s, 6H).

**Step 4:**

1-(5-aminopyridin-2-yl)-2-methyl-2-(1-methylimidazol-4-yl)propan-1-one (**I-72-d**) (47 mg, 0.193 mmol) obtained in previous step, 2-(4-(ethylsulfonyl)phenyl)acetic acid (88 mg, 0.386 mmol) and N,N-diisopropylethylamine (124 mg, 0.963 mmol) were added to dichloromethane (5 ml), added with $T_3P$ (50% in ethyl acetate, 368 mg, 0.578 mmol), and stirred at room temperature for 18h. The reaction solution was concentrated and separated by column chromatography (dichloromethane: methanol = 25:1) to obtain 16 mg of 2-(4-(ethylsulfonyl)phenyl)-N-(6-(2-methyl-2-(1-methyl-1H-imidazol-4-yl)propionyl)py ridin-3-yl)acetamide (**I-72**) as a white solid with a yield of 18%. LCMS:(ESI, m/z): $[M+H]^+$= 455.2. $^1$H NMR (400 MHz, DMSO-$d_6$) δ 10.68 (s, 1H), 8.61 (s, 1H), 8.05 (d, J = 8.3 Hz, 1H), 7.94 - 7.78 (m, 2H), 7.77 (d, J =

8.5 Hz, 1H), 7.59 (d, J = 7.6 Hz, 2H), 7.35 (s, 1H), 6.97 (s, 1H), 3.85 (s, 2H), 3.56 (s, 3H), 3.29 - 3.20 (m, 2H), 2.50 (s, 3H), 1.60 (s, 6H), 1.09 (t, J = 7.3 Hz, 3H).

**Example 73**

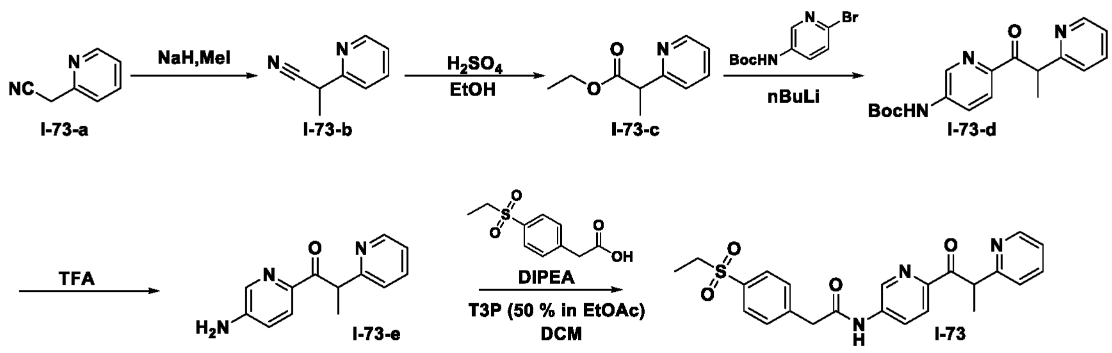


I-73-a I-73-b I-73-c I-73-d I-73-e I-73

**Step 1:**

2-(Pyridin-2-yl)acetonitrile (**I-73-a**) (5 g, 42.323 mmol) was dissolved in dry tetrahydrofuran (50 mL), cooled to 0 °C under nitrogen protection, added with NaH (2.54 g, 63.484 mmol, 60%) in batches and stirred. At this temperature, iodomethane (6.07 g, 42.746 mmol) was added dropwise to the mixture, and after the addition, the mixture was slowly raised to room temperature and stirred for 2h. LCMS showed that the reacton was complete, and the raction mixture was cooled to 0 °C, quenched with saturated aqueous ammonium chloride (100 mL), added with water (100 mL), and extracted with ethyl acetate (3×300 mL). The organic phases were combined, dried over anhydrous $Na_2SO_4$ and filtered, and the filtrate was concentrated and separated by column chromatography (PE:EA=10:1) to obtain 2-(pyridin-2-yl)propionitrile (**I-73-b**) (3.2 g, 57%) as a yellow oil. LCMS: (ESI, m/z): $[M+1]^+$= 133.3.

**Step 2:**

2-(Pyridin-2-yl)propionitrile (**I-73-b**) (3.2 g, 24.212 mmol) was dissolved in ethanol (10 mL), added slowly with $H_2SO_4$ (11.87 g, 121.060 mmol) in an ice bath, raised to 80 °C and stirred overnight. LCMS showed that the reaction was complete, and the reaction system was poured into an ice water, and extracted with ethyl acetate (3×300 mL). The organic phases were combined, dried over anhydrous $Na_2SO_4$ and filtered, and the filtrate was concentrated and separated by column chromatography (PE:EA=5:1) to obtain ethyl 2-(pyridin-2-yl)propionate (**I-73-c**) (2.4 g, 55%) as a yellow oil. LCMS: (ESI, m/z): $[M+1]^+$=180.0.

**Step 3:**

Tert-butyl (6-bromopyridin-3-yl)carbamate (4.39 g, 16.069 mmol) was added into 25 ml of anhydrous tetrahydrofuran, slowly added with n-butyllithium (2.5 M in n-hexane, 16.07 mL, 40.173 mmol), stirred at this temperature for 1 h, slowly added with a solution of ethyl 2-(pyridin-2-yl)propionate (**I-73-c**) (2.4 g, 13.391 mmol) in tetrahydrofuran (5 ml), and stirred at -78 °C for 2h. The reaction solution was quenched with saturated aqueous $NH_4Cl$ (50 ml), and extracted with ethyl acetate (3×100 mL). The organic phase was washed once with saturated brine, dried over anhydrous sodium sulfate and concentrated, and the residue was separated by reverse phase column chromatography (C18 silica gel; mobile phase A: acetonitrile, mobile phase B: water (0.1% trifluoroacetic acid), the volume ratio of A: 60% to 80%) to obtain tert-butyl N-{6-[2-(pyridin-2-yl)propanoyl]pyridin-3-yl}carbamate (**I-73-d**) (320 mg, 7%) as a yellow solid. LCMS: (ESI, m/z): $[M+1]^+$= 328.2.

**Step 4:**

Tert-butyl N-{6-[2-(pyridin-2-yl)propionyl]pyridin-3-yl}carbamate (**I-73-d**) (320 mg, 0.977 mmol) was added to 6 ml of dichloromethane, added with 3 ml of trifluoroacetic acid, and stirred at room temperature for 1h. The reaction solution was concentrated, added with 10 ml of saturated aqueous sodium bicarbonate, and extracted with dichloromethane (2×20 ml). The organic phase was dried over anhydrous sodium sulfate and concentrated to obtain a crude, which

was separated by reverse phase column chromatography (C18 silica gel; mobile phase A: acetonitrile; mobile phase B: water (0.1% $NH_3 \cdot H_2O$), the volume ratio of A: 10% to 50%) to obtain 1-(5-aminopyridin-2-yl)-2-(pyridin-2-yl)propyl-1-one (**I-73-e**) (102.6 mg, 46%) as a yellow solid. LCMS: (ESI, m/z): $[M+H]^+$= 228.05. LCMS: (ESI, m/z): $[M+1]^+$= 228.05. $^1$H NMR (400 MHz, deuterated chloroform) δ 8.59 - 8.48 (m, 1H), 8.03 (m, 1H), 7.93 (m, 1H), 7.75 - 7.62 (m, 1H), 7.41 (m, 1H), 7.21 - 7.10 (m, 1H), 6.94 (m, 1H), 5.58 (m, 1H), 4.14 (m, 2H), 1.60 (d, *J*= 7.1 Hz, 3H).

**Step 5:**

1-(5-aminopyridin-2-yl)-2-(pyridin-2-yl)propyl-1-one (**I-73-e**) (50 mg, 0.22 mmol) obtained in the previous step, 2-(4-(ethylsulfonyl)phenyl)acetic acid (50 mg, 0.22 mmol) and N,N-diisopropylethylamine (120 mg, 0.93 mmol) were added to dichloromethane (5 ml), added with $T_3P$ (50% in ethyl acetate, 270 mg, 0.42 mmol), and stirred at room temperature for 4h. The reaction solution was concentrated and separated by column chromatography to obtain 24 mg of 2-(4-(ethylsulfonyl)phenyl)-N-(6-(2-(pyridin-2-yl)propanoyl)pyridin-3-yl)acetamide (**I-73**) as a white solid. LC-MS: m/z: $(M+H)^+$ =438.2, $^1$HNMR(400 MHz, DMSO-$d_6$) δ 10.77 (s, 1H), 8.74 (d, J = 2.2 Hz, 1H), 8.33 (ddd, J = 4.8, 1.8, 0.8 Hz, 1H), 8.17 (dd, J = 8.6, 2.5 Hz, 1H), 7.96 (d, J = 8.6 Hz, 1H), 7.88 - 7.79 (m, 2H), 7.71 (td, J = 7.7, 1.9 Hz, 1H), 7.60 (d, J = 8.4 Hz, 2H), 7.42 (d, J = 7.9 Hz, 1H), 7.15 (ddd, J = 7.5, 4.9, 1.0 Hz, 1H), 5.37 (q, J = 7.0 Hz, 1H), 3.87 (s, 2H), 3.27 (q, J = 7.3 Hz, 2H), 1.42 (d, J = 7.0 Hz, 3H), 1.09 (t, J = 7.4 Hz, 3H).

**Example 75**

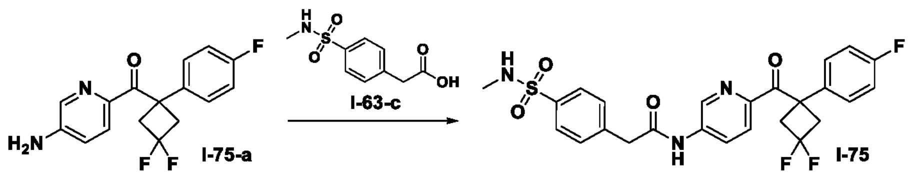


I-75-a

I-75

Similar to the synthesis method of (5-aminopyridin-2-yl)(1-(4-chlorophenyl)-3,3-difluorocyclobutyl)methanone (**I-35-f**) in Example 35, (5-aminopyridin-2-yl)(1-(4-fluorophenyl)-3,3-difluorocyclobutyl)methanone (**I-75-a**) was synthesized. (5-aminopyridin-2-yl)(1-(4-fluorophenyl)-3,3-difluorocyclobutyl)methanone (**I-75-a**) (50 mg, 0.163 mmol), 2-(4-(N-methylsulfamoyl)phenyl)acetic acid (**I-63-c**)(75 mg, 0.327 mmol) and N,N-diisopropylethylamine (100 mg, 0.775 mmol) were added into 10 ml of dichloromethane, added with a 50% solution of $T_3P$ (260 mg, 0.408 mmol) in ethyl acetate, and stirred overnight at room temperature. The reaction solution was washed with water, dried over anhydrous sodium sulfate, concentrated, and separated by column chromatography (DCM:MeOH=10:1) to obtain 16 mg of N-(6-(3,3-difluoro-1-(4-fluorophenyl)cyclobutane-1-carbonyl)pyridin-3-yl)-2-(4-(N-met hylsulfamoyl)phenyl)acetamide (**I-75**) as a white solid with a yield of 19%. LC-MS: m/z: $(M+H)^+$ = 518.2, $^1$HNMR (400 MHz, MeOD) δ 8.74 (d, J = 2.2 Hz, 1H), 8.12 (dd, J = 8.6, 2.5 Hz, 1H), 7.95 (d, J = 8.6 Hz, 1H), 7.79 (d, J = 8.4 Hz, 2H), 7.63 - 7.41 (m, 4H), 7.08 - 6.92 (m, 2H), 3.83 (s, 2H), 3.59 (dd, J = 27.9, 13.1 Hz, 2H), 3.29 - 3.16 (m, 2H), 2.51 (s, 3H).

Referring to the above examples, the compounds listed in Table 1 were prepared as follows:

Table 1 Compound list

| Compound | Formula | Characterized data | Referred method |
|---|---|---|---|
| I-15 | 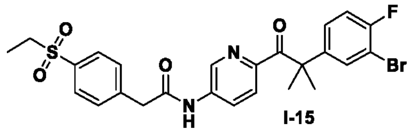 I-15 | LC-MS: m/z: $(M+H)^+$= 547.1, 549.1. $^1$H NMR (400 MHz, $CDCl_3$) δ 8.30 (d, J = 2.4 Hz, 1H), 8.12 (dd, J = 8.7, 2.5 Hz, 1H), 7.99 (d, J = 8.7 Hz, 1H), 7.85 (t, J = 6.8 Hz, 2H), 7.69 (s, 1H), 7.52 - 7.42 (m, 3H), 7.12 - 7.04 (m, 1H), 6.94 (t, J = 8.5 Hz, 1H), 3.80 (s, 2H), 3.17 - 3.05 (q, J = 7.4 Hz, 2H), 1.69 (s, 6H), 1.28 (t =7.8 Hz, 3H). | I-13 |

(continued)

| Compound | Formula | Characterized data | Referred method |
|---|---|---|---|
| I-28 | 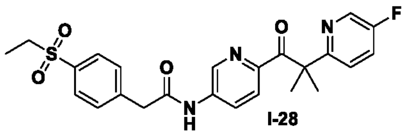 I-28 | LC-MS: m/z: $(M+H)^+$= 470.2. $^1H$ NMR (400 MHz, $CDCl_3$) δ 8.35 (s, 2H), 8.13 (s, 1H), 7.98 (d, J = 8.4 Hz, 1H), 7.92 (d, J = 8.3 Hz, 1H), 7.81 (d, J = 8.0 Hz, 2H), 7.48 (d, J = 7.9 Hz, 2H), 7.37 (d, J = 5.2 Hz, 2H), 3.82 (s, 2H), 3.10 (q, J = 7.4 Hz, 2H), 1.71 (s, 6H), 1.27 (t, J = 7.4 Hz, 3H). | I-27 |
| I-29 | 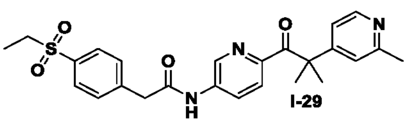 I-29 | LC-MS: m/z: $(M+H)^+$= 466.1. $^1H$ NMR (400 MHz, $CDCl_3$) δ 9.74 (s, 1H), 8.40 - 8.23 (m, 2H), 8.18 (s, 1H), 8.02 (d, J = 8.6 Hz, 1H), 7.80 (d, J = 7.3 Hz, 2H), 7.47 (d, J = 7.3 Hz, 2H), 7.05 (s,2H), 3.83 (s, 2H), 3.10 (q, J = 7.3 Hz, 2H), 2.48 (s, 3H), 1.66 (s, 3H), 1.26 (t, J = 7.4 Hz, 3H). | I-27 |
| I-30 | 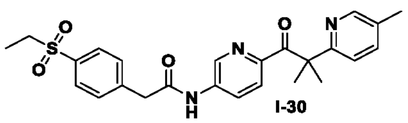 I-30 | LC-MS: m/z: $(M+H)^+$=466.2. $^1H$ NMR (400 MHz, $CDCl_3$) δ 9.72 (s, 1H), 8.56 (s, 1H), 8.15 (s, 1H), 7.93 - 7.85 (m, 3H), 7.73 (dd, *J* = 8.9, 2.4 Hz, 1H), 7.58 (dd, *J* = 8.8, 7.0 Hz, 3H), 7.31 (s, 1H), 3.93 (s, 2H), 3.12 (q, *J* = 7.4 Hz, 2H), 2.30 (s, 3H), 1.75 (s, 6H), 1.29 (t, *J* = 7.5 Hz, 3H). | I-27 |
| I-31 | 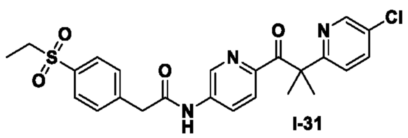 I-31 | LC-MS: m/z: $(M+H)^+$=486.1. $^1H$ NMR (400 MHz, $CDCl_3$) δ 8.40 (s, 1H), 8.30 (d, *J* = 2.5 Hz, 1H), 8.25 (s, 1H), 8.01 (d, *J* = 2.2 Hz, 2H), 7.93 - 7.85 (m, 2H), 7.69 (dd, *J* = 8.6, 2.5 Hz, 1H), 7.56 (d, *J* = 8.0 Hz, 2H), 7.38 (d, *J* = 8.5 Hz, 1H), 3.88 (s, 2H), 3.13 (q, *J* = 7.4 Hz, 2H), 1.74 (s, 6H), 1.30 (t, *J* = 7.5 Hz, 3H). | I-27 |
| I-34 | 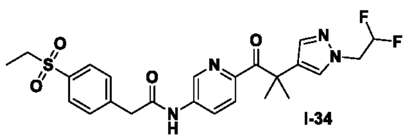 I-34 | LC-MS: m/z: $(M+H)^+$ =505, $^1H$ NMR (400 MHz, $CDCl_3$) δ 8.55 (d, 2H), 8.14 (dd, *J* = 8.7, 2.4 Hz, 1H), 7.86 (d, *J* = 8.7 Hz, 1H), 7.82 (d, *J* = 8.3 Hz, 2H), 7.54 - 7.45 (m, 3H), 7.40 (s, 1H), 6.00 (tt, *J* = 55.4, 4.2 Hz, 1H), 4.38 (td, *J* = 13.7, 4.2 Hz, 2H), 3.84 (s, 2H), 3.14 (q, *J* = 7.4 Hz, 2H), 1.76 (s, 6H), 1.30 (t, *J* = 7.4 Hz, 3H). | I-33 |
| I-50 | 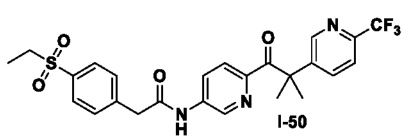 I-50 | LC-MS: 520.1$[M+H]^+$. 1H NMR (400 MHz, DMSO-$d_6$) δ 8.62 (s, 1H), 8.48 (d, J = 1.8 Hz, 1H), 8.16 (dd, J = 8.6, 2.1 Hz, 1H), 8.03(d, J = 8.7 Hz, 1H), 7.89 (d, J = 7.3 Hz, 1H), 7.80 (dd, J = 19.8, 8.2 Hz, 3H), 7.58 (d, J = 8.1 Hz, 2H), 3.30- 3.24 (m, 2H), 1.71 (s, 6H), 1.20 - 1.13 (m, 2H), 1.09 (t, J = 7.3 Hz, 3H). | I-48 |
| I-52 | 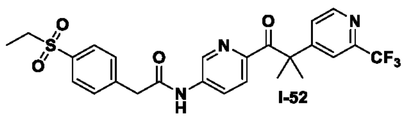 I-52 | LC-MS: 520.2$[M+H]^+$ 1H NMR (400 MHz, $CDCl_3$) δ 8.50 (d, J = 4.1 Hz, 1H), 8.42 (s, 1H), 8.24 (d, J = 6.5 Hz, 2H), 8.03 (d, J = 8.5 Hz, 1H), 7.78 (d, J = 7.7 Hz, 2H), 7.59 (s, 1H), 7.44 (d, J = 7.8 Hz, 2H), 7.29 (s, 1H), 3.79 (s, 2H), 3.10 (q, J = 7.4 Hz, 2H), 1.71 (s, 6H), 1.26 (t, J = 7.4 Hz, 3H). | I-3 |

(continued)

| Compound | Formula | Characterized data | Referred method |
|---|---|---|---|
| I-55 | 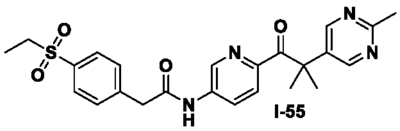 I-55 | LC-MS: m/z: $(M+H)^+$ = 466.9. 1H NMR (400 MHz, $CDCl_3$) δ 8.69 (s, 2H), 8.35 (s, 2H), 8.30 (d, J = 8.6 Hz, 1H), 8.05 (d, J = 8.6 Hz, 1H), 7.88 (d, J = 7.9 Hz, 2H), 7.56 (d, J = 8.1 Hz, 2H), 3.87 (s, 2H), 3.11 (q, J = 7.4 Hz, 2H), 2.81 (s, 3H), 1.78 (s, 6H), 1.29 (t, J = 7.4 Hz, 3H). | I-44 |
| I-57 | 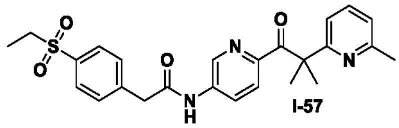 I-57 | LC-MS: 466.1$[M+H]^+$. 1H NMR (400 MHz, DMSO-$d_6$) δ 10.61 (s, 1H), 8.41 (d, J = 2.3 Hz, 1H), 8.09 (dd, J = 8.7, 2.5 Hz, 1H),7.96 (d, J = 8.7 Hz, 1H), 7.84 (d, J = 8.3 Hz, 2H), 7.60 (dd, J = 15.6, 8.0 Hz, 3H), 7.25 (d, J = 7.8 Hz, 1H),6.94 (d, J = 7.6 Hz, 1H), 3.84 (s, 2H), 3.27 (q, J = 7.4 Hz, 2H), 2.17 (s, 3H), 1.61 (s, 6H), 1.10 (t, J = 7.4Hz, 3H). | I-40 |
| I-61 | 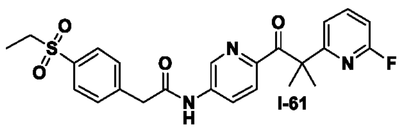 I-61 | LCMS $[M+H]^+$=470.2. $^1$H NMR (400 MHz, MeOD) δ 8.40 (d, J = 2.3 Hz, 1H), 8.11 (dd, J = 8.7, 2.3 Hz, 1H), 8.01 (d, J = 8.7 Hz, 1H), 7.90 - 7.79 (m, 3H), 7.59 (d, J = 8.1 Hz, 2H), 7.34 (dd, J = 7.6, 2.3 Hz, 1H), 6.74 (dd, J = 8.1, 2.5 Hz, 1H), 3.84 (s, 2H), 3.19 (q, J = 7.4 Hz, 2H), 1.67 (s, 6H), 1.21 (t, J = 7.4 Hz, 3H). | I-41 |
| I-62 | 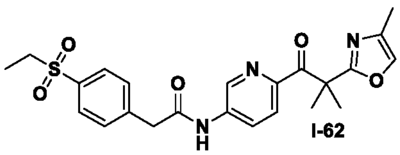 I-62 | LCMS: $[M+H]^+$= 456.2. $^1$H NMR (400 MHz, $CDCl_3$) δ 10.73 (s, 1H), 8.56 - 8.37 (m, 2H), 8.06 (d, *J* = 8.7 Hz, 1H), 7.89 (d, *J* = 8.2 Hz, 2H), 7.56 (d, *J* = 8.2 Hz, 2H), 7.19 (d, *J* = 0.8 Hz, 1H), 3.96 (s, 2H), 3.11 (q, *J* = 7.4 Hz, 2H), 2.03 (s, 3H), 1.73 (s, 6H), 1.28 (t, *J* = 7.4 Hz, 3H). | I-56 |
| I-66 | 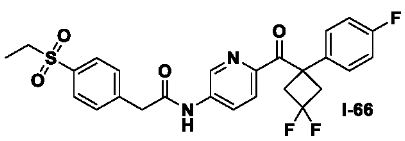 I-66 | LCMS: $[M+H]^+$ = 517.2. $^1$H NMR (400 MHz, DMSO-$d_6$) δ 10.79 (s, 1H), 8.78 (d, J = 2.4 Hz, 1H), 8.11 (dd, J = 8.7, 2.5 Hz, 1H), 7.96 (d, J = 8.6 Hz, 1H), 7.84 (d, J = 8.3 Hz, 2H), 7.59 (d, J = 8.3 Hz, 2H), 7.51 (dd, J = 8.8, 5.3 Hz, 2H), 7.12 (t, J = 8.9 Hz, 2H), 3.87 (s, 2H), 3.56 (dd, J = 27.5, 14.7 Hz, 2H), 3.27 (m, 4H), 1.09 (t, J = 7.4 Hz, 3H). | 1-35 |
| I-67 | 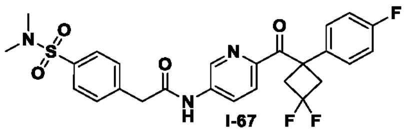 I-67 | LCMS $[M+H]^+$=532.2. $^1$HNMR (400 MHz, DMSO-$d_6$) δ 10.79 (s, 1H), 8.79 (d, J = 2.3 Hz, 1H), 8.12 (dd, J = 8.7, 2.5 Hz, 1H), 7.96 (d, J = 8.6 Hz, 1H), 7.71 (d, J = 8.4 Hz, 2H), 7.58 (d, J = 8.4 Hz, 2H), 7.55 - 7.44 (m, 2H), 7.12 (t, J = 8.9 Hz, 2H), 3.86 (s, 2H), 3.57 (dd, J = 27.5, 14.6 Hz, 2H), 3.28 (m, 2H), 2.60 (s, 6H). | I-64 |

(continued)

| Compound | Formula | Characterized data | Referred method |
|---|---|---|---|
| I-69 | 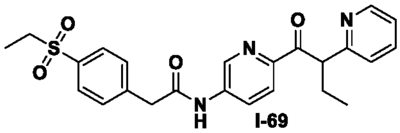 I-69 | LC-MS: 452.2[M+H]$^+$. $^1$HNMR (400 MHz, DMSO-$d_6$) δ 10.79 (s, 1H), 8.78 (d, J = 2.2 Hz, 1H), 8.37 (d, J = 4.0 Hz, 1H), 8.17 (dd,J = 8.7, 2.4 Hz, 1H), 7.96 (d, J = 8.6 Hz, 1H), 7.85 (d, J = 8.3 Hz, 2H), 7.71 (td, J = 7.7, 1.8 Hz, 1H), 7.61(d, J = 8.2 Hz, 2H), 7.43 (d, J = 7.9 Hz, 1H), 7.16 (dd, J = 6.7, 5.0 Hz, 1H), 5.26 (t, J = 7.3 Hz, 1H), 3.88(s, 2H), 3.28 (q, J = 7.3 Hz, 2H), 2.08 (dt, J = 14.3, 7.2 Hz, 1H), 1.94 - 1.80 (m, 1H), 1.10 (t, J = 7.3 Hz,3H), 0.84 (t, J = 7.4 Hz, 3H). | I-73 |
| I-70 | 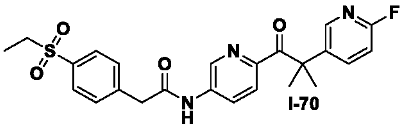 I-70 | LC-MS: 467.2[M+H]$^+$. $^1$HNMR (400 MHz, DMSO-$d_6$) δ 10.70 (s, 1H), 8.42 (s, 1H), 8.03 (d, J = 9.0 Hz, 3H), 7.89 -7.74 (m, 4H),7.58 (d, J = 7.0 Hz, 2H), 3.84 (s, 2H), 3.27 (d, J = 6.9 Hz, 2H), 1.63 (s, 6H), 1.10 (d, J = 6.6 Hz, 3H). | I-20 |
| I-74 | 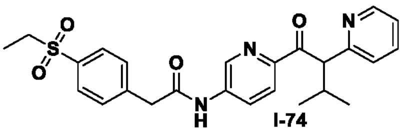 I-74 | LC-MS: m/z: (M+H)$^+$ =466.2, $^1$H NMR (400 MHz, $CDCl_3$) δ 10.07 (s, 1H), 8.89 (d, J = 2.4 Hz, 1H), 8.46 (dd, J = 5.0, 0.9 Hz, 1H), 8.36 (dd, J = 8.7, 2.4 Hz, 1H), 8.03 (d, J = 8.7 Hz, 1H), 7.84 (d, J = 8.3 Hz, 2H), 7.71 (td, J = 7.7, 1.8 Hz, 1H), 7.57 (d, J = 7.9 Hz, 1H), 7.50 (d, J = 8.3 Hz, 2H), 7.19 (ddd, J = 7.4, 5.0, 1.0 Hz, 1H), 5.39 (d, J = 10.4 Hz, 1H), 3.84 (s, 2H), 3.12 (q, J = 7.4 Hz, 2H), 2.71 (qd, J = 13.2, 6.6 Hz, 1H), 1.29 (t, J = 7.4 Hz, 3H), 1.02 (t, J = 7.9 Hz, 3H), 0.84 (d, J = 6.7 Hz, 3H). | I-73 |
| I-76 | 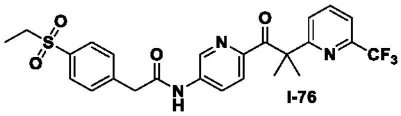 I-76 | LC-MS: 520.2[M+H]$^+$. $^1$HNMR (400 MHz, DMSO-$d_6$) δ 10.68 (s, 1H), 8.41 (s, 1H), 8.12 (d, J = 7.4 Hz, 1H), 8.01 (d, J = 7.7 Hz,2H), 7.82 (dd, J = 24.2, 7.3 Hz, 3H), 7.60 (s, 3H), 3.86 (s, 2H), 3.27 (d, J = 6.8 Hz, 2H), 1.66 (s, 6H), 1.11(d, J = 6.3 Hz, 3H). | I-20 |

**Experimental example 1**

I. The inhibitory activity of the compound on RORγt

1. Test method:

Fluorescence resonance energy transfer (FRET) experiment was performed to assay the inhibitory activity of the compound of the present invention on RORγt.

2. Materials and Reagents:

| Materaials and reagents | Supplier | Product Catalog No. |
|---|---|---|
| SRC(Biotinyl-CPSSHSSLTERHKIL HRL) | Sangon Biotech | P13475 |
| RORgt-LBD, GST | Invitrogen | PV5887 |
| Europium anti-GST | Cisbio | 61GSTKLB |
| streptavidin-D2 | Cisbio | 610SADAB |

(continued)

| Materaials and reagents | Supplier | Product Catalog No. |
|---|---|---|
| TR-FRET Comodulator Buffer D | Invitrogen | PV4420 |
| DTT | Invitrogen | P2325 |
| DMSO | Sigma | D8418 |
| Consumables and instruments | Supplier | Product Catalog No. |
| 384-well plate | Greiner | 784075 |
| 96-well plate | Nunc | 249944 |
| Shock-shaking bed | Thermo | 4625-1 CECN/THZ Q |
| Centrifuge | Eppendorf | 5810R |
| Multilabel Plate Reader | PerkinElmer | 2104-0010 |
| Vortex instrument | IKA | MS3 digital |

3. Experimental steps:

3.1 Preparation and treatment of the compounds

1) Preparation of DMSO stock solutions of the compounds: All compounds were dissolved in DMSO to prepare 10 mM stock solutions.
2) Compound storage: After the compound was dissolved in DMSO, the solution can be stored in a desiccator at room temperature for three months. For long-term storage, the solution should be put in a -20°C refrigerator.
3) Preparation of working solution:

a) Positive control compound AZD0284: Starting from 500 μM, the solution was diluted serially by 3-folds with DMSO to obtian a 10-concentration gradient, each concentration with 3 replicate wells. 2 wells were pipetted. The gradiently diluted compound was diluted in a reaction buffer to obtain a 4X working solution (starting from 40 μM, diluted gradiently by 3-folds) of the positive control compound.
b) Compounds to be tested: Starting from 500 μM, the solution was diluted serially by 3-folds with DMSO to obtain a 10-concentration gradient, each concentration with 3 replicate wells. 2 wells were pipetted. The gradiently diluted compound was diluted in a reaction buffer to obtain a 4X working solution (4, diluted gradiently by 3-folds) of the compounds to be tested.
c) Prepare a 4X negative control (8% DMSO).
d) Centrifuge the plate at 1000 rpm for Imin.

3.2 Compound screening

1) Prepare 4x serially diluted compounds in 1x buffer.
2) Add 5ul of the 4x serially diluted compound (prepared in step 1) to a 384 assay plate (784075, Greiner).
3) Prepare 4x RORgt-LBD in 1x buffer.
4) Add 5ul of the 4x RORgt-LBD (prepared in step 3) to the 384 assay plate (prepared in step 2).
5) Incubate the assay plate for 15min at room temperature, protected from light.
6) Prepare a 2x mixture of SRC, anti-GST Eu and streptavidin-d2 in 1x freezing buffer.
7) Add 10ul of the 2x mixture (prepared in step 6) to the 384 assay plate (prepared in step 4).
8) Centrifuge the 384 assay plates at 1000g for Imin.
9) Incubate for 3h at room temperature, protected from light.
10) Read the plate at wavelengths of 665nm and 615nm on an Envision 2104 plate reader.

4. Data analysis:

Relative ratio (RR): Calculate the relative ratio (response value at 665nm/ response value at 615nm - response value in blank background) of each well.

Percent Inhibition Rate:

Inhibition rate%=[1-(fluorescence detection value of the compound - fluorescence detection average of the positive compound)/(fluorescence detection average of the negative control - fluorescence detection average of the positive compound)]x100

Calculate the $IC_{50}$ and dose-response curve of the compound: The $IC_{50}$ and dose-response curve of the compound were obtained via Graphpad 8.0 by calculating the inhibition rate of the compound and the log value of the compound concentration.

2. Test result data.

Table 2 Determination of Inhibitory Activity of Examplary Compounds on RORyt

| Compound No. | $IC_{50}$ | Compound No. | $IC_{50}$ | Compound No. | $IC_{50}$ |
|---|---|---|---|---|---|
| I-1 | +++ | I-2 | ++ | I-3 | ++ |
| I-4 | +++ | I-5 | +++ | I-6 | +++ |
| I-7 | ++ | I-8 | +++ | I-9 | +++ |
| I-10 | +++ | I-11 | +++ | I-12 | +++ |
| I-13 | +++ | I-14 | +++ | I-15 | +++ |
| I-19 | +++ | I-20 | ++ | I-23 | ++ |
| I-24 | ++ | I-25 | +++ | I-26 | +++ |
| I-27 | +++ | I-28 | ++ | I-29 | +++ |
| I-30 | +++ | I-31 | +++ | I-32 | ++ |
| I-33 | ++ | I-34 | ± | I-35 | +++ |
| I-36 | ++ | I-37 | ± | I-38 | - |
| I-39 | - | I-40 | +++ | I-41 | +++ |
| I-42 | ++ | I-43 | ++ | I-44 | +++ |
| I-45 | ± | I-46 | ++ | I-47 | +++ |
| I-48 | + | I-50 | + | I-52 | +++ |
| I-54 | +++ | I-55 | ± | I-56 | ++ |
| I-57 | +++ | I-58 | +++ | I-59 | ++ |
| I-60 | ± | I-61 | +++ | I-62 | + |
| I-63 | ++ | I-64 | - | I-65 | +++ |
| I-66 | +++ | I-67 | ± | I-68 | ± |
| I-69 | ++ | I-70 | ++ | I-71 | + |
| I-72 | + | I-73 | ++ | I-74 | ++ |
| I-75 | +++ | I-76 | +++ | | |
| Note: "-" means $IC_{50}$ > 1000nM, "±" means 100nM < $IC_{50}$≤ 1000nM, "+" means 50nM ≤ $IC_{50}$ ≤ 100nM, "++" means 10nM ≤ $IC_{50}$ < 50nM, "+++" means 1nM ≤ $IC_{50}$ < 10nM. | | | | | |

Conclusion: It can be seen from Table 1 that the compounds of the present invention have significant inhibitory effect on RORyt.

Although the specific embodiments of the present invention have been described above, those skilled in the art should understand that these are only examples for illustrating, and various changes or modifications can be made to these embodiments without departing from the principle and essence of the present invention. Accordingly, the protection scope of the present invention is defined by the appended claims.

## Claims

1. A compound represented by formula I, or a pharmaceutically acceptable salt, solvate, solvate of the pharmaceutically acceptable salt, metabolite or prodrug thereof:

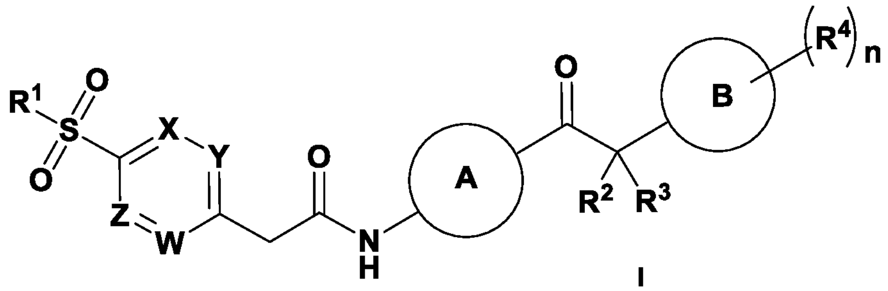

I

wherein, n is 0, 1, 2, 3, 4 or 5;
W, X, Y and Z are independently CH or N;
ring A and ring B are each independently $C_3$-$C_{14}$ cycloalkyl, 3- to 14-membered heterocycloalkyl, $C_3$-$C_{14}$ cycloalkenyl, 3- to 14-membered heterocycloalkenyl, $C_6$-$C_{12}$ aryl, or 5- to 10-membered heteroaryl, the 3- to 14-membered heterocycloalkyl, the 3- to 14-membered heterocycloalkenyl and the 5- to 10-membered heteroaryl independently contain one or more heteroatoms selected from the group conisisting of boron, silicon, oxygen, sulfur, selenium, nitrogen and phosphorus, and the number of heteroatoms is independently 1, 2, 3 or 4; particularly, ring A and ring B are each independently $C_3$-$C_{10}$ cycloalkyl, 3- to 10-membered heterocycloalkyl, $C_6$-$C_{10}$ aryl or 5- to 6-membered heteroaryl, the 5- to 6-membered heteroaryl and the 3- to 10-membered heterocycloalkyl each independently contain one or more heteroatoms selected from the group consisting of oxygen, sulfur and nitrogen, and the number of heteroatoms is independently 1, 2 or 3; more particularly, ring A and ring B are each independently cyclopropyl, cyclobutyl, cyclopentyl, cyclohexyl, oxetanyl, pyrrolidinyl, tetrahydrofuryl, tetrahydrothiophenyl, tetrahydropyranyl, tetrahydrothiopyranyl, piperidinyl, piperazinyl, morpholinyl, phenyl, naphthyl, pyrazolyl, imidazolyl, thiazolyl, oxazolyl, indolyl, furyl, thienyl, benzothienyl, benzofuryl, pyrazinyl, pyridazinyl, pyridyl, pyrimidyl or pyrrolyl;
wherein, ring A is optionally substituted by 1 to 3 substituents selected from the group consisting of halogen, $C_1$-$C_4$ alkyl, $C_1$-$C_4$ alkoxy, hydroxyl and cyano;
$R^1$ is independently $-OR^{1-1}$, $-NR^{1-2}R^{1-3}$, $C_1$-$C_7$ alkyl, $C_3$-$C_{14}$ cycloalkyl, 3- to 14-membered heterocycloalkyl, $R^{1-4}$-substituted $C_1$-$C_7$ alkyl, or $R^{1-4}$-substituted 3- to 14-membered heterocycloalkyl, said 3- to 14-membered heterocycloalkyl independently contains one or more heteroatoms selected from the group consisting of boron, silicon, oxygen, sulfur, selenium, nitrogen and phosphorus, and the number of heteroatoms is independently 1, 2, 3 or 4; particularly, $R^1$ is independently $-OR^{1-1}$, $C_1$-$C_4$ alkyl, $-NR^{1-2}R^{1-3}$, or $R^{1-4}$-substituted $C_1$-$C_4$ alkyl; more particularly, $R^1$ is independently methoxy, ethoxy, propoxy, methyl, ethyl, n-propyl, isopropyl, methylamino, dimethylamino, halogen-substituted methyl, halogen-substituted ethyl, halogen-substituted n-propyl, halogen-substituted isopropyl;
$R^2$ and $R^3$ are each independently hydrogen, halogen, $-OR^{2-1}$, -CN, $-NR^{2-2}R^{2-3}$, $C_1$-$C_7$ alkyl, $C_3$-$C_{14}$ cycloalkyl, 3- to 14-membered heterocycloalkyl, $R^{2-4}$-substituted $C_1$-$C_7$ alkyl, $R^{2-4}$-substituted $C_3$-$C_{14}$ cycloalkyl, or $R^{2-4}$-substituted 3- to 14-membered heterocycloalkyl, or $R^2$ and $R^3$ together form oxo, the 3- to 14-membered heterocycloalkyl group independently contains one or more heteroatoms selected from the group consisting of boron, silicon, oxygen, sulfur, selenium, nitrogen and phosphorus, and the number of heteroatoms is independently 1, 2, 3 or 4; particularly, $R^2$ and $R^3$ are each independently hydrogen, halogen, -CN, $C_1$-$C_4$ alkyl, $C_3$-$C_8$ cycloalkyl, or $R^{2-4}$-substituted $C_1$-$C_4$ alkyl; more particularly, $R^2$ and $R^3$ are each independently hydrogen, halogen, -CN, methyl, ethyl, n-propyl, isopropyl, cyclopropyl, cyclobutyl, halogen-substituted methyl, halogen-substituted ethyl;
or, $R^2$ and $R^3$ together with the carbon atom attached thereto form a $C_3$-$C_{14}$ cycloalkyl, a 3- to 14-membered heterocycloalkyl, a $R^{3-1}$-substituted $C_3$-$C_{14}$ cycloalkyl, or a $R^{3-1}$-substituted 3- to 14-heterocycloalkyl, the 3- to

14-membered heterocycloalkyl independently contains one or more heteroatoms selected from the group consisting of boron, silicon, oxygen, sulfur, selenium, nitrogen and phosphorus, and the number of the heteroatoms is independently 1, 2, 3 or 4; particularly, $R^2$ and $R^3$ together with the carbon atom attached thereto form a $C_3$-$C_8$ cycloalkyl, a 3- to 8-membered heterocycloalkyl, a $R^{3-1}$-substituted $C_3$-$C_8$ cycloalkyl, or a $R^{3-1}$-substituted 3- to 8-membered heterocycloalkyl, the 3- to 8-membered heterocycloalkyl independently contains one or more heteroatoms selected from the group consisting of oxygen, sulfur and nitrogen, and the number of heteroatoms is independently 1, 2 or 3; more particularly, $R^2$ and $R^3$ together with the carbon atom attached thereto form cyclopropyl, halogen-substituted cyclopropyl, cyclobutyl, halogen-substituted cyclobutyl, cyclopentyl, cyclohexyl, oxetanyl, pyrrolidinyl, tetrahydrofuranyl, tetrahydrothienyl, tetrahydropyranyl, piperidinyl or morpholinyl;

Each $R^4$ is independently hydrogen, halogen, $-OR^{4-1}$, -CN, $-NR^{4-2}R^{4-3}$, $-C(=O)R^{4-4}$, $-S(=O)_2R^{4-5}$, $C_3$-$C_{14}$ cycloalkyl, 3- to 14-membered heterocycloalkyl, $C_1$-$C_7$ alkyl, $R^{4-6}$-substituted $C_1$-$C_7$ alkyl, $R^{4-6}$-substituted $C_3$-$C_{14}$ cycloalkyl, $R^{4-6}$-substituted 3- to 14-membered heterocycloalkyl, or oxo, the 3- to 14-membered heterocycloalkyl independently contains one or more heteroatoms selected from the group consisting of boron, silicon, oxygen, sulfur, selenium, nitrogen and phosphorus, and the number of heteroatoms is independently 1, 2, 3 or 4; particularly, each $R^4$ is independently hydrogen, halogen, $-OR^{4-1}$, -CN, $C_3$-$C_8$ cycloalkyl, 3- to 8-membered heterocycloalkyl, $C_1$-$C_4$ alkyl, $R^{4-6}$-substituted $C_1$-$C_4$ alkyl, or oxo, the 3- to 8-membered heterocycloalkyl independently contains one or more heteroatoms selected from the group consisting of oxygen, sulfur and nitrogen, and the number of heteroatoms is independently 1, 2 or 3; more particularly, each $R^4$ is independently hydrogen, halogen, $C_1$-$C_4$ alkoxy, halogen- or deuterium-substituted $C_1$-$C_4$ alkoxy, -CN, $C_1$-$C_4$ alkyl, or halogen- or deuterium-substituted $C_1$-$C_4$ alkyl; more particularly, each $R^4$ is independently hydrogen, halogen, methoxy, ethoxy, halogen- or deuterium-substituted methoxy or ethoxy, -CN, methyl, ethyl, or halogen- or deuterium-substituted methyl or ethyl;

alternatively, any two adjacent $R^4$s together with the ring atoms attached thereto form a substituted or unsubstituted saturated or unsaturated non-aromatic $C_3$-$C_{10}$ cyclic hydrocarbyl, a substituted or unsubstituted saturated or unsaturated non-aromatic 4- to 6-membered heterocyclyl, a substituted or unsubstituted $C_6$-$C_{10}$ aryl, or a substituted or unsubstituted 4- to 6-membered heteroaryl, wherein, the 4- to 6-membered heterocyclyl and the 4- to 6-membered heteroaryl each independently contain one or more heteroatoms selected from the group consisting of oxygen, sulfur and nitrogen, and the number of heteroatoms is independently 1, 2 or 3; particularly, any two adjacent $R^4$s together with the ring atoms attached thereto form a substituted or unsubstituted, saturated or unsaturated non-aromatic $C_3$-$C_6$ cyclic hydrocarbyl, a substituted or unsubstituted saturated or unsaturated non-aromatic 5- to 6-membered heterocyclyl, a substituted or unsubstituted $C_6$-$C_{10}$ aryl, or a substituted or unsubstituted 5- to 6-membered heteroaryl, wherein, the 5- to 6-membered heterocyclyl and the 5- to 6-membered heteroaryl each independently contain one or more heteroatoms selected from the group consisting of oxygen, sulfur and nitrogen, and the number of heteroatoms is independently 1, 2 or 3; wherein, the substituent for the "substituted" includes 1, 2 or 3 groups selected from the group consisting of $C_1$-$C_6$ alkyl, $C_1$-$C_6$ alkoxy, halogen, -CN, amino, -OH, halogen-substituted $C_1$-$C_6$ alkyl, $C_1$-$C_6$ alkyl-monosubstituted or disubstituted amino, $C_3$-$C_6$ cycloalkyl, 3-6 membered heterocycloalkyl containing one or more heteroatoms independently selected from the group consisting of oxygen, sulfur and nitrogen, and oxo;

$R^{1-1}$, $R^{2-1}$ and $R^{4-1}$ are each independently hydrogen, deuterium, $C_1$-$C_7$ alkyl, halogen-substituted $C_1$-$C_7$ alkyl, $C_3$-$C_{14}$ cycloalkyl, or 3- to 14-membered heterocycloalkyl, the 3- to 14-membered heterocycloalkyl contains one or more heteroatoms selected from the group consisting of boron, silicon, oxygen, sulfur, selenium, nitrogen and phosphorus, and the number of heteroatoms is independently 1, 2, 3 or 4; particularly, $R^{1-1}$, $R^{2-1}$ and $R^{4-1}$ are each independently hydrogen, deuterium, $C_1$-$C_7$ alkyl, or halogen-substituted $C_1$-$C_7$ alkyl; more particularly, $R^{1-1}$, $R^{2-1}$ and $R^{4-1}$ are each independently hydrogen, deuterium, methyl, ethyl, n-propyl, isopropyl, halogen-substituted methyl, halogen-substituted ethyl, halogen-substituted n-propyl, halogen-substituted isopropyl;

$R^{1-2}$, $R^{1-3}$, $R^{2-2}$, $R^{2-3}$, $R^{4-2}$, $R^{4-3}$ are independently hydrogen, $C_1$-$C_7$ alkyl, halogen-substituted $C_1$-$C_7$ alkyl, $C_3$-$C_{14}$ cycloalkyl or 3- to 14-membered heterocycloalkyl; the 3- to 14- membered heterocycloalkyl contains one or more heteroatoms selected from the group consisting of boron, silicon, oxygen, sulfur, selenium, nitrogen and phosphorus, and the number of heteroatoms may be 1, 2, 3 or 4;

alternatively, $R^{1-2}$ and $R^{1-3}$ together with the nitrogen atom attached thereto form a 3- to 14-membered heterocycloalkyl, or a $R^{1-2-1}$-substituted 3- to 14-membered heterocycloalkyl; the 3- to 14-membered heterocycloalkyl and the $R^{1-2-1}$-substituted 3-to 14-membered heterocycloalkyl independently contain one or more heteroatoms selected from the group consisting of boron, silicon, oxygen, sulfur, selenium, nitrogen and phosphorus, and the number of heteroatoms is independently 1, 2, 3 or 4; said $R^{1-2-1}$ is independently hydroxyl, halogen, oxo, -CN, $C_1$-$C_7$ alkyl or $C_1$-$C_7$ alkoxy;

alternatively, $R^{2-2}$ and $R^{2-3}$ together with the nitrogen atom attached thereto form a 3- to 14-membered heterocycloalkyl or a $R^{2-2-1}$-substituted 3- to 14-membered heterocycloalkyl; the 3- to 14-membered heterocycloalkyl

and the $R^{2-2-1}$-substituted 3-to 14-membered heterocycloalkyl independently contain one or more heteroatoms selected from the group consisting of boron, silicon, oxygen, sulfur, selenium, nitrogen and phosphorus, and the number of heteroatoms is independently 1, 2, 3 or 4; said $R^{2-2-1}$ is independently hydroxyl, halogen, oxo, -CN, $C_1$-$C_7$ alkyl or $C_1$-$C_7$ alkoxy;

alternatively, $R^{4-2}$ and $R^{4-3}$ together with the nitrogen atom attached thereto form a 3- to 14-membered heterocycloalkyl or a $R^{4-2-1}$-substituted 3- to 14-membered heterocycloalkyl; the 3- to 14-membered heterocycloalkyl and the $R^{4-2-1}$-substituted 3-to 14-membered heterocycloalkyl independently contain one or more heteroatoms selected from the group consisting of boron, silicon, oxygen, sulfur, selenium, nitrogen and phosphorus, and the number of heteroatoms is independently 1, 2, 3 or 4; said $R^{4-2-1}$ is independently hydroxyl, halogen, oxo, -CN, $C_1$-$C_7$ alkyl or $C_1$-$C_7$ alkoxy;

$R^{1-4}$, $R^{2-4}$, $R^{3-1}$ and $R^{4-6}$ represent 1 to 3 substituents, and are each independently one or more selected from the group consisting of hydrogen, deuterium, -CN, halogen, -$OR^{1-4-1}$, $NR^{1-4-2}R^{1-4-3}$, $C_1$-$C_7$ alkyl, $C_3$-$C_{14}$ cycloalkyl and 3- to 14-membered heterocycloalkyl; the 3- to 14-membered heterocycloalkyl contains one or more heteroatoms selected from the group consisting of boron, silicon, oxygen, sulfur, selenium, nitrogen and phosphorus, and the number of heteroatoms is independently 1, 2, 3 or 4;

$R^{4-4}$ and $R^{4-5}$ are independently hydrogen, -$OR^{4-4-1}$, $NR^{4-4-2}R^{4-4-3}$, $C_1$-$C_7$ alkyl, $C_3$-$C_{14}$ cycloalkyl, or 3- to 14-membered heterocycloalkyl; the 3- to 14-membered heterocycloalkyl contains one or more heteroatoms selected from the group consisting of boron, silicon, oxygen, sulfur, selenium, nitrogen and phosphorus, and the number of heteroatoms is independently 1, 2, 3 or 4;

$R^{1-4-1}$ and $R^{4-4-1}$ are independently hydrogen, halogen, $C_1$-$C_7$ alkyl, $C_3$-$C_{14}$ cycloalkyl, or 3- to 14-membered heterocycloalkyl, the 3- to 14-membered heterocycloalkyl contains one or more heteroatoms selected from the group consisting of boron, silicon, oxygen, sulfur, selenium, nitrogen and phosphorus, and the number of heteroatoms is independently 1, 2, 3 or 4;

$R^{1-4-2}$, $R^{1-4-3}$, $R^{4-4-2}$ and $R^{4-4-3}$ are independently hydrogen, $C_1$-$C_7$ alkyl, $C_3$-$C_{14}$ cycloalkyl, or 3- to 14-membered heterocycloalkyl, the 3- to 14-membered heterocycloalkyl contains one or more heteroatoms selected from the group consisting of boron, silicon, oxygen, sulfur, selenium, nitrogen and phosphorus, and the number of heteroatoms is independently 1, 2, 3 or 4;

alternatively, $R^{1-4-2}$ and $R^{1-4-3}$ together with the nitrogen atom attached thereto form a 3- to 14-membered heterocycloalkyl, or a $R^{1-4-2-1}$-substituted 3- to 14-membered heterocycloalkyl; the 3- to 14-membered heterocycloalkyl and the $R^{1-4-2-1}$-substituted 3-to 14-membered heterocycloalkyl independently contain one or more heteroatoms selected from the group consisting of boron, silicon, oxygen, sulfur, selenium, nitrogen and phosphorus, and the number of heteroatoms is independently 1, 2, 3 or 4; the $R^{1-4-2-1}$ is independently hydroxyl, halogen, oxo, -CN, $C_1$-$C_7$ alkyl or $C_1$-$C_7$ alkoxy;

alternatively, $R^{4-4-2}$ and $R^{4-4-3}$ together with the nitrogen atom attached thereto form a 3- to 14-membered heterocycloalkyl, or a $R^{4-4-2-1}$-substituted 3- to 14-membered heterocycloalkyl; the 3- to 14-membered heterocycloalkyl and the $R^{4-4-2-1}$-substituted 3-to 14-membered heterocycloalkyl independently contain one or more heteroatoms selected from the group consisting of boron, silicon, oxygen, sulfur, selenium, nitrogen and phosphorus, and the number of heteroatoms is independently 1, 2, 3 or 4; the $R^{4-4-2-1}$ is independently hydroxyl, halogen, oxo, -CN, $C_1$-$C_7$ alkyl or $C_1$-$C_7$ alkoxy.

**2.** The compound represented by formula I according to claim 1, or the pharmaceutically acceptable salt, solvate, solvate of pharmaceutically acceptable salt, metabolite or prodrug thereof, wherein,

n is 0, 1, 2, 3, 4 or 5;

W, X, Y and Z are independently CH or N;

ring A is 5- to 10-membered heteroaryl, preferably 5- to 6-membered heteroaryl, wherein the 5- to 10-membered heteroaryl or 5- to 6-membered heteroaryl independently contains one or more heteroatoms selected from the group conisisting of oxygen, sulfur, and nitrogen, and the number of heteroatoms is independently 1, 2, 3 or 4; wherein, ring A is optionally substituted by 1 to 3 substituents selected from the group consisting of halogen, $C_1$-$C_4$ alkyl, $C_1$-$C_4$ alkoxy, hydroxyl and cyano;

ring B is $C_3$-$C_{14}$ cycloalkyl, 3- to 14-membered heterocycloalkyl, $C_6$-$C_{12}$ aryl, or 5-to 10-membered heteroaryl, the 5- to 10-membered heteroaryl and 3- to 14-membered heterocycloalkyl each independently contain one or more heteroatoms selected from the group consisting of oxygen, sulfur and nitrogen, and the number of heteroatoms is independently 1, 2, 3 or 4;

$R^1$ is -$NR^{1-2}R^{1-3}$ or $C_1$-$C_7$ alkyl, $R^{1-2}$ and $R^{1-3}$ are each as defined in claim 1;

$R^2$ and $R^3$ are each independently hydrogen, halogen, $C_1$-$C_7$ alkyl, or $C_3$-$C_{14}$ cycloalkyl; or, $R^2$ and $R^3$ together with the carbon atom attached thereto form a $C_3$-$C_{14}$ cycloalkyl, a $R^{3-1}$-substituted $C_3$-$C_{14}$ cycloalkyl, or a 3- to 14-membered heterocycloalkyl, wherein $R^{3-1}$ is as defined in claim 1, the 3- to 14-membered heterocycloalkyl

independently contains one or more heteroatoms selected from the group consisting of boron, silicon, oxygen, sulfur, selenium, nitrogen and phosphorus, and the number of heteroatoms is independently 1, 2, 3 or 4;
Each $R^4$ is independently hydrogen, halogen, $-OR^{4-1}$, -CN, $C_1$-$C_7$ alkyl, or $R^{4-6}$-substituted $C_1$-$C_7$ alkyl;
$R^{4-1}$s are each independently hydrogen, deuterium, $C_1$-$C_7$ alkyl, or halogen-substituted $C_1$-$C_7$ alkyl; $R^{4-6}$ is as defined in claim 1, preferably independently one or more selected from the group consisting of halogen and deuterium.

**3.** The compound represented by formula I according to claim 1, or the pharmaceutically acceptable salt, solvate, solvate of pharmaceutically acceptable salt, metabolite or prodrug thereof, wherein,

n is 0, 1, 2, 3, 4 or 5;
W, X, Y and Z are independently CH or N;
ring A is 5- to 6-membered heteroaryl, the 5- to 6-membered heteroaryl contains one or more heteroatoms selected from the group consisting of oxygen, sulfur and nitrogen, and the number of heteroatoms is independently 1 or 2; wherein, ring A is optionally substituted by 1 to 3 substituents selected from the group consisting of halogen, $C_1$-$C_4$ alkyl, $C_1$-$C_4$ alkoxy, hydroxyl, and cyano;
ring B is $C_3$-$C_{10}$ cycloalkyl, 3- to 10-membered heterocycloalkyl, $C_6$-$C_{10}$ aryl or 5-to 6-membered heteroaryl, the 5- to 6-membered heteroaryl and 3- to 10-membered heterocycloalkyl each independently contain one or more heteroatoms selected from the group consisting of oxygen, sulfur and nitrogen, and the number of heteroatoms is independently 1 or 2;
$R^1$ is $-NR^{1-2}R^{1-3}$ or $C_1$-$C_7$ alkyl, wherein $R^{1-2}$ and $R^{1-3}$ are each as defined in claim 1;
$R^2$ and $R^3$ are each independently hydrogen, halogen, $C_1$-$C_7$ alkyl, or $C_3$-$C_{14}$ cycloalkyl; or, $R^2$ and $R^3$ together with the carbon atom attached thereto form a $C_3$-$C_8$ cycloalkyl, a $R^{3-1}$-substituted $C_3$-$C_8$ cycloalkyl, or a 3- to 8-membered heterocycloalkyl, wherein $R^{3-1}$ is as defined in claim 1, the 3- to 8-membered heterocycloalkyl independently contains one or more heteroatoms selected from the group consisting of oxygen, sulfur and nitrogen, and the number of heteroatoms is independently 1 or 2;
Each $R^4$ is independently hydrogen, halogen, $-OR^{4-1}$, -CN, $C_1$-$C_7$ alkyl, or halogen- or deuterium-substituted $C_1$-$C_7$ alkyl;
$R^{4-1}$s are each independently hydrogen, deuterium, $C_1$-$C_7$ alkyl, or halogen-substituted $C_1$-$C_7$ alkyl.

**4.** The compound represented by formula I according to claim 1, or the pharmaceutically acceptable salt, solvate, solvate of pharmaceutically acceptable salt, metabolite or prodrug thereof, wherein,

n is 0, 1, 2, 3, 4 or 5;
W, X, Y and Z are independently CH or N;
ring A is pyridyl, pyrimidyl or thiazolyl;
ring B is $C_3$-$C_8$ cycloalkyl, 4- to 8-membered heterocycloalkyl, $C_6$-$C_{10}$ aryl or 5- to 6-membered heteroaryl, the 5- to 6-membered heteroaryl and 4- to 8-membered heterocycloalkyl each independently contain one or more heteroatoms selected from the group consisting of oxygen, sulfur and nitrogen, and the number of heteroatoms is independently 1 or 2;
$R^1$ is $-NR^{1-2}R^{1-3}$ or $C_1$-$C_7$ alkyl, wherein $R^{1-2}$ and $R^{1-3}$ are each as defined in claim 1;
$R^2$ and $R^3$ are each independently hydrogen, halogen, $C_1$-$C_7$ alkyl or $C_3$-$C_{14}$ cycloalkyl; or, $R^2$ and $R^3$ together with the carbon atom attached thereto form a $C_3$-$C_8$ cycloalkyl, a $R^{3-1}$-substituted $C_3$-$C_8$ cycloalkyl, or a 3- to 8-membered heterocycloalkyl, wherein $R^{3-1}$ is as defined in claim 1, the 3- to 8-membered heterocycloalkyl independently contains one or more heteroatoms selected from the group consisting of oxygen, sulfur and nitrogen, and the number of heteroatoms is independently 1 or 2;
Each $R^4$ is independently hydrogen, halogen, $-OR^{4-1}$, -CN, $C_1$-$C_7$ alkyl, or halogen- or deuterium-substituted $C_1$-$C_7$ alkyl;
$R^{4-1}$s are each independently hydrogen, deuterium, $C_1$-$C_7$ alkyl, or halogen-substituted $C_1$-$C_7$ alkyl.

**5.** The compound represented by formula I according to claim 1, or the pharmaceutically acceptable salt, solvate, solvate of pharmaceutically acceptable salt, metabolite or prodrug thereof, wherein,

n is 0, 1, 2, 3, 4 or 5;
W, X, Y and Z are independently CH or N;
ring A is pyridyl, pyrimidyl or thiazolyl;
ring B is cyclopropyl, cyclobutyl, cyclopentyl, cyclohexyl, oxetanyl, pyrrolidinyl, tetrahydrofuryl, tetrahydrothiophenyl, tetrahydropyranyl, tetrahydrothiopyranyl, piperidyl, piperazinyl, morpholinyl, phenyl, pyrimidinyl, thiazolyl, pyrazolyl, imidazolyl, oxazolyl or pyridyl;

$R^1$ is $-NR^{1-2}R^{1-3}$ or $C_1$-$C_7$ alkyl, wherein $R^{1-2}$ and $R^{1-3}$ are each as defined above;

$R^2$ and $R^3$ are each independently hydrogen, halogen or $C_1$-$C_7$ alkyl; or, $R^2$ and $R^3$ together with the carbon atom attached thereto form a $C_3$-$C_6$ cycloalkyl, a $R^{3-1}$-substituted $C_3$-$C_6$ cycloalkyl, or a 3- to 6-membered heterocycloalkyl, wherein $R^{3-1}$ is as defined in claim 1, the 3- to 6-membered heterocycloalkyl independently contains one or more heteroatoms selected from the group consisting of oxygen, sulfur and nitrogen, and the number of heteroatoms is independently 1 or 2;

Each $R^4$ is independently hydrogen, halogen, $-OR^{4-1}$, -CN, $C_1$-$C_7$ alkyl, or halogen-or deuterium-substituted $C_1$-$C_7$ alkyl;

$R^{4-1}$s are each independently hydrogen, deuterium, $C_1$-$C_7$ alkyl, or halogen-substituted $C_1$-$C_7$ alkyl;

preferably,

n is 1, 2, 3 or 4;

W, X, Y and Z are independently CH or N;

ring A is selected from the group consisting of

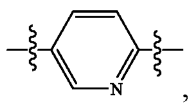, 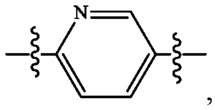,

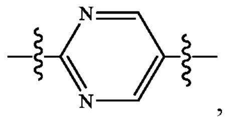, and 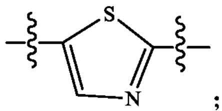;

and it is connected to the amido group at the left side and to the carbonyl group at the right side,

ring B is selected from the group consisting of

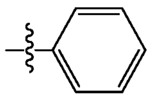, 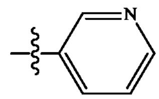,

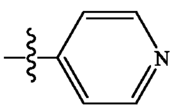, 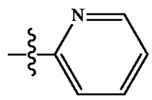, 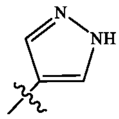, 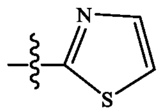, 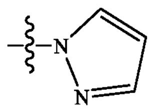, 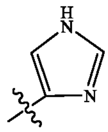,

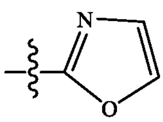, 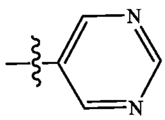, 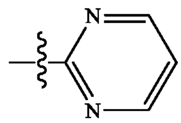;

$R^1$ is methyl, ethyl, n-propyl, methylamino, ethylamino or dimethylamino;

$R^2$ and $R^3$ are each independently hydrogen, fluorine, chlorine, methyl, ethyl, n-propyl or isopropyl; or, $R^2$ and $R^3$ together with the carbon atom attached thereto form

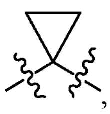, 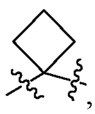, 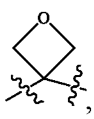, or 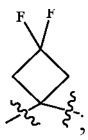;

Each $R^4$ is independently hydrogen, fluorine, chlorine, bromine, trifluoromethoxy, -CN, methyl, ethyl, trifluoromethyl, 2,2,2-trifluoroethyl, 1,1-difluoroethyl group, or trideuteromethyl.

**6.** The compound represented by formula I according to claim 1, or the pharmaceutically acceptable salt, solvate, solvate of pharmaceutically acceptable salt, metabolite or prodrug thereof, wherein, the compound represented by formula I is selected from the group consisting of:

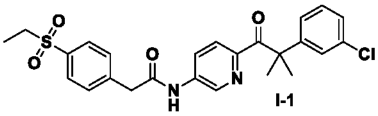
I-1 ,
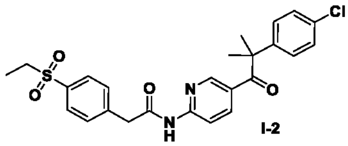
I-2 ,
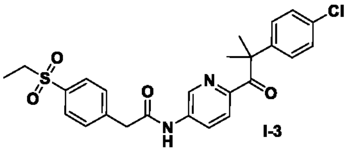
I-3 ,
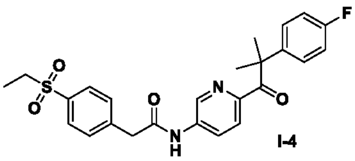
I-4 ,
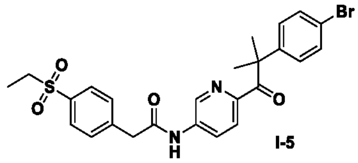
I-5 ,
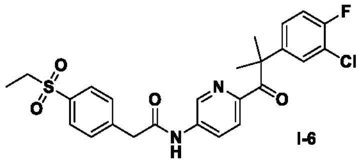
I-6 ,
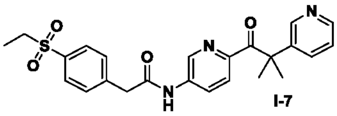
I-7 ,
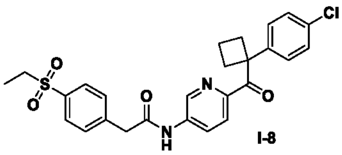
I-8 ,
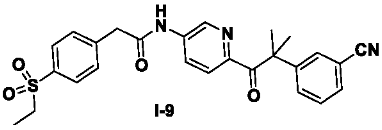
I-9 ,
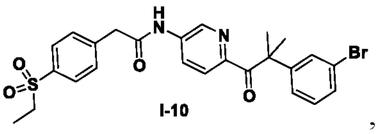
I-10 ,
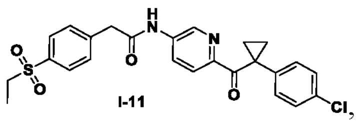
I-11 ,
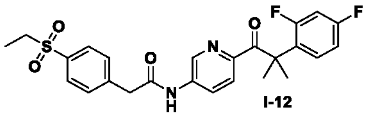
I-12 ,
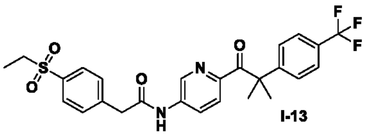
I-13 ,
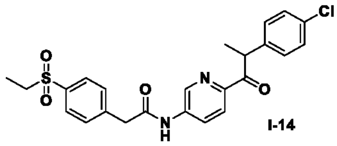
I-14 ,
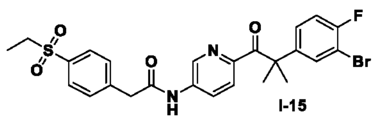
I-15 ,
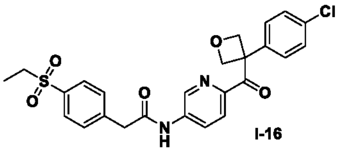
I-16 ,
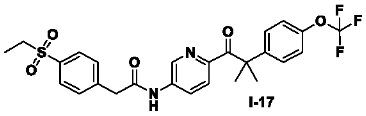
I-17 ,
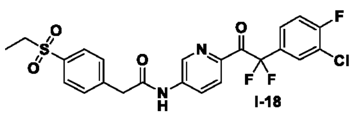
I-18 ,
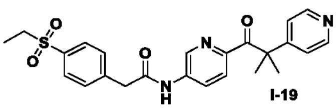
I-19 ,
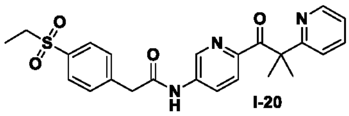
I-20 ,
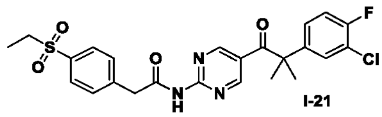
I-21 ,
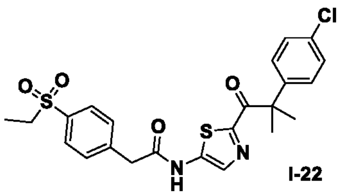
I-22 ,
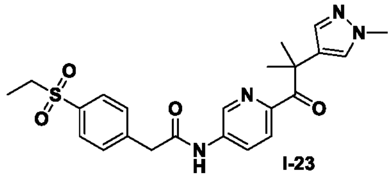
I-23

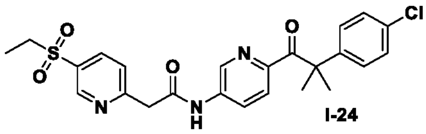
I-24 ,

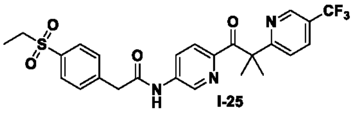
I-25 ,

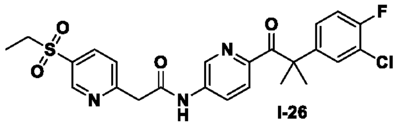
I-26

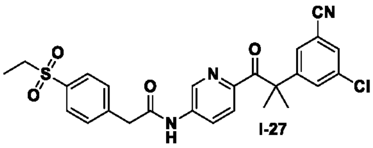
I-27

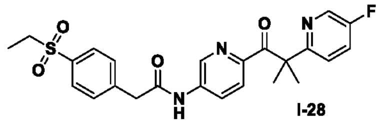
I-28 ,

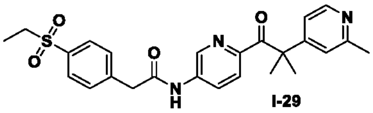
I-29 ,

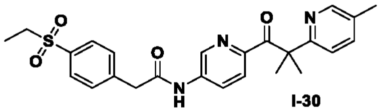
I-30

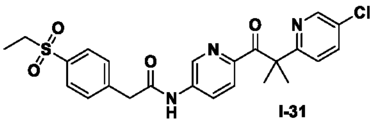
I-31 ,

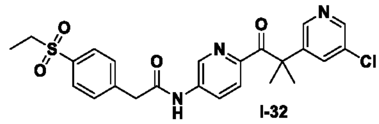
I-32

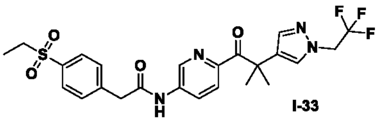
I-33 ,

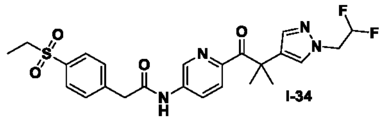
I-34

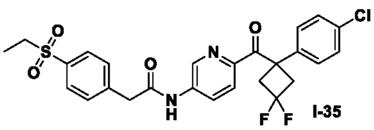
I-35 ,

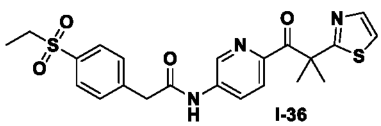
I-36

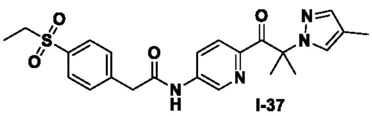
I-37 ,

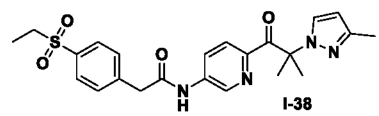
I-38

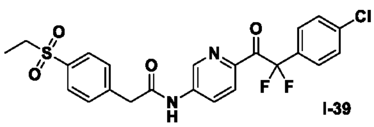
I-39 ,

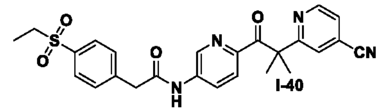
I-40

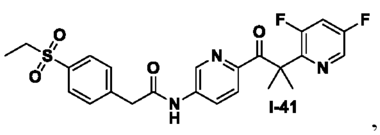
I-41 ,

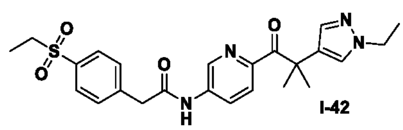
I-42 ,

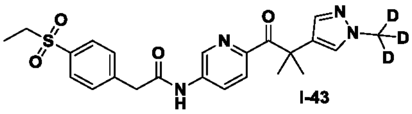
I-43 ,

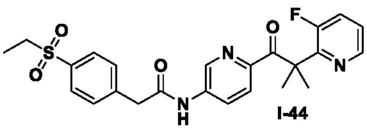
I-44

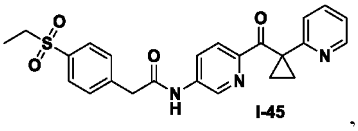
I-45 ,

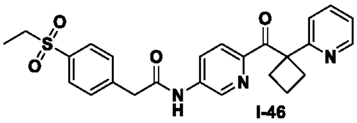
I-46 ,

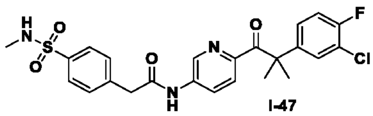
I-47

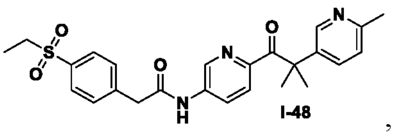
I-48 ,

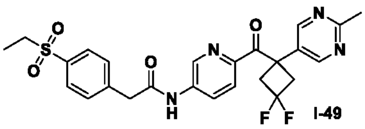
I-49 ,

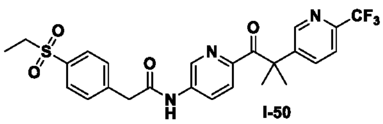
I-50 ,

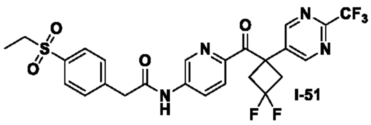
I-51 ,

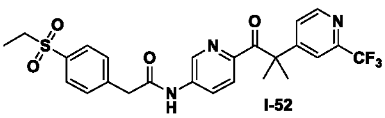
I-52 ,

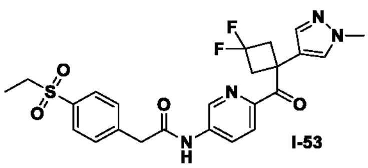
I-53 ,

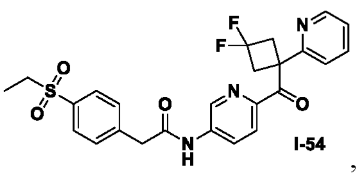
I-54 ,

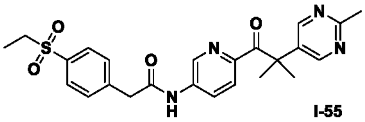
I-55 ,

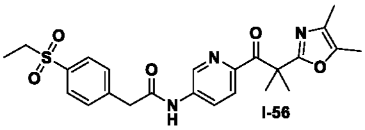
I-56

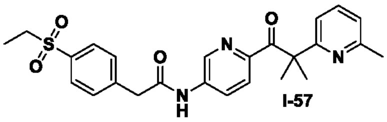
I-57 ,

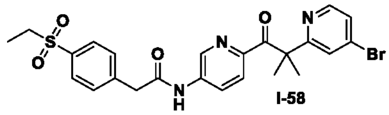
I-58 ,

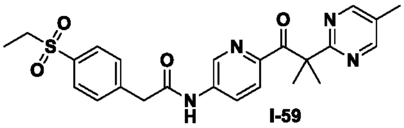
I-59 ,

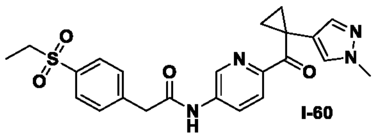
I-60 ,

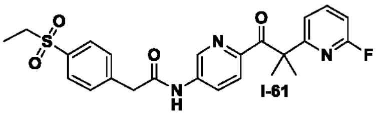
I-61 ,

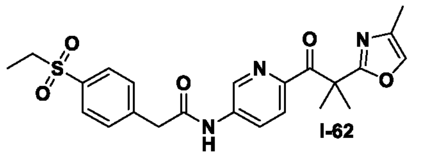
I-62 ,

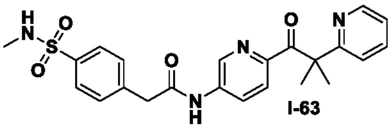
I-63 ,

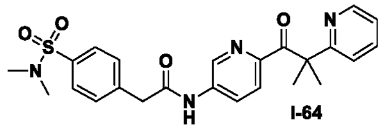
I-64 ,

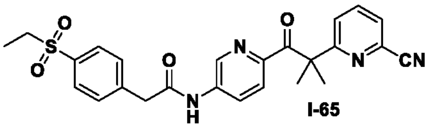
I-65 ,

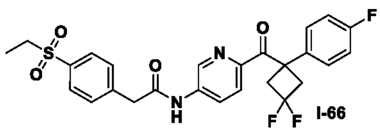
I-66 ,

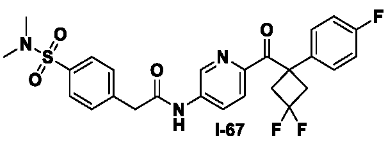
I-67 ,

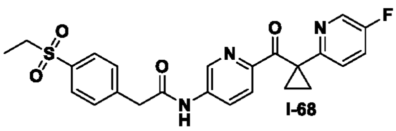
I-68 ,

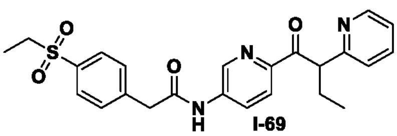
I-69 ,

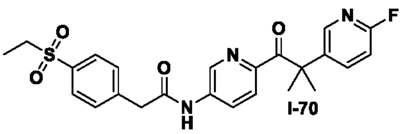
I-70 ,

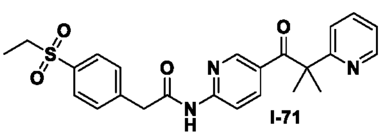
I-71 ,

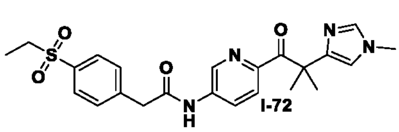
I-72 ,

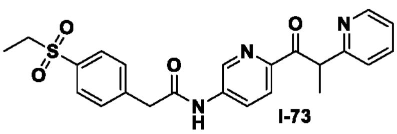
I-73 ,

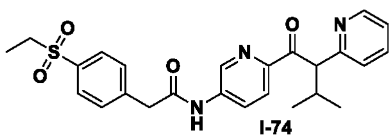
I-74 ,

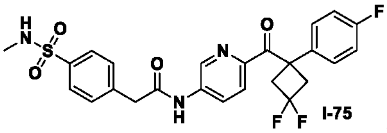

I-75 , and

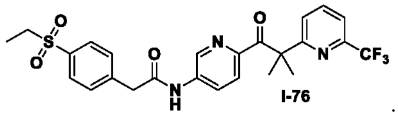

I-76 .

7. A pharmaceutical composition, which comprises one or more selected from the group consisting of the compound represented by formula I according to any one of calims 1 to 6, the pharmaceutically acceptable salt, solvate, solvate of the pharmaceutically acceptable salt, metabolite and prodrug thereof, and optionally, a pharmaceutically acceptable carrier.

8. A use of the compound represented by formula I according to any one of calims 1 to 6, or the pharmaceutically acceptable salt, solvate, solvate of pharmaceutically acceptable salt, metabolite or prodrug thereof, or the pharmaceutical composition according to calim 7 in the preparation of a RORγt protein receptor modulator.

9. A use of the compound represented by formula I according to any one of calims 1 to 6, or the pharmaceutically acceptable salt, solvate, solvate of pharmaceutically acceptable salt, metabolite or prodrug thereof, or the pharmaceutical composition according to calim 7 in the preparation of a medicament for preventing or treating a disease associated with RORγt protein receptor.

10. The use according to claim 9, wherein the disease associated with RORγt protein receptor is one or more selected from the group consisting of psoriasis, multiple sclerosis, rheumatoid arthritis, inflammatory bowel disease, ankylosing spondylitis, systemic lupus erythematosus, Behcet's disease, and chronic obstructive pulmonary disease.

## INTERNATIONAL SEARCH REPORT

International application No.
**PCT/CN2021/142477**

**A. CLASSIFICATION OF SUBJECT MATTER**

C07D 213/75(2006.01)i; C07D 213/84(2006.01)i; C07D 401/06(2006.01)i; C07D 405/06(2006.01)i; C07D 417/06(2006.01)i; C07D 239/04(2006.01)i; A61K 31/44(2006.01)i; A61K 31/4402(2006.01)i; A61K 31/4427(2006.01)i; A61K 31/4439(2006.01)i; A61K 31/505(2006.01)i; A61K 31/506(2006.01)i; A61P 17/06(2006.01)i; A61P 25/00(2006.01)i; A61P 1/00(2006.01)i; A61P 29/00(2006.01)i; A61P 37/00(2006.01)i; A61P 11/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

TWTXT; GBTXT; SGTXT; TWABS; EPTXT; TWMED; VEN; USTXT; CHTXT; HKABS; WOTXT; CNABS; CATXT; ATTXT; LEXTXT; MOABS; CNTXT; CNKI; STN CaPlus; STN Reg; STN CasReact; CNKI: structural formula search, RORγt蛋白受体调节剂, 维甲酸受体相关的孤儿受体, 维甲酸受体相关孤儿受体, 银屑病, 牛皮癣, 多发性硬化症, 类风湿性关节炎, 炎症性肠炎, 强直性脊椎炎, 系统性红斑狼疮, 白塞氏病, 白塞病, 贝赫切特综合征, 慢性阻塞性肺病, retinoic acid receptor-related orphan receptor γt, retinoic acid receptor-related orphan receptor?, psoriasis, multiple sclerosis, rheumatoid arthritis, inflammatory bowel disease?, ankylosing spondylitis, systemic lupus erythematosus, Behcet's Disease, Behcet syndrome, COPD, chronic obstructive pulmonary disease

**C. DOCUMENTS CONSIDERED TO BE RELEVANT**

| Category* | Citation of document, with indication, where appropriate, of the relevant passages | Relevant to claim No. |
|---|---|---|
| X | WO 2015101928 A1 (AURIGENE DISCOVERY TECHNOLOGIES LIMITED) 09 July 2015 (2015-07-09)<br>claims 1-22, in particular claim 13 or compound 76 in embodiment 16 | 1-10 |
| X | WO 2014179564 A1 (VITAE PHARMACEUTICALS INC.) 06 November 2014 (2014-11-06)<br>claims 1-37, description page 205, first compound | 1-10 |
| A | CN 101827840 A (NOVARTIS AG.) 08 September 2010 (2010-09-08)<br>claims 1-10, description paragraphs 0486, 0488 | 1-10 |

☐ Further documents are listed in the continuation of Box C.　☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 March 2022** | **30 March 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/142477**

| Patent document cited in search report | Publication date (day/month/year) | Patent family member(s) | Publication date (day/month/year) |
|---|---|---|---|
| WO 2015101928 A1 | 09 July 2015 | None | |
| WO 2014179564 A1 | 06 November 2014 | US 2017008910 A1 | 12 January 2017 |
| | | US 9868748 B2 | 16 January 2018 |
| | | WO 2014179564 A8 | 04 December 2014 |
| | | EP 2991994 A1 | 09 March 2016 |
| | | EP 2991994 B1 | 15 August 2018 |
| CN 101827840 A | 08 September 2010 | WO 2009050200 A1 | 23 April 2009 |
| | | CL 2008003054 A1 | 15 May 2009 |
| | | TW 200927747 A | 01 July 2009 |
| | | JP 2011500632 A | 06 January 2011 |
| | | EP 2212314 A1 | 04 August 2010 |
| | | AR 068784 A1 | 02 December 2009 |
| | | US 2009099243 A1 | 16 April 2009 |
| | | PE 20090967 A1 | 10 August 2009 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015083130 A
- WO 2016193459 A
- WO 2017010399 A


### Non-patent literature cited in the description


- **BERGE et al.** Pharmaceutical Salts. *Journal of Pharmaceutical Science,* 1977, vol. 66, 1-19
- Handbook of Pharmaceutical Salts: Properties, Selection, and Use. Wiley-VCH, 2002
- Pharmacopoeia of the People's Republic of China. 2015, vol. 4
- **RAYMOND C ROWE.** Handbook of Pharmaceutical Excipients. 2009